# EUROPEAN PATENT SPECIFICATION

(11) **EP 0 431 943 B1**
(45) Date of publication and mention of the grant of the patent: **15.07.1998**
(21) Application number: 90313262.9
(22) Date of filing: 06.12.1990
(51) Int. Cl.: C07D 491/10, C07D 491/20, C07D 471/10, C07D 495/10, C07D 495/20, A61K 31/445

(54) **Spiro(pyran)piperidine derivatives**
Spiro(pyran)Piperidine-Derivate
Dérivés de spiro(pyran)pipéridine

(30) Priority: 08.12.1989 US 447950; 16.11.1990 US 612091
(43) Date of publication of application: 12.06.1991
(73) Proprietor: Merck & Co., Inc., Rahway New Jersey 07065-0900 (US)
(72) Inventor: Baldwin, John J., Gwynedd Valley, PA 19437 (US); Claremon, David A., Audubon, PA 19403 (US); Elliott, Jason M., Blue Bell, PA 19422 (US); Ponticello, Gerald S., Lansdale, PA 19446 (US); Remy, David C., North Wales, PA 19454 (US); Selnick, Harold G., Ambler, PA 19002 (US)
(74) Representative: Cole, William Gwyn

(56) References cited:
- EP-A- 0 121 972
- FR-A- 2 248 047
- FR-A- 2 558 835
- US-A- 3 649 635
- US-A- 3 686 186
- US-A- 4 166 119
- US-A- 4 353 900
- CHEMICAL ABSTRACTS, vol. 89, no. 15, 9 October 1978, Columbus, Ohio, US; abstract no. 129425J, K. ONO ET AL.: '" Spiroamine derivatives "' page 582 ; & JP-A-53068784

## Description

### SUMMARY OF THE INVENTION

This invention is concerned with novel spirocycles of general structural formula: 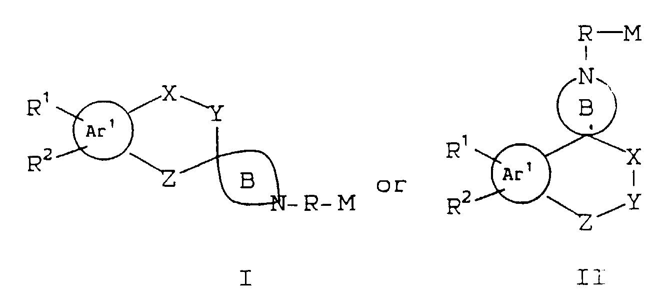 which are Class III antiarrhythmic agents, and positive inotropic or cardiotonic agents,
wherein Ar¹ is a carbocyclic or heterocyclic group, M is a carbocyclic or heterocyclic group, or a functional group, R is a bridging group and X, Y and Z are independently a carbon or a heteroatom.

The invention is also concerned with pharmaceutical formulations comprising one or more of the novel compounds as active ingredient, either alone or in combination with one or more of a Class I, Class II or Class IV antiarrhythmic agent.

The invention is also concerned with a method of treatment of arrhythmia and impaired cardiac pump functions with the above-described novel compounds and formulations thereof.

The invention is further concerned with processes for preparing the novel compounds.

### BACKGROUND OF THE INVENTION

Arrhythmias often occur as complications to cardiac diseases such as myocardial infarction and heart failure. In a serious case, arrhythmias give rise to a ventricular fibrillation and can cause sudden death.

Though various antiarrythmic agents are now available on the market, those, having both satisfactory effects and high safety, have not been obtained. For example, antiarrythmic agents of Class I according to the classification of Vaughan-Williams which cause a selective inhibition of the maximum velocity of the upstroke of the action potential (Vmax) are inadequate for preventing ventricular fibrillation. In addition, they have problems regarding safety, namely, they cause a depression of the myocardial contractility and have a tendency to induce arrythmias due to an inhibition of the impulse conduction. Beta-adrenoceptor blockers and calcium antagonists which belong to Class II and IV respectively, have a defect that their effects are either limited to a certain type of arrhythmia or are contraindicated because of their cardiac depressant properties in certain patients with cardiovascular disease. Their safety, however, is higher than that of the antiarrhythmic agents of Class I.

Antiarrythmic agents of Class III are drugs which cause a selective prolongation of the duration of the action potential without a significant depression of the Vmax. Drugs in this class are limited. Examples such as sotalol and amiodarone have been shown to possess Class III properties. Sotalol also possesses Class II effects which may cause cardiac depression and be contraindicated in certain susceptible patients. Also, amiodarone is severly limited by side effects. Drugs of this class are expected to be effective in preventing ventricular fibrillations. Pure Class III agents, by definition, are not considered to cause myocardial depression or an induction of arrhythmias due to the inhibition of the action potential conduction as seen with Class I antiarrhythmic agents.

A number of antiarrhythmic agents have been reported in the literature, such as those disclosed in:
(2) EP 300,908-A,
(3) EP 307,121,
(4) US 4,629,739,
(5) US 4,544,654,
(6) US 4,788,196,
(7) EP application 88302597.5,
(8) EP application 88302598.3,
(9) EP application 88302270.9,
(10) EP application 88302600.7,
(11) EP application 88302599.1,
(12) EP application 88300962.3,
(13) EP application 235,752,
(14) DE 3633977-A1,
(15) US 4,804,662,
(16) US 4,797,401,
(17) US 4,806,555,
(18) US 4,806,536.

Japanese patent publication 78-68784 of Sumitomo Chemical Co., Ltd. (see also Chem. Abs. 89:129425j) describes spiroamine derivatives having antiarrythmic activity.

Compounds of similar structure are found in Japanese patent publication 88-63533-B of Daiichi Pharmaceutical Co.; J. Med. Chem., 19, 1315 (1976) by Bauer et al; Iorio et al in Il. Farmaco-Ed Sci., 32, 212-219 (1977): Houlihan et al, U.S. Patent 3,686,186; Davis et al, U.S. Patent 4,420,485; Kealey, U.S. Patent 4,810,792; Parham et al, J. Org. Chem., 41, 2629 (1976), however, none of these compounds are alleged to have antiarrhythmic activity.

Now with the present invention, there is provided a new use as antiarrhythmic agent for many of these known compounds and a group of new compounds of similar structure with an increased degree of activity.

### DETAILED DESCRIPTION OF THE INVENTION

The present invention relates to the use of a compound of the structural formulae I and II: 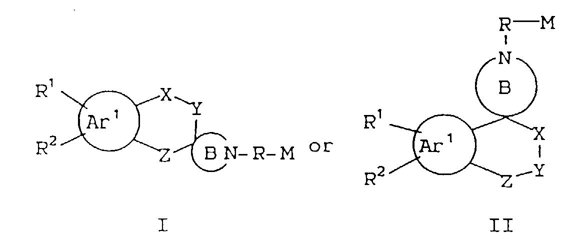 or a pharmaceutically acceptable salt thereof, wherein Ar¹ is an aromatic ring selected from
1) benzo, and
2) thieno,
the ring system comprising X, Y and Z is selectedfrom: 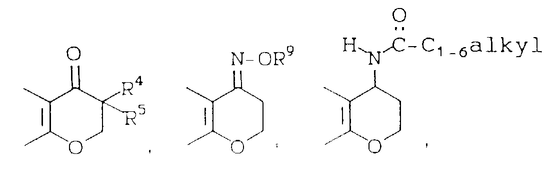 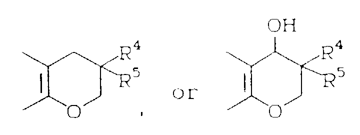 wherein:
R⁴ and R⁵ are independently
   a) hydrogen, or
   b) C₁₋₆alkyl ;
R⁹ is
   a) hydrogen, or
   b) C₁₋₆alkyl, unsubstituted or substituted with -COOR⁵;

- R-M is: n-hexyl, unsubstituted or substituted with hydroxy or cyano; or
- R is: a bond or C₁₋₈alkyl and
- M is: Ar²R³, wherein Ar²R³ is:
phenyl,
4-fluorophenyl, 4-cyanophenyl, benzofurazanyl, 2- or 4-pyridyl, 4-methoxyphenyl, 4-chlorophenyl, 3,4-methylenedioxyphenyl, 4-sulfamoylphenyl 4-methanesulfonamidophenyl, cyanotetralin or 2-methyl-6-pyridyl, especially benzofurazanyl, phenyl, 4-fluorophenyl, tetralin-2-yl, 6-cyano-tetralin-2-yl or 4-cyanophenyl;

R¹ and R² are independently selected from:
a) -NO₂,
b) -NHSO₂(C₁₋₆alkyl),
c) -SO₂(C₁₋₆alkyl),
d) -NHSO₂-C₆H₄-R4,
e) -SO₂-C₆H₄-R4,
f) hydrogen, or
R¹ and R² taken together with the Ar¹ group to which they are attached represent: 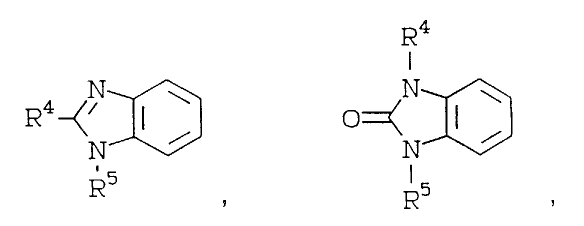 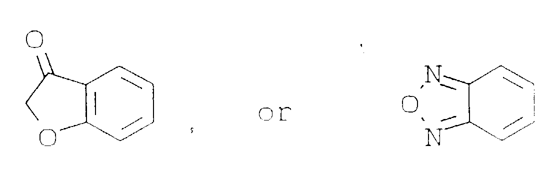
- B is: a piperidine ring;
with the proviso that if Ar¹ is benzo, and R¹ and R² are hydrogen, hydroxyalkyl or alkoxy, then M is other than unsubstituted phenyl or hydrogen; in the manufacture of a medicament for the treatment of arrythmia and impaired cardiac function.

The present invention also relates to novel compounds of the structural formulae I and II in which Ar¹ is benzo or thieno, especially benzo substituted with an electron withdrawing group such as nitro or methanesulfonamido.

It is also preferred that the spirocycle B is spiro-piperidine, especially wherein the nitrogen atom is in the 3'- or 4'- position, and more especially in the 4'- position.

It is most preferred that the cyclic moiety comprising X, Y and Z is:

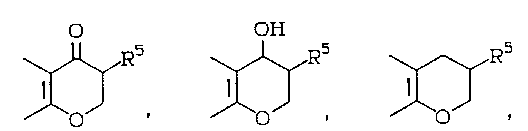

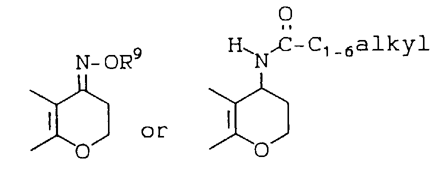

Preferred compounds include:
3,4-dihydro-1'-[(6-cyano-1,2,3,4-tetrahydronaphthalene)-2-yl]-6-methanesulfonyl-spiro[(2H)-1-benzopyran-2,4'-piperidine]-4-one;
3,4-dihydro-1'-[(6-cyano-1,2,3,4-tetrahydronaphthalene)-2-yl]-6-methanesulfonamido-spiro[(2H)-1-benzopyran-2,4'-piperidine]-4-ol;
3,4-dihydro-1'-[(6-cyano-1,2,3,4-tetrahydronaphthalene)-2-yl]-6-methanesulfonamido-spiro[(2H)-1-benzopyran-2,4'-piperidine];
1-[(5,6,7,8-tetrahydro-2-naphthalenecarbonitrile)-6-yl]-7',8'-dihydro-8'-oxo-spiro[piperidine-4,6'(1'H)-pyrano[2,3-f]benzimidazole];
1-[(5,6,7,8-tetrahydro-2-naphthalenecarbonitrile)-6-yl]-2',3',7',8'-tetrahydro-2',8'-dioxo-spiro[piperidine-4,6'(1'H)-pyrano[2,3-f]benzimidazole];
3,4-dihydro-1'-[2-(benzofurazan-5-yl)ethyl]-6-methanesulfonamido-spiro[(2H)-1-benzopyran-2,4'-piperidine]-4-ol;
3,4-dihydro-3-methyl-6-methanesulfonamido-1'-[2-(benzofurazan-5-yl)ethyl]spiro[(2H)-1-benzopyran-2,4-piperidine]-4-ol;
3,4-dihydro-1'-[2-(4-cyanophenyl)ethyl]-6-methanesulfonamido-3-methyl-spiro[(2H)-1-benzopyran-2,4'-piperidine]-4-one;
6-methanesulfonamido-1'-hexyl-4-hydroxy-3,4-dihydrospiro[(2H)-1-benzopyran-2,4'-piperidine];
3,4-dihydro-3-methyl-6-methanesulfonamido-1'-[2-(benzofurazan-5-yl)ethyl]spiro[(2H)-1-benzopyran-2,4'-piperidine]-4-one;
methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-;
cis or trans-3,4-dihydro-1'-[(6-cyano-1,2,3,4-tetrahydro-1-hydroxy-naphthalene)-2-yl]-6-methanesulfonamido-spiro[(2H)-1-benzopyran-2,4'-piperidine]-4-one;
4-acetamido-3,4-dihydro-1'-[(6-cyano-1,2,3,4-tetrahydronaphthalene)-2-yl]-6-methanesulfonamido-spiro-[(2H)-1-benzopyran-2,4'-piperidine];
3,4-dihydro-1'-[(6-cyano-1,2,3,4-tetrahydronaphthalene)-2-yl]-6-methanesulfonyl-spiro[(2H)-1-benzopyran-2,4'-piperidine]-4-ol;
3,4-dihydro-1'-[(6-cyano-1,2,3,4-tetrahydronaphthalene)-2-yl]-6-methanesulfonyl-spiro[(2H)-1-benzopyran-2,4'-piperidine];
1-[2-(4-cyanophenyl)ethyl]-7',8'-dihydro-8'-oxospiro[piperidine-4,6'(1'H)-pyrano[2,3-f]benzimidazole];
1-[2-(4-cyanophenyl)ethyl]-2',3',7',8'-tetrahydro-2',8'-dioxo-spiro[piperidine-4,6'(1'H)-pyrano[2,3-f]-benzimidazole;
3,4-dihydro-1'-[2-(4-cyanophenyl)ethyl]-6-methanesulfonyl-spiro[(2H)-1-benzopyran-2,4'-piperidine]-4-one;
3,4-dihydro-1'-hexyl-6-methanesulfonamido-spiro[(2H)-1-benzopyran-2,4'-piperidine];
3,4-dihydro-1'-hexyl-6-methanesulfonamido-spiro[(2H)-1-benzopyran-2,4'-piperidine]-4-one;
1-hexyl-2',3',7',8'-tetrahydro-2',8'-dioxospiro-[piperidine-4,6'(1H)-pyrano[2,3-f]benzimidazole];
3,4-dihydro-1'-[2-(benzofurazan-5-yl)ethyl]-6-methanesulfonamido-spiro[2H)-1-benzopyran-2,4'-piperidine]-4-one;
3,4-dihydro-1'-[2-(benzofurazan-5-yl)ethyl]-6-methanesulfonamido-spiro[(2H)-1-benzopyran-2,4'-piperidine];
7',8'-dihydro-1-hexyl-8'-oxospiro[piperidine-4,6'-(1H)-pyrano[2,3-f]benzimidazole];
3,4-dihydro-1'-(7-hydroxyheptyl)-6-methanesulfonamidospiro[(2H)-1-benzopyran-2,4'-piperidine]-4-one;
3,4-dihydro-1'-(6-hydroxyhexyl)-6-methanesulfonamidospiro[(2H)-1-benzopyran-2,4'-piperidine]-4-one; or
3,4-dihydro-1'-heptyl-6-methanesulfonamido-spiro[(2H)-1-benzopyran-2,4'-piperidine]-4-one;
3,4-dihydro-1'-[2-(4-acetylphenyl)ethyl]-6-methanesulfonamido-spiro[(2H)-1-benzopyran-2,4'-piperidine]-4-one;
3,4-dihydro-1'-hexyl-6-methanesulfonyl-spiro[(2H)-1-benzopyran-2,4'-piperidine]-4-one;
1-[(5,6,7,8-tetrahydro-2-naphthalenecarbonitrile)-6-yl]-7',8'-dihydro-8'-hydroxy-spiro[piperidine-4,6'(1'H)-pyrano[2,3-f]benzimidazole];
1-[(5,6,7,8-tetrahydro-2-naphthalenecarbonitrile)-6-yl]-2',3',7',8'-tetrahydro-8'-hydroxy-2'-oxo-spiro-[piperidine-4,6'(1'H)-pyrano[2,3-f]benzimidazole];
1-[(5,6,7,8-tetrahydro-2-naphthalenecarbonitrile)-6-yl]-7',8'-dihydro-spiro[piperidine-4,6'(1'H)-pyrano[2,3-f]benzimidazole];
1-[(5,6,7,8-tetrahydro-2-naphthalenecarbonitrile)-6-yl]-2',3',7',8'-tetrahydro-2'-oxo-spiro[piperidine-4,6'(1'H)-pyrano[2,3-f]benzimidazole];
4-acetamido-3,4-dihydro-1'-[(6-cyano-1,2,3,4-tetrahydronaphthalene)-2-yl]-6-methanesulfonyl-spiro-[(2H)-1-benzopyran-2,4'-piperidine];
cis or trans-3,4-dihydro-1'-[(6-cyano-1,2,3,4-tetrahydro-1-hydroxy-naphthalene)-2-yl]-6-methanesulfonylspiro[(2H)-1-benzopyran-2,4'-piperidine];
3,4-dihydro-1'-[(6-cyano-1,2,3,4-tetrahydronaphthalene)-2-yl]-6-methanesulfonamido-3-methyl-spiro[(2H)-1-benzopyran-2,4'-piperidine]-4-one;
3,4-dihydro-1'-[(6-cyano-1,2,3,4-tetrahydronaphthalene)-2-yl]-6-methanesulfonyl-3-methyl-spiro[(2H)-1-benzopyran-2,4'-piperidine]-4-one;
cis or trans-3,4-dihydro-1'-[(6-cyano-1,2,3,4-tetrahydro-1-hydroxy-naphthalene)-2-yl]-6-methanesulfonamido-spiro[(2H)-1-benzopyran-2,4'-piperidine];
and pharmaceutically acceptable salts thereof.

The term "alkyl", if the number of carbons is unspecified, means C₁₋₆alkyl and "alkyl" of three or more carbon atoms includes straight chain, branched chain and cycloalkyl.

Also included within the scope of the present invention are the non-toxic pharmaceutically acceptable salts of the novel compounds. Acid addition salts are formed by mixing a solution of the compound with a solution of a pharmaceutically acceptable non-toxic acid such as hydrochloric acid, fumaric acid, maleic acid, succinic acid, acetic acid, citric acid, tartaric acid, phosphoric acid, or the like. Also included within the scope of this invention are N-oxides and C₁₋₆alkyl quaternary ammonium salts.

Also included within the scope of this invention are diastereomers and enantiomers of the novel compounds and mixtures thereof. In particular if the two bridging groups joining the spiro carbon with the nitrogen of B are not identical, such as in a spiro-3-piperidine, chirality is introduced and the racemate and both enantiomers thereof are embraced within the scope of this invention.

Nomenclature utilized herein is intended to unambiguously identify specific chemical entities. However, there may be more than one way to refer to a particular compound. For example, the compound: 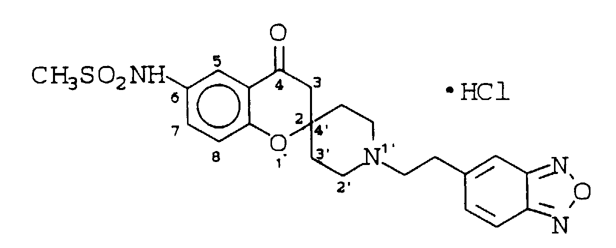 may be named as:
3,4-dihydro-1'-[2-(benzofurazan-5-yl)-ethyl]-6-methanesulfonamido-spiro[(2H)-1-benzopyran-2,4'-piperidine]-4-one hydrochloride and also named as:
N-[1'-[2-(5-benzofuranzanyl)ethyl]-3,4-dihydro-4-oxo-spiro[2H-1-benzopyran-2,4'-piperidin]-6-yl]-methanesulfonamide, monohydrochloride.
Both methods of nomenclature have been utilized herein.

The novel processes of this invention can be exemplified by the following Reaction Schemes:

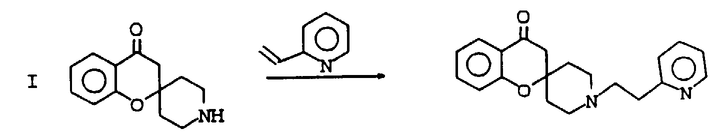

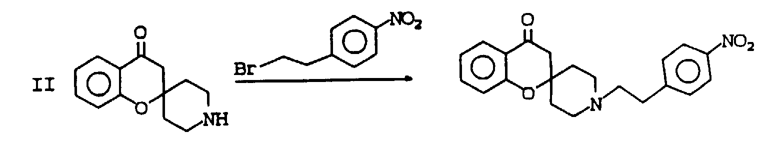

The process of Scheme I comprises addition of the piperidine across the vinyl group by heating a mixture of 1 part of the piperidine compound with about 1-2 parts of the vinyl compound in an aqueous lower alkanol such as methanol or ethanol in the presence of 1-2 parts of sodium or potassium acetate for about 2-20 hours at about reflux temperature.

Reaction Scheme II comprises N-alkylation of the piperidine with a slight excess of the phenalkyl alkylating agent. Although the leaving group exemplified is bromo, equivalent leaving groups are chloro, mesyl, tosyl, or the like. The two reagents are stirred at about 15°C to about reflux temperature in a suitable solvent, preferably one in which the reagents are soluble such as a lower alkanol, such as acetonitrile, methanol, ethanol or propanol in the presence of an acid scavenger such as sodium or potassium bicarbonate or carbonate, an organic amine or an appropriate ion exchange resin for about 10 to 40 hours.

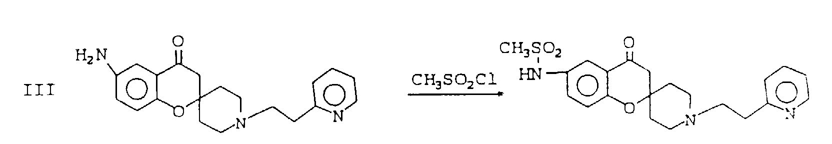

Reaction Scheme III depicts alterations of substituents such as sulfonylation with an alkanesulfonyl chloride by standard procedures.

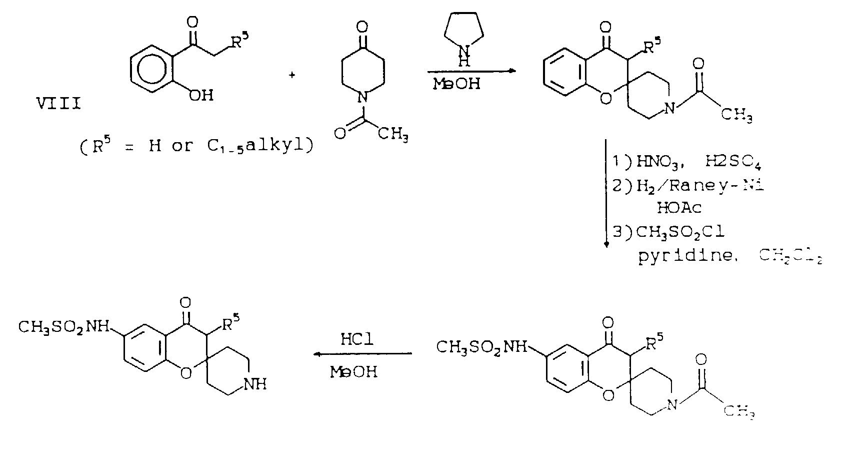

Reaction Scheme VIII illustrates the preparation of compounds bearing a substituent on the benopyranone by condensation of an ortho-hydroxyacetophenone with 1-acetyl-4-piperidone in the presence of pyrrolidine in methanol at or near solvent reflux temperature for a period of about 1 to 14 days. Reaction Scheme VIII further illustrates the incorporation of substitutents on the aromatic rings. Nitration with nitric acid, catalytic reduction of the nitro group with hydrogen and Raney-Nickel catalyst in acetic acid (or by standard procedures) gives the amine which may be sulfonylated with an alkansulfonyl chloride by standard procedures. The acetamide may be cleaved by treatment with hydrochloric acid in methanol at or near solvent reflux temperature for about 2 to 10 hours. The 4-piperidinyl compound so obtained may be utilized for the processs of Reaction Scheme I or II.

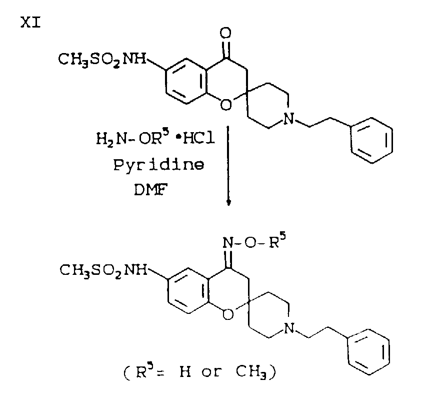

Reaction Scheme XI illustrates the preparation of oximes of the ketone on the benzopyranone ring. Treatment of the ketone with hydroxylamine hydrochloride or methylamine hydrochloride in N,N-dimethylformamide in the presence of pyridine gives the corresponding hydroxylimine, or methoxylimine, respectively.

The novel compounds of the present invention, have the pharmacological properties required for the antiarrhythmic agents of Class III, namely the prolongation of the myocardial action potential in vitro, without a significant depression of the Vmax, and the prolongation of QTc-interval in anesthetized dogs.

In addition those compounds wherein R³ represents hydrogen, when M is Ar² and Ar² is phenyl, Ar¹ is benzo and R¹ and R² are independently hydrogen, hydroxyalkyl or alkoxy also have the pharmacological properties required for the antiarrhythmic agents of Class III. Moreover, the members of both groups of compounds in general are much more potent than the reference drug, sotalol.

These compounds are effective in treating and preventing all types of arrhythmias including ventricular and atrial (supraventricular) arrhythmias. The compounds of the present invention are especially useful to control reentrant arrhythmias and prevent sudden death due to the ventricular fibrillation. These compounds are also effective in treating and preventing impaired cardiac pump funcitons.

In the novel method of this invention of treating arrhythmia, one of the compounds or pharmaceutically acceptable salt thereof, is administered in an amount ranging from about 0.0001 to about 20 mg per kg of body weight per day, preferably from about 0.001 to about 10 mg per kg of body weight per day in a single dose or in 2 to 4 divided doses.

These compounds can be administered as the sole active ingredient or in combination with other antiarrhythmic agents or other cardiovascular agents.

These compounds, or pharmaceutically acceptable salts thereof, in the described dosages, are administered orally, intraperitoneally, subcutaneously, intramuscularly, transdermally, sublingually or intravenously. They are preferably administered intravenously or orally, for example in the form of tablets, troches, capsules, elixirs, suspensions, syrups, wafers, chewing gum, or the like prepared by art recognized procedures. The amount of active compound in such therapeutically useful compositions or preparations is such that a suitable dosage will be obtained.

The following examples are given for the purpose of illustrating the present invention and shall not be construed as being limitations on the scope or spirit of the instant invention.

### SYNTHESIS METHOD (EXAMPLE 28)

### 1-(2-(4-Methanesulfonamidophenyl)ethyl)spiro-(piperidine-4,6'-(6H)-thieno[2,3-b]thiopyran)-4(5'H)-one

A solution of 850 mg (2.37 mmoles) of 1-(2-(4-aminophenyl)ethyl)spiro(piperidine-4,6'-thieno-[2,3-b]thiopyran)-4'5(H)-one in 10 ml methylene chloride was treated with 500 µl pyridine and 220 ml 2.85 mmole) of methanesulfonyl chloride at room temperature. The reaction was stirred at room temperature for ½ hour and then poured into 200 ml saturated sodium bicarbonate solution. The aqueous mixture was extracted several times with ethyl acetate. The organics were dried over MgSO₄, filtered and concentrated in vacuo. The residue was chromatographed on silica gel eluting with 3% CH₃OH/ CHCl₃ to give 690 mg. The free base was crystallized from CH₃OH to give 605 mg of product, m.p. 173-174°C

| Elemental analysis for C₂₀H₂₄N₂O₃S₃•CH₃OH | | | |
|---|---|---|---|
| | N | C | H |
| Calculated | 5.97 | 53.81 | 6.02 |
| Found | 5.95 | 54.03 | 6.08 |

### EXAMPLE 52

### 1'-(2-(2-Pyridyl)ethyl)-3,4-dihydrospiro-[(2H)-1-benzopyran-2,3'-piperidine]-4-one hydrochloride

### Step A: Preparation of 3,4-Dihydrospiro-1'-benzyl-2H-1-benzopyran-2,3'-piperidine-4-one

A solution of 5g (36 mmole) of ortho hydroxyacetophenone and 8.2g (36 mmole) of N-benzyl-3-piperidone in 200 mL methanol was treated with 5.24g (72 mmole) of pyrrolidine and heated to reflux for 4 hours. The reaction was cooled to room temperature concentrated in vacuo and partitioned between ethyl acetate and saturated sodium bicarbonate. The ethyl acetate solution was washed with 1N HCl which was then basified to pH 9 and reextracted with ethyl acetate. The ethyl acetate layer was dried over magnesium sulfate, filtered and concentrated in vacuo. The residue was chromatographed on silca gel eluting with 20% ethyl acetate/hexanes to give 8.1 g product. A small portion was treated with excess ethanolic HCl and crystallized from CH₃OH/C₂H₅OH to give product with mp = 255-258°C.

| Elemental analysis for C₂₀H₂₁NO₂•HCl | | | |
|---|---|---|---|
| Calcd | N, 4.07; | C, 69.85; | H, 6.45. |
| Found | N, 4.05; | C, 69.73; | H, 6.54. |

In a manner analogous to the above Step A except substituting 5-Acetamido-2-hydroxyacetophenone for ortho hydroxyacetophenone there was produced 6-Acetamido-3,4-dihydrospiro-1'-benzyl-2H-1-benzopyran-2,3'-piperidine-4-one, mp = 262-265°C.

### Step B: Preparation of 3,4-Dihydrospiro-[(2H)-1-benzopyran-2,3'-piperidine]-4-one

A solution of 20.3g g (66 mmole) of 3,4-Dihydrospiro-1'-benzyl-2H-1-benzopyran-2,3'-piperidine in 200 mL dichloroethane at room temperature and was treated with 7.4 mL (10.38g, g, 72 mmole) of α-chloroethyl chloroformate. The reaction was heated to reflux for 1/2 hour, cooled to room temperature concentrated in vacuo. The residue was dissolved in 200 mL of methanol and heated to reflux for 1/2 hour. The reaction was then cooled to room temperature and concentrated to 1/4 volume. The solid was collected to give 14.1 g of product, mp = 273-274°C.

### Step C: Preparation of 1'-(2-(2-pyridyl)ethyl)-3,4-dihydrospiro-[(2H)-1-benzopyran-2,3'-piperidine] 4-one hydrochloride

A solution of 520 mg (2.05 mmole) of 3,4-Dihydro-2H-1-benzopyran-2,3'-piperidine-4-one hydrochloride in 15 mL methanol was treated with 237 mg (2.25 mmole) of 2-vinylpyridine and heated to reflux for 48 hours. The reaction was cooled to room temperature, poured into 50 mL of saturated sodium bicarbonate and extracted with ethyl acetate. The ethyl acetate solution was dried over magnesium sulfate, filtered and concentrated in vacuo. The hydrochloride salt was prepared in ethanol with excess ethanolic HCL. The solid was collected to give 188 mg of product; mp 195-198°C.

| Elemental analysis for C₂₀H₂₂N₂O₂•2HCL•1/2H₂O | | | |
|---|---|---|---|
| Calcd | N, 6.93; | C, 59.40; | H, 6.23. |
| Found | N, 6.40; | C, 59.53; | H, 6.20. |

### EXAMPLE 53

### 1'-(2-(4-Methanesulfonamidophenyl)ethyl)-3,4-dihydrospiro-[(2H)-1-benzopyran-2,3'-piperidine]-4-one oxalate

A solution of 560 mg (258 mmole) of 3,4-dihydrospiro-[(2H)-1-benzopyran-2,3'-piperidine]4-one in 20 mL ethanol was treated with 880 mg (3 mmoles) of 2-(4-methanesulfonamidophenyl)ethyl mesylate and 0.5g sodium bicarbonate and heated to reflux for 48 hours. The reaction was cooled to room temperature, poured into saturated sodium bicarbonate and extracted with ethyl acetate. The ethyl acetate solution was dried over magnesium sulfate, filtered and concentrated in vacuo. The crude material was treated with 1 eq. oxalic acid and the salt was recrystallized from acetone to give product with mp = 204-210°C.

| Elemental analysis for C₂₂H₂₆N₂O₄S•C₂H₂O₄•1/2H₂O | | | |
|---|---|---|---|
| Calcd | N, 5.45; | C, 56.12; | H, 5.69. |
| Found | N, 5.52; | C, 56.35; | H, 5.54. |

### EXAMPLE 54

### 1'-(2-(4-Methanesulfonamidophenoxy)ethyl)-3,4-dihydrospiro-[(2H)-1-benzopyran-2,3'-piperidine]-4-one oxalate hemihydrate

Prepared in a manner analogous to the previous Example 53 except that 2-(4-Methanesulfonamidophenoxy)ethyl bromide was used instead of2-(4-methanesulfonamidophenyl)ethyl mesylate. The product had mp = 175°C.

| Elemental analysis for C₂₂H₂₆N₂O₅S•C₂H₂O₄•1/2H₂O | | | |
|---|---|---|---|
| Calcd | N, 5.32; | C, 54.43; | H, 5.51. |
| Found | N, 5.20; | C, 54.22; | H, 5.44. |

### EXAMPLE 55

### 6-Methanesulfonamido-1'-(2-(2-pyridinyl)ethyl)-3,4-dihydrospiro-[(2H)-1-benzopyran-2,3'-piperidine]-4-one hydrochloride

### Step A: Preparation of 6-Methanesulfonamido-1'-benzyl-3,4-dihydrospiro-[(2H)-1-benzopyran-2, 3'-piperidine]-4-one

A solution of 12.2g (33.47 mmole) of 6-acetamido-3,4-dihydrospiro-1'-benzyl-2-H-1-benzopyran-2,3'-piperidine-4-one in 200 mL ethanol was treated with 80 mL of 6N HCl and heated to reflux for 5 hours and then stirred at room temperature overnight. The reaction was concentrated to 1/4 volume and then poured into 300 mL water. The pH was adjusted to 9 with 2N NaOH and saturated sodium bicarbonate, and extracted with ethyl acetate. The ethyl acetate solution was dried over magnesium sulfate filtered and concentrated in vacuo. The 10.2g of crude material was dissolved in 100 mL pyridine and treated with 2.7 mL (3.99g, 34 mmole) methanesulfonyl chloride. The reaction was stirred at room temperature for 3 hours, evaporated to ~ 1/4 volume and poured into a mixture of ethyl acetate and saturated sodium bicarbonate. The layers were separated and the aqueous phase extracted several times with ethyl acetate.

The combined organics were dried over magnesium sulfate, filtered and concentrated in vacuo. The residue was chromatographed on silica gel eluting with 3% methanol/chloroform, to give 12.2g product which was crystallized from ethyl acetate to yield 9.2 g, mp = 150°C.

| Elemental analysis for C₂₁H₂₄N₂O₄S•1/4C₄H₈O₂ | | | |
|---|---|---|---|
| Calcd | N, 6.63; | C, 62.53; | H, 6.15. |
| Found | N, 6.64; | C, 62.52; | H, 6.17. |

### Step B: Preparation of 6-Methanesulfonamido-3,4-dihydrospiro-[(2H)-1-benzopyran-2,3'-piperidine]-4-one hydrochloride

A solution of 7 g (17.47 mmols) of 6-methanesulfonamido-1'-benzyl-3,4-dihydrospiro-[(2H)-1-benzopyran-2,3'-piperidine]-4-one in 250 mL dichloroethane was treated with 5.6g (43.7 mmole) of diisopropylethylamine, and 6.24g (43.7 mmoles) of 1-chloroethyl chloroformate. The reaction was stirred at room temperature for 24 hours and then poured into 500 mL saturated sodium bicarbonate, and extracted with ethyl acetate. The ethyl acetate layers were washed with dilute acid to remove excess diisopropylethylamine. The ethyl acetate solution was then dried over MgSO₄, filtered and concentrated in vacuo. The crude material was dissolved in 250 mL CH₃OH and heated to reflux for 24 hours. The reaction mixture was cooled to room temperature and the solid collected by filtration to give 4.1g of product, mp = 290°C.

### Step C: Preparation of 6-Methanesulfonamido-1'-(2-(2-pyridyl)ethyl)-3,4-dihydrospiro-[(2H)-1-benzopyran-2,3'-piperidine]-4-one hydrochloride hydrate

Prepared according to the procedure as described previously in Example 52 Step C starting with 6-methanesulfonamido-3,4-dihydrospiro-2H-1-benzopyran-2,3'-piperidine-4-one. mp = 205-210°C.

| Elemental analysis for C₂₁H₂₅N₃O₄S•2HCl•H₂O | | | |
|---|---|---|---|
| Calcd | N, 8.29; | C, 49.80; | H, 5.77. |
| Found | N, 8.28; | C, 49.95; | H, 5.62. |

### EXAMPLE 56

### 6-Acetamido-3,4-dihydrospiro-1'-(2-(6-methyl)pyrid-2-yl)ethyl)[(2H)-1-benzopyran-2,4'-piperidine]-4-one dihydrochloride

A solution of 1.3g (4.7 mmoles) of 6-acetamido-3,4-dihydrospiro(2H-1-benzopyran-2,4'-piperidine)-4-one in 20 mL CH₃OH was treated with 560mg (4.7 mmole) of 2-vinyl-6-methylpyridine and 5 drops of acetic acid and heated to reflux for 36 hours. The reaction was cooled to room temperature, poured into 100 mL sat'd NaHCO₃, and extracted with ethyl acetate. The ethyl acetate solution was dried over magnesium sulfate, filtered and concentrated at reduced pressure. The free base was dissolved in ethanol and treated with ethanolic HCl. The dihydrochloride separated and was collected by filtration to give 610 mg of product. mp = 214-217°C.

| Elemental analysis for C₂₃H₂₇N₃O₃•2HCl•3/4H₂O | | | |
|---|---|---|---|
| Calcd | N, 8.75; | C, 57.56; | H, 6.40. |
| Found | N, 8.74; | C, 57.76; | H, 6.08. |

### EXAMPLE 58

### 6-Methanesulfonamido-3,4-dihydro-1'-(2-(2-(6-methyl)pyridyl)ethyl)spiro[(2H-)1-benzopyran-2,4'piperidine]-4-one

A solution of 1.3g of 6-acetamido-3,4-dihydro-1'-(2-(2-(6-methyl)pyridyl)ethyl)(2H-1benzopyran-2,4'-piperidine)-4-one in 20 mL methanol was treated with 10 mL 6N HCl heated to reflux for 4 hours. The reaction was cooled to room temperature, concentrated to dryness, and partitioned between ethyl acetate and saturated sodium bicarbonate. The ethyl acetate layers were dried over magnesium sulfate, filtered and concentrated. The crude material was dissolved in 10 mL pyridine and treated with 460 mg (4 mmoles) of methanesulfonyl chloride. The reaction was stirred at room temperature for 5 hours, concentrated to 1/2 volume and poured into saturated sodium bicarbonate solution. The mixture was extracted with several portions of ethyl acetate. The ethyl acetate layers were dried over magnesium sulfate, filtered and concentrated in vacuo . The free base crystallized from ethyl acetate to give 206 mg of product mp = 176-178°C.

| Elemental analysis for C₂₂H₂₇N₃O₄S | | | |
|---|---|---|---|
| Calcd | N, 9.78; | C, 61.51; | H, 6.33. |
| Found | N, 9.65; | C, 61.32; | H. 6.34. |

### EXAMPLE 59

### 6-Methanesulfonamido-3,4-dihydro-1'-(2-(4-methanesulfonamidophenyl)ethyl)spiro[(2H)-1-benzopyran 2,4'-piperidine]-4-one

This compound was prepared in a manner analogous to that of Example 58 and isolated as the hydrochloride salt to give 240mg of product. mp = 285-288°C.

| Elemental analysis for C₂₃H₂₉N₃O₆S•HCl | | | |
|---|---|---|---|
| Calcd | N, 7.72; | C, 50.77; | H, 5.55. |
| Found | N, 7.70; | C, 50.84; | H, 5.62. |

### EXAMPLE 67

### 3,4-Dihydro-1'-[2-(2-pyridyl)ethyl]spiro[(2H)-1-benzopyran-2-4'-piperidine]-4-one hydrochloride

### Step A: Preparation of 3,4-Dihydro-spiro[(2H)-1-benzopyran-2,4'-piperidine]-4-one hydrochloride

### Method 1

A solution of 2-acetylphenol (5g, 0.037 mmol), l-benzoyl-4-piperidone (7.5g, 0.037 mmol), pyrrolidine (2.6g, 0.037 mol) and CH₃OH (50 ml) was heated at reflux for 8 hours. After cooling to room temperature overnight, the solid was filtered to yield 8.2g of 1'-benzoyl-3,4-dihydro-spiro[(2H)-1-benzopyran-2,4'-piperidin]-4-one. The mother liquor was concentrated to dryness and the residue triturated with ether to yield an additional 1.7 g (83% total yield).

A solution of the benzoyl compound (3.2g, 0.01 mol), in ethanol (50 ml) and 6N HCl (50 ml) was heated at reflux. After 18 hours, the reaction was concentrated to dryness and the residue was flushed with ethanol (3X). The residue was crystallized from ethanol to yield 2.4g of title compound (92%); mp 238-240°C (ethanol).

| Analysis for C₁₃H₁₅NO₂•HCl | | | |
|---|---|---|---|
| Calcd | N, 5.52; | C, 61.54; | H, 6.36. |
| Found | N, 5.38; | C, 61.62; | H, 6.42. |

### Method 2

Utilizing the chemistry described in Method 1 but substituting l-methyl-4-piperidone in place of 1-benzoyl-4-piperidone provided an 82% yield of the corresponding 1'-methyl compound; mp >300°C (ethanol).

| Analysis for C₁₄H₁₇NO₂•HCl | | | |
|---|---|---|---|
| Calcd | N, 5.23; | C, 62.80; | H, 6.78. |
| Found | N, 5.02; | C, 62.51; | H, 6.90. |

A solution of the 1'-methyl compound (1.45g, 0063 mol) in benzene (30 ml) was added dropwise to a solution of 5M CNBr in CH₃CN (1.5 ml) in benzene (30 ml). The solution was stirred overnight at room temperature and then concentrated to dryness. The residue was treated with acetic acid (66 ml), 12N HCl (8 ml) and H₂O (35 ml). The solution was heated at reflux for 4 hours. After stirring at room temperature, the reaction was concentrated to dryness and the residue crystallized from ethanol to yield 1.6 g (75%) of title compound.

### Step B: Preparation of 3,4-Dihydro-1'-[2-(2-pyridyl)-ethyl]spiro[(2H)-1-benzopyran-2,4'-piperidin] -4-one hydrochloride

A solution of the product from Step A (0.4g, 0.0018 mol) in ethanol (10 ml) and 2-vinylpyridine (0.39g, 0.0037 mmol) was heated at reflux. After 18 hours, the reaction was concentrated to dryness and the residue was chromatographed on silica gel (Still column, 50 mm). The product was eluted with 20% CH₃OH-CHCl₃ with 2% aqueous NH₃. The product was crystallized as the hydrochloride salt from CH₃CN to yield 0.37g (51%) of title compound; mp 229-230°C.

| Analysis for C₂₀H₂₂N₂O₂•2HCl. | | | |
|---|---|---|---|
| Calcd | N, 7.09; | C, 60.76; | H, 6.12. |
| Found | N, 7.06; | C, 60.70; | H, 6.17. |

### EXAMPLE 68

### 3,4-Dihydro-1'-[2-p-nitrophenyl)ethyl]spiro[(2H)-1-benzopyran-2,4'-piperidine]-4-one hydrochloride hemihydrate

Under N₂, a mixture of the product of Example 67, Step A (0.91 g, 0.0042 mol), 4-nitrophenethylbromide(1.2 g, 0.005 mol), NaHCO₃ (1.3 g, 0.015 mol) in ethanol (25 ml) was heated at reflux. After 1 day, 4-nitrophenylethylbromide (1.2 g) and NaHCO₃ (1.3 g) was added and the mixture heated at reflux an additional 24 hr. The reaction was then concentrated to dryness and the residue was chromatographed on silica gel (Still column, 50 mm). The product was eluted with 10% CH₃OH-CHCl₃ and the product crystallized as the hydrochloride salt to yield 0.45 g (25%) of the title compound; mp 250-251°C (CH₃CN).

| Analysis for C₂₁H₂₂N₂O₄•HCl•1/2H₂O | | | |
|---|---|---|---|
| Calcd | N, 6.70; | C, 60.98; | H, 5.51. |
| Found | N, 6.80; | C, 61.23; | H, 5.87. |

### EXAMPLE 69

### 3,4-Dihydro-1'-(4-pyridyl)spiro[(2H)-1-benzopyran-2-4'-piperidine]-4-one·hydrochloride

A mixture of product from Example 67, Step A (1.0 g, 0.0046 mol), 4-bromopyridine (1.99, 0.0092 mol), NaHCO₃ (2.3 g, 0.027 mol) in ethanol (40 ml) was heated at reflux. After 24h, K₂CO₃ (3.7 g, 0.0027 mol) was added and the reaction was heated at reflux. After 24 hour, DMF (25 ml) was added, the ethanol boiled off and additional 4-bromopyridine (0.5 g) was added. After 24 hours at reflux, the reaction was poured into H₂O and the aqueous phase extracted with ethyl acetate (3x). The organic layers were dried, filtered and concentrated to dryness. The residue was chromatographed on Silica gel (Still column (50 mn) and the product was eluted with 10% CH₃OH-CHCl₃. The product was crystallized as the hydrochloride salt from ethanol to yield 0.4 g (27%) of product mp 251-253°C

| Analysis for C₁₈H₁₈N₂O₂•HCl. | |
|---|---|
| Calc'd | Obs. |
| N 8.47 | 8.30 |
| C 65.35 | 65.06 |
| H 5.79 | 5.72 |

### EXAMPLE 70

### 3,4-Dihydro-1'-[2-(2-pyridyl)ethyl-6-methanesulfonamido-spiro[2H-1-benzopyran-2-4'-piperidin]-4-one dihydrochloride mono-hydrate

### METHOD 1

### Step A: Preparation of 6-acetamido-3,4-dihydro-spiro [2H-1-benzopyran-2-4'-piperidine]-4-one

A solution of 4-acetamido-2-acetylphenol (13 g, 0.067 mol), CH₃OH (90 ml), piperidine-4-one ·HCl·H₂O (11 g, 0.072 mol) and pyrrolidine (10.2 g, 0.14 mol) was heated at reflux. After 20 hr, piperidine-4-one.HCL.H₂O (1.5 g, 0.01 mol) and pyrrolidine (1.7 g, 0.024 mol) were added and the reaction was heated at reflux. After 18 hr, the reaction was concentrated to dryness and the residue was dry packed with silica gel. The dry pack was added to a Still column (100 mm) and the residue eluted with 10% CH₃OH-CHCl₃ with 1% aq. NH₃ to yield 3.5g of phenolic starting material and then 11.8g (64%) of title compound.

### Step B: 6-Acetamido-3,4-Dihydro-1'-[2-(2-pyridyl)-ethyl]spiro[(2H)-1-benzopyran-2,4'-piperidin] -4-one-hydrochloride

A solution of product from Step A (7.0g, 0.026mol), CH₃OH (150ml), 2-vinylpyridine (8.4ml, 8.2g, 0.078mol) and acetic acid (10 drops) was heated at reflux. After 24 hr, the reaction mixture was poured into saturated NaHCO₃ and the mixture extracted with ethyl acetate (4x). The organic extracts were dried, filtered and concentrated to dryness (quantitative yield). An analytical sample was prepared by crystallization of the HCl salt from ethanol, m.p. 228-230°(dec).

| Analysis for C₂₂ H₂₅ N₃ O₃•2HCl•¾ H₂O | |
|---|---|
| Calc'd | Found |
| N 9.02 | 9.08 |
| C 56.71 | 56.74 |
| H 5.78 | 6.08 |

### Step C: Preparation of 3,4-Dihydro-1'-[2-(2-pyridyl) ethyl]-6-methanesulfonamido-spiro[(2H)-1-benzopyran-2-4'-piperidin]-4-one• dihydrochloride. monohydrate

A solution of product from Step B (9.7g, 0.025 mol) in ethanol (150 ml) and 6 N HCL (150 ml) was heated at reflux for 19h. The reaction was then concentrated to dryness and the residue was flushed (2x) with ethanol toluene to yield 8.0g (71%) of deacetylated product. The residue was treated with CH₂Cl₂. (250 ml), pyridine (40 ml) and methanesulfonyl chloride (2 ml, 2.96 g, 0.026 mol). After stirring overnight at room temperature, the reaction mixture was washed with 2.5 N NaOH (3x), the basic layer was adjusted to pH 8.5 and extracted with ethyl acetate (5x). The organic extracts were dried, filtered and concentrated to dryness to yield a 6.1 g residue which was crystallized as the dihydrochloride salt to yield the title compound.

### METHOD 2:

### Step A: Preparation of 1'-benzoyl-3,4-dihydro-8-nitro-spiro[(2H)-1-benzopyran-2-4'piperidine-4-one and 1'-benzoyl-3,4-dihydro-6-nitrospiro [(2H)-1-benzopyran-2-4'-piperidene]4-one

Under N₂, HNO₃ (15 ml, 1.49 density, 22.3 g, 0.35 mol) was added dropwise to a cooled solution of the 1'-benzoyl product from Example 67, Step A of Method 1 (12.6 g, 0.039 mol) in acetic anhydride (120 ml). After the addition, the solution was stirred at room temperature. After 3 hours, the reaction was poured into sat'd Na₂CO₃ and ice and the mixture was extracted with ethyl acetate (3x). The organic extract was dried, filtered and concentrated to dryness. The residue was chromatographed on silica gel (Still column, 100 mm) to yield 1.2 g (8.2%) of the 8-nitro product and then with 50% ethyl acetate-hexane to yield 6.4 g (44%) of the 6-nitro product.

### Step B: Preparation of 6-amino-1'-benzoyl-3,4-dihydro-spiro[(2H)-1-benzopyran-2-4'-piperidine] -4-one

A suspension of the 6-nitro compound from Step A (3.2g, 0.0087 mol) in ethanol (200 ml) and acetic acid (50 ml) with one teaspoonful of Raney nickel was hydrogenated at 5 psi. After the theoretical amount of H₂ was absorbed, the mixture was filtered under N₂ through a super cel pad and the solution was concentrated to dryness. The residue was partitioned between saturated NaHCO₃ and CHCl₃ (3x) and the organic extracts were dried, filtered and concentrated to dryness to yield 2.8g (95%) of title compound.

### Step C: Preparation of 1'-benzoyl-3,4-dihydro-6-methanesulfonamido-spiro[(2H)-1-benzopyran-2-4'-piperidine]-4-one

Under N₂, methanesulfonyl chloride (0.7 ml, 1 g, 0.009 mol) was added dropwise at room temperature to a solution of product from Step B (2.6 g, 0.0077 mol) and pyridine (2 ml) in CH₂Cl₂ (80 ml). After 18 hours, the reaction was poured into dilute acid and the mixture was extracted with ethyl acetate (5x). The organic extracts were dried, filtered and concentrated to dryness. The mixture was crystallized from CH₃OH-C₂H₅OH to yield 2.2g (69%) of product .

### Step D: Preparation of 3,4-dihydro-6-methanesulfonamido-spiro[(2H)-1-benzopyran-2-4'-piperidin]-4-one

A solution of product from Step C (2.6 g, 0.0063 mol) in ethanol and 6 N HCl (40 ml) was heated at reflux. After 18 hours, the solution was concentrated to dryness. The residue was crystallized from CH₃OH-C₂H₅OH to yield 1.8 g (82%) of product mp 285-287°C.

| Analysis for C₁₄H₁₈N₂O₄S•HCl. | |
|---|---|
| Calc'd | Found |
| N 8.08 | 7.97 |
| C 48.48 | 48.42 |
| H 5.52 | 5.49 |

### Step E: Preparation of 3,4-dihydro-1'-[2-(2-pyridyl)ethyl]-6-methanesulfonamido-spiro[(2H)-1-benzopyran-2-4'-piperidin]-4-one dihydrochloride •monohydrate

Under N₂, product from Step D (0.5 g, 0.0014 mol), 2-vinylpyridine (0.36 g, 0.0034 mol), sodium acetate trihydrate (0.45 g, 0.0033 mol), and 1:1 CH₃OH: H₂O (5.4 ml) was heated at reflux for 10 hours. The reaction was then partitioned between 10% NaOH and ethyl acetate and the organic layer was discarded. The aqueous layer was then adjusted to pH 8.5 and extracted with ethyl acetate (3x). The organic extract was dried, filtered and concentrated to dryness. The residue was crystallized as the hydrochloride salt from CH₃OH to yield 0.4 g (55%) of product mp 214-215°C.

| Analysis for C₂₁H₂₅N₃O₄S•2HCl•H₂O | |
|---|---|
| Calc'd | Found |
| N 8.30 | 8.41 |
| C 49.70 | 50.00 |
| H 5.33 | 5.51 |

### METHOD 3

### Step A: Preparation of 3,4-Dihydro-1'-methyl-6-nitrospiro-[(2H)-1-benzopyran-2,4'-piperidin]-4-one

Sulfuric acid (55 ml) was cooled to 0°C and was stirred as 3,4-dihydro-1'-methyl-spiro[2H-1-benzopyran-2,4'-piperidin]-4-one (11.56 g, 0.05 mol) was added over 20 minutes. Some gum separated. A solution of 90% nitric acid (2.5 ml, 0.0525 mol) in sulfuric acid (5 ml) was added over 20 minutes. The mixture was stirred for 2 hours at 0°C and then for 1 hour at room temperature. A solution resulted but nitration was not complete. 90% Nitric acid (0.3 ml, 0.007 mol) was added and stirring was continued for 1½ hours at room temperature. The reaction solution was poured into a mixture of ice, ethyl acetate, and sodium bicarbonate (210 g). The basic mixture was filtered, the ethyl acetate layer was separated and the aqueous layer was extracted with ethyl acetate (150 ml). The combined ethyl acetate extracts were washed with saturated NaCl, dried, filtered and concentrated in vacuo to yield 10.6g (77%) of product

### Step B: Preparation of 3,4-dihydro-6-amino-1'-methylspiro[(2H)-1-benzopyran-2,4'-piperidin]-4-one

The 3,4-dihydro-1'-methyl-6-nitro-spiro[2H-1-benzopyran-2,4'-piperdin]-4-one (11.2g, 0.04 mol) was dissolved in acetic acid (200 ml) and was hydrogenated using two "lab spoon" spatulas of Raney nickel catalyst in a Parr shaker. The theoretical amount of hydrogen was absorbed in 4 hours. The reaction mixture was filtered under nitrogen and the acetic acid was removed in vacuo. The residual oil was taken up in 3N HCl (150 ml), washed with ethyl acetate and neutralized with solid sodium bicarbonate. The product was extracted into ethyl acetate (3x100 ml), washed with sat'd. NaCl, dried, filtered and concentrated in vacuo to yield 7.4g (75%) of product.

### Step C: Preparation of 3,4-dihydro-6-methanesulfonamido-1'-methyl-spiro[(2H)-1-benzopyran-2,4'-pireridin]-4-one

3,4-Dihydro-6-amino-1'-methyl-spiro[2H-1-benzopyran-2,4'-piperidin]-4-one(5.6g, 0.0027 mol) was stirred in methylene chloride (50 ml) and pyridine (16 ml) at room temperature under a nitrogen atmosphere as methanesulfonyl chloride (2.7 ml, 0.035 mol) was added dropwise over several minutes. Some gum separated so additional pyridine (25 ml) was added. The mixture was stirred at ambient temperature for 16 hours. The methylene chloride and pyridine were removed in vacuo and the residue was taken up in saturated NaHCO₃ (150 ml) and methylene chloride (150 ml). The methylene chloride layer was separated and the aqueous phase was again extracted with methylene chloride. The combined extracts were washed with sodium bicarbonate, dried filtered and concentrated in vacuo. The residual mixture was chromatographed on silica gel (500g) using 15% methanol/chloroform to yield 3.67g (50%) of product.

### Step D: Preparation of 3,4-dihydro-6-methanesulfonamido-1'-spiro[2H-1-benzopyran-2,4'-piperidine]-4-one hydrochloride

3,4-Dihydro-6-methanesulfonamido-1'-methylspiro[2H-1-benzopyran-2,4'-piperidin]-4-one (324 mg, 0.001 mol) was dissovled in dry tetrahydrofuran (10 ml) and 1-chloroethyl chloroformate (0.3 ml, 0.003 mol) was added at room temperature followed by triethylamine (0.37 ml, 0.003 mol). The mixture was stirred in an oil bath at 70°C for 1 hour. Additional triethylamine (0.13 ml, 0.001 mol) was added and stirring at 70°C was continued for 1 hour. The reaction mixture was concentrated to dryness in vacuo. The residue was taken up in chloroform (25 ml) and was washed with cold 3N HCL and with water, dried, filtered and concentrated in vacuo to an amber oil. This oil was refluxed in methanol (15 ml) over night. The methanol was removed in vacuo. The residual oil was triturated in ethanol (5 ml) the solid that separated, was diluted with ether (10 ml) and then filtered to obtain 246 mg (71%) of product.

### Step E: Preparation of 3,4-dihydro-1'-[2-(2-pyridyl)-ethyl]6-methanesulfonamido-spiro[2H-1-benzopy ran-2,4'-piperidine]-4-one dihydrochloride monohydrate

Prepared as described in Method 2, Step E of this Example

### EXAMPLE 83

### 6-Methanesulfonamido-1'-[(2-(benzofurazan-5-yl)ethyl]-3,4-dihydrospiro[(2H)-1-benzopyran-2,3'-piperidine]-4-one

A solution of 500 mg (1.44 mmole) of 6-methanesulfonamido-3,4-dihydrospiro-2H-benzopyran-2,3'-piperidine-4-one hydrochloride in 10 mL acetonitrile was treated with 400 mg (1.7 mmoles) of 5-(2-bromoethyl)benzofurazan, 190 mg (1.44 mmole) of lithium iodide and 250 mg (3 mmoles) of sodium bicarbonate. The mixture was heated to reflux for 48 hrs, cooled to room temperature and poured into 50 mL H₂O. The mixture was acidified to pH 1 and extracted with 2 x 50 mL portions of ethyl acetate which were discarded. The aqueous phase was basified to pH 8-9 and extracted with ethyl acetate. The organic layers were dried over magnesium sulfate, filtered and concentrated in vacuo. The crude material was chromatographed on silica gel eluting with 5% methanol/chloroform to give 188 mg product. This material was dissolved in ethanol and treated with excess ethanolic HCl. The solid was collected and dried in vacuo to give 108 mg of the product with m.p. 255-257°C.

| Elemental analysis for C₂₂H₂₄N₄O₅S•HCl•¼H₂O | | | |
|---|---|---|---|
| | N | C | H |
| Calc'd | 11.26 | 53.11 | 5.16 |
| Found | 11.12 | 53.20 | 5.13 |

### EXAMPLE 84

### 6-Methanesulfonamido-1'-(2-(4-methanesulfonamidophenyl)ethyl-3,4-dihydrospiro-[(2H)-1-benzopyran-2,3'-piperidine]-4-one oxalate

The title compound was prepared following the procedure substantially as described in Example 83 but using 4-methanesulfonamidophenylethyl mesylate. The product has mp. 195-199°C.

| Elemental analysis for C₂₃H₂₉N₃O₆S₂•½C₂H₂O₄•H₂O | | | |
|---|---|---|---|
| | N | C | H |
| Calc'd | 7.49 | 51.42 | 5.57 |
| Found | 7.52 | 51.32 | 5.23 |

### EXAMPLE 88

### 3,4-Dihydro-1'-(benzofurazan-5-methyl)spiro[2H-1-benzopyran-2,4'-piperidine]-4-one:

In the same way, 5-(methanesulfonyloxymethyl) benzofurazan (91 mg, 0.4 mmol), 3,4-dihydrospiro[2H-1-benzopyran-2,4'-piperidine]-4-one (130 mg, 0.6 mmol) and diisopropylethylamine (0.35 ml, 0.26 g, 2 mmol) in DMF (1.5 ml) gave, after purification by flash column chromatography on silica gel eluting with EtOAc/40% hexane and crystallization from EtOAc/hexane, the benzopyranone as rods (70 mg, 50%), m.p. 126-127°C.
'H NMR (CDCl₃) δ 7.86 (1H, dd, J=8.0, 1.7 Hz), 7.78 (1H, d, J 9.3 Hz), 7.70 (1H, s), 7.5 (2H m), 7.0 (2H, m), 3.62 (2H, s), 2.74 (2H, s), 2.6 (4H, m), 2.1 (2H, m) 1.8 (2H, m).

| Elemental analysis for C₂₀H₁₉N₃O₃•0.33H₂O: | | | |
|---|---|---|---|
| Calculated; | C 67.59; | H 5.58; | N 11.82. |
| Found; | C 67.45; | H 5.40; | N 11.82. |

### EXAMPLE 89

### 3,4-Dihydro-1'-(benzofurazan-5-yl)ethyl]spiro[2H-1-benzopyran-2,4'-piperidine]-4-one:

### Step A: Preparation of 5-(2-Hydroxyethyl)benzofuroxan

4-(2-Acetoxyethyl)-2-nitroacetanilide (4.16 g, 15.6 mmol) was dissolved in methanol (25 ml) and methanolic potassium hydroxide (20% 25 ml) was added dropwise. The mixture was stirred at room temperature for 40 min., then cooled in ice. Aqueous sodium hypochlorite (Ca. 5%, 50 ml) was added dropwise over 30 min., with vigorous stirring. Water (50 ml) was added and the mixture was extracted with dichloromethane (3x100 ml). The combined organic fractions were dried (Na₂SO₄) and evaporated under reduced pressure to give the benzofuroxan as a yellow oil (2.76 g, 98%).
'H NMR (CDCl₃) δ 7.6-6.1 (3H, br m), 3.95 (2H, t, J=6.2 Hz), 2.91 (2H, t J=6.2 Hz), 2.1 (1H br s).

### Step B: Preparation of 5-(2-Hydroxyethyl)benzofurazan

5-(2-Hydroxyethyl)benzofuroxan (2.23 g, 12.4 mmol) was dissolved in methanol (25 ml) and trimethylphosphite (4.38 ml, 4.60 g, 37.1 mmol) was added. The mixture was heated under reflux for 3h., cooled and the solvent was evaporated under reduced pressure. The residue was purified by flash column chromatography on silica gel eluting with Et₂O to give the benzofurazan as a yellow oil (1.52 g, 75%). ¹H NMR (CDCl₃) δ 7.78 (1H, d, J=9.2 Hz), 7.65 (1H, s) 7.34 (1H, d, J 9.2 Hz), 3.98 (2H, t J=6.3 Hz), 2.98 (2H, t J=6.3 Hz), 1.9 (1H, br s).

### Step C: Preparation of 5-[2-(Methanesulfonyloxy)ethyl]benzofurazan

5-(2-Hydroxyethyl)benzofurazan (1.48 g, 9 mmol) was dissolved in dichloromethane (45 ml) and triethylamine (1.88 ml, 1.37 g, 13.5 mmol) was added. The mixture was cooled to -30°C and methanesulfonyl chloride (0.84 ml, 1.24 g, 10.8 mmol) was added dropwise over 5 min. The mixture was stirred at -30°C for 15 min. and dichloromethane (100 ml) was added. The mixture was washed with water (100 ml), dried (Na₂SO₄) and evaporated under reduced pressure to give the benzofurazan as a yellow solid (2.10 g, 96%).
¹H NMR (CDCl₃) δ 7.83 (1H, d, J=9.5 Hz), 7.70 (1H, s), 7.32 (1H, d, J=9.5 Hz), 4.53 (2H, t, J=6.4 Hz) 3.19 (2H, t, J=6.4 Hz), 2.99 (3H, s).

### Step D: Preparation of 3,4-Dihydro-1'-[2-benzofurazan-5-yl)ethyl]spiro[2H-1-benzopyran-2,4'-piperidine]-4-one:

5-[2-(Methanesulfonyloxy)ethyl]benzofurazan (169 mg, 0.7 mmol) in DMF (1 ml) was added to a stirred mixture of 3,4-dihydrospiro[(2H)-1-benzopyran-2,4'-piperidine]-4-one (266 mg, 1.05 mmol) and diisopropylethylamine (0.61 ml, 0.45 g, 3.5 mmol) in DMF (1 ml). The mixture was stirred at room temperature for 28 h, then at 50°C for 1 hr. The solvent was evaporated under reduced pressure and the residue was purified by flash column chromatography on silica gel, eluting with CH₂Cl₂/MeOH/NH₃(Aq.) (96:4:0.4). The appropriate fractions were combined and concentrated to dryness and the residue was recrystallized from ethyl acelate/hexane to give the benzopyranone as pale yellow microcrystals (20 mg, 8%), m.p. 117-119°C.
¹H NMR (CDCl₃) δ 7.86 (1H, dd, J 8.2, 2.7 Hz) 7.76 (1H, d, J 9.3 Hz), 7.61 (1H, br s), 7.50 (1H, dt, Jt 7.8, Jd 1.7 Hz), 7.30 (1H, dd, J 9.3, 1.2 Hz), 7.0 (2H, m), 2.92 (2H, t, J 7.5 Hz), 2.73 (6H m), 2.54 (2H, m) 2.09 (2H, m), 1.79 (2H, m).

| Elementary analysis for C₂₁H₂₁N₃O₃•0.1H₂O: | | | |
|---|---|---|---|
| Calculated; | C 69.06; | H 5.85; | N 11.50. |
| Found; | C 68.97; | H 5.90; | N 11.16. |

### EXAMPLE 92

### 3,4-Dihydro-1'-[2-(benzofurazan-5-oxy)ethyl]-6-methanesulfonamido-spiro[(2H)-1-benzopyran-2,4'-piperidine]-4-one hydrochloride

### Step A: Preparation of 5-(2-Bromoethoxy)benzofurazan:

Sodium hydroxide (40 mg, 1 mmol) was added to a solution of 5-hydroxybenzofurazan (136 mg, 1 mmol) in ethanol (2 ml) and the mixture was stirred at room temperature for 30 min. 1,2-dibromoethane (0.26 ml, 0.56 g, 3 mmol) was added and the mixture was heated under reflux for 20 hr., cooled and the solvent was evaporated under reduced pressure. Aqueous sodium bicarbonate (saturated, 10 ml) was added and the mixture was extracted with dichloromethane (3x10 ml). The combined organic fractions were dried (Na₂SO₄) and evaporated under reduced pressure. The residue was purified by flash column chromatography on silica gel, eluting with hexane/10% EtOAc to give the benzofurazan as a pale yellow oil (99 mg, 41%).
¹H(CDl₃) δ 7.74 (1H, d J 9.6 Hz), 7.16 (1H, dd, J 9.6, 2.0 Hz), 6.85 (1H, d, J 2.0 Hz) 4.39 (2H, t J 6.0 Hz), 3.72 (2H, t, J 6. 0 Hz).

### Step B: Preparation of 3,4-Dihydro-1'-[2-(benzofurazan-5-oxy)ethyl]-6-methanesulfonamidospiro [(2H)-1-benzopyran-2,4'-piperidine]-4-one hydrochloride:

5-(2-Bromoethoxy)benzofurazan (87 mg, 0.36 mmol), 3,4-Dihydro-6-methanesulfonamidospiro[(2H)-1-benzopyran-2,4'-piperidine]-4-one hydrochloride (125 mg, 0.36 mmol), sodium bicarbonate (91 mg, 1.08 mmol) and potassium iodide (30 mg, 0.18 mmol) in acetonitrile (4 ml) were heated under reflux for 24 hr, cooled and aqueous sodium bicarbonate (saturated, 10 ml) was added. The mixture was extracted with dichloromethane (3 x 10 ml) and the combined organic fractions were dried (Na₂SO₄) and evaporated under reduced pressure. The residue was purified by flash column chromatography on silica gel, eluting with CH₂Cl₂/MeOH/NH₃ (97: 3: 0.3), to give a yellow foam which was dissolved in EtOAc (2 ml). Ethanolic HCl (Ca. 1M, 2 ml) was added, the mixture was stirred for 2 h. and the solid was collected and dried in vacuo to give the hydrochloride as a white solid (114 mg, 62%), m.p.>250°C.
¹H NMR (DMSO) δ 11.2-10.8 (1H, br m), 9.68 (1H br s), 8.0-7.1 (6H, m), 4.6 (2H, br s), 3.6-3.2 (6H, m) 2.92 (3H, s), 2.84 (2H s), 2.1 (4H, m).

| Elementary analysis for C₂₂H₂₄N₄O₆S•HCl•0.25H₂O | | | |
|---|---|---|---|
| Calculated: | C 51.46; | H 5.01; | N 10.91. |
| Found | C 51.45; | H 5.05; | N 10.61. |

### EXAMPLE 94

### 3,4-Dihydro-1'-[2-(benzofurazan-5-yl)ethyl]-6-methanesulfonamido spiro[(2H)-1-benzopyran-2,4'-piperidine]-4-one hydrochloride

### Step A: Preparation of 5-(2-Bromoethyl) benzofurazan

A solution of 5-(2-hydroxyethyl)benzofurazan (1.0g, 6.1 nmol) and carbon tetrabromide (2.6g, 7.9 nmol)in methylene chloride (10ml) was cooled to 0°C. A solution of triphenyl phosphine (1.9g, 7.3 nmol) in methylene chloride (10 ml) was added dropwise and the reaction was stirred for 5 min. Solvent evaporation and flash chromatography (silica gel, ethyl acetate-hexane, 5/95) gave 5-(2 bromoethyl)benzofurazan (1.2g, 86%); 'H NMR (CDCl₃) δ: 3.32 (t, 2H), 3.69 (t, 2H), 7.32 (d, 1H), 7.69 (s, 1H), 7.84 (d, 1H).

### Step B: Preparation of 3,4-Dihydro-1'-[2-(benzofurazan-5-yl)ethyl]-6-methanesulfonamidospiro[(2H)-1-benzopyran-2,4'-piperidine]-4-one hydrochloride

A suspension of 5-(2-bromoethyl)benzofurazan (0.39 g, 1.72 mmol), 3,4-dihydro-6-methanesulfonamido spiro[(2H)-1-benzopyran-2,4'-piperidine]-4-one hydrochloride (0.24 g, 0.69 mmol) and sodium bicarbonate (0.21 g, 2.55 mmol) in ethanol (13.2 ml) was heated at reflux temperature for 24 hr. Solvent evaporation and chromatography (silica gel, chloroform-methanol-ammonium hydroxide, 97/3/0.3) gave an oil; conversion to the hydrochloride gave the hydrochloride (0.14 g, 41%); mp > 250°C.

| Anal. Calcd for C₂₂H₂₄N₄O₃•HCl | | | |
|---|---|---|---|
| | C, 53.59; | H, 5.12, | N, 11.37. |
| Found | C, 53.55; | H, 5.15; | N. 11.12. |

### EXAMPLE 98

### 3,4-Dihydro-6-acetamido-1'-(4-nitrobenzyl)spiro[(2H)-1-benzopyran-2,4'-piperidine]-4-one hydrochloride

A solution of 4-nitrobenzyl bromide (0.73 g, 3.4 mmol), 3,4-dihydro-6-acetamido spiro[(2H)-benzopyran-2,4'-piperidine]-4-one (0.94 g, 3.4 mmol) and diisopropylethylamine (0.61 ml, 3.5 mmol) in dimethylformamide (15 ml) was stirred at room temperature for 3 hr. The reaction mixture was then concentrated under reduced pressure. The residue was diluted with methylene chloride and washed with saturated sodium bicarbonate solution, water and brine. Drying and solvent evaporation gave an oil; flash chromatography (silica gel, methanol-chloroform 2/98) gave 3,4-dihydro-6-acetamido-1'-(4-nitrobenzyl) spiro[(2H)-1-benzopyran-2,4'-piperidine]-4-one (0.83 g, 59%); 75 mg converted to hydrochloride; mp > 250°C.

| Anal. Calcd for C₂₂H₂₃N₃O₅•HCl·½ H₂O: | | | |
|---|---|---|---|
| | C, 58.08, | H, 5.55; | N, 9.24. |
| Found | C, 57.88; | H, 5.33; | N, 9.18. |

### EXAMPLE 99

### 3,4-Dihydro-6-methanesulfonamido-1'-(4-methanesulfonamidobenzyl)spiro[(2H)-1-benzopyran-2,4'-piperidine]-4-one

### Step A: Preparation of 3,4-Dihydro-6-acetamido-1'-(4-aminobenzyl)spiro[(2H)-1-benzopyran-2,4'-piperidine]

To a solution of 3,4-dihydro-6-acetamido-1'-(4-nitrobenzyl)spiro[(2H)-1-benzopyran-2,4'-piperidine]-4-one (0.58 g, 1.4 mmol) in acetic acid (6.4 ml) was added titanium (III) chloride (15% wt. in 20-30% wt. hydrochloric acid, 7 ml) dropwise. The reaction mixture was stirred at room temperature for 1 hr, cooled to 0°C and basified with saturated sodium bicarbonate and 20% sodium hydroxide solutions. Extraction with ethyl acetate, drying and solvent evaporation gave an oil (0.42g).
Chromatography (silica gel, chloroform-methanol-ammonium hydroxide, 95/2/0.5) gave 3,4-dihydro-6-acetamido-1'-(4-aminobenzyl)spiro[(2H)-1-benzopyran-2, 4'-piperidine]-4-one (136mg, 26%);
'H NMR (CDCl₃) d 1.71 (m, 2H), 2.04 (m, 2H), 2.18 (s, 3H), 2.42 (t, 2H), 2.62 (m, 2H), 2.69 (s, 2H), 3.45 (s, 2H), 6.66 (d, 2H, J= 8.3 Hz), 6.98 (d, 1H, J= 8.8 Hz), 7.10 (d, 2H, J= 8.3 Hz),7.63 ((d, 1H, J= 2.7 Hz), 7.93 (dt, 1H, J= 8.8 Hz, J = 2.8 Hz).

### Step B: Preparation of 3,4-Dihydro-6-amino-1'-(4-aminobenzyl)spiro[(2H)-1-benzopyran-2,4'-piperidine]-4-one

A solution of 3,4-dihydro-6-acetamido-1'-(4-aminobenzyl)spiro[(2H)-1-benzopyran-2,4'-piperidine](136mg, 0.36 mmol) and hydrochloric acid (6N solution, 13. 6 ml) in methanol (13.6 ml) was heated at reflux temperature for 1 hr. The reaction mixture was cooled to 0°C and basified with 20% sodium hydroxide solution. Extraction with methylene chloride, drying and solvent evaporation gave 3,4-dihydro-6-amino-1'-(4-aminobenzyl)spiro[(2H)-1-benzopyran-2,4'-piperidine]-4-one (107 mg, 88%);
'H NMR (CDCl₃) d 1.73 (m, 2H), 2.01 (m, 2H), 2.40 (m, 2H), 2.60 (m, 2H), 2.66 (s, 2H), 3.42 (s, 2H), 3.52 (bs, 2H), 3.63 (bs, 2H,) 6.65 (d, 2H, J= 8.4 Hz), 6.86 (m, 2H,), 7.09 (d, 2H, J= 8.3 Hz), 7.13 (d, 1H, J= 2.7 Hz).

### Step C: Preparation of 3,4-Dihydro-6-methanesulfonamido-1'-(4-methanesulfonamidobenzyl)spiro [(2H)-1-benzopyran-2,4'-piperidine]-4-one

A solution of 3,4-dihydro-6-amino-1'-(4-aminobenzyl) spiro [(2H)-1-benzopyran-2,4'-piperidine]-4-one (107 mg, 0.32 mmol) and methanesulfonyl chloride (78ml, 1.0 mmol) in pyridine (3 ml) was stirred at room temperature for 3h. Solvent evaporation gave a foam; flash chromatography (silica gel, chloroform-methanol-ammonium hydroxide, 96/4/0.4) and trituration with ethanol gave 3,4-dihydro-6-methanesulfonamido-1'-(4-methanesulfonamidobenzyl) spiro [(2H)-1-benzopyran-2,4'-piperidine-4-one (32 mg, 20%); mp 209-210°C
'H NMR (DMSO) δ 1.70 (m, 2H), 1.88 (m, 2H), 2.34 (t, 2H), 2.55 (m, 2H), 2.81 (s, 2H), 2.94 (s, 2H), 2.98 (s, 3H), 3.46 (s, 2H,) 7.06 (d, 1H, J= 8.8 Hz), 7.16 (d, 2H, J= 8.3 Hz), 7.27 (d, 2H, J= 8.5 Hz), 7.43 (dd, 1H, J= 8.8 Hz, J= 2.7 Hz), 7.57 (d, 1H, J= 2.7 Hz), 9.63 (s, 1H), 9.69 (s, 1H).

| Anal. Calcd. for C₂₂H₂₇N₃O₆S₂: | | | |
|---|---|---|---|
| | C, 53.52; | H, 5.52, | N, 8.51. |
| Found | C, 53.25, | H, 5.50, | N, 8.41. |

### EXAMPLE 100

### 3,4-Dihydro-6-methanesulfonamido-1'-(5-methanesulfon- amidobenzofuryl)-2-methyl-4-oxospiro[(2H)-1-benzopyran-2,4'-piperidine]

### Step A: Preparation of 6-Acetamido-3,4-dihydro-1'-(5-nitrobenzofuryl-2-methyl)-4-oxospiro[(2H)-1-benzopyran-2,4'-piperidine]

A mixture of 1.12 g (3.6 mmoles)-6-acetamido-3,4-dihydro-4-oxospiro[(2H)-1-benzopyran-2,4'-piperidine], 0.83 g (6.0 mmoles) potassium carbonate, 0.61 g (4.1 mmoles) sodium iodide, and 1.05 g (4.1 mmoles) 2-bromoethyl-5-nitro-benzofuran (European Patent Application no. 88300962.3, 5-2-88) in 75 ml acetonitrile was heated at reflux for 7 hours. The solvent was removed in vacuo and the residue was partitoned between 75 ml methylene chloride and 15 ml dilute sodium bicarbonate solution. The layers were separated and the aqueous layer was extracted with 75 ml methylene chloride. The combined extracts were washed with 15 ml water and brine, dried, and the CH₂Cl₂ was removed in vacuo to give 2.08 g crude product. The compound was purified by flash chromatography on silica gel eluting with 2.5: 97.5 methanol: chloroform to give 1.12g (69%) of product as a solid foam, m.p. 99-104°C.
¹H NMR (CDCl₃): δ 1.81 (m, 2H), 2.08 (d, 2H), 2.17 (s, 3H), 2.56 (m, 2H), 2.70 (s, 2H), 2.72 (m, 2H), 3.76 (s, 2H), 6.75 (s, 1H), 6.97 (d, 1HO, 7.21 (s, 1H-NH), 7.55 (d, 1H), 7.63 (d, 1H), 7.92 (d of d, 1H), 8.20 (d of d 1H), 8.47 (d, 1H).

### Step B: Preparation of 6-acetamido-1'-(5-aminobenzofuryl-2-methyl)-dihydro-4-oxospiro[(2H)-1-benzopyran-2,4'-piperidine]

To a solution of 1.08g (2.4 mmoles) of the compound from Step A in 10 ml acetic acid was added 7.7 ml (8.8 mmoles) of 15% titanium (III) chloride solution in 20-30%. aqueous hydrochloride acid dropwise in portions over 1 hour. The reaction mixture was made basic (pH 10) with saturated sodium bicarbonate solution and 10 N sodium hydroxide solution diluted in 300 ml water, and extracted with 3x300 ml ethyl acetate. The combined extracts were washed with 60 ml water and brine, dried and the solvent was removed in vacuo to give 0.99g (98%) product.
'H NMR (deuteriochloroform): δ 1.65 (broad s, 2H, NH₂), 1.80 (m, 2H), 2.04 (d, 2H), 2.16 (s, 3H), 2.51 (m, 2H) 2.68 (s, 2H), 2.70 (m, 2H), 3.66 (s, 2H), 6.44 (s, 1H), 6.64 (d of d 1H), 6.80 (d, 1H), 6.97 (d, 1H), 7.26 (d, 1H), 7.62 (d, 1H), 7.92 (d of d, 1H).

### Step C: Preparation of 6-Amino-1'-(5-aminobenzofuryl-2-methyl)-3,4-dihydro-4-oxospiro[(2H)-1-benzopyran-2,4'-piperidine]trihyarochloride

A solution of 1.01 g (2.4 mmoles) of the compound from Step B in 30 ml ethanol and 10 ml (30 mmoles) of 3N hydrochloric acid was heated at reflux for 6 hours. The solution was cooled and the solvent was removed in vacuo. The residue was stirred under 20 ml ethanol to give a solid which was filtered off and dried to give 0.90g (77%) product, mp 277-279°C.
'H NMR (DMSO-d₆): δ 2.18 (m, 4H), 2.92 (m, 2H), 3.35 (m, 6H), 3.68 (m, 2H), 4.69 (s, 2H), 7.18 (d, 2H), 7.34 (s, 1H), 7.40 (d of d, 1H), 7.58 (d of d, 1H), 7.67 (d, 1H), 7.75 (d, 1H), 7.77 (s, 1H) 10.4 (broad s, 3H).

### Step D: Preparation of 3,4-Dihydro-6-methanesulfonamido-1'-(5-methanesulfonamidobenzofuryl-2-methyl-4-oxospiro[(2H)-1-benzopyran-2,4'-piperidine]

To a solution of 0.24 g (0.50 mmole) of the compound from Step C and 0.21 ml (1.5 mmoles) triethylamine in 2 ml pyridine was added 0.081 ml (1.05 mmoles) methanesulfonyl chloride. The resulting red mixture was stirred 5 hours. The solvent was removed in vacuo and the residue was partitioned between 5 ml saturated sodium bicarbonate solution and 15 ml ethyl acetate. The layers were separated and the aqueous layer was extracted with 2x15 ml ethyl acetate. The combined extracts were washed with 3 ml water and brine, dried, and the solvent was removed in vacuo to give 0.28 g (97%) crude product which was purified by flash chromatography on silica gel eluting with 5:95 methanol: chloroform (saturated with ammonia) to give 0.167g (63%) of product as a foam.

To a solution of 0.167 g (0.313 mmole) of product in 3 ml ethanol was added o.058 ml (0.34 mmole) 5.9N hydrogen chloride in ethanol. The mixture was stirred 1 hour and the resulting yellow precipitate was filtered off to give 0.114g (64%) hydrochloride salt, mp 199-202°C.

| Anal Calcd for C₂₄H₂₇N₃O₇S₂•HCl•0.30 C₂H₅OH•0.65 H₂O: | | | |
|---|---|---|---|
| | C, 49.60; | H, 5.26; | N, 7.06. |
| Found | C, 49.63; | H, 5.25; | N, 7.04. |

### SYNTHESIS METHOD (EXAMPLE 101)

### 1,4-Dihydro-1'-[(6-methanesulfonamidoquinolin-2-yl) methyl]-7-methanesulfonamido-spiro-[(3H)-2-benzopyran-3,4'-piperidine] dihydrochloride

To a solution of 100 mg (0.3 mmole) 1.4 dihydro-7-methanesulfonamido-spiro-[(3H)-2-benzopyran-3,4;-piperidine]hydrochloride and 122 mg (0.45 mmole) 2-chloromethyl-6-methanesulfonamidoquinoline in 10 ml acetonitrile was added 76 mg (0.90 mmole) sodium bicarbonate. The mixture was heated at reflux under nitrogen for 19 hours. The reaction mixture was concentrated in vacuo to dryness. The residue was treated with a small amount of water and the crude product was filtered off. The crude product was chromatographed (silica gel), 5% methanol-chloroform). The free base was converted to the dihydrochloride salt by the addition of ethanolic hydrogen chloride solution and recrystallization from ethanol-methanol-ether to give the title compound (80 mg, 44.2%), m.p. 207°C.

| Analysis Calculated for C₂₅H₃₀N₄O₅S₂•2HCl: | | | |
|---|---|---|---|
| | C, 49.75; | H, 5.34; | N, 9.28. |
| Found | C, 49.44; | H, 5.29; | N, 9.22. |

Employing the procedure substantially as described in Example 101, but substituting for the 2-benzopyranspiropiperidine used therein, there were produced the spiropiperidines described in Table IV,

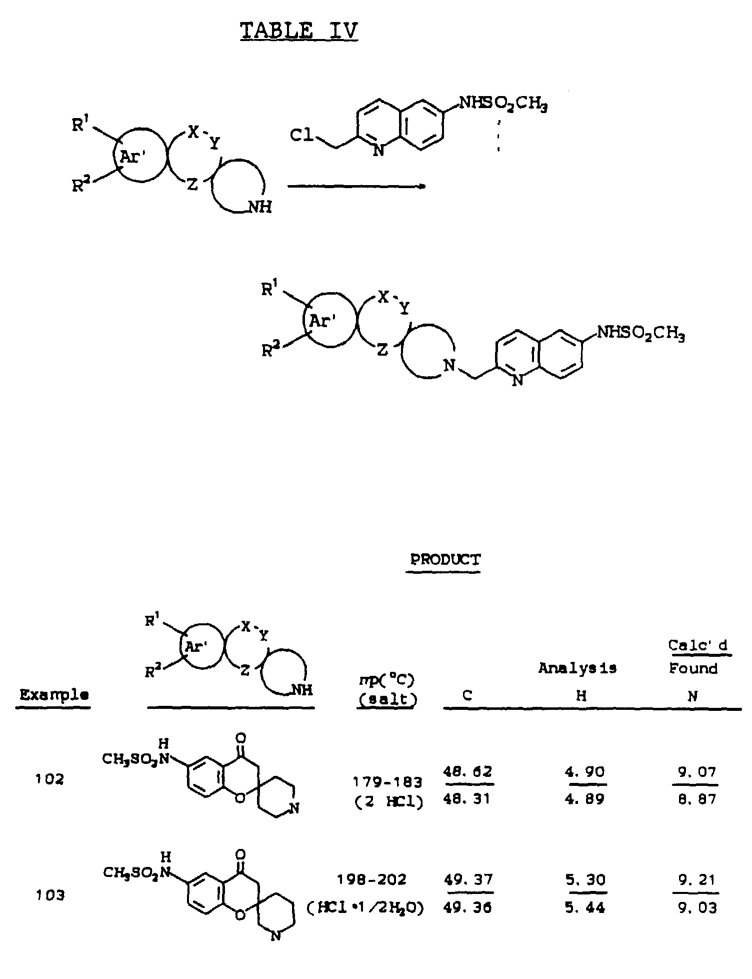

Employing the procedure substantially as described in Example 83 but substituting 3,4-dihydro-6-methanesulfonamidospiro[(2H)-1-benzopyran-2,4'-piperidine]-4-one (see Example 70, Method 2, Step D) for the 3,4,-dihydro-6-methanesulfonamidospiro[(2H)-1-benzopyran-2,3'-piperidine]-4-one used therein, and substituting an appropriate electrophile (wherein the leaving-group is methanesulfonyl, bromo or chloro) for the 5-(2-bromoethyl)benzofurazan, the following compounds of Tables V, VI, VII, VIII, IX, and X were prepared:

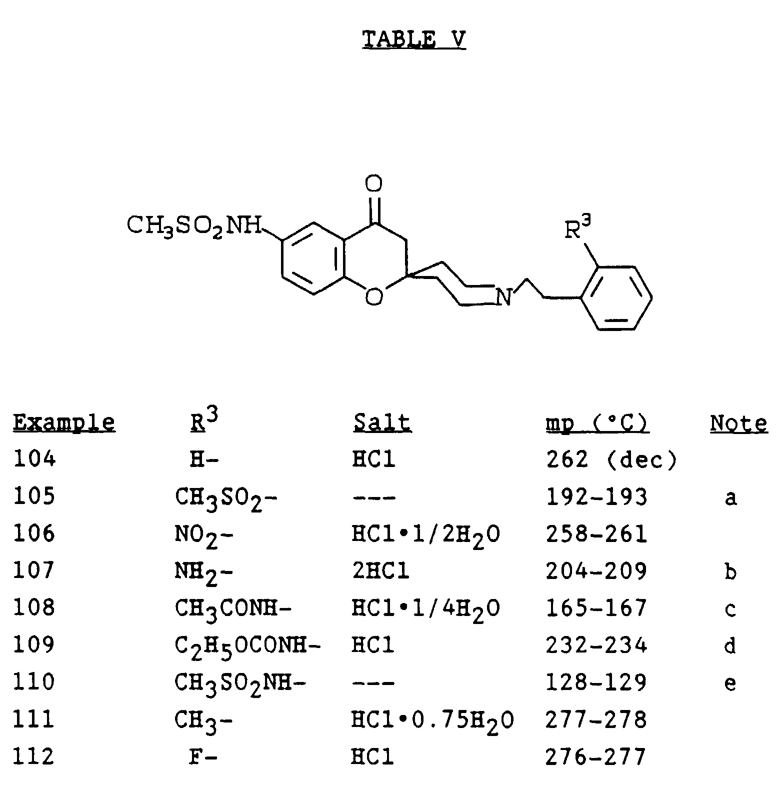

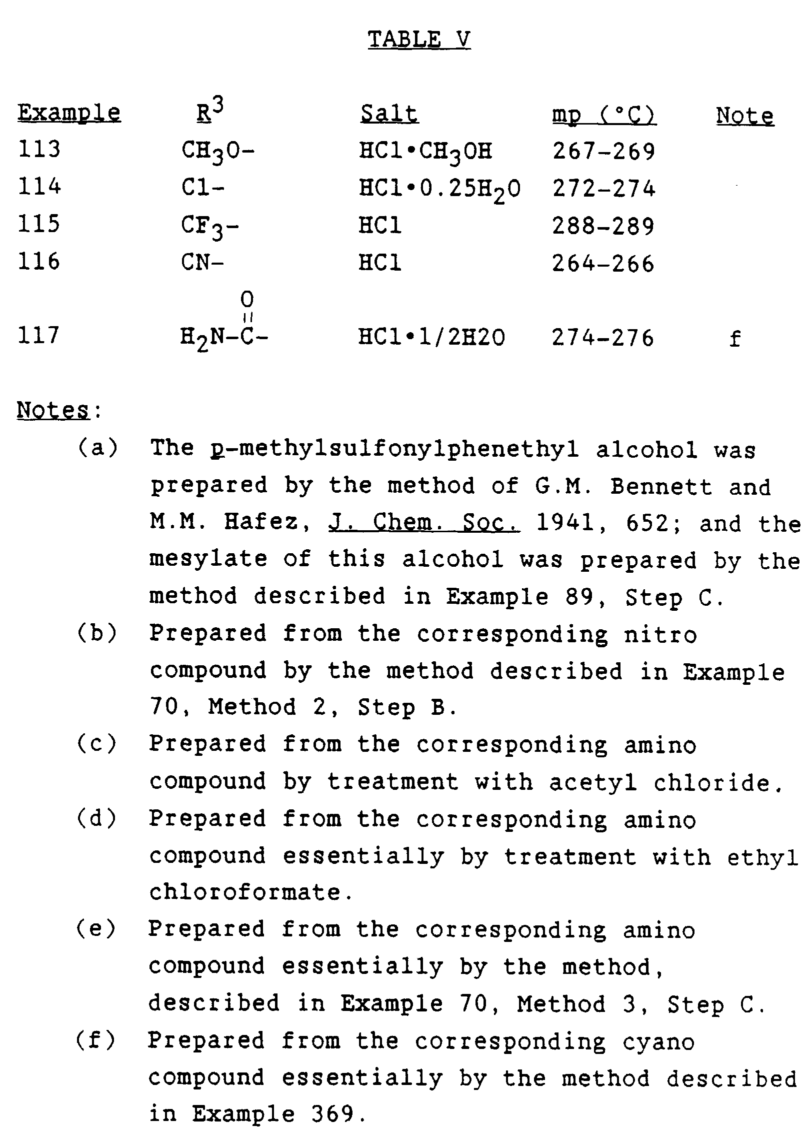

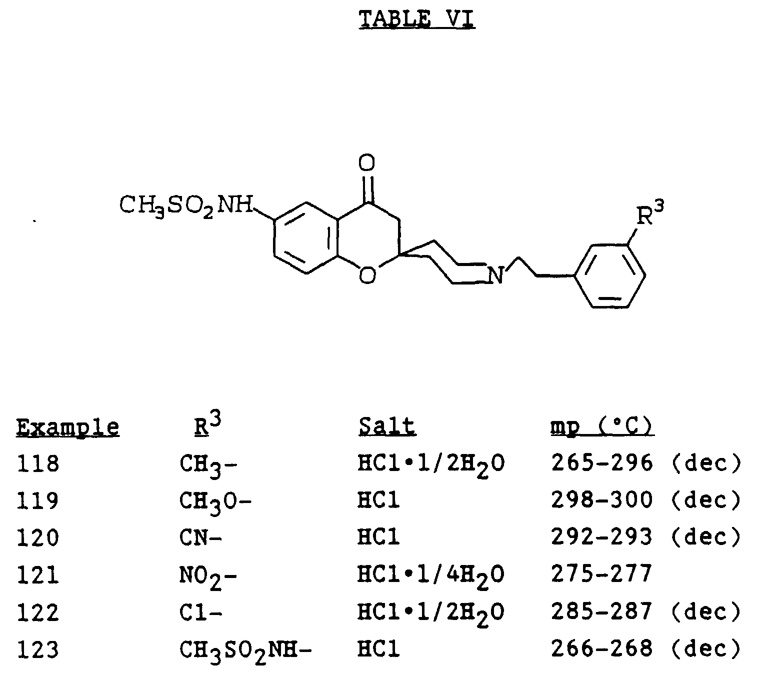

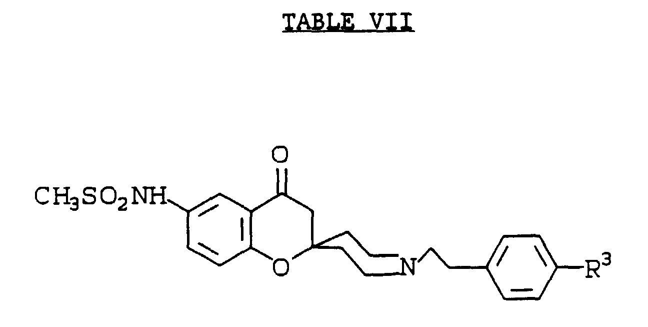

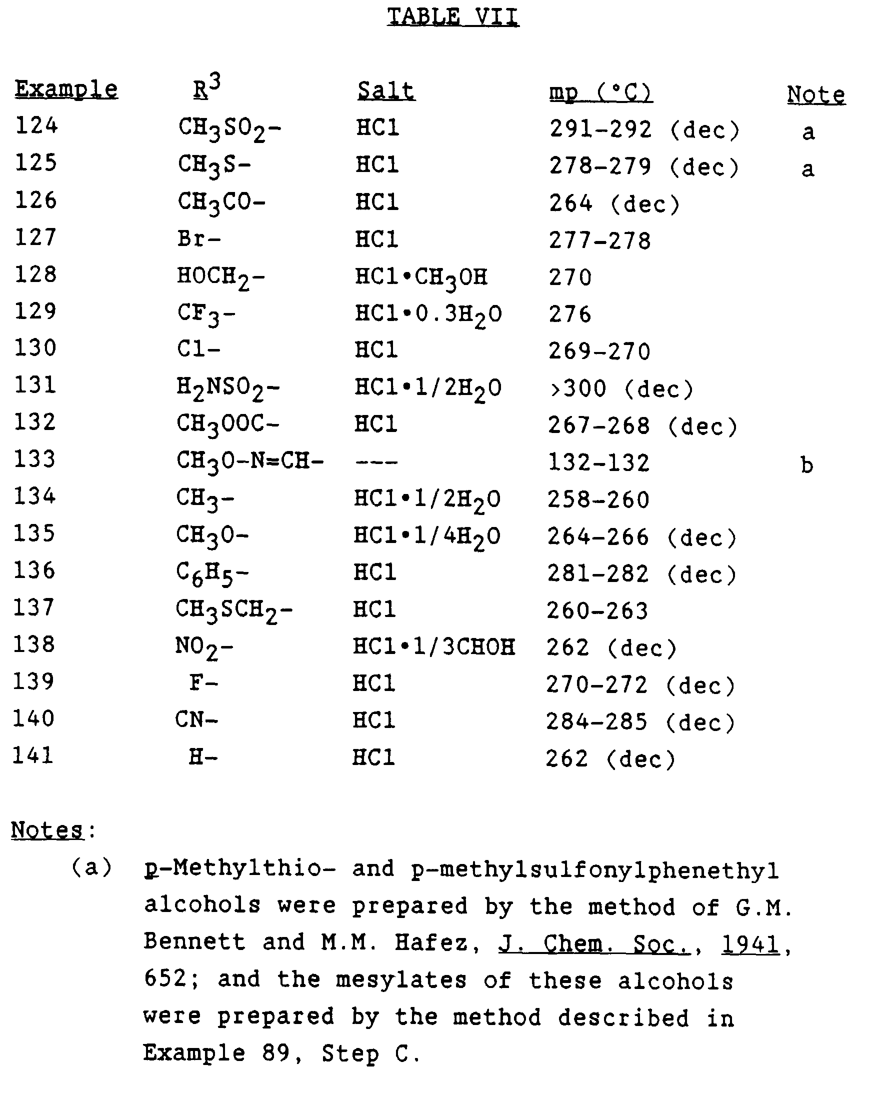

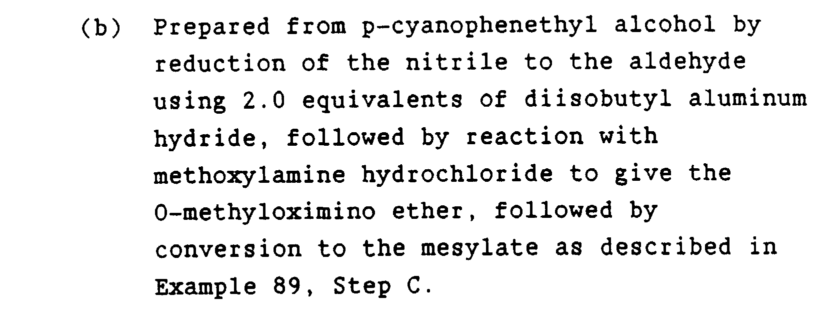

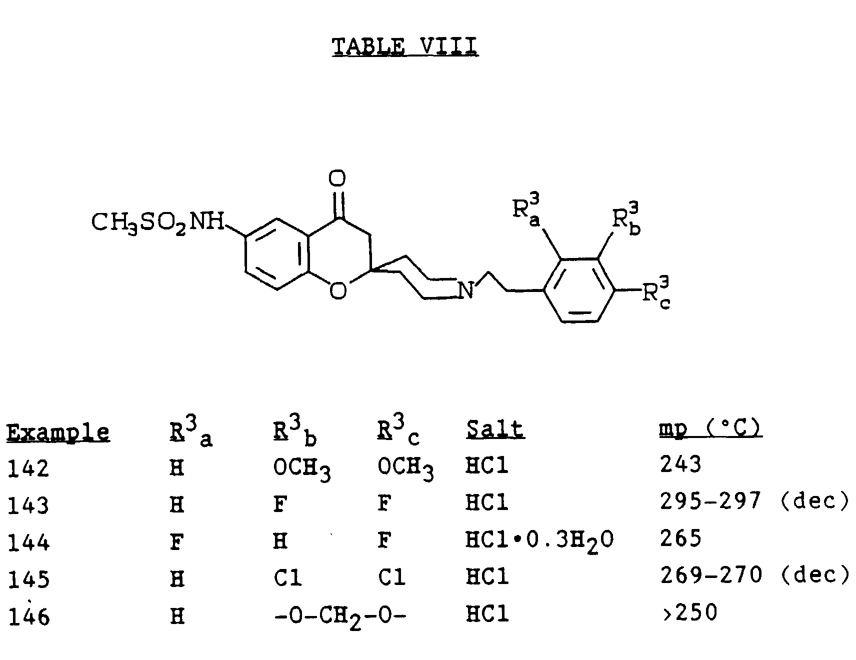

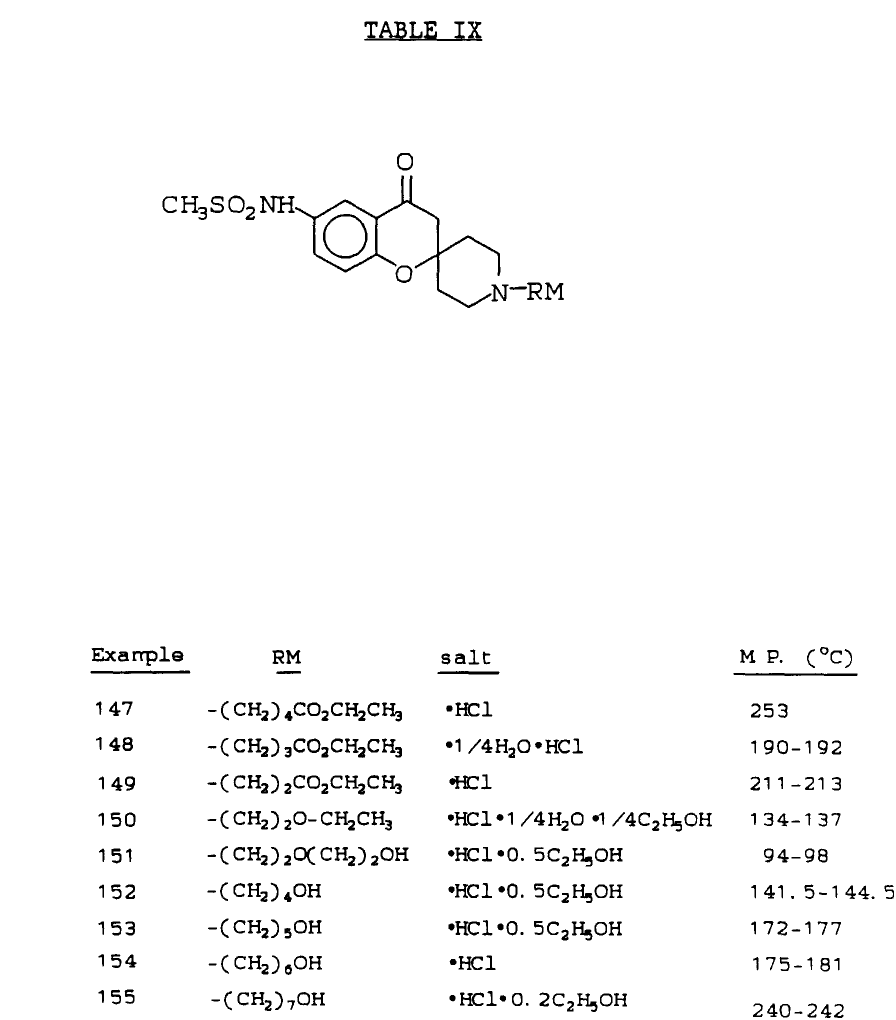

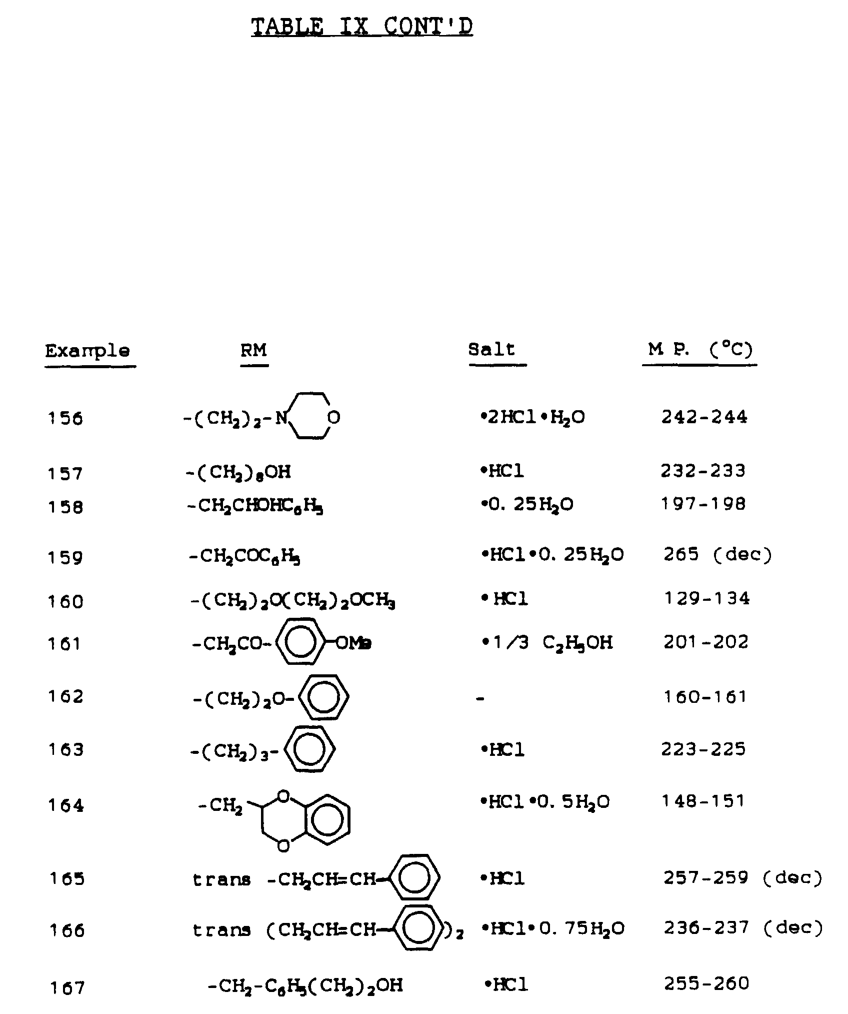

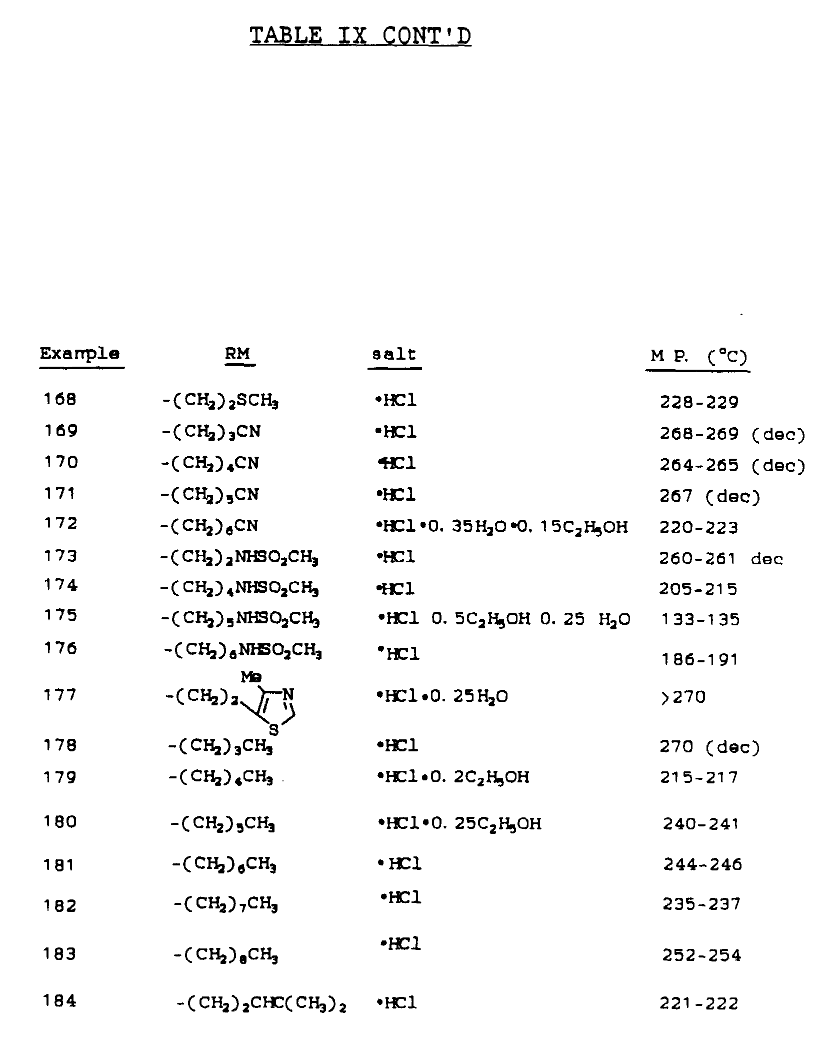

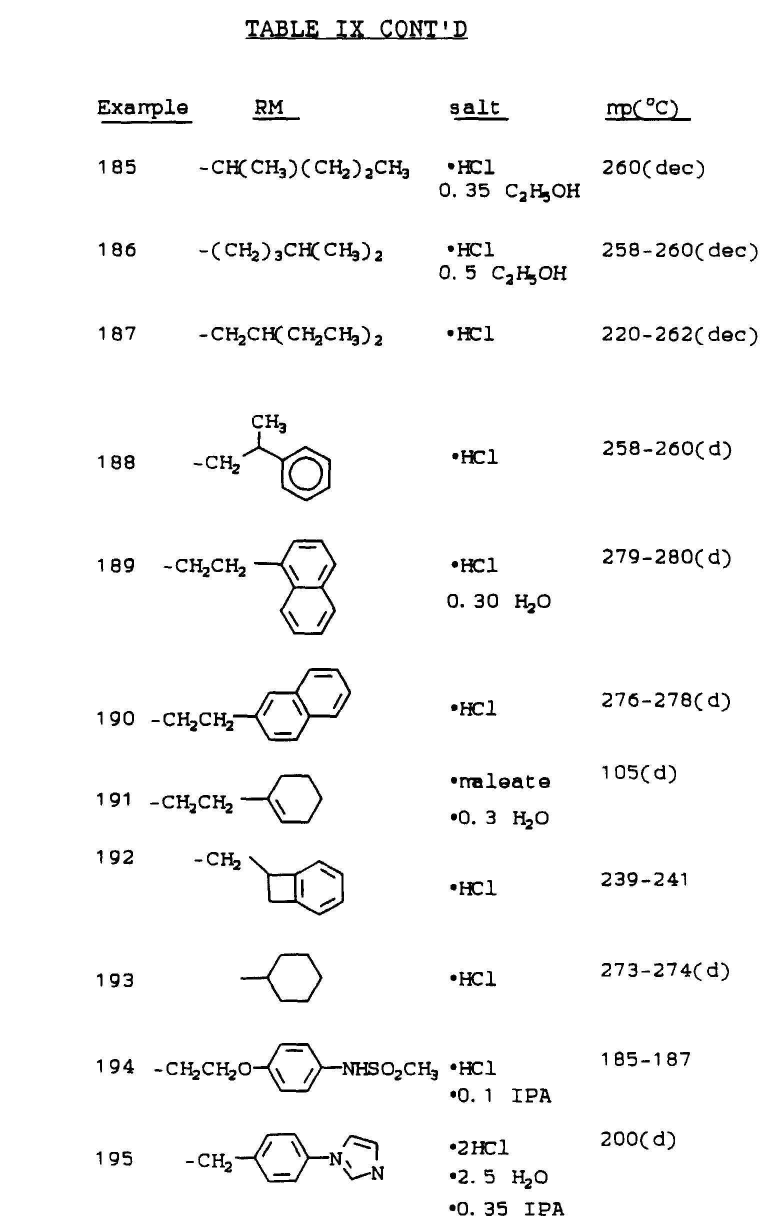

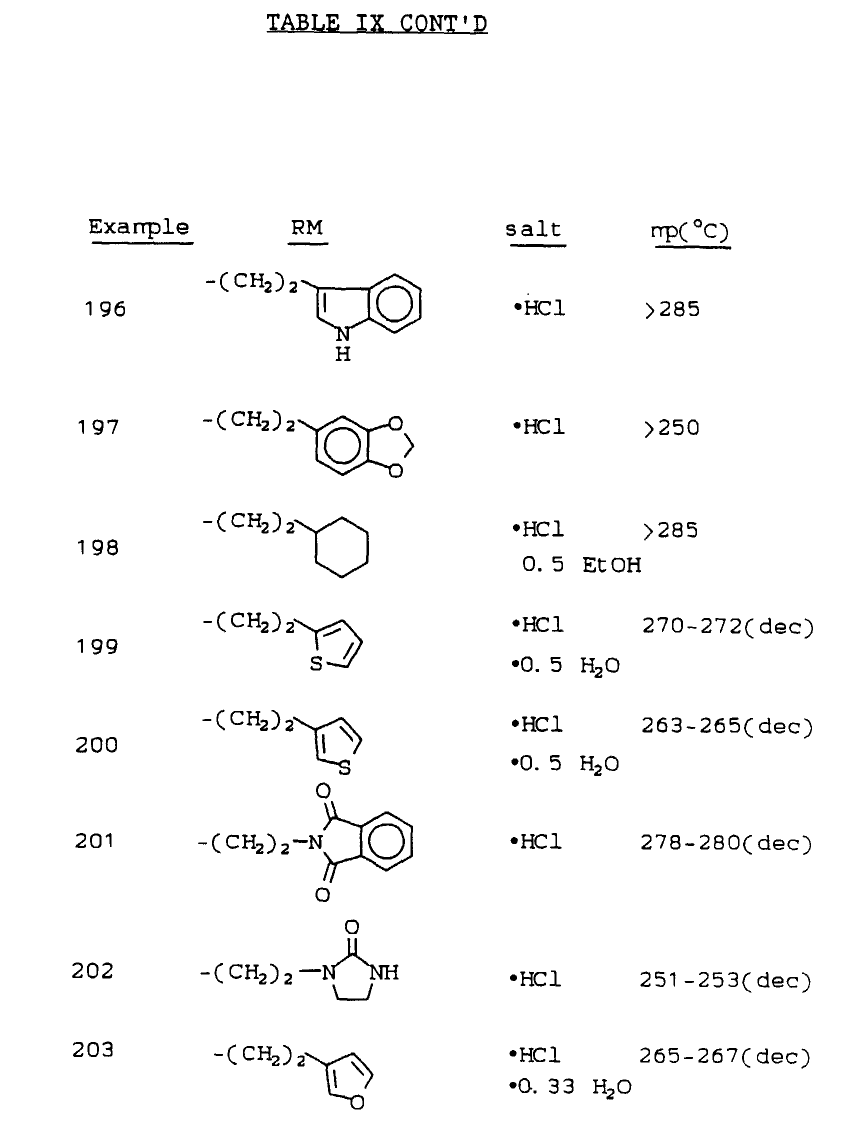

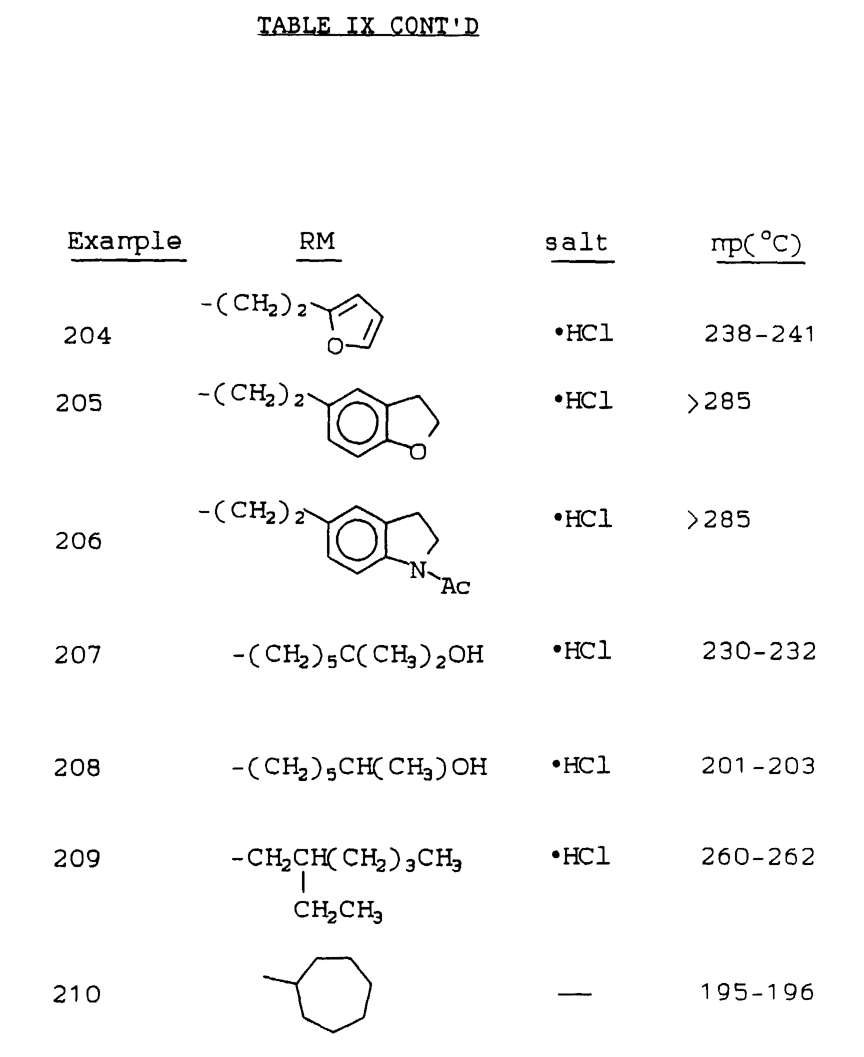

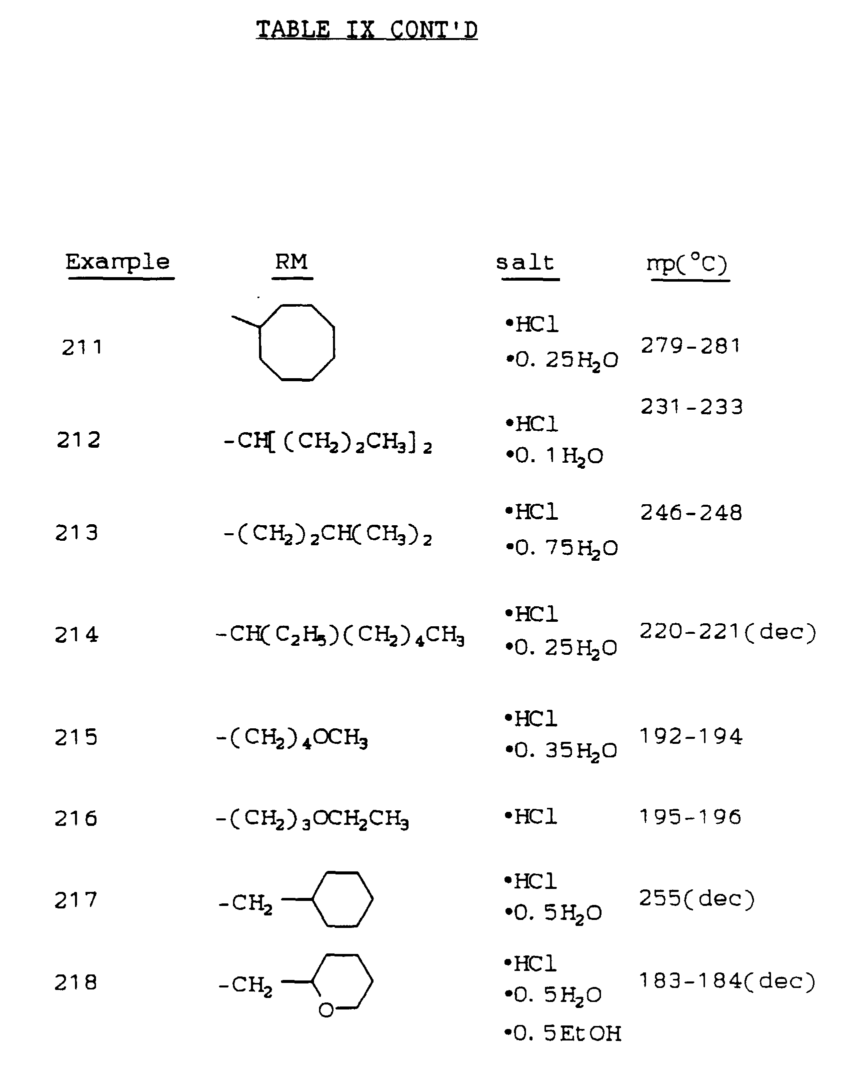

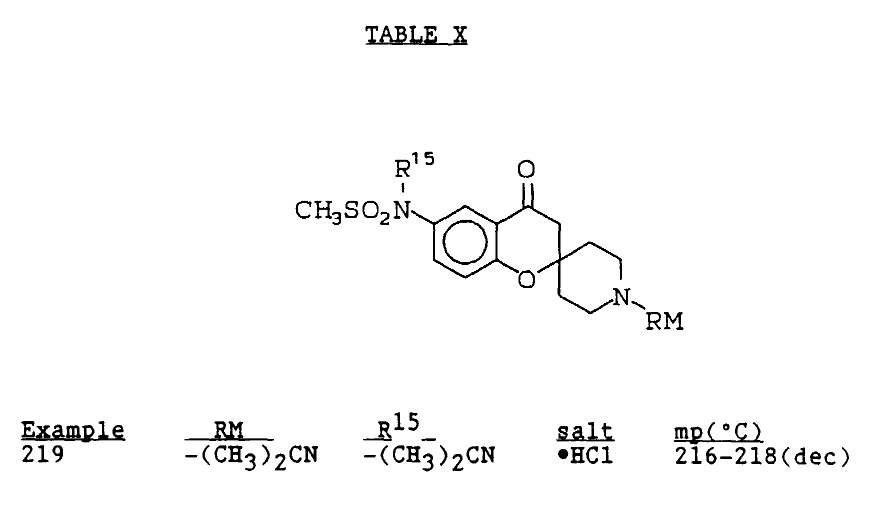

### EXAMPLE 220

### Preparation of 6-Methanesulfonamido-3,4-dihydro-spiro-[(2H)-1-benzopyran-2,4'-piperidin]-4-one-3-methyl hydrochloride

### Step A:

A solution of 2-hydroxypropiophenone (15 g, 0.1 mol), 1-acetyl-4-piperidone (14.2 g, 0.1 mol), and pyrrolidine (7.1 g, 0.1 mol) in CH₃OH (150 ml) was heated at reflux for 6 days. After cooling to room temperature, the reaction was concentrated to dryness. The residue was chromatographed on silica gel using a Still column (90 mm) and the mixture eluted with 30% EtOAc-hexanes to yield 9.5 g of starting material and then 60% EtOAc-hexanes to yield 10 g (37%) of 1'-acetamido-3,4-dihydro-3-methyl-spiro-[(2H)-1-benzopyran-2-4'-piperidin]-4-one.

### Step B:

To a solution of concentrated H₂SO₄ (18 mol) was added the product of Step A (5 g, 0.018 mol) at room temperature with stirring. After 0.5 hours, the mixture was cooled to -10°C and nitric acid (0.9 ml, 1.2 g, 0.019 mol) in (1.8 ml concentrated H₂SO₄) was added via syringe. After stirring for 0.5 hours at 0-4°C, the mixture was carefully added to NaHCO₃ (76 g) in and EtOAc. After separation, the aqueous layer was further extracted with EtOAc (2 x). The combined organic layers were dried, filtered, and concentrated to dryness to yield 5.8 g (88%) of 1'-acetamido-3,4-dihydro-3-methyl-6-nitro-spiro[(2H)-1-benzopyran-2-4'-piperidin]-4-one.

### Step C:

Under N₂, Ra-Ni (2 scoops in H₂O) was added to the product of Step B (5.1 g, 0.016 mol) in AcOH (50 ml) and hydrogenated on a Parr shaker under 12 psi. After 8 hours, the theoretical amount of H₂ was absorbed and the reaction was then filtered through super cel under a blanket of N₂ and the filtrate was concentrated to dryness. The residue was dissolved in CH₂Cl₂ (275 ml) and piperidine (13.6 ml, 13.2 g, 0.17 mol) and then methanesulfonyl chloride (2.2 g, 0.017 mol) was added at room temperature. After 15 hours, the reaction was washed with H₂O cold 1.5 N HCl, saturated NaHCO₃ dried, filtered and concentrated to dryness to yield 5.2 g (89%) of 1'-acetamido-3,4-dihydro-6-methanesulfonamido-3-methyl-spiro[(2H)-1-benzopyran-2-4'-piperidin]-4-one.

### Step D:

The product of Step C (5.2 g, 0.014 mol) in CH₃OH (80 ml) and 6N HCl (80 ml) was heated at reflux. After 7 hours, the reaction was concentrated to dryness and the residue flushed with EtOH (4 x) and then toluene to yield 5.0 g (98%) of the title compound.

Employing the procedure substantially as described in Example 83 but substituting 6-methanesulfonamido-3,4-dihydro-spiro[(2H)-1-benzopyran-2,4'-piperidin]-4-one-3-methyl hydrochloride for the 3,4-dihydro-6-methanesulfonamido-spiro[(2H)-1-benzopyran-2,3'-piperidine]-4-one hydrochloride used therein, and substituting an appropriate electrophile for the 5-(2-bromoethyl)benzofurazan, the following compounds of Table XI were prepared:

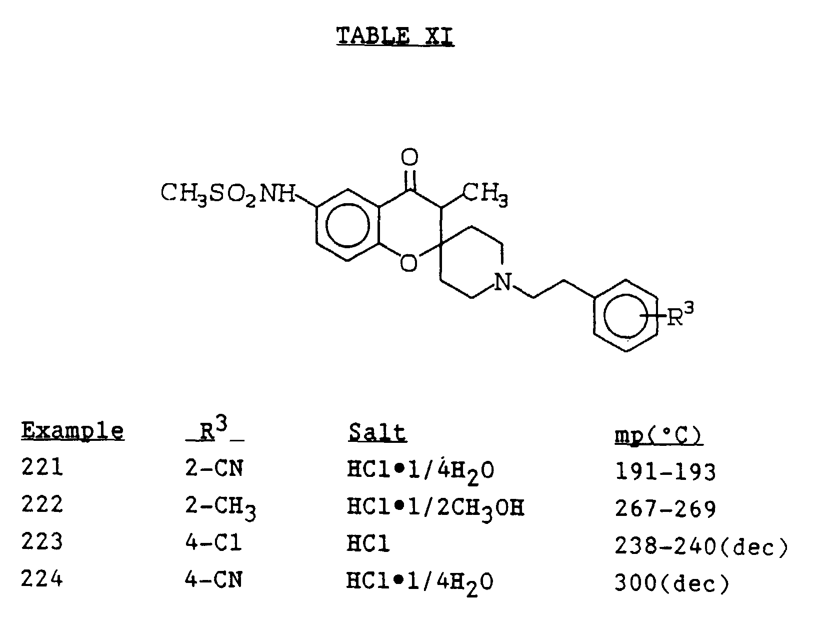

### EXAMPLE 225 R4

### 3,4-Dihydro-6-methanesulfonamido-3-methyl-1'-[2-(2-pyridyl)ethyl]-spiro[(2H)-1-benzopyran-2,4'-piperidine]-4-one

The title compound was prepared from 3,4-dihydro-6-methanesulfonamido-3-methyl-spiro[(2H)--benzopyran-2,4'-piperidine]-4-one hydrochloride in a manner analogous to that described in Example 52, Step C, mp 210-211°C.

| Anal. Calc'd for C₂₂H₂₇N₃O₄S•2HCl•1/2H₂O | | | |
|---|---|---|---|
| | C, 51.72; | H, 5.79; | N, 8.22. |
| Found | C, 51.66; | H, 5.91; | N, 8.22. |

### EXAMPLE 225A

### 3,4-Dihydro-6-methanesulfonamido-3-methyl-1'-hexylspiro[(2H)-1-benzopyran-2,4'-piperidine-4-one

The title compound was prepared from 3,4-dihydro-6-methanesulfonamido-3-methyl-spiro [(2H)-benzopyran-2,4'-piperidine]-4-one hydrochloride and hexyl bromide in a manner similar to that described in Example 83; mp = 261-264°C.

### EXAMPLE 226

### 3,4-Dihydro-3-methyl-6-methanesulfonamido-1'-[2-(benzofurazan-5-yl)ethyl]spiro[(2H)-1-benzopyran-2,4'-piperidine]-4-one hydrochloride

The title compound was prepared from 3,4-dihydro-3-methyl-6-methanesulfonamido-spiro[(2H)-1-benzopyran-2,4'-piperidine-4-one hydrochloride (Example 220) and 5-(2-bromoethyl)benzofurazan in a manner analogous to that described in Example 83; mp 240-243°C.

| Anal. Calc'd for C₂₃H₂₆N₄O₅S•HCl: | | | |
|---|---|---|---|
| | C, 54.48; | H, 5.37; | N, 11.05. |
| Found | C, 54.14; | H, 5.36; | N, 11.01. |

### EXAMPLE 227

### 3,4-Dihydro-3-methyl-6-methanesulfonamido-1'-[2-(benzofurazan-5-yl)ethyl]spiro[(2H)-1-benzopyran-2,4'-piperidine-4-ol hemihydrate

3,4-Dihydro-3-methyl-6-methanesulfonamido-1'-[2-(benzofurazan-5-yl)ethyl]spiro[(2H)-1-benzopyran-2,4'-piperidine-4-one was reduced with sodium borohydride in a manner analogous to that described in Example 429 to give the title compound as a mixture of diastereomers.

| Anal. Calc'd for C₂₃H₂₈N₄O₅S•1/2H₂O: | | | |
|---|---|---|---|
| | C, 57.36; | H, 6.07; | N, 11.64. |
| Found | C, 57.17; | H, 5.75; | N, 11.42. |

### EXAMPLE 228 (INTERMEDIATE COMPOUND)

### 1-(Hydroxymethyl)-4-(1H-imidazol-1-yl)benzene

To a mixture of 4-(1H-imidazol-1-yl)benzoic acid methyl ester (prepared as described in U.S. Patent No. 4,804,662) (1.5 g) in THF (25 ml) at -15°C was added a solution of 1M LAH/THF (10.4) ml). The mixture was stirred for 1/2 hour and allowed to warm to room temperature. The reaction was quenched by addition of water (0.5 ml), and then diluted with methanol (50 ml) and filtered. Concentration of the filtrate yielded 1.2 g (98%) of 1-(hydroxymethyl)-4-(1H-imidazol-1-yl)-benzene.
¹H NMR (300MHz, CDCl₃): δ 4.75 (s, 3H), 7.15 (s, 1H), 7.25 (s, 1H), 7.32 (d, 2H, J=12Hz), 7.48 (d, 2H, J=12Hz), 7.75 (s, 1H).

### EXAMPLE 229

### 6-Methanesulfonamido-3,4-dihydro-1'-(2-methylsulfinylethyl)spiro[(2H)-1-benzopyran-2,4'-piperidine]-4-one

A solution of 6-methanesulfonamido-3,4-dihydro-1'-(2-methylthioethyl)spiro[(2H)-1-benzopyran-2,4'-piperidine]-4-one (0.30 g, 0.78 mmol) in 15 mL of methanol was treated dropwise with a solution of sodium periodate (0.17 g, 0.78 mmol) in 15 mL of water. The solution was stirred at 25°C for 3 hours, and was then poured into 25 ml of water and extracted with a total of 100 mL of ethyl acetate. The organic phase was dried (MgSO₄), filtered and concentrated in vacuo to give the product as a pale yellow foam. The foam was dissolved in 30 mL of absolute ethanol and was treated with excess 4.8N ethanolic HC1. The resulting solid was collected and dried for 4 hours at 100°C under vacuum to give 0.083 g (24%) of product, mp 181-183°C.

| Anal. Calc'd. for C₁₇H₂₅ClN₂O₅S₂: | | | |
|---|---|---|---|
| | C, 46.72; | H, 5.77; | N, 6.41. |
| Found | C, 46.60; | H, 5.69; | N, 6.42. |

### EXAMPLE 230

### 6-Methanesulfonamido-3,4-dihydro-1'-(2-methanesulfonylethyl)spiro[(2H)-1-benzopyran-2,4'-piperidine]-4-one

A solution of 6-methanesulfonamido-3,4-dihydro-1'-(2-methylthioethyl)spiro[(2H)-1-benzopyran-2,4'-piperidine]-4-one (0.335 g, 0.871 mmol) in 15 mL of methanol was treated dropwise with a solution of Oxone® (0.803 g, 1.31 mmol) in 15 mL of water. The resulting mixture was stirred at 25°C for 2 hours. The reaction mixture was then poured into 50 mL of saturated aqueous NaHCO₃ and extracted with a total of 100 mL of ethyl acetate. The organic phase was dried (MgSO₄), filtered, and concentrated in vacuo to a pale yellow oil which was shown to be a mixture by then layer analysis. The mixture was chromatographed on silica gel using 5% methanol in methylene chloride eluant. The pure oil was dissolved in 20 mL of absolute ethanol and was treated with excess 4.8N ethanolic HCl. The solid was collected, washed with diethyl ether, and dried for 4 hours at 100°C under vacuum to give 0.176 g (44.6%) of product, mp 250-251°C.

| Anal. Calc'd. for C₁₇H₂₆ClN₂O₅S₂: | | | |
|---|---|---|---|
| | C, 45.07; | H, 5.56; | N, 6.19. |
| Found | C, 45.26; | H, 5.65; | N, 6.21. |

### EXAMPLE 231

### 3,4-Dihydro-6-methoxy-spiro[(2H)-1-benzopyran-2,4'-piperidine]-4-one hydrochloride

The title compound was prepared according to the procedure described in Example 67, Step A but substituting 5-methoxy-2-hydroxyacetophenone for 2-acetyl-phenol; mp 235-237°C.

| Anal. Calc'd. for C₁₄H₁₈NO₂•HCl: | | | |
|---|---|---|---|
| | C, 59.25; | H, 6.39; | N, 4.93. |
| Found | C, 59.00; | H, 6.20; | N, 4.84. |

### EXAMPLE 232

### 5-Methoxy-3,4-dihydro-1'-(2-(4-methanesulfonamidophenyl)ethyl)spiro[(2H)-1-benzopyran-2,4'-piperidine-4-one

A mixture of 3,4-dihydro-5-methoxy-spiro [(2H)-benzopyran-2,4'-piperidine]-4-one hydrochloride (1 g, 3.52 mmol) and 2-[4-(methanesulfonamido)phenyl)-ethylmethanesulfonate (1.5 g, 5.1 mmol) and sodium bicarbonate (excess) and LiI (680 mg, 5.1 mmole) in 30 mL acetonitrile was heated to reflux for 8 hours. The reaction was cooled to room temperature and poured into 200 mL saturated sodium bicarbonate and extracted with ethyl acetate. The ethyl acetate layers were dried over MgSO₄, filtered and concentrated in vacuo. The residue was chromatographed on silica gel using 2% to 5% MeOH/CHCl₃ as eluant to give 1.2 g. The solid was dissolved in ethanol and treated with excess ethanolic HCl. The solid was collected and dried under vacuum to give 0.900 g product, mp 255°C.

| Anal. Calc'd. for C₂₃H₂₈N₂O₅•HCl: | | | |
|---|---|---|---|
| | C, 56.95; | H, 6.02; | N, 5.77. |
| Found | C, 56.81; | H, 5.91; | N, 5.76. |

Employing the procedure substantially as described in Example 232, but substituting for the 2-[4-(methanesulfonamido)phenyl]ethylmethanesulfonate used therein, the appropriate electrophiles there were produced the compounds of Table XII.

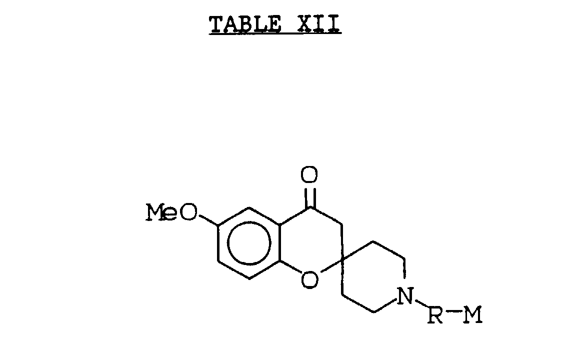

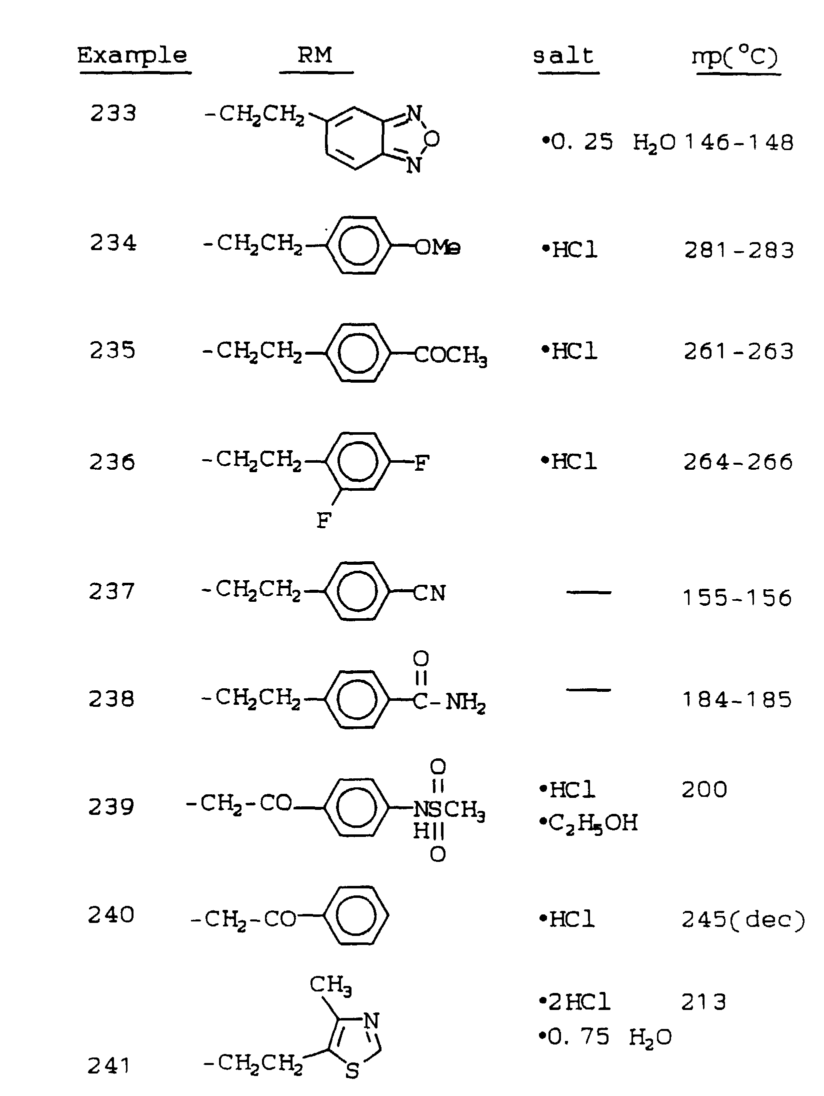

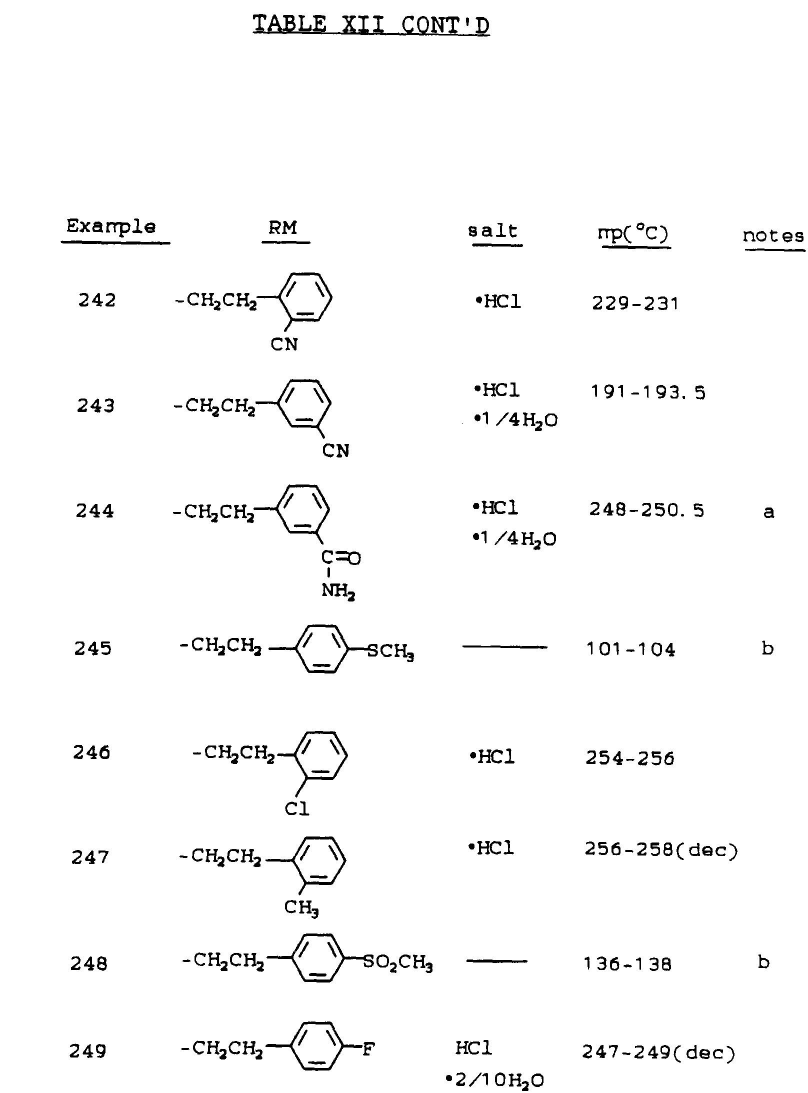

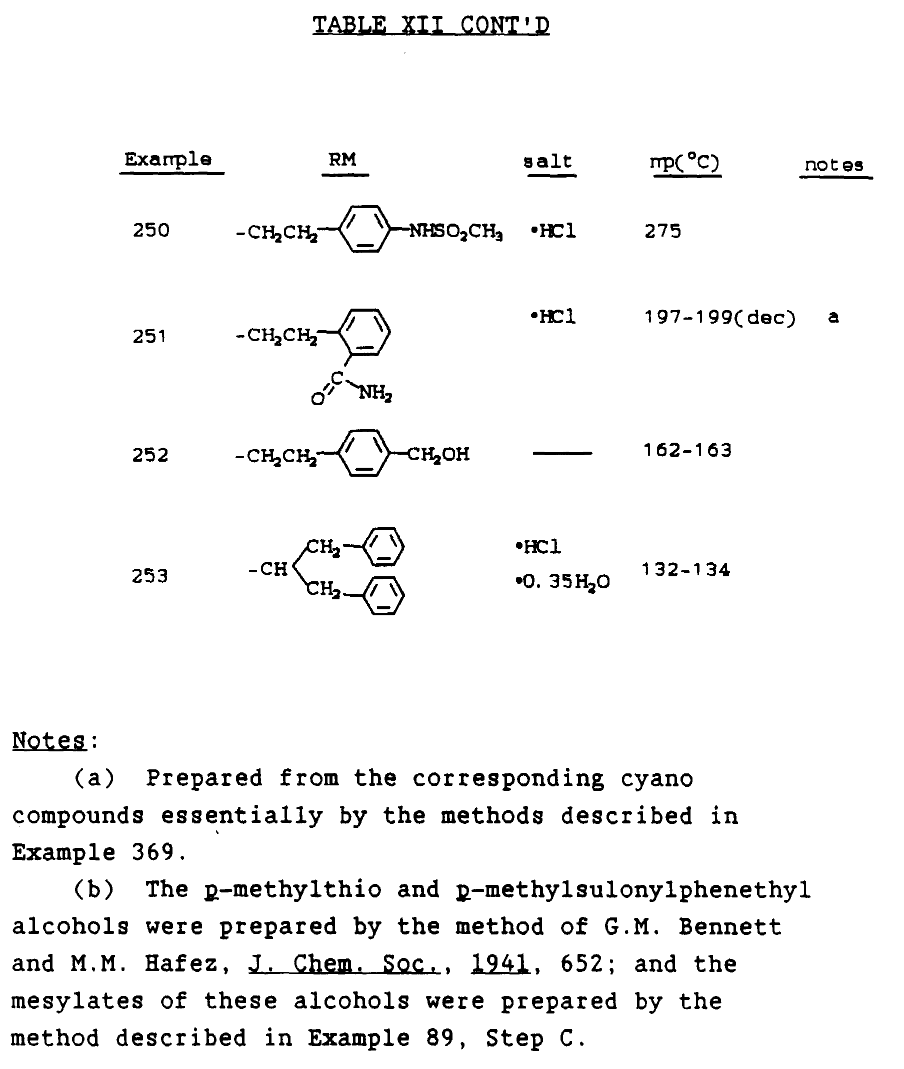

### EXAMPLE 254

### 6-Methoxy-3,4-dihydro-spiro(2H)-1-benzopyran-2,3'-piperidine-4-one hydrochloride

Employing a procedure analogous to that described for Example 52, Steps A and B, but substituting 5-methoxy-2-hydroxyacetophenone for the orthohydroxyacetophenone used therein there was obtained 6-methoxy-3,4-dihydro-spiro(2H)-1-benzopyran-2,3'-piperidine-4-one hydrochloride, mp 276-278°C (dec).

Employing the procedure substantially as described in Example 53, but substituting 6-methoxy-3,4-dihydro-spiro(2H)-1-benzopyran-2,3'-piperidine-4-one for the 3,4-dihydro-spiro(2H)-1-benzopyran-2,3'-piperidine]-4-one used therein, and substituting the appropriate electrophiles for the 2-(4-methanesulfonamidophenyl)ethyl mesylate used therein, there were obtained the spiropiperidines described in the following table.

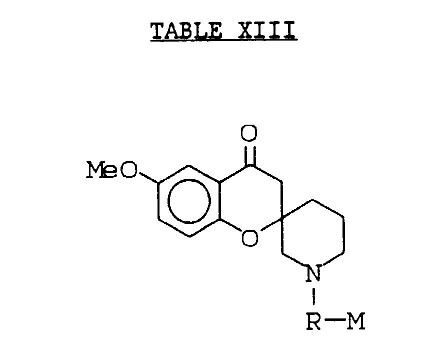

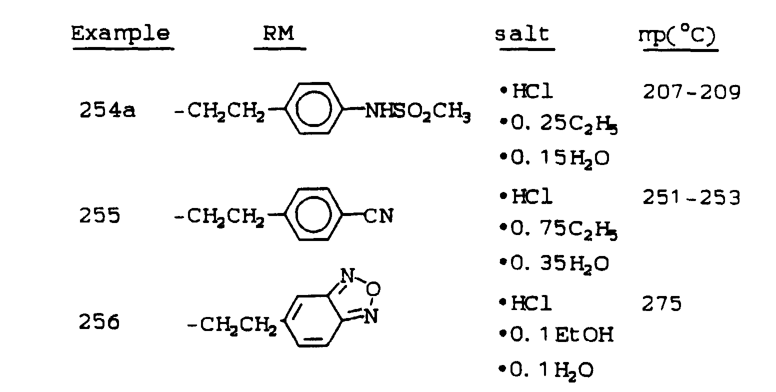

Employing the procedure substantially as described in Example 52, Step C, but starting with 6-methanesulfonamido-3,4-dihydrospiro-2H-1-benzopyran-2,3'-piperidine-4-one and the appropriate vinyl pyridines there were obtained the following compounds.

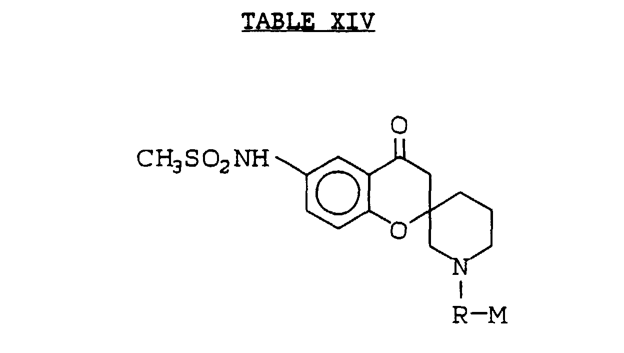

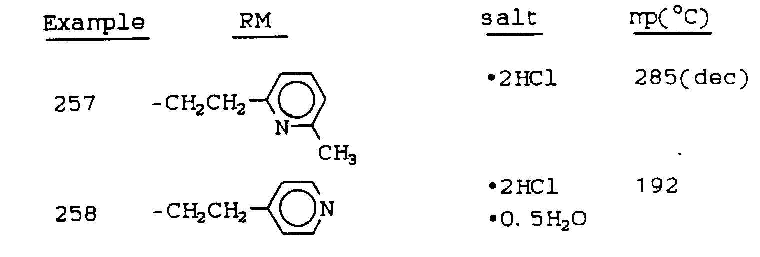

Employing the procedure substantially as described in Example 83, but substituting for the 5-(2-bromoethyl)benzofurazan used therein, an appropriate electrophile, there were produced the following compounds.

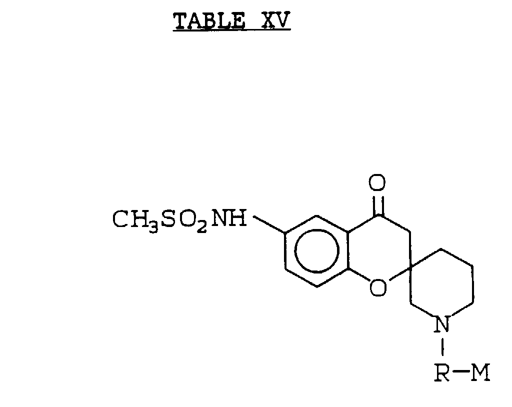

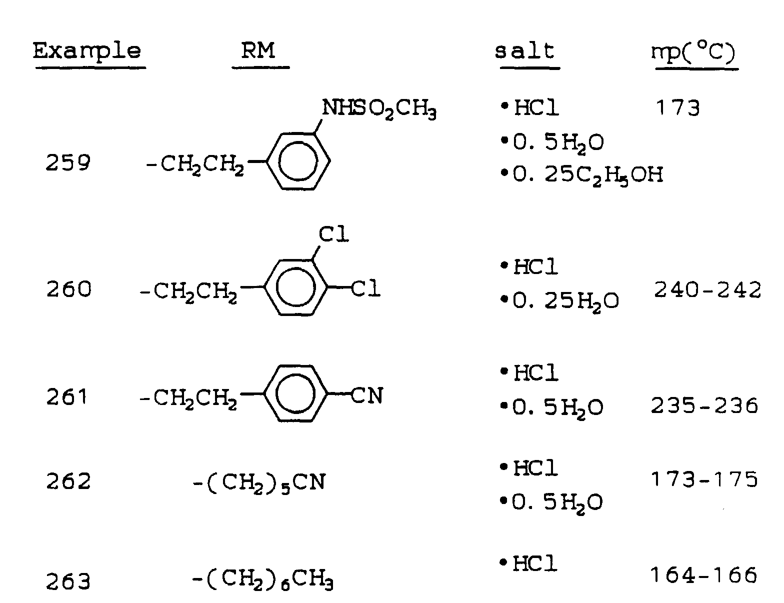

### EXAMPLE 264

### 6-Methanesulfonamido-3,4-dihydro-1'-(2-(4-aminophenyl)ethyl)-spiro[(2H)-1-benzopyran-2,4'-piperidine]-4-one

The title compound was prepared from 6-methanesulfonamido-3,4-dihydro-1'-(2-(4-nitrophenyl)ethyl)-spiro[(2H)-1-benzopyran-2,4'-piperidine]-4-one by a procedure analogous to that described in Example 25, mp 271°C(dec).

Employing a procedure substantially as described in Example 28 but substituting 6-methansulfonamido-3,4-dihydro-1'-(2-(4-aminophenyl)ethyl)-spiro[(2H)-1-benzopyran-2,4'-piperindine]-4-one for the 1-(2-(4-aminophenyl)ethyl) spiro(piperidine-4,6'-thieno[2,3-b]thiopyran)-4'5(H)-one used therin and the appropriate alkyl or aryl sulfonyl chlorides, there were produced the following compounds.

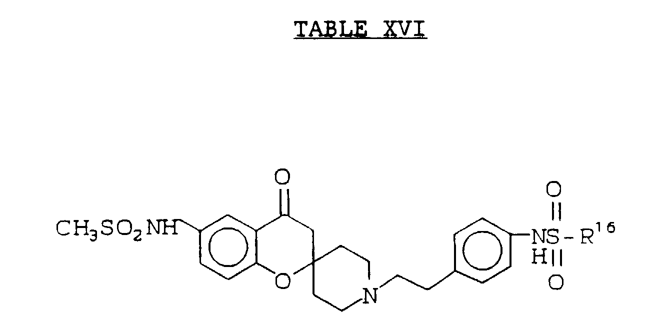

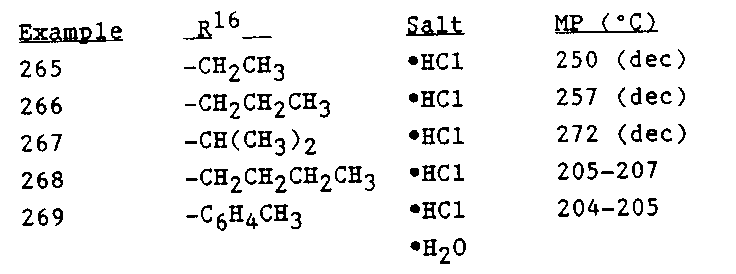

### EXAMPLE 270

### 6-Methanesulfonamido-3,4-dihydro-1'-(2-(4-acetamidophenyl)ethyl)-spiro[(2H)-1-benzopyran-2,4'-piperidine]-4-one

A suspension of 6-methanesulfonamido-3,4-dihydro-1'-(2-(4-aminophenyl)ethyl)-spiro[(2H)-1-benzopyran-2,4'-piperidine]-4-one (0.50 g, 1.2 mmol), and pyridine (0.09 mL, 1.2 mmol) in 25 mL of methylene chloride was cooled in an ice bath and treated dropwise with acetyl chloride (0.09 mL, 1.2 mmol). The mixture was stirred at 0°C for 2 hours, and then filtered. The tan solid was washed with methylene chloride and dried in the air. The solid was found to be a mixture by thin layer analysis, and was chromatographed on silica gel using 10% methanol in methylene chloride eluant. The pure oil was dissolved in 20 mL of absolute ethanol and was treated with excess 4.8N ethanolic HCl. The solution was then concentrated to 10 mL. The resulting ivory colored solid was collected, washed with diethyl ether, and dried 3 hours at 100°C under vacuum to give 0.10 g (18%) of product, mp 294°C(dec).

| Anal. Calc'd. for C₂₄H₃₀ClN₃O₅S: | | | |
|---|---|---|---|
| | C, 56.74; | H, 5.95; | N, 8.27. |
| Found | C, 56.70; | H, 5.90; | N, 8.08. |

### EXAMPLE 271

### 6-Methanesulfonamido-3,4-dihydro-1'-(2-(4-(methylsulfonylmethyl)phenyl)ethyl)-spiro[(2H)-1-benzopyran-2,4'-piperidine]-4-one (A) and 6-Methanesulfonamido-3,4-dihydro-1'-(2-(4-(methylsulfinylmethyl)phenyl)-ethyl)-spiro[(2H)-1-benzopyran-2,4'-piperidine]-4-one (B)

A solution of 6-methanesulfonamido-3,4-dihydro-1'-(2-(4-(methylthiomethyl)phenyl)ethyl)-spiro [(2H)-1-benzopyran-2,4'-piperidine]-4-one (450 mg, 0.88 mmol) in 10 mL methanol was treated with a solution of Oxone® (450 mg, 0.71 mmol) in 10 mL water. The reaction was stirred at room temperature for 1 hour and then poured into 100 mL saturated sodium bicarbonate and extracted with ethyl acetate. The ethyl acetate layers were dried over anhydrous magnesium sulfate, filtered and concentrated in vacuo. The residue was chromatographed on silica gel eluting with 2% MeOH/CHCl₃ to give in order of elution 218.8 mg of (A) free base and 170 mg of (B) free base. Each was dissolved in ethanol and treated with excess ethanolic HCl. The solids were collected and dried in vacuo to give 180 mg (A)•HCl•0.3H₂O, mp 285°C.

| Anal. Calc'd. for C₂₄H₃₀N₂O₆S₂•HCl•0.3 H₂O: | | | |
|---|---|---|---|
| | C, 52.55; | H, 5.81; | N, 5.11. |
| Found | C, 52.54; | H, 5.75; | N, 4.96. |

The sample of (B) was treated in a similar manner to give 140 mg (B) •HCl•0.35H₂O, mp 274°C.

| Anal. Calc'd. for C₂₄H₃₀N₂O₅S₂•HCl•0.35 H₂O: | | | |
|---|---|---|---|
| | C, 54.04; | H, 5.99; | N, 5.25. |
| Found | C, 54.08; | H, 5.78; | N, 5.23. |

### EXAMPLE 272

### 6-Methanesulfonamido-3,4-dihydro-1'-(2-(4-pyridyl)-ethyl)spiro[(2H)-1-benzopyran-2,4'-piperidine]-4-one

A solution of 6-methanesulfonamido-3,4-dihydrospiro[(2H)-1-benzopyran-2,4'-piperidine]-4-one (920 mg, 2.65 mmol3) and 4-vinyl-pyridine (1 g) in 15 mL methanol and 15 mL water was treated with NaOAc (200 mg) and heated to reflux for 16 hours. The reaction was cooled to room temperature and poured into ~100 mL of 1N NaOH. The mixture was extracted with ethyl acetate. The ethyl acetate extracts were discarded and the pH of the aqueous phase was adjusted to pH ~8-9 and extracted with ethyl acetate. The organic layer was dried over magnesium sulfate, filtered and concentrated in vacuo. The product was crystallized from ethyl acetate to give 470 mg of material that melted at 202-204°C.

| Anal. Calc'd. for C₂₁H₂₅N₃O₄S: | | | |
|---|---|---|---|
| | C, 60.70; | H, 6.06; | N, 9.67. |
| Found | C, 60.71; | H, 6.04; | N, 9.89. |

Employing the procedure described in Example 58, but substituting the appropriate alkyl or aryl sulfonyl chlorides for the methanesulfonyl chloride used therein, there were obtained the following compounds.

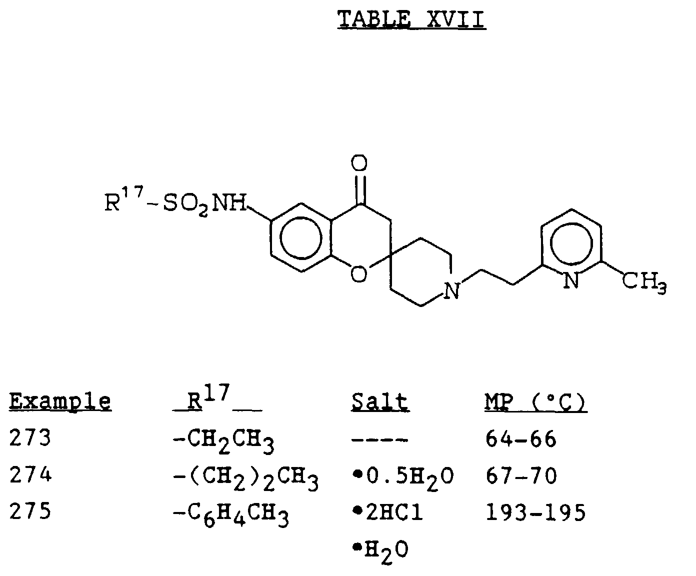

### EXAMPLE 276

### 6-Methanesulfonamido-3,4-dihydro-1'-[2-(2-chloropyrid-5-yl)ethyl]-spiro[(2H)-1-benzopyran-2,4'-piperidine]-4-one

The title compound was prepared from 3,4-dihydro-6-methanesulfonamidospiro[(2H)-1-benzopyran-2,4'-piperidine]-4-one hydrochloride and 2-chloro-5-chloroethylpyridine hydrochloride by a procedure analogous to that described in Example 92, Step B. [The 2-chloro-5-chloroethylpyridine hydrochloride used in this reaction was obtained by treating 6-chloropyrid-3-yl acetic acid (L.A. Carlson, Acta Pharm. Suecica, 9, 411 (1972)) with borane-tetrahydrofuran complex followed by reaction with thionyl chloride.] mp 213-215°C.

| Anal. Calc'd. for C₂₁H₂₄ClN₃O₄S: | | | |
|---|---|---|---|
| | C, 56.05; | H, 5.38; | N, 9.34. |
| Found | C, 55.93; | H, 5.41; | N, 9.20. |

### EXAMPLE 277

### 6-Methanesulfonamide-3,4-dihydro-1'[2-(2-chloro-1-oxopyrid-5-yl)ethyl]-spiro[(2H)-1-benzopyran-2,4'-piperidine]-4-one

The N-oxide of the product of Example 276 was prepared by treating 6-methanesulfonamido-3,4-dihydro-1'-[2-(2-chloro-pyrid-5-yl)ethyl]spiro [(2H)-1-benzopyran-2,4'-piperidine]-4-one (0.170 g, 0.000378 mol) in 120 mL of chloroform with 0.276 g (0.0008 mol) of m-chloroperbenzoic acid at room temperature overnight. Calcium hydroxide (0.12 g, 0.0016 mol) was added, the mixture was stirred one hour at room temperature and was filtered and concentrated in vacuo. Flash chromatography on silica gel using CH₂Cl₂: methanol: concentrated NH₄OH (90: 10: 2) gave the title compound, mp 170-171°C.

| Anal. Calc'd. for C₂₁H₂₄ClN₃O₅S: | | | |
|---|---|---|---|
| | C, 54.13; | H, 5.19; | N, 9.02. |
| Found | C, 54.11; | H, 5.29; | N, 8.94. |

### EXAMPLE 278

### 6-Methanesulfonamido-3,4-dihydro-1'-(2-[2-(1H)- pyridon-1-yl]ethyl)spiro[(2H)-1-benzopyran-2,4'- piperidine]-4-one hydrochloride

A mixture of 0.752 g of 6-methanesulfonamido-3,4-dihydro-spiro[(2H)-1-benzopyran-2,4'-piperidine]-4-one hydrochloride, 0.912 g sodium bicarbonate (s), 0.463 g of 1-(β-chloroethyl)-2-(1H)-pyridone hydrochloride (E. Wilson and M. Tishler, J. Am. Chem. Soc., 73, 3635 (1951)), a small crystal of potassium iodide and 25 mL of dry acetonitrile was stirred and refluxed overnight. The solvent was removed in vacuo, and the residue was partitioned between ethyl acetate and water. The organic phase was separated and was washed an additional five times with water. The organic phase was dried (MgSO₄), filtered, and evaporated, and the residual oil was converted to a hydrochloride salt. Recrystallization from methanol gave the title compound, mp 258-260°C(dec).

| Anal. Calc'd. for C₂₁H₂₅N₃O₅S•HCl. | | | |
|---|---|---|---|
| | C, 53.90; | H, 5.60; | N, 8.98. |
| Found | C, 53.62; | H, 5.61; | N, 8.91. |

The compounds of Table XVIII were prepared by heating a solution of 6-methanesulfonamido-3,4-dihydro-spiro[(2H)-1-benzopyran-2,4'-piperidine]-4-one (Example 70, Method 2, Step D) or 6-methoxy-3,4-dihydrospiro[(2H)-1-benzopyran-2,4'-piperidine]-4-one with an equimolar amount of 2-(tetrazolo[1,5-a]pyrid-6-yl)oxirane (U.S. Patent No. 4,358,455) in ethanol to reflux for 1.5 hours. The reaction mixture was evaporated and the residue crystallized first from a minimum of ethyl acetate and recrystallized from ethanol to give the desired tetrazolopyridine (essentially as described in U.S. Patent No. 4,358,455, Example 4, Step D).

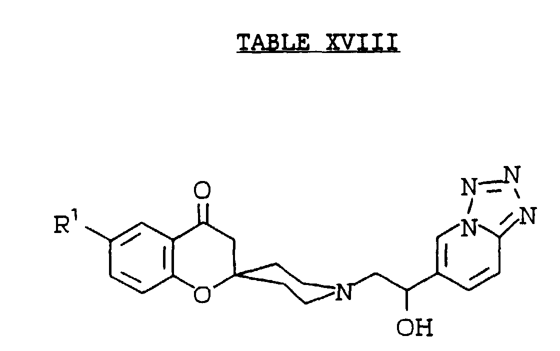

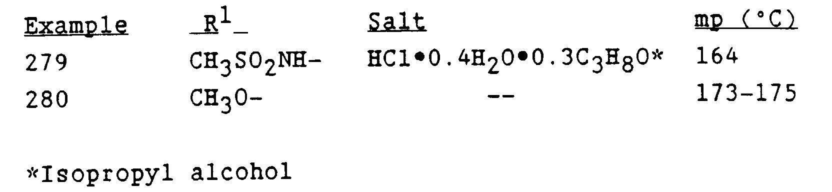

The compounds of Table XIX were prepared by heating a solution of 35.4 mmoles of the corresponding compound of Table XVIII and 24 g (106 mmoles) of stannous chloride dihydrate in 60 ml of 12 N HCl to reflux for 2 hours. The reaction was evaporated to dryness, the residue suspended in 150 ml of methanol and basified with concentrated NH₄OH. The precipitated salts were removed by filtration and the product in the filtrated chromatographed over 600 g of silica gel, eluting with a mixture of CH₂Cl₂: CH₃OH: concentrated NH₄OH (15: 5: 4) to give, upon evaporation of the eluate, the desired aminopyridine compound (essentially as described in U.S. Patent No. 4,358,455, Example 4, Step E).

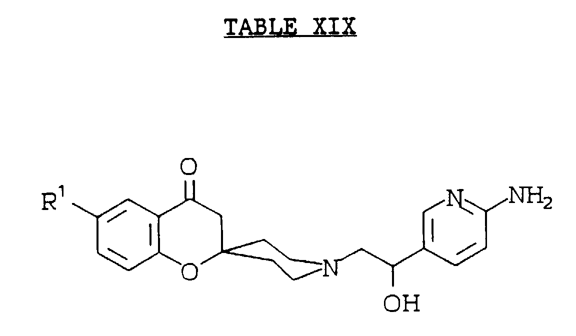

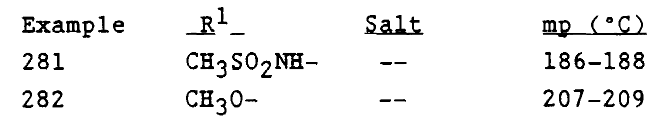

The compounds of Table XX were prepared from the corresponding compounds of Table XIX essentially by the method described in the J. org. Chem., 26, 122 (1961) for the conversion of 2-amino-5-methylpyridine to 2-hydroxy-5-methylpyridine.

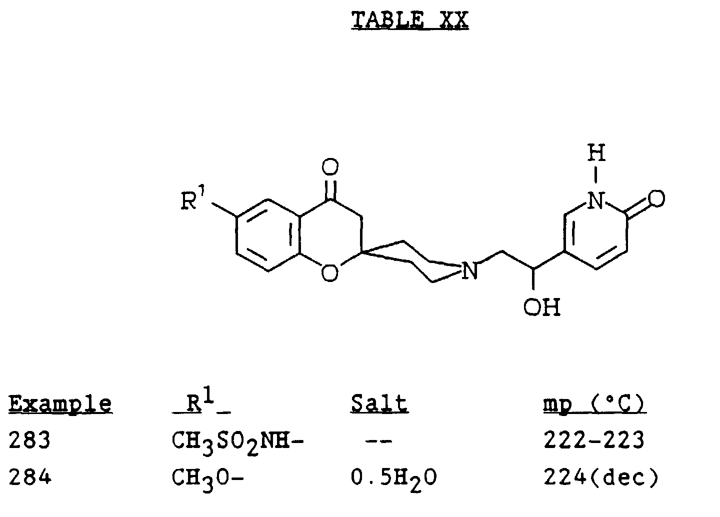

### EXAMPLE 288

### 3,4-Dihydro-6-methoxy-1'-(2-[2-benzimidazolon-1-yl]-ethyl)spiro[(2H)-1-benzopyran-2,4'-piperidine]-4-one hydrochloride

Step 1: 1-Isopropenylbenzimidazolone (J. Davol, J. Chem. Soc., 1960, 308) was alkylated with 1,2-dibromoethane using sodium hydride in DMF. The product was purified by chromatography and crystallization from cyclohexane to give 1-isopropenyl-3-(2-bromoethyl)benzimidazolone.

| Anal. Calc'd. for C₁₂H₁₃BrN₂O: | | | |
|---|---|---|---|
| | C, 51.26; | H, 4.66; | N, 9.97. |
| Found | C, 51.44; | H, 4.67; | N, 9.98. |

Step 2: Using the procedure described for the preparation of 6-methoxy-3,4-dihydro-1'-(2-(2-methanesulfonamidophenyl)ethyl]spiro-[(2H)-1-benzopyran-2,4'-piperidine]-4-one but substituting 1-isopropenyl-3-(2-bromoethyl)benzimidazolone for the 2-[4-methanesulfonamidophenyl]ethyl methanesulfonate used therein, there is produced an isopropenyl derivative of the title compound. The isopropenyl protecting group was removed by stirring an ethanolic solution of the compound with concentrated hydrochloric acid at room temperature. The title compound crystallized from the reaction mixture and was purified by recrystallization from ethanol, mp 267-269°C(dec).

| Anal. Calc'd. for C₂₃H₂₅N₃O₄•HCl: | | | |
|---|---|---|---|
| | C, 62.23; | H, 5.90; | N, 9.47. |
| Found | C, 62.34; | H, 5.82; | N, 9.34. |

### EXAMPLE 289

### 3,4-Dihydro-6-methoxy-1'-[2-(4-N-cyano-N'-benzyl-S-methylthiourea)ethyl]spiro[(2H)-1-benzopyran-2,4'-piperidine]-4-one hydrochloride

### Step A: Preparation of 4-(2-hydroxyethyl)benzylamine

A solution of 0.74 g (0.005 mol) of p-cyanophenethyl alcohol in 50 ml of ethanol containing 10 ml of concentrated hydrochloric acid was hydrogenated at 30 psi using 0.1 g 5% Pd/C for 72 hours. The catalyst was removed by filtration and the solvent was removed under reduced pressure.

### Step B: Preparation of N-cyano-N'-[4-(2-hydroxyethyl)benzyl]-S-methylisothiourea

The residue obtained from the hydrogenation reaction (0.88 g, 0.007 mol) was dissolved in 20 ml of acetonitrile and 0.4 g (0.0047 mol) of sodium bicarbonate and 0.687 (0.0047 mole) of dimethylcyanodithioimidocarbonate were added. The mixture was refluxed overnight. After cooling, the white solid was removed by filtration and dried to give 0.93 g of N-cyano-N'-[4-(2-hydroxyethyl)benzyl]-S-methylisothiourea, mp 164-165°C.

| Anal. Calc'd. for C₁₂H₁₅N₃OS: | | | |
|---|---|---|---|
| | C, 57.80; | H, 6.06; | N, 16.86. |
| Found | C, 57.58; | H, 5.88; | N, 16.80. |

### Step C: Preparation of 3,4-dihydro-6-methoxy-1'-[2-(4-N-cyano-N'-benzyl-S-methylthiourea)ethyl]-spiro[(2H)-1-benzopyran-2,4'-piperidine]-4-one hydrochloride

N-Cyano-N'-[4-(2-hydroxyethyl)benzyl]-S-methylisothiourea (0.47 g, 0.00189 mol) and triethylamine (0.48 g, 0.0047 mol) were dissolved in dichloromethane. Methanesulfonyl chloride (0.43 g, 0.00378 mol) was added and the mixture was stirred at reflux overnight. The cooled reaction mixture was diluted with methylene chloride and was washed with an aqueous solution of sodium bicarbonate. The organic phase was separated, dried (Na₂SO₄), filtered and evaporated. To the residue was added 0.4 g (0.00475 mol) of sodium bicarbonate and 0.426 g (0.0015 g) of 3,4-dihydro-6-methoxy-spiro[(2H)-1-benzopyran-2,4'-piperidine]-4-one hydrochloride and 20 ml of acetonitrile. The mixture was heated at reflux for 60 hours. The cooled mixture was diluted with water and was extracted with methylene chloride. This organic phase was dried over MgSO₄, filtered, and the solvent removed under reduced pressure. The residue was purifed by flash column chromatography on silica gel eluting with 5% methanol in chloroform. The purified product was converted to the hydrochloride salt. Recrystallization from methanol gave the title compound, mp 263-264°C.

| Anal. Calc'd. for C₂₆H₃₀N₄O₃S•HCl: | | | |
|---|---|---|---|
| | C, 60.63; | H, 6.07; | N, 10.88. |
| Found | C, 60.56; | H, 5.98; | N, 10.84. |

### EXAMPLE 290

### 3,4-Dihydro-6-methoxy-1'-[2-(4-N-cyano-N'-methyl-N"-guanidinobenzyl)ethyl]spiro[(2H)-1-benzopyran-2,4'-piperidine]-4-one hydrochloride

### Step A: Preparation of N-cyano-N'-methyl-N"-[4-(2-hydroxyethyl)benzyl]guanidine

A solution of 0.42g (0.00168 mol) of N-cyano-N'-[4-(2-hydroxyethyl)benzyl]-S-methylisothiourea in 50 ml of methanol and 10 g of methylamine was stirred at room temperature overnight. Evaporation of the solvent gave the title compound.

### Step B: Preparation of 3,4-dihydro-6-methoxy-1'-[2-(4-N-cyano-N'-methyl-N"-guanidinobenzyl)-ethyl]spiro[(2H)-1-benzopyran-2,4'-piperidine]-one hydrochloride

To a solution of the N-cyano-N'-methyl-N"-[4-(2-hydroxyethyl)benzyl]guanidine in 10 ml of pyridine was added 0.25 g (0.00218 mol) of methanesulfonyl chloride and the mixture was stirred at room temperature for 5 hours. The solvent was removed under reduced pressure and the residue was dissolved in methylene chloride and was washed with a saturated solution of sodium bicarbonate. The organic phase was separated, dried (MgSO₄) filtered, and evaporated. To the residue was added 0.426g (0.0015 mol) of 3,4-dihydro-6-methoxy-spiro[(2H)-1-benzopyran-2,4'-piperidine]-4-one hydrochloride, 0.315 g (0.00375 mol) of sodium bicarbonate, and 25 ml of acetonitrile. The mixture was heated under reflux for 24 hours. After evaporating the solvent, the residue was extracted into methylene chloride. This organic phase was washed with water, dried (Na₂SO₄), filtered and evaporated to dryness. The residue was purified by flash column chromatography on silica gel eluting with 5% methanol in methylene chloride. The purified product was converted to the hydrochloride salt. Recrystallization from isopropyl alcohol/methanol gave the title compound, mp about 204°C (dec).

| Anal. Calc'd. for C₂₆H₃₁N₅O•HCl•0.5 H₂O: | | | |
|---|---|---|---|
| | C, 61.59; | H, 6.56; | N, 13.81. |
| Found | C, 61.59; | H, 6.57; | N, 13.62. |

### EXAMPLE 293

### 3,4-Dihydro-6-methoxy-1'-[1,3-dimethylpyrimidin-2,4-(1H,3H)-dione-6-yl]spiro[(2H)-1-benzopyran-2,4'-piperidinel-4-one

A solution of 3,4-dihydro-6-methoxyspiro-[(2H)-1-benzopyran-2,4'-piperidin]-4-one hydrochloride (0.568 g, 2 mmol), 6-chloro-1,3-dimethylpyrimidine-2,4-(1H,3H)-dione (0.349 g, 2 mmol) and sodium bicarbonate (0.42 g, 5 mmol) in acetonitrile was heated at reflux for 42 hours. After cooling, the reaction mixture was concentrated in vacuo to dryness. The residue was partitioned between chloroform and water. The chloroform solution was washed twice with water and once with saturated brine solution and then dried over sodium sulfate and concentrated to dryness in vacuo. The solid residue was recrystallized from methanol/ethanol to give the title compound (0.47 g, 61% yield) mp 202-203°C.

| Anal. Calc'd for C₂₀H₂₃N₃O₅: | | | |
|---|---|---|---|
| | C, 62.33; | H, 6.02; | N, 10.90. |
| Found | C, 62.46; | H, 6.08; | N, 10.87. |

### EXAMPLE 294

### 3,4-Dihydro-6-methanesulfonamide-1'-[1,3-dimethylpyrimidine-2,4(1H, 3H)-dione-6-yl]spiro[(2H)-1-benzopyran-2,4'-piperidine]-4-one Utilizing the methodolgy of Example 293, the

title compound was prepared, mp 259.5-262°C.

| Anal. Calc'd for C₂₀H₂₄N₄O₆S: | | | |
|---|---|---|---|
| | C, 53.56; | H, 5.39; | N, 12.49. |
| Found | C, 53.16; | H, 5.47; | N, 12.24. |

### EXAMPLE 295 3,4-Dihydro-6-methoxy-1'-[2-(1,3-dimethylpyrimidin-2,4(1H,3H)dione-6-yl)ethylmercapto]spiro[(2H)-1-benzopyran-2,4'-piperidine]-4-one

A solution of 6-chloro-1,3-dimethyluracil (0.87 g, 5 mmol), 2-mercaptoethanol (0.39 g, 5 mmol) and sodium bicarbonate (0.63 g, 7.5 mmol) in acetonitrile (15 ml) was heated at reflux for 24 hours. After cooling, the reaction mixture was concentrated in vacuo to dryness. The residue was partitioned between chloroform and water. The combined chloroform solution was dried over magnesium sulfate, filtered and concentrated to dryness. The residue was triturated with ether and filtered off.

A solution of the residue (0.83 g, 3.8 mmol), triethylamine (0.58 g, 5.7 mmol) and methanesulfonyl chloride (0.48 g, 4.2 mmol) in methylene chloride (30 ml) was stirred in an ice bath for 3 hours. The resulting reaction was washed with water and brine, dried over sodium sulfate and concentrated in vacuo to dryness. The residue was triturated with ether, and the solid mesylate was filtered off.

A solution of the mesylate (0.44 g, 1.5 mmol), 3,4-dihydro-6-methoxyspiro[(2H)-1-benzopyran-2,4'-piperidin]-4-one (0.425 g, 1.5 mmol) and sodium bicarbonate (0.315 g, 3.75 mmol) in acetonitrile (15 ml) was heated at reflux for 23 hours. After cooling, the reaction mixture was concentrated in vacuo to dryness. The residue was partitioned between chloroform and water. The chloroform solution was washed with water, brine and dried over sodium sulfate and concentrated in vacuo to dryness. The residue was triturated with hot ethanol/methanol and filtered off to give the title compound, mp 191-193.5°C (0.31 g, 46.4%).

| Anal. Calc'd for C₂₂H₂₇N₃O₅S: | | | |
|---|---|---|---|
| | C, 59.31; | H, 6.11; | N, 9.43. |
| Found | C, 59.07; | H, 6.08; | N, 9.38. |

### EXAMPLE 296

### 3,4-Dihydro-6-methanesulfonamido-1'-[2-(1,3-dimethylpyrimidin-2,4(1H,3H)dione-6-yl)ethylmercapto]spiro-[(2H)-1-benzopyran-2,4'-piperidine]-4-one

Utilizing the methodology of Example 295, the title compound was prepared, mp 198-201°C.

| Anal. Calc'd for C₂₂H₂₈N₄O₆S₂: | | | |
|---|---|---|---|
| | C, 51.95; | H, 5.55; | N, 11.02. |
| Found | C, 51.62; | H, 5.42; | N, 10.99. |

### EXAMPLE 297

### 3,4-Dihydro-6-methanesulfonamido-1'-[2-(1,3-dimethylpyrimidine-2,4(1H,3H)-dione-6-yl)ethylamino]spiro[(2H)-1-benzopyran-2,4'-piperidine]-4-one

1,3-Dimethyl-6-[2-(methylsulfonyloxy)ethylamino]-2,4(1H,3H)-pyrimidinedione (prepared as described in EPO Publication 0,369,627) (0.4 g, 2 mmol) was suspended in pyridine (2 ml) and cooled to 0°C, methanesulfonyl chloride (0.286 g, 2.5 mmol) was added at <5°C. the reaction mixture was stirred in an ice bath for 3 hours and was concentrated in vacuo to dryness. The residue was partitioned between chloroform and saturated sodium bicarbonate. The chloroform layer was dried over sodium sulfate, filtered, and concentrated to give the mesylate.

A solution of the mesylate (0.26 g, 0.94 mmol), 3,4-dihydro-6-sulfamidospirop[(2H)-1-benzopyran-2,4'-piperidin]-4-one (0.347 g, 1 mmol) and sodium bicarbonate (0.25 g, 2.5 mmol) in acetonitrile (30 ml) was heated at reflux for 2 hours. After cooling, the reaction mixture was concentrated in vacuo to dryness. The residue was stirred in chloroform and methanol and filtered off solid. This solid was heated with hot ethanol. After cooling, the title compound was filtered off.

| Anal. Calc'd for C₂₂H₂₉N₅O₆S: | | | |
|---|---|---|---|
| | C, 53.76; | H, 5.95; | N, 14.25. |
| Found | C, 53.50; | H, 5.94; | N, 14.13. |

### EXAMPLE 298

### 3,4-Dihydro-6-methoxy-1'-[2-(1,3-dimethylpyrimidine-2,4(1H,3H)-dione-6-yl)ethylamino]spiro[(2H)-1-benzopyran-2,4'-piperidine]-4-one

A suspension of 3,4-dihydro-6-methoxyspiro-[(2H)-1-benzopyran-2,4'-piperidin]-4-one (0.284 g, 1 mmol) and sodium bicarbonate (0.42 g, 5 mmol) in acetonitrile was heated at reflux for 1 hour, 2-chloro ethylamine hydrochloride (0.116 g, 1 mmol) was added the solution was heated at reflux for 17 hours, 6-chloro-1,3-dimethyluracil (0.175 g, 1 mmol) and sodium bicarbonate (0.16 g, 2 mmol) were added. Refluxing was continued for 28 hours. After cooling, the reaction mixture was concentrated in vacuo to dryness. The residue was partitioned between chloroform and water. The chloroform solution was washed with water and dried over sodium sulfate. The resulting residue was chromatographed through a pressure column (silica gel) eluting with 5% methanol/ chloroform to give the title compound (45 mg, 10.5% yield), mp 235-238°C dec.

| Anal. Calc'd for C₂₂H₂₇N₄O₅: | | | |
|---|---|---|---|
| | C, 61.67; | H, 6.59; | N, 13.08. |
| Found | C, 61.40; | H, 6.69; | N, 12.68. |

### EXAMPLE 299

### 1'-[2-(3,4-Diaminophenyl)ethyl]-3,4-dihydro-6-methanesulfonamidospiro[(2H)-(1-benzopyran-2,4'-piperidine]-4-one and dihydrochloride

Step 1: A suspension of 5.24 g (15.0 mmol) of 1-(8-[2-(4-acetamido-3-nitrophenyl)ethyl]-1,4-dioxa-8-azaspiro[4.5]decane in 30 mL 2N hydrochloric acid was heated at reflux for 1.5 hours. The cooled mixture was basified (pH 9-10) with saturated sodium carbonate solution and 10 N sodium hydroxide, diluted with 100 ml water, and extracted with methylene chloride (3 x 150 ml). The extract was washed with 50 ml water and brine, dried over sodium sulfate and filtered. The filtrate was concentrated in vacuo to give 3.95 g (100%) of 1-[2-(4-amino-3-nitrophenyl)ethyl]-4-piperidone as an oil.
¹H NMR CDCl₃: δ 7.98 (d,1H), 7.25 (d of d, 1H), 6.77 (d, 1H), 6.01 (broad s, 2H), 2.82 (t, 4H), 2.76 (m, 2H), 2.70 (m, 2H), 2.48 (t, 4H).
Step 2: A solution of 3.44 g (15.0 mmol) 5-methanesulfonamido-2-hydroxy-acetophenone 0.53 g (7.5 mmol) pyrrolidine, and 3.95 g (15.0 mmol) 1-[2-(4-amino-3-nitrophenyl)ethyl])piperidin-4-one in 30 mL methanol was heated at reflux for 30 hours. The solvent was removed in vacuo and the residue was partitioned between 50 mL ethyl acetate and 25 mL 1 N hydrochloric acid. The layers were separated and the ethyl acetate layer was extracted with 1 N hydrochloric acid (2 x 25 mL). The combined aqueous extract was basified with 10N sodium hydroxide to pH 10, then extracted with methylene chloride (3 x 175 mL). The extract was washed with 50 mL water and brine, dried over sodium sulfate and filtered. The filtrate was concentrated in vacuo to give a residue which was purified by flash chromatography on silica gel eluting with 2.5:97.5 methanol:chloroform saturated with ammonia to give 3.72g (52%) of 1'-[2-(4-amino-3-nitrophenyl)ethyl]-3,4-dihydro-6-methanesulfonamidospiro[(2H)-1-benzopyran-2,4'-piperidine]-4-one as an orange solid, m.p. 69-78°C. ¹H NMR (CDCl₃): δ 7.96 (d, 1H), 7.64 (d, 1H), 7.52 (d of d, 1H), 7.24 (d of d, 1H), 7.03 (d, 1H), 6.76 (d, 1H), 5.99 (broad s, 2H), 3.01 (s, 1H), 2.73 (s, 2H), 2.72 (m, 4H), 2.62 (m, 2H), 2.50 (t, 2H), 2.08 (d, 2H), 1.78 (m, 2H).
Step 3: To a solution of 3.56 g (7.5 mmol) 1'-[2-(4-amino-3-nitrophenyl)ethyl]-3,4-dihydro-6-methanesulfonamidospiro[(2H)-(1-benzopyran-2,4'-piperidine]-4-one in 30 mL acetic acid was added dropwise over 50 minutes a solution of 22.7 mL (26.3 mmol) titanium trichloride (15%) in 20-30% hydrochloric acid. The solution was stirred 20 minutes, basified (pH 9-10) with saturated sodium carbonate solution and 10 N sodium hydroxide, diluted with 200 mL water, and extracted with methylene chloride (3 x 300 mL). The combined extract was washed with 50 mL water and brine, dried over sodium sulfate and filtered. The filtrate was concentrated in vacuo to give the crude product which was re-crystallized from methylene chloride:ether (1:2) to give 2.16 g (65%) 1'-[2-(3,4-diaminophenyl)ethyl]-3,4-dihydro-6-methanesulfonamidospiro[(2H)-1-benzopyran-2,4'-piperidine]-4-one as a yellow solid mp. 109-112°C.
Step 4: To a solution of 0.222 g (0.50 mmol) 1'-[2-(3,4-diaminophenyl)ethyl]-3,4-dihydro-6-methanesulfonamidospiro[(2H)-1-benzopyran-2,4'-piperidine]-4-one in 5 ml ethanol was added 0.27 ml (1.6 mmol) 5.9N hydrogen chloride in ethanol. A precipitate formed which was filtered off and dried in vacuo to yield 0.226 g (87%) dihydrochloride salt of 1'-[2-(3,4-diaminophenyl)ethyl]-3,4-dihydro-6-methanesulfonamidospiro[(2H)-1-benzopyran-2,4'-piperidine]-4-one containing 0.65 water as a solvate, as a white solid, mp. 255-256°C.

| Anal Calcd for C₂₂H₂₈N₄O₄•HCl•0.65 H₂O: | | | |
|---|---|---|---|
| | C, 49.93; | H, 5.96; | N, 10.59. |
| Found | C, 49.91; | H, 5.86; | N, 10.48. |

### EXAMPLE 300

### 1'-[2-(3,4-Bis(methanesulfonamido)phenyl)ethyl]-3,4-dihydro-6-methanesulfonamidospiro[(2H)-1-benzopyran-2,4'-piperidine)]-4-one hydrochloride

To a solution of 0.222 g (0.50 mmol) 1'-[2-(3,4-diaminophenyl)ethyl]-3,4-dihydro-6-methanesulfonamidospiro[(2H)-1-benzopyran-2,4'-piperidine]-4-one and 0.16 g (2.0 mmol) pyridine in 8 mL methylene chloride was added 0.120 g (1.05 mmol) methanesulfonyl chloride. The resulting mixture was stirred one hour, then diluted with 10 mL methylene chloride. The mixture was washed with 10 mL saturated sodium bicarbonate solution, 3 mL water, and brine, dried over sodium sulfate, and filtered. The filtrate was concentrated in vacuo to give a gum which was purified by flash chromatography on silica gel eluting with 5: 95 methanol: chloroform saturated with ammonic to give 0.125 g of the free base.

To a solution of 0.12 g (0.20 mmol) of the free base in 10 mL ethanol was added 0.037 mL (0.22 mmol) 5.9 N hydrogen chloride in ethanol to give a white precipitate. The precipitate was filtered off and dried in vacuo to give 0.074g (24%) hydrochloride as a 0.20 ethanol and 0.75 water solvate as a off-white solid m.p. 178-181°C.

| Anal Calcd for C₂₄H₃₂N₄O₈S₃•HCl•0.20 C₂H₅OH•0.75 H₂O: | | | |
|---|---|---|---|
| | C, 44.41; | H, 5.45; | N, 8.49. |
| Found | C, 44.41; | H, 5.35; | N, 8.49. |

### EXAMPLE 301

### 1'-[2-(1H-Benzimidazol-5-yl)ethyl]-3,4-dihydro-6-methanesulfonamidospiro[(2H)-1-benzopyran-2,4'-piperidine)]-4-one dihydrochloride

A solution of 0.36 g (0.80 mmol) 1'-[2-(3,4-diaminophenyl)ethyl]-3,4-dihydro-6-methane-sulfonamidospiro[(2H)-1-benzopyran-2,4'-piperidine]-4-one and 1 mL (6 mmol) triethyl orthoformate in 1 mL ethanol was heated at reflux for 2 hours. The solvent was evaporated in vacuo to give a residue which was purified by flash chromatography on silica gel eluting with 5: 95 methanol: chloroform saturated with ammonia to give 0.38 g of the free base as a foam.

To a solution of the free base in 4 mL ethanol was added 0.27 mL (1.6 mmol) 5.9 N hydrogen chloride in ethanol to give a gummy precipitate. The mixture was diluted with 10 mL ether, stirred two hours, and the precipitate filtered off and dried in vacuo to give 0.317 g (75%) of the title compound as a 0.35 ethanol and 1.35 water solvate, as a pale yellow solid m.p. 221-225°C.

| Anal Calcd. for C₂₃H₂₆N₄O₄S•2HCl•0.35 C₂H₅OH•1.35 H₂O: | | | |
|---|---|---|---|
| | C, 50.12; | H, 5.82; | N, 9.87. |
| Found | C, 50.08; | H, 6.00; | N, 9.96. |

### EXAMPLE 302

### 1'-[2-(2,2-Dioxo-1H,3H-2,1,3-benzothiadiazolin-5-yl)ethyl]-3,4-dihydro-6-methanesulfonamidospiro[(2H)-1-benzopyran-2,4'-piperidine]-4-one hydrochloride

To 0.7 mL refluxing diglyme was added dropwise over 10 minutes a solution of 0.31 g (0.70 mmol) 1'-[2-(3,4-diaminophenyl)ethyl]-3,4-dihydro-6-methanesulfonamidospiro[(2H)-1-benzopyran-2,4'-piperidine]-4-one and 0.067 g (0.70 mmol) sulfamide in 1.4 mL diglyme. The mixture was heated at reflux for four hours and the diglyme was distilled off under reduced pressure leaving a solid residue. The residue was placed under 5 mL ethanol, the mixture was heated at reflux for 10 minutes, cooled to 40°C, and the solid was filtered off. The solid was extracted with hot methanol (4 x 25 mL) and filtered. The filtrate was concentrated in vacuo to give 0.042 g impure free base.

To a suspension of the free base in 5 mL ethanol was added 0.015 ml (0.088 mmol) 5.9 N hydrogen chloride in ethanol. The solid slowly dissolved. The solution was concentrated in vacuo to 1 ml and diluted with 2 ml ether to give a precipitate. The precipitate was filtered off and recrystallized from ethanol to give 0.012 g (3%) of the title compound as a 0.30 water solvate, as a tan solid, mp 270-275°C.

| Anal. Calcd. for C₂₂H₂₆N₄O₆S₂•HCl•0.30 H₂O: | | | |
|---|---|---|---|
| | C, 48.17; | H, 5.07; | N, 10.20 |
| Found | C, 48.14; | H, 5.36; | N, 10.02. |

### EXAMPLE 303

### 1'-[2-(2,1-3-Benzothiadiazol-5-yl)ethyl]-3,4-dihydro-6-methanesulfonamidospiro[(2H)-1-benzopyran-2,4'-piperidine]-4-one hydrochloride

To a mixture of 0.267 g (0.60 mmol) 1'-[2(3,4-diaminophenyl)ethyl]-3,4-dihydro-6-methanesulfonamidospiro[(2H)-1-benzopyran-2,4'-piperadine]-4-one and 0.30 g (3.0 mmol) triethylamine in 5 mL methylene chloride was added 0.083 g (0.70 mmol) thionyl chloride. The resulting solution was stirred 90 minutes and 0.083 g (0.70 mmol) thionyl chloride was added. The solution was stirred 90 minutes, diluted with 50 mL methylene chloride, and washed with 15 mL saturated sodium bicarbonate. The aqueous layer was extracted with 25 mL methylene chloride. The combined organic layer was washed with 15 mL water and brine, dried over sodium sulfate, and filtered. The filtrate was concentrated in vacuo to give a residue which was purified by flash chromatography on silica gel eluting with 5: 95 methanol: chloroform saturated with ammonia. The solid obtained was recrystallized from ethyl acetate to give 0.180 g free base.

To a suspension of the free base in 5 mL ethanol was added 0.064 mL (0.38 mmol) 5.9N hydrogen chloride in ethanol to give a yellow precipitate. The precipitate was filtered off and dried in vacuo to give 0.171 g (56%) of the title compound as a yellow solid mp 259-260°C.

| Anal. Calcd. for C₂₂H₂₄N₄O₄S₂•HCl: | | | |
|---|---|---|---|
| | C, 51.91; | H, 4.95; | N, 11.01. |
| Found | C, 51.61; | H, 4.86; | N, 10.95. |

### EXAMPLE 304

### 1'-[2-(2,3-Dihydro-2-oxo-1H-benzimidazol-5-yl)ethyl]-3,4-dihydro-6-methanesulfonamidospiro[(2H)-1-benzoyran-2,4'-piperidine]-4-one hydrochloride

To a mixture of 0.266 g (0.60 mmol) 1'-[2-(3,4-diaminophenyl)ethyl]-3,4-dihydro-6-methanesulfonamidospiro[(2H)-1-benzopyran-2,4'-piperidine]-4-one in 4.5 mL tetrahydrofuran was added a solution of 0.126 g (0.78 mmol) carbonyldiimidazole in 2.5 mL tetrahydrofuran. The resulting solution was stirred for two hours and a precipitate formed which was filtered off to give 0.181 g free base.

To a suspension of 0.176 g (0.38 mmol) free base in 4 mL ethanol was added 0.046 mL (0.38 mmol) 5.9 N hydrogen chloride in ethanol. The solid dissolved and a precipitate formed which was filtered off and dried in vacuo to give 0.173 g (58%) of the title compound as a 0.10 ethanol and 0.35 water solvate, as a white solid mp 270-272°C.

| Anal. Calcd. for C₂₃H₂₆N₄O₅S•HCl•0.10 C₂H₅OH•0.35 H₂O: | | | |
|---|---|---|---|
| | C, 53.80; | H, 5.51; | N, 10.82; |
| Found | C, 53.75; | H, 5.64; | N, 10.84. |

### EXAMPLE 305

### 1'-[2-(1-Methyl-1H-benzimidazol-5-yl)ethyl]-3,4-dihydro-6-methanesulfonamidospiro-[(2H)-1-benzopyran-2,4'-piperidine]-4-one dihydrochloride and 1'-[2-(1-Methyl-1H-benzamidazol-6-yl)ethyl]-3,4-dihydro-6-methanesulfonamidospiro-[(2H)-1-benzopyran-2,4'-piperidine]-4-one

To a mixture of 0.204 g (0.45 mmol) 1'-[2-(3,4-diaminophenyl)ethyl]-3,4-dihydro-6-methanesulfonamidospiro[(2H)-1-benzopyran-2,4'-piperidine]-4-one free base and 0.093 g (0.80 mmol) diisopropylethylamine in 1.5 mL methanol was added 0.069g (0.52 mmol) dimethylsulfate. The mixture was heated at reflux for 2 hours, 0.078 g (0.60 mmol) dimethyl sulfate was added, and the solution was heated at reflux overnight. The solution was concentrated in vacuo and the residue was partitioned between 5 mL saturated sodium bicarbonate solution and 20 mL chloroform. The layers were separated and the aqueous layer was extracted with chloroform (2 x 10 mL). The combined extract was washed with 5 mL water and brine, dried over sodium sulfate, and filtered. The filtrate was concentrated in vacuo to give a residue which was purified by flash chromatography on silica gel eluted with 5: 95 methanol: chloroform saturated with ammonia to give a gum. This was further purified by preparative thin layer chromatography using the above eluent to give 0.018 g free base.

To a solution of the free base in 0.2 ml ethanol was added 0.0136 ml (0.080 mmol) 5.9 N hydrogen chloride in ethanol to give a gummy precipitate. The mixture was diluted with 2 ml ether and stirred 2 hours to give a fine yellow solid. The solid was filtered off and dried in vacuo to give 0.014 g (6%) of the title compound as a 0.20 ethanol and 1.00 water solvate as a pale orange solid 220-232°C.

| Anal. Calcd. for C₂₄H₂₈N₄O₄S•2HCl•0.20 C₂H₅OH•1.00 H₂O: | | | |
|---|---|---|---|
| | C, 51.53; | H, 5.88; | N, 9.85. |
| Found | C, 51.61; | H, 5.86; | N, 9.84. |

### EXAMPLE 306

### 1'[2-(2-methyl-1H-benzimidazol-5-yl)ethyl]-3,4-dihydro-6-methanesulfonamidospiro[(2H)-1-benzopyran-2,4'-piperidine]-4-one dihydrochloride

A solution of 0.355 g (0.80 mmol) 1'-[2(3,4-diaminophenyl)ethyl]-3,4-dihydro-6-methanesulfonamidospiro[(2H)-1-benzopyran-2,4'-piperidine]-4-one and 1 mL (6 mmol) triethyl orthoacetate in 1 mL ethanol was heated at reflux for two hours. The solution was concentrated in vacuo to give a residue. The residue was dissolved in 5 mL chloroform, there was added 0.334 g (1.76 mmol) p-toluenesulfonic acid hydrate, and the resulting solution was stirred two hours. The solution was concentrated in vacuo to give a residue which was purified by flash chromatography on silica gel eluting with 5: 95 methanol: chloroform saturated with ammonia to give 0.226 g of the free base as a foam.

To a solution of 0.202 g (0.43 mmol) free base in 2 ml ethanol was added 0.152 ml (0.90 mmol) 5.9 N hydrogen chloride in ethanol. The resulting solution was stirred 30 minutes, diluted with 5 mL ether, and stirred 3 hours to give a white precipitate. The precipitate was filtered off and dried in vacuo to give 0.181 g (47%) of the title compound as a 0.20 ethanol solvate as a white solid, mp 224-227°C.

| Anal. Calc for C₂₄H₂₈N₄O₄S•2HCl•0.20 C₂H₅OH: | | | |
|---|---|---|---|
| | C, 53.21; | H, 5.71; | N, 10.12. |
| Found | C, 52.85; | H, 5.64; | N, 10.16. |

### EXAMPLE 307

### 3,4-Dihydro-6-methanesulfonomide-1'-[2-(6-quinoxolinyl)ethyl]spiro[(2H)-1-benzopyran-2,4-piperidine]-4-one hydrochloride

To a mixture of 0.356 g (0.80 mmol) 1'-[2-(3,4-diaminophenyl)ethyl]-3,4-dihydro-6-methanesulfonamidospiro[(2H)-1-benzopyran-2,4'-piperidine]-4-one in 1.3 ml water heated to 70°C was added a hot solution of 0.250 g (0.88 mmol) of glyoxal sodium bisulfite addition compound monohydrate (Aldrich Chemical Co.) in 1 mL water and 0.083 g (0.80 mmol) sodium bisulfite. The mixture was stirred at 70°C for two hours, cooled to room temperature, diluted with 5 mL water, and extracted with chloroform (3x25 ml). The extract was washed with 5 mL water and brine, dried over sodium sulfate, and filtered. The filtrate was concentrated in vacuo to give a residue which was purified by flash chromatography eluting with 5: 95 methanol: chloroform saturated with ammonia to give 0.174 g of the free base.

To a solution of the free base in 2 ml ethanol was added 0.127 ml (0.75 mmol) 5.9 N hydrogen chloride in ethanol to give a precipitate. The precipitate was filtered off and dried in vacuo to give 0.133 g (33%) of the title compound as a 0.40 water solvate as a white solid, mp 262-264°C.

| Anal. Calcd. for C₂₄H₂₆N₄O₄S•HCl.0.40 H₂O: | | | |
|---|---|---|---|
| | C, 56.49; | H, 5.49; | N, 10.98. |
| Found | C, 56.51; | H, 5.53; | N, 11.19. |

### EXAMPLE 308

### 3,4-Dihydro-1'-[2-(2,3-dihydroxy-6-quinoxolinyl)ethyl]-6-methanesulfanomidospiro[(2H)-1-benzopyran-2,4'-piperidine]-4-one hydrochloride

A solution of 0.31 g (0.70 mmol) 1'-[2-(3,4-diaminophenyl)ethyl]-3,4-dihydro-6-methanesulfonamidospiro[(2H)-1-benzopyran-2,4'-piperidine]-4-one and 0.083 g (0.74 mmol) oxalic acid dihydrate in 1.5 ml of 4N hydrochloric acid was heated at reflux for one hour to give a precipitate. The mixture was diluted with 1 mL water, heated at reflux for 30 minutes, and cooled to room temperature. The precipitate was filtered off, washed with water, and dried in vacuo to give 0.308 g (82%) of the title compound as a 0.75 water solvate as a white solid, mp 283-285°C.

| Anal. Calcd. for C₂₄H₂₆N₄O₆S•HCl•0.75 H₂O: | | | |
|---|---|---|---|
| | C, 52.55; | H, 5.24; | N, 10.21. |
| Found | C, 52.60; | H, 5.28; | N, 10.39. |

### EXAMPLE 309

### 1'-[2-(2-(5-cyanopyridyl))ethyl]-3,4-dihydro-6-methanesulfonamidospiro[(2H)-1-benzopyran-2,4'-piperidine]-4-one monohydrochloride

Step 1: A solution of 4.13 g (35.0 mmol) 5-cyano-2-picoline (Fairfield Chemicals) and 0.28 g (7.0 mmol) sodium hydroxide in 13.2 mL (135 mmol) of 37% aqueous formaldehyde solution was heated at 160°C in a sealed pressure tube overnight. The cooled mixture was basified with 6 mL saturated sodium carbonate solution and extracted with methylene chloride (3 x 60 mL). The extract was washed with 10 mL bine, dried over Na₂SO₄, and filtered. The filtrate was concentrated in vacuo to give a residue which was partially purified by flash chromatography eluting with 2: 98 methanol: chloroform to give 0.21 g of a solid. The solid was recrystallized from ether: hexane (1: 2) to give 0.150 g (3%) 5-cyano-2-(2-hydroxyethyl)-pyridine, mp 92-93°C. ¹HNMR CDCl₃: δ 8.81 (d, 1H), 7.89 (d of d, 1H), 7.33 (d, 1H), 4.06 (t, 2H), 3.22 (broad s, 1H), 3.12 (t, 2H).
Step 2: A mixture of 0.163 g (1.1 mmol) 5-cyano-2(2-hydroxyethyl)pyridine and 0.029 g (0.15 mmol) p-toluenesulfonic acid hydrate in 10 mL toluene was heated to reflux for five hours in a flask equipped with a Dean-Stark trap. The cooled mixture was neutralized with 5 mL saturated sodium bicarbonate solution and extracted with ether (3 x 15 mL). The extract was washed with 3 mL brine, dried over sodium sulfate, and filtered. The filtrate was concentrated to give an oil which was purified by flash chromatography on silica gel elating with 1:3 ethyl acetate: hexane to give 0.052 g (36%) 5-cyano-2-vinylpyridine, mp 29-30°C.
¹HNMR CDCl₃: δ 8.83 (d, 1H), 7.92 (d of d, 1H), 7.42 (d of d, 1H), 6.84 (d of d, 1H), 6.40 (d of d, 1H), 5.70 (d of d, 1H).
Step 3: In a manner similar to that described in Example 70, Method 2, Step E, a solution of 3,4-dihydro-6-methanesulfonamido-spiro[(2H)-1-benzopyran-2,4'-piperidine]-4-one hydrochloride (0.121 g, 0.35 mmol), 5-cyano-2-vinylpyridine (0.046 g, 0.35 mmol), sodium acetate trihydrate (0.095 g, 0.70 mmol) in 1:1 methanol:water water was heated at reflux for 13 hours. Purification and crystallization as the hydrochloride salt from ethanol gave 0.067 g (40%) of the title compound as a white solid, mp 203-204°C.

| Analy. Calcd. for C₂₂H₂₄N₄O₄S•HCl•0.05 C₂H₅OH•0.30 H₂O: | | | |
|---|---|---|---|
| | C, 54.76; | H, 5.39; | N, 11.56. |
| Found | C, 54.72; | H, 5.10; | N, 11.51. |

### EXAMPLE 310

### 1'-[2-(6-benzothiazolyl)ethyl]-3,4-dihydro-6-methanesulfonamidospiro[(2H)-1-benzopyran-2,4'-piperidine]-4-one hydrochloride

Step 1: To a solution of t-butyl nitrite (6.60 g, 64.0 mmol) in 60 mL dimethylformamide heated to 65°C was added a solution of 2-amino-6-benzothiazoleacetic acid (Indian J. Chem., 16B, 605 (1978)) (8.95 g, 43.0 mmol) in 140 mL dimethylformamide. The mixture was stirred at 65°C for 15 minutes, cooled to room temperature, diluted with 600 mL 6N hydrochloric acid, and extracted with 1:99 methanol:chloroform (3 x 600 mL). The extract was washed wtih 30 mL water and brine, dried over sodium sulfate and filtered. The filtrate was concentrated to give crude product which was purified by flash chromatography eluting with 1:2:97 acetic acid:methanol:chloroform to give 1.1 g material. This was stirred under 20 mL ether to give a solid which was filtered off to give 0.42 g (5%) 6-benzothiazoleacetic acid, mp 147-149°C. ¹HNMR DMSO-d₆: δ 9.35 (s, 1H), 8.04 (m, 2H), 7.44 (d, 1H), 3.75 (s, 2H).
Step 2: To a solution of 6-benzothiazoleacetic acid (0.39 g, 2.0 mmol) in 10 mL dry tetrahydrofuran cooled in an ice-bath was added 4.0 ml (4.0 mmol) 1.0M borane/tetrahydrofuran was added, and the solution was stirred two hours with cooling. The reaction was quenched with 4 mL of 1:1 tetrahydrofuran: water, 2 mL water, and 5 mL saturated sodium potassium tartrate. The mixture was partially concentrated in vacuo and the aqueous residue was extracted with methylene chloride (3 x 15 mL). The extract was washed with 3 mL saturated sodium bicarbonate solution and brine, dried over sodium sulfate and filtered. The filtrate was concentrated to give a residue which was partially purified by flash chromatography eluting with 1: 99 methanol: chloroform to give 0.18 g of a gum. A solution of the gum in 5 mL tetrahydrofuran and 3 mL 6N hydrochloric acid was stirred at 60°C for two hours, cooled to room temperature, neutralized with saturated sodium bicarbonate solution, and extracted with methylene chloride (3 x 20 mL). The extract was concentrated in vacuo to give a residue which was re-chromatographed to give 0.100 g (28%) 6-benzothiazolylethanol as an oil.
¹HNMR CDCl₃: δ 8.85 (s, 1H), 7.96 (d, 1H), 7.74 (s, 1H), 7.30 (dd, 1H), 3.85 (t, 2H), 2.94 (t, 2H), 2.1 (broad s, 1H).
Step 3: To a solution of 6-benzothiazolylethanol (0.099 g, 0.55 mmol) and triethylamine (0.121 g, 1.20 mmol) in 2 mL methylene chloride cooled in an ice bath was added dropwise a solution of methanesulfonyl chloride (0.086 g, 0.75 mmol) in 0.5 mL methylene chloride. The mixture was stirred one hour, diluted with 10 ml methylene chloride and washed with 3 mL saturated sodium bicarbonate solution. The aqueous layer was extracted with 5 mL methylene chloride and the combined extract was washed with 2 mL water and brine, dried over sodium sulfate and filtered. The filtrate was concentrated in vacuo to give crude product which was purified by flash chromatography on silica gel eluting with 1:1 ethyl acetate:hexane to give 0.118 g (83%) 6-benzothiazolylethyl methanesulfonate as an oil.
¹HNMR CDCl₃: δ 8.99 (s, 1H), 8.10 (d, 1H), 7.85 (d, 1H), 7.40 (dd, 1H), 4.49 (t, 2H), 3.22 (t, 2H), 2.89 (s, 3H).
Step 4: In a manner similar to that described in Example 92, Step B a mixture of 3,4-dihydro-6-methanesulfonamido-spiro[(2H)-1-benzopyran-2,6'-piperidine]-4-one hydrochloride (0.146 g, 0.42 mmol), sodium bicarbonate (0.143 g, 1.70 mmol), 6-benzothiazolylethyl methanesulfonate (0.108 g, 0.42 mmol),
potassium iodide (0.070 g, 0.42 mmol) and acetonitrile (5 mL) was heated at reflux for 6.5 hours. Purification and crystallization as the hydrochloride salt from ethanol gave 0.102 g (48%) of the title compound as a white solid, mp 214-215°C.

| Analy. Calcd. for C₂₃H₂₅N₃O₄S₂•HCl•0.25 H₂O: | | | |
|---|---|---|---|
| | C, 53.89; | H, 5.21; | N, 8.20. |
| Found | C, 53.94; | H, 5.06; | N, 8.21. |

### EXAMPLE 311 (INTERMEDIATE COMPOUNDS)

### Preparation of 5'-(substituted methyl)-2'-hydroxyacetophenones: 5'-[(Dimethylamino)methyl]-2'-hydroxyacetophenone

### Ref: A. Warshawsky, R. Kalir, A. Patchornik, Ger. Offen. 2,733,251 (1978).

### 2-Hydroxy-5'-(methylthiomethyl)acetophenone

To a solution of 5'-(chloromethyl)-2'-hydroxyacetophenone (prepared by method of Acta Pharm. Suec., 15, 13 (1978))(5.54 g, 30.0 mmol) in 90 mL dimethylformamide was added sodium thiomethoxide (2.31 g, 33.0 mmol). The mixture was stirred one hour and solid iodine was added in portions until a purple color was maintained to oxidize residual methylmercaptan. The mixture was diluted with 100 mL saturated ammonium chloride solution and 2 mL 3N hydrochloric acid and extracted with ether (3 x 300 mL). The extract was washed with water (2 x 60 mL) and brine, dried over Na₂SO₄ and filtered. The filtrate was concentrated in vacuo to give after drying 5.89 (100%) product as an oil.
¹HNMR CDCl₃: δ 12.20 (s, 1H), 7.66 (d, 1H), 7.43 (dd, 1H), 6.95 (d, 1H), 3.65 (s, 2H), 2.65 (s, 3H), 2.02 (s, 3H).

### 2'-Hydroxy-5'-(methylsulfinylmethyl)acetophenone

To a solution of 2'-hydroxy-5'-(methylthiomethyl)acetophenone (2.20 g, 11.0 mmol) in 70 mL MeOH cooled in an ice bath was added a solution of sodium metaperiodate (2.49 g, 12.1 mmol) in 115 mL water. The resulting cloudy mixture was stirred two hours with cooling then partially concentrated in vacuo to remove methanol. The remaining aqueous mixture was extracted with chloroform (3 x 125 mL). The extract was washed with 50 mL water and brine, dried over sodium sulfate, and filtered. The filtrate was concentrated in vacuo to give crude product which was recrystallized from n-butylchloride to give 1.39 g (60%) product, as a pale yellow solid, mp 95-97°C. ¹HNMR CDCl₃: δ 12.30 (s, 1H), 7.70 (d, 1H), 7.40 (dd, 1H), 7.02 (d, 1H), 3.92 (dd, 2H), 2.66 (s, 3H), 2.50 (s, 3H).

### 2'-Hydroxy-5'-(methylsulfonylmethyl)acetophenone

To a solution of 2'-hydroxy-5'-(methylthiomethyl)acetophenone (2.20 g, 11.0 mmol) in 88 mL methanol cooled in an ice bath was added a solution of OXONE (commercial potassium peroxymonosulfate mixture, Aldrich Chemical Co.)(20.28 g, 33.0 mmol) in 88 mL water. The mixture was stirred two hours with cooling then partially concentrated in vacuo. The product was isolated in the same manner employed in the previous example to give after recrystallization from n-butylchloride 1.70 g (68%) product, as a white solid, mp 142-144°C:
¹HNMR CDCl₃: δ 12.35 (s, 1H), 7.82 (d, 1H), 7.49 (dd, 1H), 7.04 (d, 1H), 4.22 (s, 2H), 2.82 (s, 3H), 2.67 (s, 3H).

### 2'-Hydroxy-5'-(methanesulfonamidomethyl)acetophenone

To a mixture of sodium hydride (60% dispersion in mineral oil, 1.60 g, 40.0 mmol) in 25 mL DMSO under Argon was added dropwise a solution of methanesulfonamide (3.80 g, 40.0 mmol) in 10 mL DMSO. The resulting mixture was stirred 30 minutes and a solution of 5'-(chloromethyl)-2'-hydroxyacetophenone (3.69 g, 20.0 mmol) in 10 mL DMSO was added. The resulting mixture was stirred three hours, poured into 150 mL ice water, acidified with 14 mL 3N hydrochloric acid, and the mixture extracted with methylene chloride (3 x 200 mL). The extract was washed with water (50 mL) and brine, dried over sodium sulfate, and filtered. The filtrate was concentrated in vacuo to give an oil which was partially purified by flash chromatography eluting with 3: 97 methanol: chloroform to give a gum. The gum was stirred under 25 mL water to remove residual DMSO, the aqueous supernatant was decanted, and the residue was dried in vacuo. The resulting material was crystallized from ethylacetate to remove 0.67 g of a by-product identified as N,N-bis[(3-acetyl-4-hydroxyphenyl)methyl]methanesulfonamide. The mother liquor was concentrated in vacuo to give a residue which was re-chromatography to give a solid. The solid was recrystallized from n-butylchloride to give 0.60 g (12%) product, as a white solid, mp 85-86.5°C.
¹HNMR CDCl₃: δ 12.38 (s, 1H), 7.73 (d, 1H), 7.47 (dd, 1H), 7.00 (d, 1H), 4.60 (broad t, 1H, NH), 4.29 (d, 2H), 2.92 (s, 3H), 2.66 (s, 3H).

### 2'-Hydroxy-5'-[(N-methyl-N-acetyl)aminomethyl]-acetophenone

To a mixture of sodium hydride (60% dispersion in mineral oil, 1.60 g, 40.0 mmol) in 25 mL DMSO under Argon was added dropwise a solution of N-methylacetamide (2.92 g, 40.0 mmol) in 10 mL DMSO. The resulting mixture was stirred 30 minutes and a solution of 5'-(chloromethyl)-2'-hydroxyacetophenone (3.69, 20.0 mmol) in 10 mL DMSO was added. The resulting mixture was stirred three hours, poured into 150 ml ice water, acidified with 14 mL 3N hydrochloric acid, and the mixture extracted with methylene chloride (3 x 200 mL). The extract was washed with water (50 mL) and brine, dried over sodium sulfate, and filtered. The filtrate was concentrated in vacuo to give an oil which was partially purified by flash chromatography eluting with 3: 97 methanol: chloroform to give an oil. The oil was stirred under 25 mL water to remove residual DMSO, the aqueous supernatant was decanted and the resulting gum was dried in vacuo. The gum was stirred under 50 mL ether and filtered. The filtrate was concentrated in vacuo to give a residue which was re-chromatographed eluting with 3: 97 ethyl acetate: hexane to give 0.93 g (21%) product as an oil which crystallized on standing, mp 60-65°C.
¹HNMR CDCl₃: major rotamer: δ 12.22 (s, 1H), 7.66 (d, 1H), 7.38 (dd, 1H), 6.95 (d, 1H), 4.52 (s, 2H), 2.95 (s, 3H), 2.64 (s, 3H), 2.16 (s, 3H); minor rotamer: δ 12.25 (s, 1H), 7.50 (d, 1H), 7.31 (dd, 1H), 7.02 (d, 1H), 4.49 (s, 2H), 2.94 (s, 3H), 2.64 (s, 3H), 2.19 (s, 3H).

### 1-[(3-Acetyl-4-hydroxyphenyl)methyl]imidazole

To a mixture of 5'-(chloromethyl)-2'-hydroxyacetophenone (1.85 g, 10.0 mmol), sodium bicarbonate (1.68 g, 20.0 mmol), and 20 mL dimethylformamide was added imidazole (0.75 g, 11.0 mmol). The mixture was stirred four hours and concentrated in vacuo, The residue was stirred under 50 mL ethanol, filtered, and the filtrate concentrated in vacuo to give a residue. The residue was sitrred under 30 mL chloroform, filtered, and the filtrate concentrated in vacuo to give crude product. The crude product was purified by flash chromatography eluting with 5: 95 methanol: chloroform to give a solid. The solid was recrystallized from n-butlychloride to give 0.71 g (33%) product as a yellow crystalline solid, mp 106-107°C.
¹HNMR CDCl₃: δ 12.3 (broad s, 1H), 8.03 (s, 1H), 7.62 (d, 1H), 7.34 (dd, 1H), 7.15 (d, 1H), 7.00 (d, 1H), 6.94 (d, 1H), 5.17 (s, 2H), 2.62 (s, 3H).

### 2'-Hydroxy-5'-(methoxymethyl)acetophenone

- Ref:: P. Briet, J.J. Berthelon, J.C. Depin, E. Boschetti,
Ger. Offen. 2,549,745 (1976).

### 2'-Hydroxy-5'-(hydroxymethyl)acetophenone

- Ref:: S.J. Buckman, J.D. Pera, G.D. Mercer,
Ger. Offen. 2,051,921 (1971).
(Obtained as a by-product in following reaction.)

### 2'-Hydroxy-5'-([2-(methoxy)ethoxy]methyl)acetophenone

A mixture of 5'-(chloromethyl)-2'-hydroxyacetophenone (1.85 g, 10.0 mmol), potassium carbonate (1.38, 10.0 mmol), 2-methoxyethanol (0.91 g, 12.0 mmol) and 10 mL chloroform was stirred 24 hours, filtered, and the filtrate concentrated in vacuo to give a residue. The residue was purified by flash chromatography eluting with 30: 70 ethyl acetate: hexane to give 0.40 g (18%) product as an oil.
¹HNMR CDCl₃: δ 12.23 (s, 1H), 7.74 (d, 1H), 7.46 (dd, 1H), 6.96 (d, 1H), 4.52 (s, 2H), 3.63 (m, 2H), 3.58 (m, 2H), 3.40 (s, 3H), 2.64 (s, 3H).

Further elution with 50: 50 ethyl acetate: hexanes gave 0.41 g (25%) 2'-hydroxy-5'-(hydroxymethyl)acetophenone as an oil.

### 1-[(3-Acetyl-4-hydroxyphenyl)methyl]-2-pyrrolidinone

To a suspension of sodium hydride (60% dispersion in mineral oil, 0.80 g, 20.0 mmol) in 15 mL dry dimethylformamide under argon, cooled in an ice-bath was added dropwise a solution of 2-pyrrolidinone (1.70 g, 20.0 mmol) in 5 mL dimethylformamide. The ice-bath was removed, the mixture was stirred 30 minutes, and a solution of 5'-(chloromethyl)-2'-hydroxyacetophenone (1.85 g, 10.0 mmol) in 5 mL dimethylformamide was added dropwise. The mixture was stirred four hours, poured into 75 mL ice water, acidified with 7 mL 3N hydrochloric acid, and extracted with methylene chloride (3 x 75 mL). The extract was washed with water (25 mL) and brine, dried over Na₂SO₄, and filtered. The filtrate was concentrated in vacuo to give crude product which was purified by flash chromatography on silica gel eluting with 2:98 methanol: chloroform to give 1.92 g (82%) product as a viscous oil.
¹HNMR CDCl₃: δ 12.22 (s, 1H), 7.64 (d, 1H), 7.37 (dd, 1H), 6.95 (d, 1H), 4.40 (s, 2H), 3.28 (t, 2H), 2.64 (s, 3H), 2.45 (t, 2H), 2.01 (m, 2H).

### 1-[(3-Acetyl-4-hydroxyphenyl)methyl]-2-imidazolidone

To a suspension of sodium hydride (60% dispersion in mineral oil, 0.80 g, 20.0 mmol) in 15 mL dry dimethylformamide under argon, cooled in an ice-bath was added dropwise a solution of 2-imidazolidone (1.72 g, 20.0 mmol) in 5 mL dimethylformamide. The ice bath was removed, the mixture was stirred 30 minutes and a solution of 5'-chloromethyl-2'-hydroxyacetophenone (1.85 g, 10.0 mmol) in 5 mL dimethylformamide was added dropwise. The mixture was stirred four hours and the product was isolated in the same manner as in the previous example to give a solid. The solid was stirred under 20 mL n-butylchloride, filtered off, and dried in vacuo to give 1.01 g (43%) product as a white solid, mp 131-133°C.
¹HNMR CDCl₃: δ 12.22 (s, 1H), 7.66 (d, 1H), 7.41 (dd, 1H), 6.96 (d, 1H), 4.62 (broad s, 1H, NH), 4.32 (s, 2H), 3.42 (m, 2H), 3.32 (m, 2H), 2.64 (s, 3H).

Employing the procedure substantially as described in Example 339, Step 8, infra, but substituting for the 5-methanesulfonamido-2-hydroxyacetophenone used therein, the appropriately substituted 2'-hydroxy-acetophenone from Example 311 there were produced the spiropiperidines described in Table XXI.

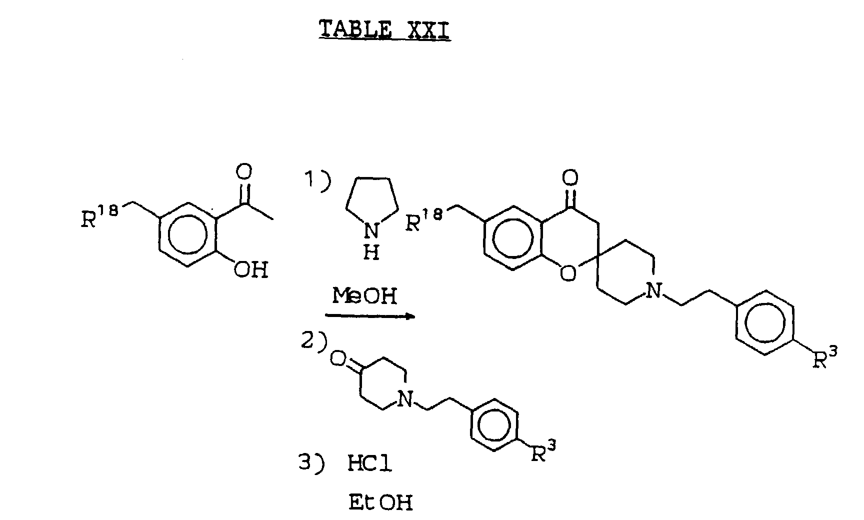

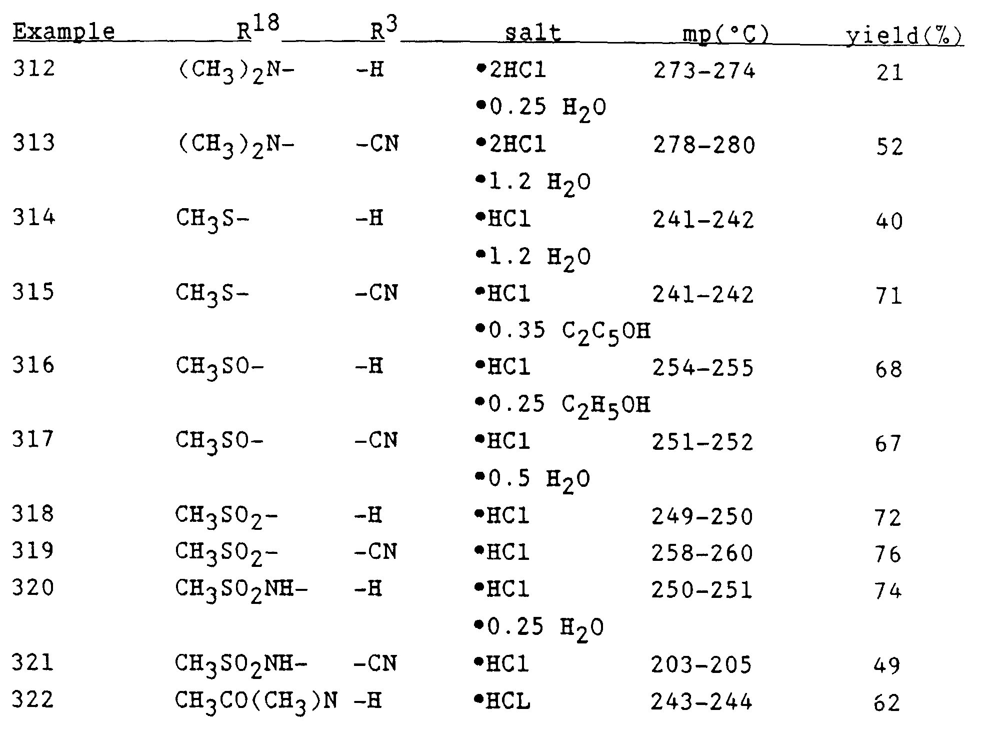

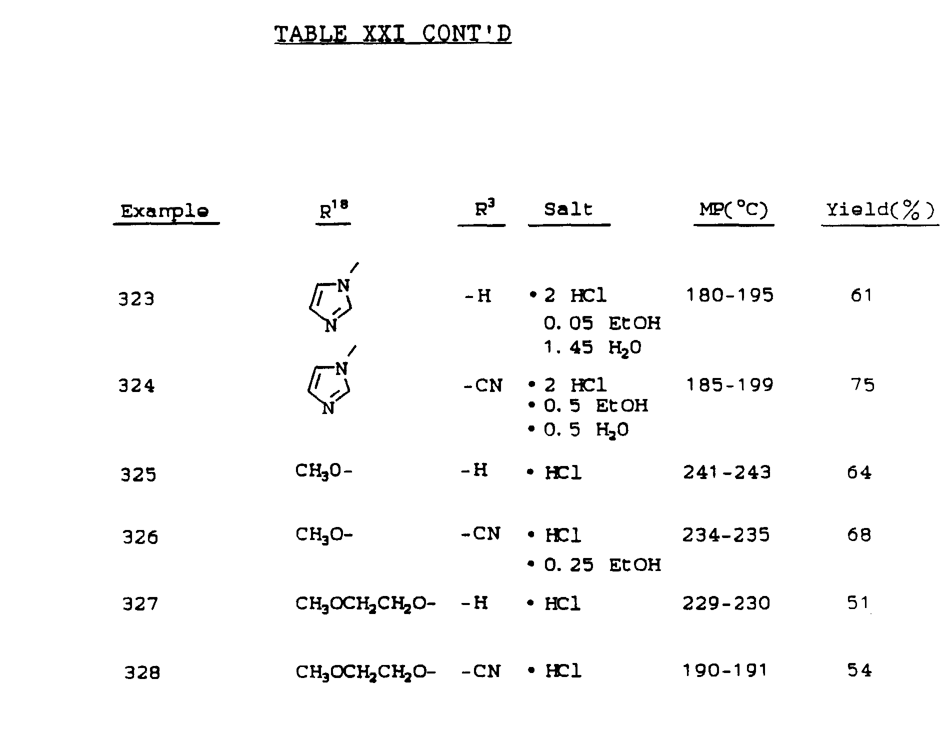

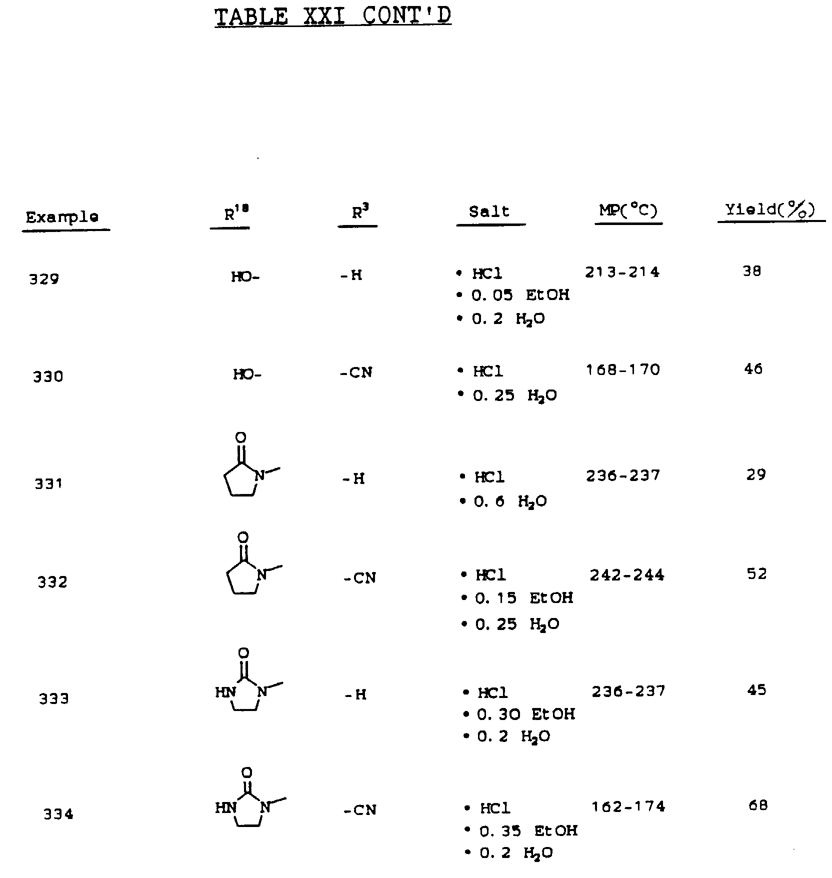

### EXAMPLE 335

### 3,4-Dihydro-6-(methylaminomethyl)-1'-(2-phenylethyl)-spiro[2H-(1-benzopyran-2,4'-piperidine)]-4-one dihydrochloride

A solution of 3,4-dihydro-6-[N-acetyl(methylaminomethyl]-1'-2(-phenylethyl)spiro[(2H)-1-benzopyran-2,4'-piperidine]-4-one (0.22 g, 0.50 mmol), 6N hydrochloric acid (0.67 ml, 4.0 mmol) and 2.0 mL ethanol was heated at reflux for 18 hours. Additional 6N hydrochloric acid (0.20 ml, 1.2 mmol) was added, the solution was heated at reflux for 4 hours, then concentrated in vacuo. The residue was placed under 5 mL ethanol and concentrated in vacuo to give a solid. The solid was stirred under 2 mL ethanol for one hour, filtered off, and dried in vacuo to give 0.143 g of the title compound product as a 0.50 water solvate as a white solid, mp 247-248°C.

| Anal. Calcd. for C₂₃H₂₈N₂O₂•2HCl•0.50 H₂O: | | | |
|---|---|---|---|
| | C, 61.88; | H, 7.00; | N, 6.28. |
| Found | C, 61.86; | H, 6.84; | N, 6.21. |

### EXAMPLE 336

### 3,4-Dihydro-6-[(N-methyl)methanesulfonamidomethyl]-1'-(2-phenylethyl)spiro[2H-1-benzopyran-2,4'-piperidine]-4-one hydrochloride

To a mixture of 3,4-dihydro-6-(methylaminomethyl)-1'-(2-phenylethyl)-spiro[(2H)-1-benzopyran-2,4'-piperidine]-4-one hydrochloride (0.162 g, 0.37 mmol), triethylamine (0.076 g, 0.75 mmol) and pyridine (2 mL) was added methanesulfonyl chloride (0.061 g, 0.53 mmol) and the resulting mixture was stirred for 18 hours. The mixture was concentrated in vacuo and the residue was partitioned between 5 mL dilute sodium bicarbonate solution and 15 mL methylene chloride. The layers were separated and the aqueous layer was extracted with methylene chloride (2 x 15 mL). The combined extract was washed with brine, dried over sodium sulfate, and filtered. The filtrate was concentrated in vacuo to give crude product which was purified by flash chromatography on silica gel eluting with 5: 95 methanol: chloroform to give 0.100 g free-base as an oil.

To a solution of the free-base in 1 mL ethanol was added 0.042 mL (0.25 mmol) 5.9 N hydrogen chloride in ethanol to give a precipitate. The precipitate was filtered off and dried in vacuo to give 0.078 g (44%) of the title compound as a 0.20 hydrate and 0.10 ethanol solvate as an off-white solid, mp 251-253°C.

| Anal. Calcd. for C₂₄H₃₀N₂O₄S•HCl•0.20 H₂O•0.10 C₂H₅OH: | | | |
|---|---|---|---|
| | C, 59.95; | H, 6.62; | N, 5.75. |
| Found | C, 59.65; | H, 6.45; | N, 5.65. |

### EXAMPLE 337

### N-Methyl[(3,4-dihydro-1'-(2-phenylethyl)-spiro[(2H)-1-benzopyran-2,4'-piperidine]-4-one)-6-yl]methanesulfonamide Hydrochloride

### Step 1: Sodium (3-acetyl-4-hydroxy)phenylmethylsulfonate

A solution of 5'-(chloromethyl)-2'-hydroxyacetophenone (1.85 g, 10.0 mmol) and sodium sulfite (1.32 g, 10.5 mmol) in water (10 mL) was heated at reflux for three hours. The cooled solution was washed with ether (2 x 10 mL) and concentrated in vacuo to give a solid which was dried in vacuo to give 2.87 g crude product containing sodium chloride and water.
¹HNMR (DMSO-d₆): δ 11.73 (s, 1H), 7.77 (d, 1H), 7.49 (dd, 1H), 6.87 (d, 1H), 3.68 (s, 2H), 2.61 (s, 3H).

### Step 2: 1'-Acetyl-3,4-dihydro-spiro[(2H)-1-benzopyran-2,4'-piperidine-4-one]-6-ylmethanesulfonic acid

A mixture of sodium (3-acetyl-4-hydroxy) phenylmethanesulfonate (2.57 g, 9 mmol) and pyrrolidine (0.64 g, 9.0 mmol) in methanol (18 mL) was heated at 60°C for 15 minutes. A solution of N-acetyl-4-piperidone (1.27 g, 9.0 mmol) in methanol (9 ml) was added and the mixture was stirred at 60°C for four hours. The mixture was concentrated in vacuo to give a residue which was dissolved in water (10 mL), acidified with 3 N hydrochloric acid, reconcentrated in vacuo, and the resulting residue was extracted with methylene chloride (3 x 50 mL). The extract was dried over Na₂SO₄, filtered, and the filtrate concentrated to give a foam. The foam was dissolved in water (25 mL), basified with 20% sodium hydroxide solution to pH 10, and the solution was washed with ether (2x20 ml) to remove residual pyrolidine. The aqueous solution was acidified with 3 N hydrochloric acid and concentrated in vacuo to give a solid. The solid was extracted with hot acetonitrile (4 x 50 mL) leaving the insoluble solid product (1.21 g, 38%), mp 233-235°C.
¹HNMR (DMSO-d₆): δ 7.64 (d, 1H), 7.51 (dd, 1H), 6.98 (d, 1H), 4.21 (d, 1H), 3.67 (s, 2H), 3.65 (d, 1H), 3.38 (m, 1H), 2.97 (m, 1H), 2.80 (s, 2H), 2.00 (s, 3H), 1.92 (m, 2H), 1.72 (m, 1H), 1.56 (m, 1H).

### Step 3: N-Methyl [(3,4-dihydro-1'-acetylspiro[(2H)-1-benzopyran-2,4'-piperidine]-4-one-6-yl]-methanesulfonamide

To a mixture of (1'-acetyl-3,4-dihydrospiro[(2H)-1-benzopyran-2,4'-piperidine-4-one]-6-yl) methanesulfonic acid (1.20 g, 2.9 mmol) and dimethylformamide (1 mL) in methylene chloride (20 mL) was added thionyl chloride (0.26 mL, 0.42 g, 3.5 mmol). The mixture was stirred three hours, then concentrated in vacuo to give the sulfonyl chloride. To a solution of the sulfonyl chloride in methylene chloride (20 mL) cooled in an ice bath was added a cold solution of methylamine (0.20 g, 6.4 mmol) in 5 mL methylene chloride. The resulting mixture was stirred three hours with cooling, diluted with methylene chloride (60 mL) washed with 1N hydrochloric acid (10 mL), water (5 mL), and brine, dried over sodium sulfate, and filtered. The filtrate was concentrated in vacuo to give a gum which was purified by flash chromatography on silica gel eluting with 3:97 methanol:chloroform to give 0.36 g (34%) product as a foam.
¹HNMR (CDCl₃): δ 7.83 (d, 1H), 7.59 (dd, 1H), 7.04 (d, 1H), 4.42 (broad s, 1H, -NH), 4.39 (d, 1H), 4.20 (s, 2H), 3.65 (d, 1H), 3.51 (m, 1H), 3.08 (m, 1H), 2.78 (d, 3H), 2.73 (s, 2H), 2.11 (s, 3H), 2.09 (m, 2H), 1.64 (m, 2H).

### Step 4: N-Methyl[(3,4-dihydrospiro[(2H)-1-benzopyran-2,4'-piperidine]-4-one)-6-yl]methanesulfonamide

A solution of N-methyl[(3,4-dihydro-1'-acetylspiro[(2H)-1-benzopyran-2,4'-piperidine]-4-one-6-yl]methanesulfonamide (0.348 g, 0.95 mmol), 6N hydrochloric acid (1.25 mL, 7.5 mmol), and 3.75 mL ethanol was heated at reflux for 18 hours. The solution was concentrated in vacuo, the residue was placed under ethanol (10 mL), reconcentrated in vacuo, placed under 1:1 ethanol:toluene (10 mL), reconcentrated in vacuo, placed under carbon tetrachloride (10 mL), reconcentrated, and dried in vacuo to give a hygroscopic foam. The foam was stirred under ether (10 mL) for one hour, filtered off, and dried in vacuo to give the hydrochloride as a white solid, mp 140-165°C.
¹HNMR (DMSO-d₆): δ 9.1 (broad s, 1H), 8.9 (broad s, 1H), 7.77 (d, 1H), 7.59 (dd, 1H), 7.15 (d, 1H), 6.94 (q, 1H, -NHSO₂), 4.35 (s, 2H), 3.25-3.02 (m, 4H), 2.93 (s, 2H), 2.58 (d, 3H), 2.11 (m, 2H), 1.92 (m, 2H).

### Step 5: N-Methyl[(3,4-dihydro-1'-(2-phenylethyl)-spiro[(2H)-1-benzopyran-2,4'-piperidine]-4-one)-6-yl]methanesulfonamide hydrochloride

A mixture of N-methyl[(3,4-dihydrospiro[(2H)-1-benzopyran-2,4'-piperidine]-4-one)-6-yl]methanesulfonamide hydrochloride (0.144 g, 0.40 mmol), sodium bicarbonate (0.134 g, 1.50 mmol), (2-bromoethyl)benzene (0,113 mL, 0.148 g, 0.80 mmol), potassium iodide (0.066 g, 0.40 mmol) and acetonitrile (5 mL) was heated at reflux for four hours. The mixture was concentrated in vacuo, the residue was diluted with saturated sodium bicarbonate solution (10 mL) and water (2 mL), and extracted with methylene chloride (3 x 15 mL). The extract was washed with water (3 mL) and brine, dried over sodium sulfate, and filtered. The filtrate was concentrated in vacuo to give a residue which was purified by flash chromatography to give 0.101 g free-base.

To a suspension of the free base in ethanol (3 mL) was added 5.9N hydrogen chloride in ethanol (0.046 ml, 0.27 mmol) to give a precipitate. The precipitate was filtered off and dried in vacuo to give the title compound as an off-white solid, (0.085 g, 45%), mp 142-145°C.

| Anal. Calcd. for C₂₃H₂₈N₂O₄S•HCl•0.65 H₂O: | | | |
|---|---|---|---|
| | C, 57.94; | H, 6.41; | N, 5.88. |
| Found | C, 58.00; | H, 6.68; | N, 5.85. |

### EXAMPLE 338

### N-Methyl[(34-dihydro-1'-[2-(4-cyanophenyl)ethyl]-spiro[(2H)-1-benzopyran-2,4'-piperidine]-4-one)-6-methanesulfonamide Hydrochloride

A mixture of N-methyl[(3,4-dihydrospiro[(2H)-1-benzopyran-2,4'-piperidine]-4-one-6-ylmethanesulfonamide hydrochloride (0.144 g, 0.40 mmol), sodium bicarbonate (0.134 g, 1.60 mmol), 2-(4-cyanophenyl)ethyl methanesulfonate (0.180 g, 0.80 mmol), potassium iodide (0.066 g, 0.40 mmol), and acetonitrile (5 mL) was heated at reflux for four hours. In the same manner as Example 337 [P27], purification and recrystallization from ethanol gave the title compound as an orange solid, (0.065 g, 33%), mp. 146-158°C.

| Anal. Calcd. for C₂₄H₂₇N₃O₄•HCl•0.25 C₂H₅OH: | | | |
|---|---|---|---|
| | C, 58.67; | H, 5.93; | N, 8.38. |
| Found | C, 58.96; | H, 5.64; | N, 8.29. |

### EXAMPLE 339

### N-[(1'-[2-(5-Benzofurazanyl)ethyl]-3,4-dihydrospiro-[2H-[1-benzopyran-2,4'-piperidine]-4-one)-6-yl]-methanesulfonamide monohydrochloride.

### Step 1: Preparation of 5-amino-2-hydroxyacetophenone hydrochloride

5-Acetamido-2-hydroxyacetophenone (19.03 g, 98.5 mmoles) (prepared as described by C.T. Chang, F.C. Chen, K.K. Hsu, T. Ueng, and M. Hung, J. Chem. Soc. 3414 (1961)) dissolved in 300 mL of ethanol and 100 mL of 6N aqueous HCl was heated at reflux for 8 hrs. The solution was concentrated in vacuo, and the residue was flushed with ethanol, and dried in vacuo to give 18.3 g (99%) of 5-amino-2-hydroxyacetophenone hydrochloride as a dark solid.

### Step 2: Preparation of 5-methanesulfonamido-2-hydroxyacetophenone

A suspension of 5-amino-2-hydroxyacetophenone hydrochloride, 18.39 g (98 mmole) in 200 mL of methylene chloride cooled to 0°C was treated with 19.4 mL (240 mmol) of pyridine. Methanesulfonyl chloride, 7.74 mL (100 mmoles) was added dropwise. The mixture was stirred an additional 30 min at 0°C and then allowed to warm to room temperature over 1 hr. The mixture was diluted with 200 mL of methylene chloride and washed with 50 mL of 1N aqueous HCl. Concentration of the organic layer and trituration with methylene chloride gave a solid which was recrystalized from methylene chloride to give 15.45 g (69%) of 5-methanesulfonamido-2-hydroxyacetophenone as a white solid (mp 121-122°C).

### Step 3: Preparation of N(2-(4-aminophenyl)ethyl-1,4-dioxa-8-azaspiro[4.5]-decane

1,4-Dioxa-8-azaspiro[4.5]-decane (18.62 g, 130 mmoles) in 50 mL of acetonitrile was stirred under argon and treated with anhydrous potassium carbonate (27.60 g, 200 mmoles). The mixture was cooled to 0°C and 2-(4-nitrophenyl)ethylbromide (23.01 g, 100 mmoles) was added in one portion. After 10 minutes, the ice bath was removed and the reaction was stirred for 14 hrs at room temperature. The mixture was diluted with 220 mL of ethyl acetate and 200 mL of water. The aqueous phase was separated, the organic phase washed with water (3 x 100 mL), and brine (2 x 50 mL). The organic portion was dried (Na2SO4), filtered, and concentrated to give 28.60 g (98%) of N(2-(4-nitrophenyl)ethyl-1,4-dioxa-8-azaspiro [4.5]-decane as a yellow solid.

The crude N(2-(4-nitrophenyl)ethyl-1,4-dioxa-8-azaspiro[4.5]-decane (28.6 g) disolved in 170 mL of tetrahydrofuran and 550 mL of absolute ethanol was treated with 4.0 grams of Raney nickel and shaken under an atmosphere of hydrogen (55 psig) for 48 hrs. Filtration through celite, and concentration in vacuo gave 25.70 g of N(2-(4-aminophenyl)ethyl)-1,4-dioxa-8- azaspiro[4.5]-decane (mp 96.5-99°C) as an off white solid (98% from 2-(4-nitrophenyl)ethylbromide).

### Step 4: Preparation of N(2-(4-acetamino-3-nitrophenyl)ethyl-1,4-dioxa-8-azaspiro[4.5]-decaneacetate

A mechanically stirred solution of N(2-(4-aminophenyl)ethyl)-1,4-dioxa-8-azaspiro[4.5]-decane in methylene chloride (400 mL) cooled to -5°C under an argon atmosphere was treated dropwise with acetic anhydride (58.8 mL, 622 mmoles). The reaction was allowed to reach room temperature and was stirred for 18 hr. After the addition of an additional 29.5 mL (312 mmoles) of acetic anhydride, the reaction was cooled to -5°C, and a solution of nitric acid (d=1.49, 22.0 mL, 468 mmoles) in methylene chloride (1:1 by volume) was added dropwise. The reaction mixture was allowed to reach room temperature and was stirred for 4 hours. The mixture was cooled in an ice bath and 100 mL of saturated brine was added. The layers were separated and the aqueous phase was extracted with methylene chloride. The combined organic portion was dried (Na₂SO₄), concentrated and flushed with toluene (3 X 200 mL). The crude product crystallized during concentration, was filtered, and triturated with ethyl acetate (550 mL) to yield 56.7 g (89%) of N(2-(4-acetamino-3-nitrophenyl)ethyl)-1,4-dioxa-8-azaspiro[4.5]-decane acetic acid salt. TLC Rf= 0.80 (silica gel, 5% methanol in methylene chloride saturated with ammonia).

### Step 5: Preparation of 8-[2-(benzofuroxan-5-yl)-ethyl]-1,4-dioxa-8-aza spiro[4.5]decane

A suspension of 4.09 g (10.0 mmoles) N(2-(4-acetamino-3-nitro-phenyl)ethyl)-1,4-dioxa-8-azaspiro-[4.5]-decane acetic acid salt in 60 mL of methanol stirred at 0°C was treated dropwise with 86% potassium hydroxide (5.22 g , 80 mmol) in 20 mL of methanol over 5 min. The temperature was allowed to warm to 25°C and the mixture was stirred 2 hr. Solid iodophenyldiacetate (3.86 g, 12 mmol) was added and the mixture was stirred 30 min. An additional portion of iodophenyldiacetate (0.32 g, 1.0 mmol) was added and stirring was continued 20 min. The mixture was concentrated in vacuo and the residue partitioned between 150 mL of ethyl acetate and 75 mL of water. The layers were separated and the aqueous layer was reextracted (2 X 100 mL) with ethyl acetate. The combined organic portions were washed with water (25 mL), and saturated brine, dried (Na₂SO₄), and concentrated to a solid orange residue. The solid was triturated with hexane and dried in vacuo to give 2.78 g (91%) of 8-[2-(benzofuroxan-5-yl)ethyl]-1,4-dioxa-8-aza spiro[4.5]decane (mp 117-119°C).

### Step 6: Preparation of 8-[2-(benzofurazan-5-yl)-ethyl]-1,4-dioxa-8-aza spiro[4,5]decane

A solution of 8-[2-(benzofuroxan-5-yl)-ethyl]-1,4-dioxa-8-aza spiro[4.5]decane (24.0 g, 78.6 mmol) in ethanol(240 mL) was treated with triethylphosphite (27.5 mL, 157 mmol) and heated at reflux for 2.5 hours. The mixture was concentrated and flushed with hexanes (2 X 200 mL), and then diluted with 400 mL of hexanes and the solids collected by filtration to yield 19.3 g (82%) of 8-[2-(benzofurazan-5-yl)ethyl]-1,4-dioxa-8-aza spiro[4.5] decane. A second crop (2.25g 13%) was obtained after chromatography of the mother liquors on silica gel (2% methanol in methylene chloride).
¹H NMR (300 MHz, CDCl₃): δ 1.75 (bt, 4H); 2.65 (bt, 4H); 2.70 (t, J=7 Hz, 2H); 2.90 (t, J=7Hz, 2H); 3.95 (s, 4H); 7.30 (d, J=9Hz, 1H); 7.60 (s,1H); 7.75 (d, J=9Hz, 1H).

### Step 7: Preparation of 1-[2-(benzofurazan-5-yl)ethyl]piperidin-4-one

A solution of 8-[2-(benzofurazan-5-yl)-ethyl]-1,4-dioxa-8-aza spiro[4.5]decane (20.0 g, 69.1 mmol) in 1N aqueous HCl (200 mL, 200 mmol) was heated to reflux for 1.5 hr. The cooled solution was adjusted to pH 8.5 with 40% aqueous sodium hydroxide and extracted with ethyl acetate (250 mL then 100 mL). The combined extracts were washed twice with 1N aqueous HCl (200 mL combined). The acid layers were heated to reflux for 1 hr and then cooled. Basification as above, extraction into ethyl acetate, drying (Na2S04), concentration, and trituration with hexanes afforded 1-[2-(benzofurazan-5-yl)ethyl]piperidin-4-one as a solid 14.9 g (88%). HPLC: Rt= 3.0 min, 90:10 (0.1% H₃PO₄ in water: acetonitrile) flow= 2.0 mL/min, Waters C¹⁸micro bondapak 25 cmL.

### Step 8: Preparation of N-[(1'-[2-(5-Benzofurazanyl)-ethyl]-3,4-dihydro-spiro[2H-[1-benzopyran-2,4'-piperidine-4-one)-6-yl]methane sulfonamide

A solution of 5-methanesulfonamido-2-hydroxyacetophenone (5.58 g, 24.3 mmol) and pyrrolidine (2.05 mL, 24.3 mmol) in methanol(30 mL) was warmed to 60°C and treated with 1-[2-(benzofurazan-5-yl)ethyl]piperidin-4-one (6.0 g, 24.3 mmol). The reaction was stirred at 60°C for 1 hr, cooled to room temperature, and concentrated in vacuo to an oil. The residue was chromatographed on silica gel eluting with ethyl acetate then 3% methanol in ethyl acetate. Concentration of the appropriate fractions and crystalization from isopropyl acetate provided 8.2 g (74%) of methanesulfonamide, N-[1'-[2(5-benzofurazanyl)ethyl]-3,4-dihydro-4-oxospiro[2H-[1-benzopyran-2,4'-piperidin]-6-yl]. This was converted to its hydrochloride salt, mp=285-287°C, by disolving the free base in dilute aqueous HCl at 90°C (2 eq HCl, 10 g in 1.5 L) cooling to precipitate, filtration and drying in vacuo.

### EXAMPLE 340

### 3,4 Dihydro-1'-[2-phenylethyl-6-cyano-spiro[2H-1-benzopyran-2,4'-piperidin]-4-one hydrochloride •1/2 H₂O•1/3 ethanol

A solution of 5-cyano-2-hydroxyacetophenone (806 mgs, 5 mmol) (prepared as described by N.G.P. Ellis, D. Shaw, J. Chem. Soc. Perkin I 779-783 (1972)) and pyrrolidine (0.42 mL, 5 mmol) in methanol (6 mL) was warmed to 60°C and treated with 1-[2-phenylethyl]piperidin-4-one (1.102 g, 5 mmol). The reaction was stirred at 60°C for 1 hr, cooled to room temperature, and concentrated in vacuo to an oil. The residue was chromatographed on silica gel eluting with ethyl acetate to give 500 mg of which 250 mg free base which was converted to the hydrochloride salt in ethanol at 0°C yield 270 mps of a solid after filtration and drying in vacuo mp=253-255°C(dec) •HCl•1/2 H₂O•1/3 C₂H₆O.

### EXAMPLE 341

### Spiro[2H-1-benzopyran-2,4'-piperidine]-6-aminosulfonyl-1'-[2-phenylethyl]-3,4-dihydro-N-methyl-4-oxo, monohydrochloride

### Step 1: Preparation of 3-acetyl-4-hydroxy-benzenesulfonyl chloride

A mixture of 3-acetyl-4-hydroxy-benzenesulfonic acid (3.0 g) (prepared according to T. Pfliegel et. al., Acta Chim. Acad. Sci. Hung., 98(2) 231-240(1978)) in tetramethylenesulfone (6 mL) and acetonitrile (6 mL) under argon was treated with triethylamine (1.94 mL) and N,N-dimethylacetamide (0.32 mL). This was stirred for 30 min and POCl₃ was added dropwise maintaining the temperature below 25°C. The reaction was stirred for 24 hr, cooled to 10°C and quenched with water (15 mL). This mixture was warmed to 60°C and stirred for 1 hr, at which point a precipitate formed which was then filtered and dried to give 2.34g (72%) of 3-acetyl-4-hydroxybenzenesulfonylchloride.
¹H NMR (CDCl₃, 300 MHz): δ 2.80 (s, 3H); 7.25 (d, J=12Hz, 1H); 8.05 (dd, J=3, 12 Hz, 1H); 8.4 (d, J=3Hz, 1H); 12.75 (s, 1H).

### Step 2: Preparation of N-methyl-3-acetyl-6-hydroxybenzenesulfonic acid amid

A saturated solution of monomethyl amine in acetonitrile cooled to 0°C was treated with 3-acetyl-4-hydroxy-benzenesulfonyl chloride. The mixture was stirred 5 min, concentrated in vacuo to an oil, disolved in ethyl acetate and treated with 1 N aqueous HCl. The organic portion was separated, concentrated, filtered through silica gel (eluted with ethyl acetate) to provide N-methyl-3-acetyl-4-hydroxy-benzenesulfonic acid amide after concentration.
¹H NMR (300 MHz, CDCl₃): δ 2.70 (s, 3H); 2.71 (s, 3H); 4.4 (bs, 1H); 7.10 (d, J=12Hz, 1H); 7.95 (dd, J=3,12Hz, 1H); 8.30 (d, J=3Hz, 1H); 12.75 (s 1H).

### Step 3: Preparation of Spiro[2H-1-benzopyran-2,4'-piperidine]-6-aminosulfonyl-1'-[2-phenylethyl]-3,4-dihydro-N-methyl-4-oxo, monohydrochloride

Following a procedure similar to that for Step 8 of Example 339 for the preparation of methanesulfonamide, N-[1'-[2-(5-benzofurazanyl)ethyl]-3,4-dihydro-4-oxospiro[2α-[1-benzopyran-2,4'-piperidin]-6-yl]-, monohydrochloride substituting N-methyl-3-acetyl-4-hydroxybenzenesulfonic acid amide for 5-methanesulfonamido-2-hydroxyacetophenone and 1-phenethyl-piperidin-4-one for 1-[2-(benzofurazan-5-yl)ethyl]piperidin-4-one there was obtained spiro[2H-1-benzopyran-2,4'-piperidine]-6-aminosulfonyl-1'-[2-phenylethyl]-3,4-dihydro-N-methyl-4-oxo, monohydrochloride•0.3 H₂O, mp = 232-233°C (dec).

### EXAMPLE 342

### Spiro[2H-1-benzopyran-2,4'-piperidine]-6-aminosulfonyl-1'-[2-phenylethyl]-3,4-dihydro-4-oxo, monohydrochloride

### Step 1: Preparation of 3-acetyl-4-hydroxy-benzenesulfonic acid amide

The title compound was prepared in a similar manner as for the preparation of N-methyl-3-acetyl-4-hydroxy-benzenesulfonic acid amide, but substituting ammonia for mono methyl amine.

### Step 2: Preparation of Spiro[2H-1-benzopyran-2,4'-piperidine]-6-aminosulfonyl-1'-[2-phenylethyl]-3,4-dihydro-4-oxo, monohydrochloride

This was prepared in a manner similar to the preparation of spiro[2H-1-benzopyran-2,4'-piperidine]-6-aminosulfonyl-1'-[2-phenylethyl]-3,4-dihydro-N-methyl-4-oxo, monohydrochloride but using 3-acetyl-4-hydroxy-benzenesulfonic acid amide in place of 5-methanesulfonamido-2-hydroxyacetophenone to give spiro[2H-1-benzopyran-2,4'-piperidine]-6-aminosulfonyl-1'-[2- phenyl-ethyl]-3,4-dihydro-4-oxo, monohydrochloride mp=265-266°C.

Employing the procedure described in Examples 340, 341 and 342, but substituting the appropriate 2-hydroxy-5-substituted acetophenone and the appropriate 1-substituted 4-piperidinone there were obtained the compounds of Table XXII.

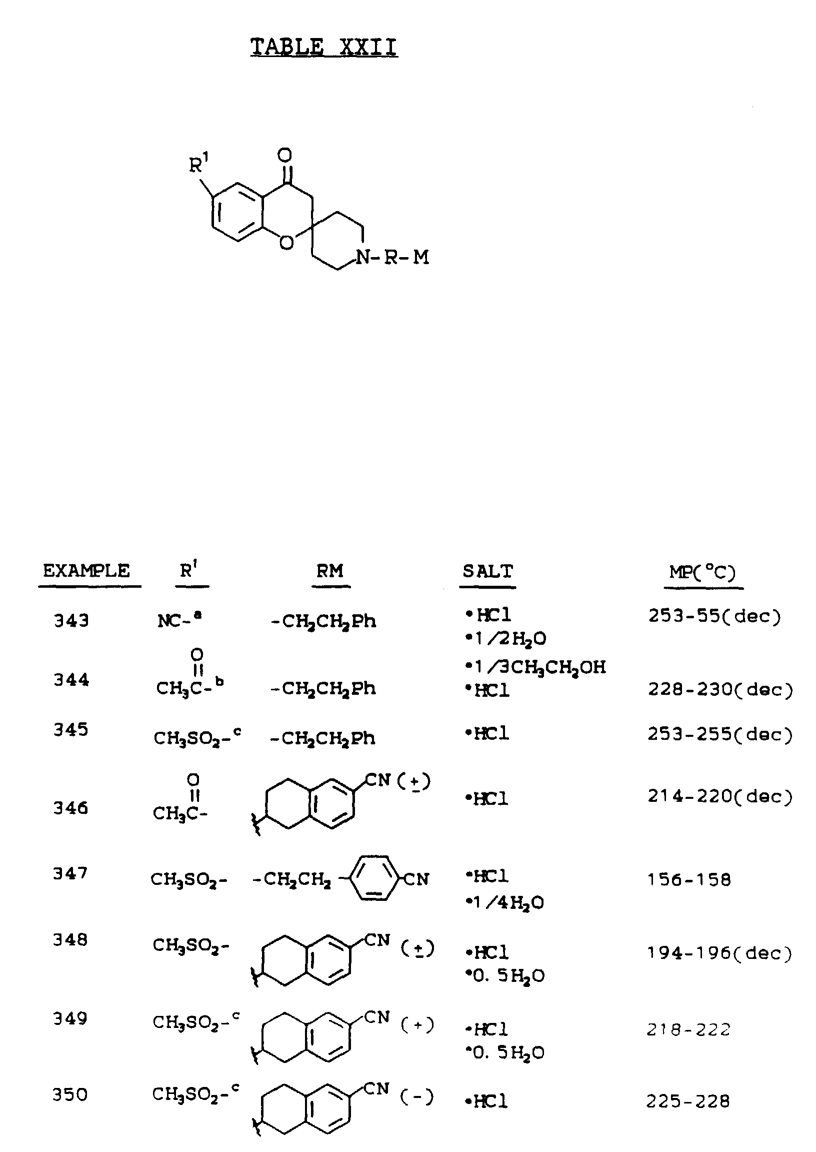

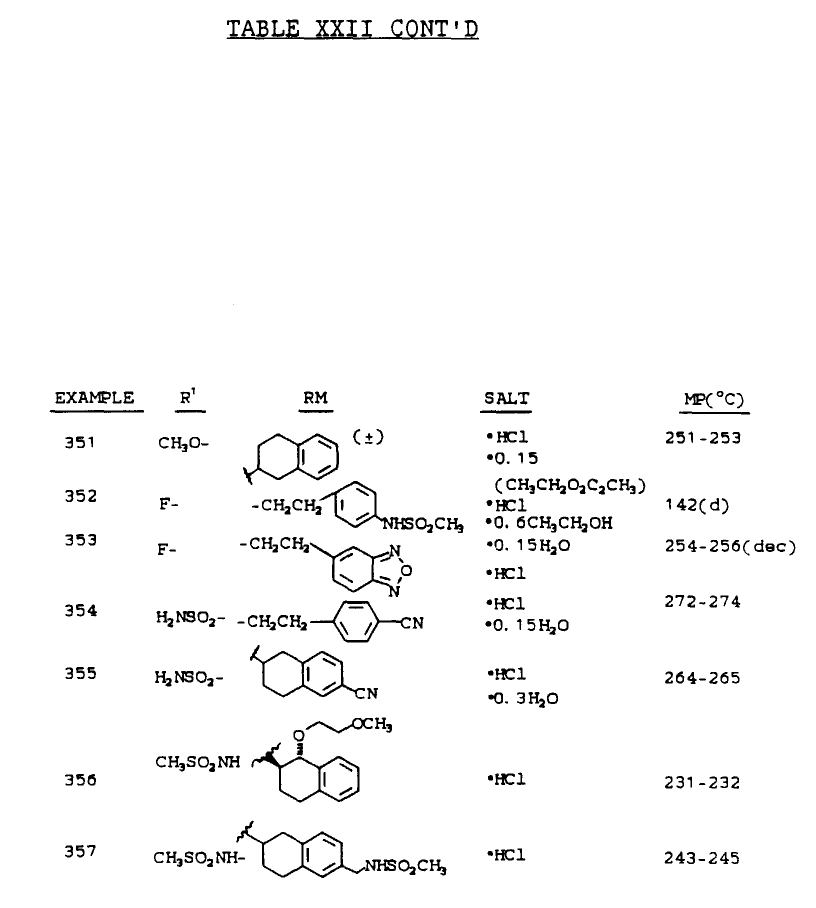

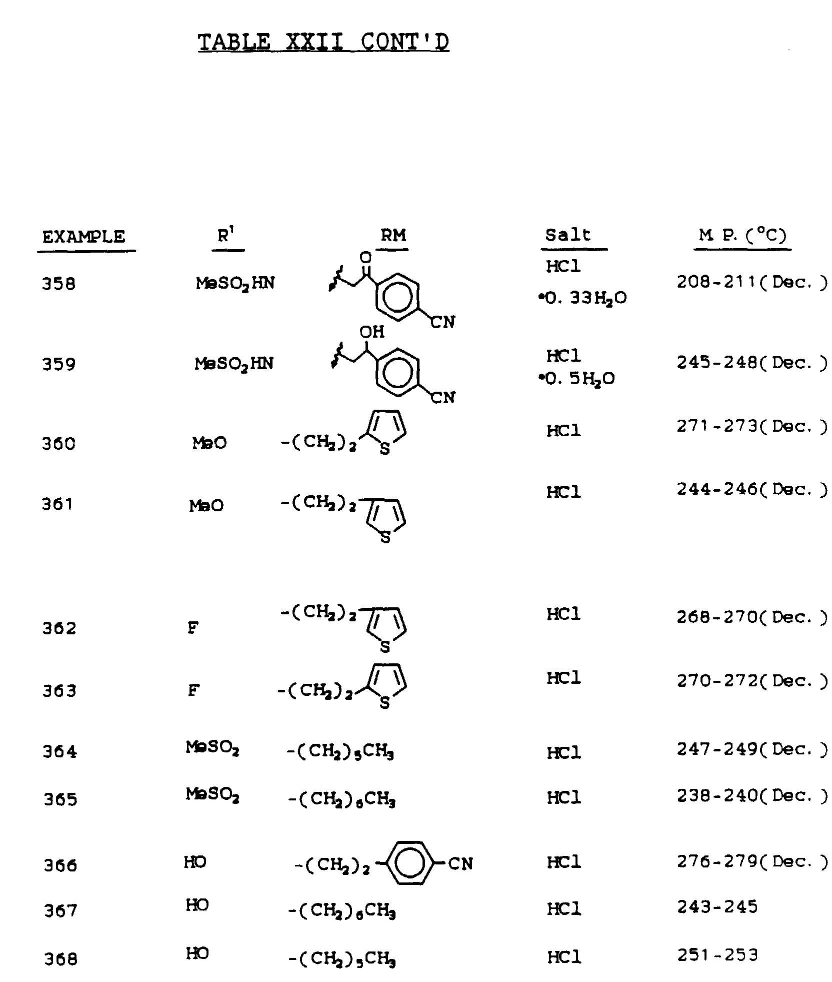

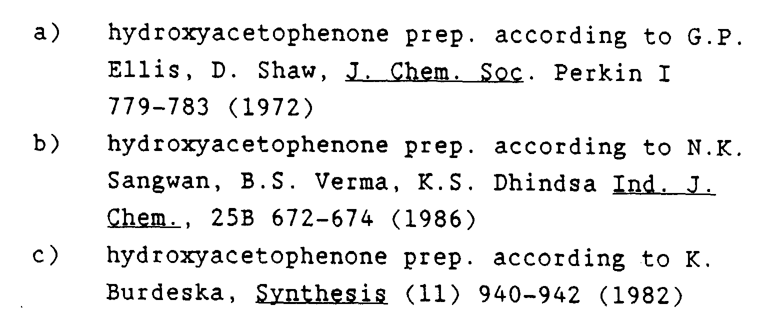

### EXAMPLE 369

### Spiro[2H-1-benzopyran-2,4'-piperidine]-6-aminocarbonyl-1'-[2-phenylethyl]-3,4-dihydro-4-oxo, monohydrochloride

A solution of 3,4-dihydro-1'-[2-phenylethylcyanospiro[2H-1-benzopyran-2,4'-piperidin]-4-one (0.30 g, 0.87 mmol) in tetrahydrofuran (10 mL) and methanol (3 mL) was stirred at 0°C. Aqueous KOH in water (1N, 6 mL) and hydrogen peroxide (0.6 mL) was added dropwise and the reaction was stirred for 2.5 hrs. The reaction was quenched with 10% aqueous sodium bisulfite and the product was extracted with ethyl acetate. The organic portion was washed with 10% aqueous sodium carbonate, water, and brine, dried (Na₂SO₄), concentrated and chromatographed (silica gel, 5% methanol in methlyene chloride) to give spiro[2H-1-benzopyran-2,4'-piperidine]-6-aminocarbonyl-1'-[2-phenylethyl]-3,4-dihydro-4-oxo, monohydrochloride (140 mg) after conversion to the salt in ethanol; mp 261-263.5°C (dec).

### EXAMPLE 370

### Spiro[2H-1-benzopyran-2,4'-piperidine]-6-carboxy-1'-[2-phenylethyl]-3,4-dihydro-4-oxo, monohydrochloride

A mixture of 3,4-dihydro-1'-[2-phenylethyl-6-cyano-spiro[2H-1-benzopyran-2,4'-piperidin]-4-one (500 mg) in 30 mL of 4N aqueous HCl was refluxed for 18 hrs. The mixture was diluted with isopropyl alcohol and concentrated. This was rediluted with isopropyl alcohol, cooled to 0°C and the resulting solid isolated by filtration and drying in vacuo. yield 0.51 g (98%) of spiro[2H-1-benzopyran-2,4'-piperidine]-6-carboxy-1'-[2-phenylethyl]-3,4-dihydro-4-oxo, monohydrochloride•0.3 H₂O, mp = 264-265°C (dec).

### EXAMPLE 371

### Spiro[2H-1-benzopyran-2,4'-piperidine]-6-[2-[4,5- dihydro-1H-imidazoyl]]-1'-[2-phenylethyl]-3,4-dihydro-4-oxo, dihydrochloride

A solution of 3,4-dihydro-1'-[2-phenylethyl-6-cyano-spiro[2H-1-benzopyran-2,4'-piperidin]-4-one (0.30 g, 0.87 mmol) in methanol (30 mL) was cooled to 0°C and treated with HCl gas until the solution was saturated. This was allowed to warm to room temperature and stirred 18 hrs. The mixture was concentrated in vacuo to an oil, redisolved in methanol (30 mL) and treated with 1,2-diamino-ethane(0.26 mL) and stirred at room temperature. The mixture was concentrated in vacuo and chromatographed (silica gel, methylene chloride/methanol/ammonium hydroxide (90:10:2)) to give after concentration of appropriate fractions and conversion to the hydrochloride salt with HCl in ethyl acetate 120 mg of spiro[2H-1-benzopyran-2,4'-piperidine]-6-[2-[4,5-dihydro-1H-imidazoyl]]-1'-[2-phenylethyl]-3,4-dihydro-4-oxo,dihydrochloride dihydrochloride•0.5 H₂O, mp=269-271°C.

### EXAMPLE 372

### Spiro[2H-1-benzopyran-2,4'-piperidine]-6-[2-[1H-imidazoyl]]-1'-[2-phenylethyl]-3,4-dihydro-4-oxo, dihydrochloride

A solution of spiro[2H-1-benzopyran-2,4'-piperidine]-6-[2-[4,5-dihydro-1H-imidazoyl]]-1'-[2-phenylethyl]-3,4-dihydro-4-oxo, dihydrochloride (0.218 g) in methylene chloride (50 mL) under an argon atmosphere was treated with BaMnO₄ (2.20 g) and the mixture stirred and refluxed for 48 hr. The mixture was filtered to remove solids and the cake washed with methanol (100 mL). The filtrate was concentrated and flash chromatographed (silica gel, methylene chloride/methanol/amonium hydroxide(90:10:2)) to give after concentration of appropriate fractions and conversion to the hydrochloride salt with HCl, 65 mg of spiro[2H-1-benzopyran-2,4'-piperidine]-6-[2-[4,5-dihydro-1H-imidazoyl]]-1'-[2-phenylethyl]-3,4-dihydro-4-oxo, dihydrochloride•0.2 H₂O, mp=262-264°C (dec).

### EXAMPLE 373

### Spiro[2H-1-benzopyran-2,4'-piperidine]-6-aminocarbonyl-1'-[2-phenylethyl]-3,4-dihydro-N-methyl-4-oxo, monohydrochloride

A suspension of spiro[2H-1-benzopyran-2,4'-piperidine]-6-carboxy-1'-[2-phenylethyl]-3,4-dihydro-4 -oxo, monohydrochloride (100 mg) in methylene chloride (10 mL) was treated with anhydrous dimethylformamide (0.005 mL) and oxalyl chloride (0.033 mL). This was stirred for 18 hrs at room temperature and then concentrated to an oil. The oil was dissolved in methylene chloride and added to a cold (0°C) stirring solution of mono methyl amine (76 mg) in methylene chloride. After 5 minutes the mixture was concentrated and disolved in methylene chloride, washed with water, dried (Na₂SO₄), and concentrated to an oil. The hydrochloride was formed and crystalized from ethanol to give 102 mgs of spiro[2H-1-benzopyran-2,4'-piperidine]-6-aminocarbonyl-1'-[2-phenylethyl]-3,4-dihydro-N-methyl-4-oxo, monohydrochloride•0.3 H₂O, mp = 246-248°C.

### EXAMPLE 374

### Spiro[2H-1-benzopyran-2,4'-piperidine]-6-aminocarbonyl-1'-[2-phenylethyl]-3,4-dihydro-N,N-dimethyl-4-oxo, monohydrochloride

In a manner similar for the preparation of spiro[2H-1-benzopyran-2,4'-piperidine]-6-aminocarbonyl-1'-[2-phenylethyl]-3,4-dihydro-N-methyl-4-oxo, monohydrochloride, but substituting dimethyl amine for monomethylamine was prepared spiro[2H-1-benzopyran-2,4'-piperidine]-6-aminocarbonyl-1'-[2-phenylethyl]-3,4-dihydro- N,N-dimethyl-4-oxo, monohydrochloride, mp = 259-261°C.

### EXAMPLE 375

### Trans-(+-)-N-[(1'-(6-Cyano-1,2,3,4-tetrahydro-1-hydroxy-2-naphthalenyl)-3,4-dihydro-spiro[(2H)-1-benzopyran-2,4'-piperidine]-4-one-6-yl-methanesulfonamide, monohydrochloride

### Step 1: Preparation of 1,4-dioxa-8-(6'-cyano-1'-oxa-1',2',3',4'-tetrahydronaphth-2'-yl)-8-azaspiro[4.5]decane

A solution of 6-cyano-tetralin-1-one (1.712 g 10 mmol) (prepared as described by N. L. Allinger and E.S. Jones, J. Org. Chem., 27 70-76(1962)) in 25 mL of anhydrous tetrahydrofuran (THF) cooled to -10°C and under an argon atmosphere was treated dropwise with a solution of phenyltrimethylammonium tribromide (3.76g, 10 mmol) in THF (25 mL) over one hour. The reaction was quenched with an aqueous solution of 10% sodium thio- sulfate and saturated aqueous sodium bicarbonate. The mixture was extracted with ethyl acetate (3 x 75 mL), the organic portion washed with water (3 x 50 mL) and brine, dried (Na₂SO₄) and concentrated to give 2.50 g of an orange solid (100%). The crude product was disolved in 25 mL of anhydrous dimethylfomamide (DMF) and added to a solution of 5.73 g (40 mmol) 1,4-dioxa-8-azaspiro [4.5]-decane in 25 mL of DMF cooled to -15°C over 15 min. The reaction was stirred an additional 2 hrs while the temperature was allowed to warm to -5°C. The mixture was diluted with ethyl acetate (200 mL) washed with water (3 x 150 mL) and then 1N aqueous HCl (2 X 40 mL). The acid washes were combined and washed with ethyl acetate (50 mL). Neutralization with saturated aqueous NaHCO₃, extraction into ethyl acetate (300 mL) drying (Na2SO4), and concentration afforded 2.2 g (67%) of 1,4-dioxa-8-(6'-cyano-1'-oxa-1',2',3',4'-tetrahydronaphth-2'-yl)-8-azaspiro [4.5]decane.

### Step 2: Preparation of Trans-1,4-Dioxa-8-(6'-cyano-1'-hydroxy-1',2',3',4'-tetrahydronaphth-2'-yl)-8-azaspiro[4.5]decane

A solution of lithium aluminum hydride (19.2 mL, 0.5 M in diglyme) was cooled to -20°C under argon and treated dropwise with 2.0 grams of 1,4-dioxa-8-(6'-cyano-1'-oxa-1',2',3',4'-tetrahydronaphth-2'-yl)-8-azaspiro[4.5]decane (6.40 mmol) in 50 mL of diglyme over 25 min. The reaction was quenched with a saturated aqueous solution of sodium potassium tartrate (25 mL) and extracted with ethyl acetate. The organic portion was washed with water, and the product extracted into 1N HCl. The acid extract was washed with ether (10 x 100 mL) and basified with saturated sodium bicarbonate. The product was extracted into ethyl acetate which was washed with water and brine, dried (Na₂SO₄), and concentrated in vacuo to give crude product yield 1.51 g. This was subjected to flash chromatography (silica gel, 75% ethyl acetate in hexane) which provided 600 mg of trans-1,4-dioxa-8-(6'-cyano-1'-hydroxy-1',2',3',4'-tetrahydronaphth-2'-yl)-8-azaspiro[4.5]decane 99.6% pure (HPLC), 300 mg 98% pure and 100 mg 90% pure. (The minor component was the cis stereoisomer.)
¹H NMR (300 MHz,CDCl₃): δ 1.55-1.90 (m, 5H); 2.04-2.15 (m, 1H); 2.55 (m, 2H); 2.66 (m, 1H); 2.80-3.03 (m, 4H); 3.97 (s, 4H); 4.58 (d, J=10Hz, 1H); 7.36 (s, 1H); 8.49 (d, J=8Hz, 1H); 7.70 (d, J=8Hz, 1H).

### Step 3: Preparation of Trans-(+-)-N-[(1'-(6-Cyano-1,2,3,4-tetrahydro-1-hydroxy-2-naphthalenyl)-3,4-dihydro-spiro[(2H)-1-benzopyran-2,4'-piperidine]-4-one)-6-yl-methanesulfonamide, monohydrochloride

A solution of 600 mg (1.91 mmol) of trans-1,4-dioxa-8-(6'-cyano-1'-hydroxy-1',2',3',4'-tetrahydronaphth-2'-yl)-8-azaspiro[4.5]decane in 25 mL of 1N HCl was heated to 100°C for 1.5 hr and cooled to room temperature. The mixture was neutralized with saturated Na₂CO₃, extracted with methylene chloride (3 x 30 mL), and the organic portion dried (Na₂SO₄). Concentration in vacuo provided 520 mg (100 %) of trans-N-(6'-cyano-1'-hydroxy-1',2',3',4'-tetrahydronaphth-2'-yl)-4-piperidinone. This material was suspended in 10 mL of methanol warmed to 60°C, and stirred under argon. In a separate flask, a solution of 5-sulfonamido-2-hydroxyacetophenone (0.876 g, 3.82 mmol) and pyrrolidine (0.319 mL, 3.82 mmol) in methanol (2.0 mL) was warmed to 60°C for 10 min. This solution was transfered to the trans-N-(6'-cyano-1'-hydroxy-1',2',3',4'-tetrahydronaphth-2'-yl)-4-piperidinone and the final mixture stirred for 1.5 hrs at 60°C. Concentration in vacuo and chromatography (silica gel, 1% methanol in methylene chloride to 2.5 % methanol in methylene chloride) provided after concentration of appropriate fractions, and recrystallization from ethanol, the free base (450 mgs) of trans-(+-)-methanesulfonamide, N-[1'-(6-cyano-1,2,3,4-tetrahydro-1-hydroxy-2-naphthalenyl)-3,4-dihydro-4-oxospiro[2H-1-benzopyran-2,4'-piperidin]-6-yl-, as a pale yellow solid. This was suspended in ethanol containing 10% methanol and treated with 1.3 N HCl in isopropanol to furnish the hydrochloride salt after concentration and trituration with ethanol. This was dried in vacuo to give 470 mgs trans-(+-)-methanesulfonamide, N-[1'-(6-cyano-1,2,3,4-tetrahydro 1-hydroxy-2-naphthalenyl)-3,4-dihydro-4-oxospiro[2H-1-benzopyran-2,4'-piperidin]-6-yl-, monohydrochloride (MP = 193-196°C) which contained 0.3 mole equivalents of ethanol by NMR and 0.35 mole equivalents of water by microanalysis.

Essentially employing the procedure described in Example 375 there were obtained the compounds of Table XXIII.

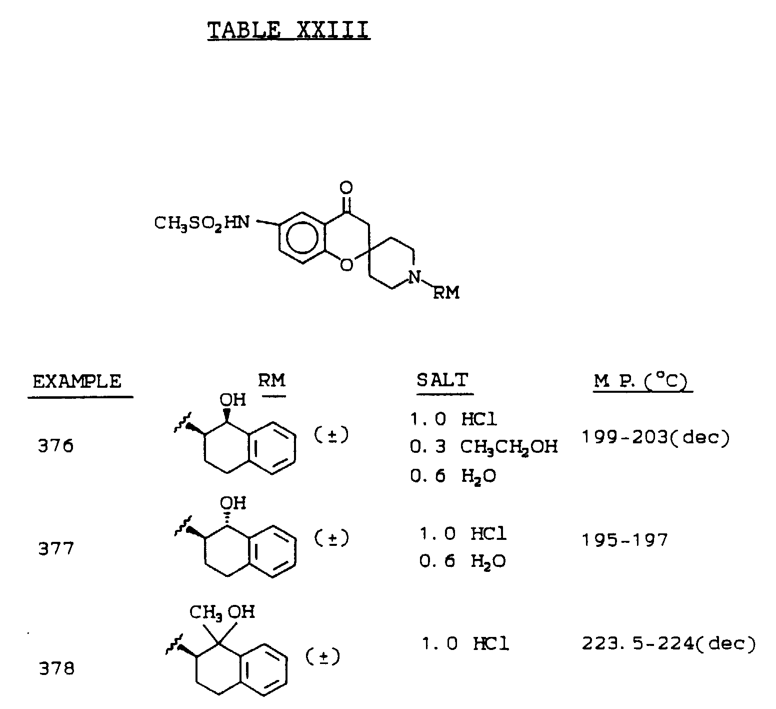

### EXAMPLE 379

### Trans(+-)-Methanesulfonamide, N-[3,4-dihydro-4-oxo-1'-(1,2,3,4-tetrahydro-3-hydroxy-2-naphthalenyl)spiro-[2H-1-benzopyran-2,4'-piperidine]-6-yl-, monohydrochloride

### Step 1: Preparation of trans-1,4-dioxa-8-(2'-hydroxy-1',2',3',4'-tetrahydronaphth-3'-yl)-8-azaspiro[4.5]decane

A stirred solution of 1,4-dihydronaphthalene (1.0 g, 7.7 mmol) in 20 mL of methylene chloride was treated with saturated aqueous sodium bicarbonate (8 mL) and meta-chloroperoxybenzoic acid (1.72 g of 80% pure, 8 mmol). After 20 hours at room temperature the mixture was diluted with methylene chloride (200 mL) and washed with saturated aqueous sodium bicarbonate, water, and brine. The organic portion was dried (Na₂SO₄), concentrated to an oil and disolved in ethanol (15 mL). 1,4-Dioxa-8-aza spiro[4.5]decane (0.99 mL, 7.7 mmol) was added and the mixture refluxed for 20 hrs. The solvent was removed in vacuo and the residue partitioned between ether and water. This was extracted with 1N HCl and the aqueous layer washed with ether (3 x 50 mL). The aqueous layer was basified with 40% NaOH and then extracted with ether (4 x 50 mL). The organic portion was dried (Na₂SO₄) and concentrate to a gummy solid which was crystrallized from ether to give 0.95 g (43%) trans-1,4-dioxa-8-(2'-hydroxy-1',2',3',4'-tetrahydronaphth-3'-yl)-8-azaspiro[4.5]decane as a white solid.
¹H NMR (CDCl₃, 300 MHz): δ 1.81 (m, 4H); 2.61 (m 2H); 2.75-2.90 (m, 6H); 3.31 (dd, 1H); 3.85 (m, 1H); 3.98 (s, 4H); 4.37 (s, 1H); 7.10 (m, 4H).

Step 2 and Step 3 are carried out in the manner of Step 7 and Step 8 for the preparaton of methanesulfonamide,N-[1'-[2-(5-benzofurazanyl)ethyl]-3,4-dihydro-4-oxospiro[2H-[1-benzopyran-2,4'-piperidin]-6-yl]-, monohydrochloride substituting trans-1,4-dioxa-8-(2'-hydroxy-1',2',3 -tetrahydronaphth-3'-yl)-8-azaspiro[4.5]decane to give trans(+-)-methanesulfonamide, N-[3,4-dihydro-4-oxo-1'-(1,2,3,4-tetrahydro-3-hydroxy-2-naphthalenyl)spiro[2H-1-benzopyran-2,4'-piperidine]-6-yl-,monohydrochloride in 40% yield, mp = 276-277°C(dec).

### EXAMPLE 380

### Trans(±)-Methanesulfonamide, N-[3,4-dihydro-4-oxo-1'-[1,2,3,4-tetrahydro-3-(2-methoxyethoxy)-2-naphthalenyl]spiro[2H-1-benzopyran-2,4'-piperidine]-6-yl-, monohydrochloride

### Step 1: Preparation of trans-1,4-dioxa-8-(2'-(2"-methoxyethoxy)-1',2',3-tetrahydronaphth-3'-yl)-8-azaspiro[4.5]decane

A solution of trans-1,4-dioxa-8-(2'-hydroxy-1',2',3,4'-tetrahydronaphth-3'-yl)-8-azaspiro[4.5]-decane (289.4 mgs,1.0 mmol) in dimethyl formamide (2.0 mL) was treated with sodium hydride( 50% by weight in oil, 225 mgs, 4.7 mmol) and stirred for 30 min. This was treated with 2-bromoethyl methyl ether (0.48 g, 3.42 mmol) and stirred at room temperature 20 hrs. This was quenched with water and extracted with ether (3 x 50 mL). The product was extracted into aqueous 1N HCl and then after basification of the aqueous layer into ether. The organic portion was dried (Na₂SO₄) and concentrated to give trans-1,4-dioxa-8-(2'-(2"-methoxy-ethoxy)-1',2',3', 4'-tetrahydronaphth-3'-yl)-8-azaspiro[4.5]decane as an oil (260 mg, 90%).
¹H NMR (300 MHz, CDCl₃): δ 1.74 (t, 4H); 2.74 (t, 4H); 2.80-3.20 (m, 5H); 3.40 (s, 3H); 3.58 (m, 1H); 3.65-3.90 (m, 3H); 3.97 (s, 4H); 7.10 (m, 4H).

Step 2 and Step 3 were carried out in the manner of Step 7 and Step 8 for the preparaton of methanesulfonamide,N-[1'-[2-(5-benzofurazanyl)ethyl]-3,4-dihydro-4-oxospiro[2H-[1-benzopyran-2,4'-piperidin]-6-yl]-,monohydrochloride, but substituting trans-1,4-dioxa-8-(2'-(2"-methoxyethoxy)-1',2',3', 4'-tetrahydronaphth-3'-yl)-8-azaspiro[4.5]decane to give trans(+-)-methanesulfonamide, N-[3,4-dihydro-4-oxo-1'-[1,2,3,4-tetrahydro-3-(2-methoxyethoxy)-2-naphthalenyl]spiro[2H-1-benzopyran-2,4'-piperidine]-6-yl-, monohydrochloride, mp = 254-256°C (dec).

### EXAMPLE 381

### Methanesulfonamide, N-[1'-(6-cyano-1,2,3,4-tetrahydro-2-naphthalenyl)-3,4-dihydro-4-oxospiro-[2H-1-benzopyran-2,4'-piperidin]-6-yl]-, monohydrochloride and separation into its enantiomers

### Step 1: Preparation of 6-cyano-2-tetralone

A flame dried 1 liter round bottom flask with argon inlet, digital thermometer, and magnetic stir bar, was charged with a 68% NaCN/alumina mixture (21.22 g, 433 mmol)(NaCN and alumina were ground together in a morter and pestle according to the procedure of Dalton et al. J. Org. Chem., 44, (24) 4443(1979)), and tetrakis(triphenylphospine) palladium(0) (10.0 g, 8.65 mmol). The flask was purged three times via vacuum with argon and a solution of 6-bromo-2-tetralone (19.46 g, 86.5 mmol) in degassed toluene (400 mL) was added. The mixture was heated to 80°C and stirred for 64 hrs at that temperature. Analysis by HPLC indicated 89% conversion (HPLC conditions, column: Waters C18 Bondapak, eluent: 80: 20 (0.1% H₃PO₄ in H₂O: CH₃CN), flow: 2 mL/min, wavelength: 220 nm, Rt: bromotetralone = 9.65 min, cyanotetralone = 3.46 min). The reaction was cooled and the mixture filtered through silica gel (elution with 60% ethyl acetate in hexane.) Concentration of the appropriate fractions gave 9.4 g (68%) of 6-cyano-2-tetralone (98.7% by HPLC).
¹H NMR (300 MHz,CDCl₃): δ 2.56 (t, J=7Hz, 2H); 3.12 (t, J=7Hz, 2H); 3.65 (s, 2H), 7.24 (d, J=9Hz, 1H); 7.52 (d, J=9Hz, 1H); 7.57 (s, 1H).

### Step 2: Preparation of 1,4-dioxa-8-(6'-cyano-1',2',3',4'-tetrahydronaphth-2'-yl)-8-azaspiro[4.5]decane hydrochloride

A 500 mL round bottom flask fitted with an argon inlet, and Dean-Stark apparatus was charged with a solution of 6-cyano-2-tetralone (8.4 g, 49.0 mmol) in toluene (150 mL). Para-toluenesulfonic acid (0.40 g, 2 mmol) and 1,4-dioxa-8-azaspiro[4.5]-decane (7.90 g, 54.0 mmol) were added and the stirred mixture heated to reflux and the water removed (4.5 hrs). The mixture was cooled, and concentrated to an oil in vacuo. The oil was disolved in anhydrous tetrahydrofuran (400 mL) and cooled to 0°C under argon. Dry HCl gas was introduced (<10°C) and a solid precipitate formed. The mixture was concentrated on a rotary evaporator to approximately 50 mL. Anhydrous tetrahydrofuran (250 mL) was added and the mixture stirred and cooled to 0°C. Sodium cyanoborohydride (4.2 g, 63 mmol) was added in two portions. The reaction was allowed to warm gradually to room temperature and stirred 16 hrs. This was quenched with 1N aqueous sodium hydroxide (160 mL) and stirred for 0.5 hr (pH=13.5). The layers were separated and the aqueous layer was washed with diethyl ether (2 x 200 mL). The combined organic layers were combined and concentrated on a rotary evaporator to an oil which was disolved into methylene chloride (200 mL). This was washed with saturated aqueous NaCl and then stirred with 1N HCl (100 mL) for 15 minutes. The layers were seperated and the aqueous was extracted with methylene chloride (2 x 100 mL). The combine methylene chloride extract was concentrated on a rotary evaporator and ethyl acetate (200 mL) was added during the distillation to effect crystallization. The resultant slurry was cooled to 0°C for 3 hrs and the 1,4-dioxa-8-(6'-cyano-1',2',3',4'-tetrahydronaphth-2'-yl)-8-azaspiro[4.5]decane hydrochloride was collected by filtration (8.70 g, 53%). A second crop was obtained upon concentration of the mother liqours and dilution with diethyl ether (3.6 g, 22%). The combined solids were 98% pure by HPLC.

### Step 3: Resolution of 1,4-dioxa-8-(6'-cyano-1',2',3',4'-tetrahydronaphth-2'-yl)-8-azaspiro[4.5]decane

To a solution of 5.00 g (0.0168 mol) of racemic 1-4-dioxa-8-(6'-cyano-1',2',3',4'-tetrahydronaphth-2'-yl)-8-azaspiro[4.5]decane in 500 mL of boiling absolute ethanol was added 6.47 g (0.01676 mol) of di-p-toluoyl-D-tartaric acid. The solution was concentrated by boiling to 450 mL and allowed to stand overnight at room temperature. The product that crystallized was removed by filtration and was washed with ethanol to give 4.45 of a salt, A. The filtrate and washings were combined and evaporated to give B.

The solid A was recrystallized three times from absolute thanol to yield 2.82 g of salt having [α]₅₈₉ = +106.1° (c = 1.151; pyridine). This salt was converted to the free base using sodium bicarbonate solution and extracting into ethyl acetate. The ethyl acetate phase was dried over magnesium sulfate, filtered, and concentrated in vacuo to give 1.15 g of the (+)-enantiomer of the title compound: mp = 124-126°C; [α]₅₈₉ = +55.4° (c = 1.605; CHCl₃).

The solid B was converted to the free base as described above to give 2.84 g of material. This solid (0.00938 mol) was dissolved in 275 mL of boiling ethanol and was treated with 3.794 g (0.00938 mol) of di-p-toluoyl-L-tartaric acid monohydrate.

The salt that crystallized on cooling was collected by filtration and was washed with ethanol to give 4.47 g of material having [α]₅₈₉ = -102.6° (c = 1.131; pyridine). Recrystallization of this material from ethanol gave 3.71 g of salt, [α]₅₈₉ = -106.1° (c = 0.985, pyridine), and further recrystallization from ethanol gave 3.24 g of salt having essentially no change in rotation, [α]₅₈₉ = -105.9° (c = 1.472, pyridine). This salt was converted to the free base as described above to give 1.34 g of the (-)-enantiomer of the title compound: mp = 124-126°C; [α]₅₈₉ = -54.5° (c = 1.954; CHCl₃).

### Step 4: Preparation of N-(6'-cyano-1',2',3',4'-tetrahydronaphth-2'-yl)piperidin-4-one

A solution of 1,4-dioxa-8-(6'-cyano-1',2',3',4'-tetrahydronaphth-2'-yl)-8-azaspiro[4.5]decane hydrochloride (10.0 g, 30.0 mmol) or the resolved free base was disolved on 1N HCl (100 mL). This was stirred and heated to 100°C under an argon atmosphere for 1.5 hrs. The solution was cooled in an ice bath to 25°C and methylene chloride added (200 mL). The mixture was stirred and basified to pH 9.0 with saturated aqueous sodium carbonate. The organic layer was separated and the aqueous extracted with methylene chloride (2 x 50 mL). The combined organic extract was dried (Na2SO4), and concentrated to a foam to give 7.5 g (99%) of N-(6'-cyano-1',2',3',4'-tetrahydronaphth-2'-yl)piperidin-4-one (98% by HPLC, HPLC conditions, column: Waters C18 Bondapak, eluent: 85:15 (0.1% H₃PO₄ in H₂O: CH₃CN), flow: 2 mL/min, wavelength: 220 nm, Rt: ketal=6.20 min, ketone=2.1 min).

### Step 5: Preparation of Methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-, monohydrochloride

A solution of 2-hydroxy-5-methanesulfonamido acetophenone (5.58 g, 24.3 mmol), and pyrolidine (2.05 mL, 24.3 mmol) in methanol (30 mL) was stirred at 60°C for 10 min. N-(6'-cyano-1',2',3',4'-tetrahydronaphth-2'-yl)piperidin-4-one (3.09 g, 12.2 mmol) was added in one portion and the mixture stirred for 1.5 hrs at 60°C. The reaction was concentrated to an oil in vacuo and flash chromatographed (silica gel, ethyl acetate) to afford the product in appropriate fractions which were combined and concentrated to 350 mL and treated with 1.3N HCl in isopropyl alcohol. The precipitate was stirred 2hrs, filtered, and dried in vacuo (60°C, 0.1 torr) to give 3.91 g (64%) of Methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-, monohydrochloride, mp = 250-252°C.
Starting from the (+) 1,4-dioxa-8-(6'-cyano-1',2', 3',4'-tetrahydronaphth-2'-yl)-8-azaspiro[4.5]decane the (+) enantiomer of methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-. monohydrochloride was obtained. [α]_{D} = + 40.7° (c=0.17 MeOH); mp = 262-264°C (dec).
Starting from the (-) 1,4-dioxa-8-(6'-cyano-1',2', 3',4'-tetrahydronaphth-2'-yl)-8-azaspiro[4.5]decane the (-) enantiomer of methanesulfonamide, N-[1'-(6-cyano-1,2,3,4-tetrahydro-2-naphthalenyl)-3,4-dihydro-4 -oxospiro[2H-1-benzopyran-2,4'-piperidin]-6-yl]-, monohydrochloride was obtained. [α]_{D} = - 41.36° (c=0.191 MeOH); mp = 263-265°C (dec).

### EXAMPLE 382

### (+) Methanesulfonamide, N-[1'-(6-cyano-1,2,3,4-tetrahydro-2-napthalenyl)-3,4-dihydro-4-oxospiro[2H-1-benzopyran-2,4'-piperidin]-6-yl]-, monohydrochloride [Alternate Method]

### Step 1: Preparation of 6-Bromo-2-tetralone

A single neck 3 liter round bottom flask under an Ar atmosphere was charged with 4-bromo-phenyl acetic acid (250.0 g, 1.15 m), methylene chloride (1.5 L), and dimethylformamide (0.5 mL). This magnetically stirred solution was cooled to 0°C and treated dropwise with oxalyl chloride (156 mL, 1.74 m). The reaction was allowed to reach room temperature and stirred 16 hrs. The reaction was concentrated on a rotary evaporator to approximately 1 L of volume. A separate dry 5 liter 3 neck round bottom flask under Ar, fitted with gas inlet tube, overhead stirrer, and digital thermometer was charged with methylene chloride (1.5 L) and AlCl₃ (312.0 g, 2.34 m). This suspension was cooled to 0°C and stirred while the above solution of acid chloride was added to it slowly via cannula. When the addition was complete, ethylene gas was introduced for 1-2 hrs to the vigorously stirred suspension while maintaining the internal temperature at 15°C. Upon completion by HPLC, the reaction was warmed to room temperature and stirred for 0.5 hrs. The mixture was recooled to 0°C and cautiously quenched slowly with water (1.5 L). The layers were separated, and the aqueous one washed with 500 mL of methylene chloride. The organic portion was washed with 2N aqueous HCl (2 x 800 mL), brine (400 mL), and saturated aqueous NaHCO₃ (2 x 800 mL). Each aqueous extract was washed with the same 500 mL methylene chloride extract from above. The methylene chloride extracts were combined, dried (Na₂SO₄), filtered, and concentrated to approximately 500 mL of volume. This was then added to 5.0 L of hexane warmed to 50°C. The methylene chloride was distilled off and the hot solution decanted from an insoluble brown tar. The solution was allowed to cool to 25°C and placed in the freezer overnight. The precipitate was collected and washed with hexane (200 mL), and dried in vacuo to give 229.0 g of the compound as a pale yellow solid (88%).

### Step 2: Preparation of (±) 1,4-dioxa-8-(6'-bromo-1',2',3',4'-tetrahydronaphth-2'-yl)-8-azaspiro[4.5]decane

A 3 L round bottom flask fitted with an argon inlet, and Deak-Stark apparatus was charged with a solution of 6-bromo-2-tetralone (100.0 g, 445 mmol) in toluene (2.0 L). Para-toluenesulfonic acid (0.50 g) and 1,4-dioxa-8-azaspiro[4,5]-decane (81.5 g, 489 mmol) were added and the stirred mixture heated to reflux and the water removed (4.5 hrs). The mixture was cooled, and concentrated to an oil in vacuo. The oil was dissolved in anhydrous tetrahydrofuran (1.5 L) and cooled to 0°C under argon. Dry HCl gas was introduced (at below 5°C) and a solid precipitate formed. Sodium cyanoborohydride (36.3 g, 578 mmol) was added in four portions. The reaction was allowed to warm gradually to room temperature and stirred 16 hrs. This was quenched with 1N aqueous sodium hydroxide (500 mL) and stirred for 0.5 hr (pH=13.5). The mixture was concentrated on a rotary evaporator to remove THF, and diluted with 1N NaOH (1.1 L) and diethyl ether (1.5 L). This mixture was stirred 15 min and the layers were separated and the aqueous layer was washed with diethyl ether (2 x 200 mL). The organic layers were combined, washed eith water (2 x 500 mL) and saturated aqueous NaCl (2 x 250 mL) and then with 1N HCl (1 x 1.0 L, 2 x 500 mL). The acid extracts were combined, stirred with methylene chloride (1.0L), and basified with 40% aq. NaOH (pH=10). The layers were separated, and the aqueous extracted with methylene chloride (500 mL). The methylene chloride extracts were combined, dried (Na₂SO₄), and concentrated to an oil. The oil was flushed with toluene (2 x 400 mL) and dried in vacuo to give the title compound as a solid on standing (128.8 g, 87%) which was greater than 98% pure by HPLC and used in the next step without purification. Note: The amount of of excess HCl gas present (pH=3-4, THF suspension on wet pH paper) critically determines the yield free amine. Additional HCl may be added during the introduction of the cyano borohydride. In runs in which the pH was not adjusted properly the yield was reduced to 50%; the balance being a borane complex which was isolated from the ether layers. This borane complex could be quantitatively converted to the free amino by heating in 40% aq NaOH/ethylene glycol (1:1) at 100°C.

### Step 3(a): Preparation of Phenyl cyanate

The title compound was prepared by a modification of the procedure described in Organic Syntheses, 61, 35 (1983). A 3-necked, 2 L R.B. flask, equipped with a 500 ml pressure equalized dropping funnel, a mechanicl stirrer and a thermometer, was charged with water and coled in an ice-salt bath. Cyanogen bromide (189.1 g, 1.78 mol) was added and the mixture was stirred for 5 min. Phenol (160.0 g., 1.7 mol) in carbon tetrachloride (535 ml) was added in one portion. The mixture was stirred vigorously while triethylamine (236.9 ml, 172.0 g, 1.7 mol) was added dropwise at a rate such that the reaction temperature did not exceed 5°C (total addition time = 45 min.). The mixture was stirred for a further 15 min. then transferred to a 2 L separatory funnel. The organic layer was separated and the aqueous layer was extracted with carbon tetrachloride (2 x 90 ml). The combined organic layers were washed with water (3 x 90 ml) then dried by stirring with phosphorus pentoxide (10 g) for 15 min. The mixture was filtered and the solvent was evaporated under reduced pressure (water aspirator) at 20°C to give a yellow oil. Polyphosphate ester (Y. Kanaoka, et al., Chem. Pharm. Bull., 13, 1065-1072 (1965)) (0.2 ml) was added and the mixture was distilled under reduced pressure through a 15 cm vigreux column to give phenyl cyanate (165.8 g, 82%) as a colorless oil, b.p. 79-82°C (16 mmHg)¹. The product was stored under nitrogen at -10°C (freezes).
¹H NMR (300 MHz, CDCl₃) δ: 7.49-7.30 (5H, m).

### Step 3 (b): Preparation of(±)1,4-Dioxa-8-(6'-cyano-1',2',3',4',-tetrahydronapath-2yl)-8-azaspiro[4.5]decane

(±)1,4-Dioxa-8-(6'-bromo-1',2',3',4-tetrahydronaphth-2-yl)-8-azaspiro[4.5]decane (70.4 g, 0.2 mol) under nitrogen in a 1 L R.B. flask was dissolved in anhydrous THF (600 ml, distilled from Na/benzophenone) and cooled to -75°C. Phenyl cyanate (26.06 ml, 28.5 g, 0.24 mol) dissolved in anhydrous THF (400 ml) under nitrogen in a 2 L R.B. flask equipped with a digital thermometer was cooled to -75°C. n-Butyl lithium (1.6M in hexane, 137.5 mL, 0.22 mmol) was added to over 5 min. to the bromide solution. Further n-butyl lithium (12.5 mL, 0.02 mmol) was added to the phenyl cyanate solution. After 5 min., the lithiated bromide solution was added over 5 min., via cannual, to the phenyl cyanate solution (reaction temperature rises to -35°C). The mixture was stirred and cooled to -75°C for 30 min. then the cooling bath was removed and HCl-H₂O (1M, 200 mL) was added with vigorous stirring. The mixture was warmed to room temperature, diluted with HCl-H₂O (1M, 1800 mL) and washed with ether (2 x 1000 mL.). Methylene chloride (1000 mL) was added and the mixture was stirred and cooled in ice during the addition of aqueous sodium hudroxide 10 M, 180 mL). The layers were separated, and the aqueous layer was extracted with methylene chloride (500 mL). The combined organic layers were dried (Na₂SO₄), and the solvent was evaporated under reduced pressure to give crude (±) 1,4-dioxa-8-(6'-cyano-1',2',3',4'-tetrahydronaphth-2'yl)-8-azaspiro[4.5]decane as a tan solid (56.2 g). Crude (±)1,4-dioxa-8-(6'-cyano-1',2',3',4'-tetrahydronaphth-2'yl)-8-azaspiro-[4.5]-decane in three batches (56.4 g, 56.2 g, 27.7 g; total 140.3 g) were separately dissolved in refluxing methyl-cyclohexane (1000 mL each) and combined by decanting into a 5 L, 4-necked flask equipped with a mechanical stirrer, thermometer, reflux condenser and a stopper. The mixture was heated to reflux (clear solution formed), then allowed to cool with stirring to room temperature, then to 5°C. The mixture was stored at -15°C for 15 hr. The solid was collected by filtration, washed with cold methylcyclohexane (2 X 150 ml) and dried in vacuo at room temperature to give the spirodecane as a pale yellow solid (121.3 g), .m.p. 136-138°C. Purity = 99.3%

### Step 4: Resolution of 1,4-dioxa-8-(6'-cyano-1'2',3'4'-tetrahydronaphth-2'-yl)-8-azaspiro[4.5]decane

A 12 L round bottom flask fitted with a reflux condenser, digital thermometer, and overhead stirrer was charged with absolute ethanol (10.6 L) and 1,4-dioxa-8-(6'-cyano-1',2',3',4'-tetrahydronaphth-2'yl)-8-azaspiro[4.5]decane 120.0 g, 402 mmol). The mixture was warmed to 65°C to give a clear solution, and 97% di-p-toluoyl-L-tartaric acid monohydrate (167.7 g, 402 mmol) was added. The resulting clear solution was seeded with this salt and allowed to cool to room temperature with stirring overnight. The precipitate was collected by filtratio and washed with absolute ethanol (600 mL). The solid was dried in vacuo to a solid and converted to free base in a stirred mixture of ethyl acetate (2.0 L) and saturated aqueous NaHCO₃(3.0L). The layers were separated, and the aqueous one washed with ethyl acetate (2 x 500 mL). The organic layers were combined, washed with brine (2 x 200 mL), dried (Na₂SO₄), and concentrated to 69.4 g of a solid (59% yield). The solid free base (69.4 g, 233 mmol) was dissolved in absolute ethanol (4.25 L) at 60°C and 97% di-p-toluoyl-D-tartaric acid (92.64 g, 233 mmol) was added. The resulting clear solution was seeded with a sample of this salt and allowed to cool to room temperature overnight. The precipitate which formed was collected, washed with absolute ethanol (800 mL), and dried in vacuo at 40°C to give 122.5 g (44.5%). This + D salt was completely dissolved in absolute ethanol (8.0L) at reflux and concentrated to approximately 7.0 L of volume by distillation at 1 atmosphere. The solution was seeded and cooled toroom temperature overnight. The solid was collected, washed with absolute ethanol (800 mL) dried in vacuo to give 100.9 g [α]_{D}= +104.7° (c= 1.0 pyridine)) (36.7%). This salt was dissolved in hot absolute ethanol (8.3L), concentrated at 1 atmosphere to 3.1 L of total volume, seeded and cooled to room temperature overnight. This solid was collected, washed with absolute ethanol (900 mL) and dried in vacuo to give 89.7 g [α]_{D}=105.4° (c=1.0 pyridine)) (32.6%). A further crystallization from 7.0 L hot ethanol concentrated to 2.9 L volume gave 74.3 g ([α]_{D}= +105.4° (c=1.0 pyridine)) (32.6%). A further recrystallization from 7.0 L hot ethanol concentrated to 2.9 L volume gave 74.3 g [α]_{D}=104.9° (c=1.0 pyridine)) (27% yield). The free base was obtained by treating a stirred mixture of saturated aq. NaHCO₃ (250 mL) and methylene chloride (250 mL) with 1,4-dioxa-8-(6'-cyano 1',2',3',4'-tetrahydronaphth-2'yl)-8-azaspiro]4.5]decane di-p-toluoyl-D-tartaric acid salt (10.0 g, 33.5 mmol). After 15 min the layers were separated, the aqueous washed with methylene chloride (100 mL), and the combined organics washed with saturated aq. NaHCO₃ (100 mL), dried Na₂SO₄) and concentrated to give 4.30 g (99%) of a solid. A sample of free base was analyzed by chiral shift reagent proton NMR to be 99.8% pure (+) enantiomer.

### Step 5: Preparation of N-(6'-Cyano-1',2',3',4'-tetrahydronaphth-2'-yl)piperidin-4-one

A solution of (+) 1,4-dioxa-8-(6'-cyano-1',2',3',4'-tetrahydronaphth-2'yl)-8-azaspiro[4.5]-decane (10.0 g, 33.5 mmol) was dissolved in 1N HCl (100 mL). This was stirred and heated to 100°C under an argon atmosphere for 1.5 hrs. the solution was cooled in an ice bath to 25°C and methylene chloride added (200 mL). The mixture was stirred and basified to pH=9.0 with saturated aqueous sodium carbonate. The organic layer was separated and the aqueous extracted with methylene chloride(2X 50 mL). The combined organic extract was dried (Na₂SO₄), and concentrated to a foam to give 7.5g(99%) of N-(6'-cyano-1',2',3',4'-tetrahydronaphth-2'-yl)-piperdin-4-one (98% by HPLC).

### Step 6: Methanesulfonamide, N-[1'-(6-cyano-1, 2,3,4-tetrahydro-2-naphthalenyl)-3,4-dihydro-4-oxospiro[2H-1-benzopyran-2,4'-piperidin]-6-yl]-, monohydrochloride

A solution of 2-hydroxy-5-methanesulfonamidoacetophenone (26.98 g, 117.7 mmol), and pyrrolidine (9.8 mL, 117.7 mmol) in methanol (480 mL) was stirred at 25 °C for 10 min. (+)-N-(6'-Cyano-1',2'3',4'-tetrahydronaphth-2'-yl)-piperidin-4-one (20.0 g, 78.4 mmol) was added in one portion and the mixture stirred for 24 hrs at 25 °C. The reaction was concentrated to an oil in vacuo and flash chromatographed (silica gel, ethyl acetate) to afford the product in appropriate fractions which were combined and concentrated to a foam. This was crystallized from isopropyl alcohol (525 mL) to give a solid which was collected by filtration, washed with IPA (50 mL) and dried in vacuo (30.8 g). This was crystallized from isopropyl alcohol (525 mL) to give a solid which was collected by filtration, washed with IPA (50 mL) and dried in vacuo (30.8 g). This was dissolved in ethyl acetate (1.5L) and treated with 1.3N HCl in IPA (55 mL). The precipitate was stirred 20 hrs, filtered and dried in vacuo (60°C, 0.1 torr) to give 32.3 g (84%) of (+) Methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-, [α]_{D} = +40.7 (c=0.17, MeOH).

### EXAMPLE 383

### Resolution of 6-Methanesulfonamido-3,4-dihydro-1'-(1,2,3,4-tetrahydronaphth-2-yl)spiro[(2H)-1-benzopyran-2,4'-piperidine]-4-one

To a warm solution of 2.112 g (0.00479 mole) of racemic 6-methanesulfonamido-3,4-dihydro-1'-(1,2,3,4-tetrahydronaphth-2-yl)spiro[(2H)-1-benzopyran-2,4'-piperidine]-4-one in 50 mL of acetone was added a solution of 1.852 g di-p-toluoyl-D-tartaric acid (0.00479 mol) in 20 mL of acetone. After standing at room temperature for 48 hours, a hard solid, A, had precipitated out. The solvent was decanted, and the solid was washed four times with acetone. The original decanted solvent and washings were combined and evaporated to afford B.

The solid A was recrystallized from absolute methanol four times to afford 0.636 g of salt having mp = 203-204° (dec.) and [α]₅₈₉^{25°} = +85.8° (c = 0.975; pyridine). This salt was converted to the free base form using sodium bicarbonate solution, and the hydrochloride salt of the amine was prepared and crystallized from isopropyl alcohol to give 0.303 g of the (+)-enantiomer of the title compound; mp 283-285°C (dec.); [α]₅₈₉ = +36.9° (c = 0.449; DMF).

| Anal. Calc'd. for C₂₄H₂₈N₂O₄S•HCl: | | | |
|---|---|---|---|
| | C, 60.43; | H, 6.13; | N, 5.87. |
| Found | C, 60.03; | H. 6.25; | N, 5.61. |

The solid B was treated with saturated sodium bicarbonate solution and extracted into ethyl acetate. The ethyl acetate phase was dried over magnesium sulfate, filtered, and concentrated in vacuo to give 1.112 g of material. This solid (0.00252 mol) was dissolved in 10 mL of warm acetone and was treated with a solution of 1.019 g (0.00252 mol) of di-p-toluoyl-L-tartaric acid in 15 mL of acetone. After standing overnight, the precipitate that formed was removed by filtration and was washed with acetone. Two recrystallizations from methanol gave 0.803 g of salt having mp = 203-204° (dec.) and [α]₅₈₉ = -85.4° (c = 0.830; pyridine.) This salt was converted to the free base form using sodium bicarbonate solution, and the hydrochloride salt of the amine was prepared and crystallized from isopropyl alcohol to give 0.146 g of the (-)-enantiomer of the title compound; mp 284-286°C (dec.); [α]₅₈₉ = -37.7° (c = 0.779; DMF).

| Anal. Calc'd. for C₂₄H₂₈N₂O₄S₈•HCl: | | | |
|---|---|---|---|
| | C, 60.43; | H, 6.13; | N, 5.87. |
| Found | C, 60.28; | H, 6.17; | N. 5.78. |

Essentially employing the procedures described in Example 381, but substituting for the 6-cyano-2-tetralone used therein, the appropriate 2-tetralone were produced the spiropiperidinones described in Table XXIV.

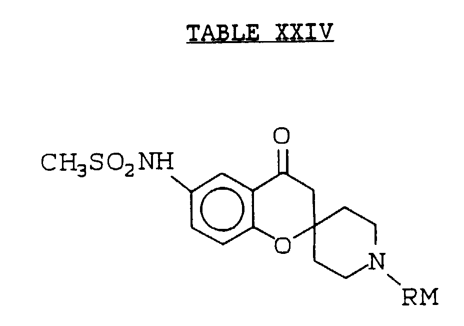

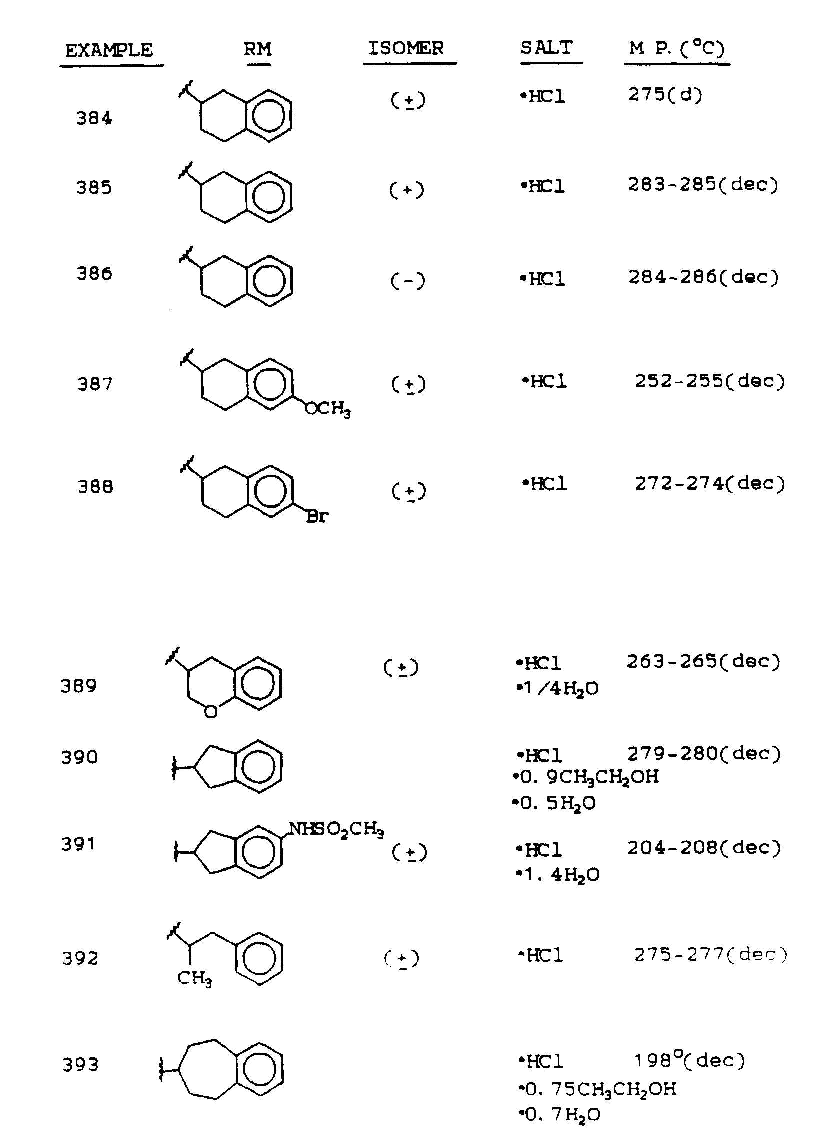

### EXAMPLE 404

### 6-Methanesulfonamide-1'-hexyl-4-hydroxy-3,4-dihydrospiro[(2H)-1-benzopyran-2,4'-piperidine]

A stirred solution of 6-methanesulfonamido-1'-hexyl-3,4-dihydrospiro[(2H)-1-benzopyran-2,4'-piperidine]-4-one(0.915 g, 2.32 mmol) in 50 ml of absolute ethanol was chilled to 0°C, blanketed in N₂, and was treated with NaBH₄ (0.878 g, 23.2 mmol). The resulting solution was allowed to stir to 25°C over 18h. The clear solution was concentrated in vacuo, and the residue was dissolved in a total of 125 ml of ethyl acetate and was washed with 50 ml of water, followed by washing with 25 ml of saturated aq. NaCl. The organic phase was dried (MgSO₄), filtered, and concentrated in vacuo to clear oil to give 0.710 g (88.3%) of the desired product. The hydrochloride salt was generated in ethanol using 1 equivalent of 3.22M ethanolic HCl to give a dense crystalline solid, mp = 249-250°C (dec.).

| Anal. Calcd. For C₂₀H₃₃ClN₂O₄S: | | | |
|---|---|---|---|
| | C, 55.48; | H, 7.68; | N, 6.47. |
| Found | C, 55.69; | H, 7.80; | N, 6.41. |

### EXAMPLE 416

### 3,4-Dihydro-1'-(1-hexyl)-6-methanesulfonamido-spiro-[(2H)-1-benzopyran-2,4'-piperidine]-4-N-acetamide Hydrochloride

To a solution of (3,4-dihydro-1'-(1-hexyl)-6-methanesulfonamido-spiro[(2H)-1-benzopyran-2,4'-piperidine]-4-ol (0.8g) in acetonitrile (16 ml) at -15°C and under argon was added sulfuric acid (0.6 ml). The reaction was aged at -15°C for 1/2 hour and allowed to warm to 25°C over 1 hour. The solution was quenched into ice/water (50 ml), concentrated to remove acetonitrile and 1N NaOH was added to adjust pH to 9.0. The product was extracted into methylene chloride, dried over sodium sulfate, concentrated and crystallized from ethanol as the hydrochloride salt to yield 0.65 g of the title compound. mp > 300°C (dec).

### EXAMPLE 417

### 3,4-Dihydro-1'-(1-hexyl)-6-methanesulfonamido-spiro-[(2H)-1-benzopyran-2,4'-piperidine]-4-N-ethylamine Dihydrochloride

To a solution of 3,4-dihydro-1'-(1-hexyl)-6-methanesulfonamido-spiro[(2H)-1-benzopyran-2,4'-piperidine]-4-N-acetamide (0.20 g) in tetrahydrofuran at 0°C under argon was added a 1.0M solution of lithium aluminum hydride in THF (1.0 ml). The reaction was warmed to 50°C for 3 hours and then quenched by addition of ice/water (20 ml). The reaction was diluted with methylene chloride (50 ml), filtered and the organic layer removed. The organic layer was washed with brine (20 ml), dried over sodium sulfate and concentrated to an oil. The oil was chromotographed on silica (methylene chloride/methanol/ammonium hydroxide 90/10/2) to give 130 mg foam upon concentration. The foam was crystallized from ethanol as its dihydrochloride salt to give the title compound. mp 220-222°C.

### EXAMPLE 418

### 3,4-Dihydro-1'[(6-cyano-1,2,3,4-tetrahydronaphthalene)-2-yl]-6-methanesulfonamido-spiro[(2H)-1-benzopyran-2,4'-piperidinel-4-N-acetamide Hydrochloride

The title compound was prepared in a manner similar to that for the preparation of 3,4-dihydro-1'-hexyl)-6-methanesulfonamido-spiro-[(2H)-1-benzopyran-2,4'-piperidine]-4-N-acetamide hydrochloride by substituting the appropriate alcohol prepared in Example 479 for the starting alcohol in the preparation of Example 416 to give the title compound as the hydrochloride, mp 317°C (dec.).

### EXAMPLE 419

### 3,4-Dihydro-1'-(benzofurazan-5-methyl)-6-methanesulfonamido-spiro[(2H)-1-benzopyran-2,4'-piperidine]-4-one hydrochloride

3,4-Dihydro-6-methanesulfonamido-spiro[(2H)-1 -benzo-pyran-2,4'-piperidine]-4-one hydrochloride was alkylated with 5-(methanesulfonyloxymethyl)benzofurazan according to the method described in Example 86, Step B to give the hydrochloride as a white solid, m.p. >285°C.

| Anal. Calc'd. for C₂₁H₂₃ClN₄O₅S•0.33H₂O: | | | |
|---|---|---|---|
| | C, 52.01; | H, 4.92; | N, 11.55. |
| Found | C, 52.09; | H, 4.94; | N, 11.35. |

### EXAMPLE 420 (INTERMEDIATE COMPOUNDS)

### Preparation of Alcohols

### (±)-1-Acetyl-2,3-dihydro-α-methyl-1H-indole-5-methanol

1-Acetyl-2,3-dihydro-1H-indole (16.1 g, 0.1 mol) in 1,2-dichloroethane (60 ml) was added dropwise to refluxing mixture of acetyl chloride (21.33 ml, 23.55 g, 0.3 mol) and aluminum chloride (40.0 g, 0.3 mol) in 1,2-dichloroethane (40 ml). The mixture was heated under reflux for 2 hours, cooled and poured onto crushed ice (1 Kg). The mixture was extracted with dichloromethane (3 x 500 ml) and the combined organic fractions were dried (Na₂SO₄) and evaporated under reduced pressure. Ethanol (400 ml) was added and the mixture was cooled in a water bath. Sodium borohydride (7.57 g, 0.2 mol) was added and the mixture was stirred for 1 hour. Water (100 ml) was added and the ethanol was evaporated under reduced pressure. Water (500 ml) was added and the mixture was extracted with dichloromethane (3 x 500 ml) and the combined organic fractions were dried (Na₂SO₄) and evaporated under reduced pressure. The residue was recrystallized from ethyl acetate to give the alcohol as an off-white solid (18.43 g, 90%).
¹H NMR (CDCl₃): δ 8.14 (1H, d, J=8.2 Hz), 7.23 (1H, s), 7.16 (1H, d, J=8.2 Hz), 4.86 (1H, br q, J=6.4 Hz), 4.06 (2H, t, J 8.4 Hz), 3.19 (2H, t, J=8.4 Hz), 2.22 (3H, s), 1.9 (1H, br s), 1.48 (3H, d, J=6.4 Hz).

### 1-Acetyl-2,3-dihydro-5-ethenyl-1H-indole

1-Acetyl-2,3-dihydro-α-methyl-1H-indole-5-ethanol (10.25 g, 50 mmol) and p-toluenesulfonic acid (monohydrate, 190 mg, 1 mmol) in toluene (250 ml) were heated under reflux with azeotropic removal of water for 20 min. The mixture was cooled, diluted with ethyl acetate (1000 ml), washed with aqueous sodium hydrogen carbonate (saturated, 500 ml) and brine (400 ml), dried (Na₂SO₄) and evaporated under reduced pressure. The residue was purified by flash column chromatography on silica gel, eluting with ethyl acetate, to give the indole as a white solid (6.55 g, 70%).
¹H NMR (CDCl₃): δ 8.14 (1H, d, J=8.3 Hz), 7.24 (2H, m), 6.66 (1H, dd, J=17.6, 11.0 Hz), 5.65 (1H, d, J 17.6 Hz), 5.15 (1H, d, J=11.0 Hz), 4.66 (2H, t, J=8.4 Hz), 3.19 (2H, t, J=8.4 Hz), 2.22 (3H, s).

### 1-Acetyl-2,3-dihydro-1H-indole-5-ethanol

Borane-tetrahydrofuran complex (1.0M in THF, 20 ml, 20 mmol) was added dropwise to an ice cooled solution of 1-acetyl-2,3-dihydro-5-ethenyl-1H-indole (5.61 g, 30 mmol) in THF (150 ml). The mixture was stirred at 0°C for 30 minute then at room temperature for 1 hr. Trimethylamine N-oxide dihydrate (8.88 g, 80 mmol) was added and the mixture was heated under reflux for 3 hr, cooled and poured into water (400 ml) and aqueous HCl (6N, 10 ml). The mixture was extracted with dichloromethane (3 x 400 ml) and the combined organic fractions were dried (Na₂SO₄) and evaporated under reduced pressure. The residue was purified by flash column chromatography on silica gel, eluting with ethyl acetate, to give the alcohol as a white solid (3.41 g, 55%).
¹H NMR (CDCl₃): δ 8.13 (1H, d, J=8.7 Hz), 7.06 (2H, m), 4.04 (2H, t, J=8.4 Hz), 3.83 (2H, t, J=6.5 Hz), 3.18 (2H, t, J=8.4 Hz), 2.82 (2H, t, J=6.5 Hz), 2.21 (3H, s), 1.61 (1H, br s).

### EXAMPLE 421 (INTERMEDIATE COMPOUNDS)

### Preparation of Methanesulfonates

The following methanesulfonates were synthesized from the corresponding alcohols by the method described in Example 89, Step C:
[2-(Methanesulfonyloxy)ethyl]cyclohexane;
   ¹H NMR (CDCl₃): δ 4.27 (2H, t, J=6.8 Hz), 3.01 (3H, s), and 1.8-0.8 (13H, m).
2-[2-(Methanesulfonyloxy)ethyl]thiophene;
   ¹H NMR (CDCl₃): δ 7.20 (1H, d, J=5.1 Hz), 6.97 (1H, dd, J=5.1, 3.4 Hz), 6.92 (1H, d, J=3.4 Hz), 4.42 (2H, t, J=6.7 Hz), 3.28 (2H, t, J=6.7 Hz), 2.93 (3H, s).
3-[2-(Methanesulfonyloxy)ethyl]thiophene;
   ¹H NMR (CDCl₃): δ 7.31 (1H, dd, J=4.9, 3.0 Hz), 7.10 (1H, br s), 6.99 (1H, dd, J=4.9, 1.2 Hz), 4.42 (2H, t, J=6.7 Hz), 3.10 (2H, t, J=6.7 Hz), 2.89 (3H, s).
1-[2-(Methanesulfonyloxy)ethyl]-2-imidazolidinone;
   ¹H NMR (CDCl₃): δ 4.8 (1H, br s), 4.36 (2H, t, J=5.1 Hz), 3.59-3.45 (6H, m), 3.05 (3H, s).
2-[2-(Methanesulfonyloxy)ethyl]furan;
   ¹H NMR (CDCl₃): δ 7.35 (1H, d, J=1.8 Hz), 6.32 (1H, dd, J=3.0, 1.8 Hz), 6.16 (1H, d, J=3.0 Hz), 4.46 (2H, t, J=6.6 Hz), 3.10 (2H, t, J=6.6 Hz), 2.92 (3H, s).
3-[2-(Methanesulfonyloxy)ethyl]furan;
   ¹H NMR (CDCl₃): δ 7.39 (1H, s), 7.33 (1H, s), 6.33 (1H, s), 4.32 (2H, t, J=6.7 Hz), 2.96 (3H, s), 2.90 (2H, t, J=6.7 Hz).
2,3-Dihydro-5-[2-(methanesulfonyloxy)ethyl]benzofuran;
   ¹H NMR (CDCl₃): δ 7.06 (1H, s), 6.95 (1H, d, J=8.1 Hz), 6.72 (1H, d, J 8.1 Hz), 4.56 (2H, t, J=8.7 Hz), 4.36 (2H, t, J=7.0 Hz), 3.19 (2H, t, J=8.7 Hz), 2.98 (2H, t, J=7.0 Hz), 2.88 (3H, s).
1-Acetyl-2,3-dihydro-5-[2-(methanesulfonyloxy)ethyl]-indole;
   ¹H NMR (CDCl₃): δ 8.14 (1H, d, J=8.8 Hz), 7.06 (2H, m), 4.38 (2H, t, J 6.8 Hz), 4.06 (2H, t, J=8.5 Hz), 3.19 (2H, t, J=8.5 Hz), 3.00 (2H, t, J=6.8 Hz), 2.88 (3H, s), 2.22 (3H, s).
4-[2-(Methanesulfonyloxy)ethyl]benzonitrile;
   ¹H NMR (CDCl₃): δ 7.64 (2H, d, J=8.3 Hz), 7.37 (2H, d, J=8.3 Hz), 4.45 (2H, t, J=6.6 Hz), 3.13 (2H, t, J=6.6 Hz), 2.92 (3H, s).

### EXAMPLE 422 (INTERMEDIATE COMPOUNDS)

### Preparation of Alcohols

### 1-Methanesulfonyloxy-6-methylheptan-6-ol

Methylmagnesium chloride (2.8M in THF, 21 ml, 60 mmol) was added dropwise to an ice-cooled solution of 2-oxepanone (2.22 ml, 2.28 g, 20 mmol) in THF (20 ml). The mixture was stirred for 1 hour, then water (100 ml) was added dropwise. The pH was adjusted to 7 with aqueous HCl (3N) and the mixture was extracted with ethyl acetate (3 x 100 ml). The combined organic fractions were dried (Na₂SO₄) and evaporated under reduced pressure. Dichloromethane (20 ml) and pyridine (3.24 ml, 3.16 g, 40 mmol) were added and the mixture was cooled in ice. Methanesulfonyl chloride (1.55 ml, 2.29 g, 20 mmol) was added dropwise and the mixture was stirred at room temperature for 3 hours. Dichloromethane (50 ml) was added and the mixture was washed with aqueous HCl (2M, 50 ml) and aqueous sodium hydrogen carbonate (saturated, 50 ml), dried (Na₂SO₄) and evaporated under reduced pressure to give the alcohol as a pale yellow oil (4.38 g, 98%).
¹H NMR (CDCl₃): δ 4.24 (2H, t, J 6.6 Hz), 3.01 (3H, s), 1.85-1.40 (9H, m), 1.22 (6H, s).

### (+/-)-1-Methanesulfonyloxyheptan-6-ol

(+/-)-Heptan-1,6-diol (1.06 g, 8 mmol) and pyridine (1.36 ml, 1.33 g, 16.8 mmol) were dissolved in dichloromethane (20 ml) and the mixture was cooled in ice. Methanesulfonyl chloride (0.65 ml, 0.96 g, 8.4 mmol) was added dropwise and the mixture was stirred at room temperature for 18 hours. Dichloromethane (50 ml) was added and the mixture was washed with aqueous HCl (1M, 50 ml) and brine (30 ml), dried (Na₂SO₄) and evaporated under reduced pressure. The residue was purified by flash column chromatography on silica gel, eluting with EtOAc/20% hexane, to give the alcohol as an oil (1.01 g, 60%).
¹H NMR (CDCl₃): δ 4.24 (2H, t, J 6.6 Hz), 3.80 (1H, m), 3.01 (3H, s), 1.77-1.22 (9H, m), 1.20 (3H, d, J=1.1 Hz).

### EXAMPLE 423 (INTERMEDIATE COMPOUNDS)

### Preparation of 1-Substituted-4-Piperidinones

The following compounds were synthesized by alkylation of 1,4-dioxa-8-azaspiro[4.5]decane with the appropriate methanesulfonate or bromide according to the method described for the synthesis of 8-[2-(nitrophenyl)ethyl]-1,4-dioxa-8-azaspiro[4.5] decane (Example 339 [C1], Step 3), followed by ketal hydrolysis according to the method described for the synthesis of 1-[2-(benzofurazan-5-yl)ethyl]-4-piperidinone (Example 339 [C1], Step 7).
1-[2-(Thiophen-2-yl)ethyl]-4-piperidinone;
   ¹H NMR (CDCl₃): δ 7.15 (1H, d, J=5.1 Hz), 6.94 (1H, dd, J=5.1, 3.4 Hz), 6.85 (1H, d, J=3.4 Hz), 3.06 (2H, t, J=7.5 Hz), 2.83 (6H, m), 2.49 (4H, t, J=6.1 Hz).
1-[2-(Thiophen-3-yl)ethyl]-4-piperidinone;
   ¹H NMR (CDCl₃): δ 7.27 (1H, m), 7.02 (1H, br s), 6.98 (1H, d, J=4.9 Hz), 2.91-2.72 (8H, m), 2.48 (4H, t, J 6.1 Hz).
1-[2-(4-Cyanophenyl)ethyl]-4-piperidinone;
   ¹H NMR (CDCl₃): δ 7.60 (2H, d, J=8.2 Hz), 7.34 (2H, d, J=8.2 Hz), 2.92-2.71 (8H, m), 2.47 (4H, t, J=6.2 Hz).
1-n-Hexyl-4-piperidinone;
   ¹H NMR (CDCl₃): δ 2.74 (4H, t, J=6.0 Hz), 2.46 (6H, m), 1.70-1.31 (8H, m), 0.90 (3H, t, J=6.6 Hz).
1-n-Heptyl-4-piperidinone;
   ¹H NMR (CDCl₃): δ 2.75 (4H, t, J=6.1 Hz), 2.47 (6H, m), 1.55-1.26 (10H, m), 0.89 (3H, t, J=6.7 Hz).

### 8-[2-Oxo-2-(4-cyanophenyl)ethyl]-1,4-dioxa-8-azaspiro [4.5]decane

Sulfuryl chloride (3.53 ml, 5.94 g, 44 mmol) was added dropwise to a solution of 4-acetylbenzonitrile (5.81 g, 40 mmol) in chloroform (40 ml). The mixture was stirred for 24 hours, diluted with chloroform (40 ml), washed with water (40 ml) and aqueous sodium hydrogen carbonate (saturated, 40 ml), dried (Na₂SO₄) and evaporated under reduced pressure. The residue was dissolved in acetonitrile (40 ml), potassium carbonate (11.06 g, 80 mmol) and 1,4 dioxa-8-azaspiro[4.5]decane (5.13 ml, 5.73 g, 40 mmol) were added. The mixture was stirred for 2.5 hours then aqueous HCl (2N, 120 ml) was added. The mixture was washed with ethyl acetate (2 x 50 ml), aqueous sodium hydroxide (20%, 80 ml) was added and the mixture was extracted with ethyl acetate (3 x 100 ml). The combined organic extracts were dried (Na₂SO₄) and evaporated under reduced pressure. The residue was purified by flash column chromatography on silica gel, eluting with CH₂Cl₂/MeOH/NH₃(Aq.); 98: 2: 0.2 to give the spirodecane as a yellow solid (3.24 g, 28%).
¹H NMR (CDCl₃): δ 8.14 (2H, d, J=8.3 Hz), 7.76 (2H, d, J=8.3 Hz), 3.96 (4H, s), 3.80 (2H, s), 2.67 (4H, t, J=5.6 Hz), 1.79 (4H, t, J=5.6 Hz).

### 1-[2-Oxo-2-(4-cyanophenyl)ethyl]-4-piperidinone

8-[2-Oxo-2-(4-cyanophenyl)ethyl]-1,4-dioxa-8-azaspiro[4.5]decane (300 mg, 1.05 mmol) was dissolved in aqueous HCl (1N, 10 ml) and the mixture was heated under reflux for 30 minutes. The mixture was cooled , aqueous sodium hydroxide (20%, 5 ml) was added and the mixture was extracted with ethyl acetate (3 x 10 ml). The combined organic fractions were dried (Na₂SO₄) and evaporated under reduced pressure to give the piperidinone as a yellow solid (157 mg, 62%).
¹H NMR (CDCl₃): δ 8.12 (2H, d, J=8.3 Hz), 7.80 (2H, d, J=8.3 Hz), 3.96 (2H, s), 2.93 (4H, t, J=6.1 Hz), 2.53 (4H, t, J=6.1 Hz).

### (+/-) 1-[2-Hydroxy-2-(4-cyanophenyl)ethyl]-4-piperidinone

Sodium borohydride (76 mg, 2 mmol) was added to an ice cooled solution of 8-[2-oxo-2-(4-cyanophenyl) ethyl]-1,4-dioxa-8-azaspiro[4.5]decane (300 mg, 1.05 mmol) in ethanol (5 ml). The mixture was stirred at room temperature for 16 hours and the solvent was evaporated under reduced pressure. Water (5 ml) and aqueous sodium hydrogen carbonate (saturated, 10 ml) were added and the mixture was extracted with dichloromethane (3 x 10 ml). The combined organic fractions were dried (Na₂SO₄) and evaporated under reduced pressure. Aqueous HCl (1N, 10 ml) was added and the mixture was heated under reflux for 30 minutes. The mixture was cooled, aqueous sodium hydroxide (20%, 5 ml) was added and the mixture was extracted with ethyl acetate (3 x 10 ml). The combined organic fractions were dried (Na₂SO₄) and evaporated under reduced pressure to give the piperidinone as a yellow gum (153 mg, 60%).
¹H NMR (CDCl₃): δ 7.66 (2H, d, J=8.3 Hz), 7.52 (2H, d, J=8.3 Hz), 4.83 (1H, dd, J=10.5, 3.5 Hz), 3.97 (1H, br s), 3.06 (2H, m), 2.81 (2H, m), 2.70 (1H, dd J=12.6, 3.5 Hz), 2.54 (5H, t, J=6.1 Hz).

### EXAMPLE 424

### 3,4-Dihydro-1'-[2-(2,3-dihydro-1H-indol-5-yl)ethyl]-6-methanesulfonamido-spiro[(2H)-benzopyran-2,4'-piperidine]-4-one dihydrochloride

1'-[2-(1-Acetyl-2,3-dihydro-1H-indol-5-yl)-ethyl]-3,4-dihydro-6-methanesulfonamido-spiro[(2H)benzopyran-2,4'-piperidine]-4-one (1.96 g, 3.9 mmol) was dissolved in ethanol (40 ml) and aqueous HCl (6N, 40 ml). The mixture was heated under reflux for 2 hours, cooled and the ethanol was evaporated under reduced pressure. Water (100 ml) was added and the pH was adjusted to 8 with aqueous sodium hydroxide (20%). The mixture was extracted with dichloromethane and the combined organic fractions were dried (Na₂SO₄) and evaporated under reduced pressure to give the crude pyranone as a tan solid (1.85 g). A sample (200 mg) was purified by flash column chromatography on silica gel, eluting with CH₂Cl₂/MeOH/NH₃(Aq.) (96: 4: 0.4) to give a pale yellow foam which was dissolved in ethanol (5 ml) and treated with ethanolic HCl (6N, 0.5 ml). The mixture was stirred for 1 hour, cooled and the precipitate was collected and dried in vacuo to give the dihydrochloride as a white solid (157 mg, 70%), m.p. 250-252°C.

| Anal. Calc'd for C₂₄H₃₁Cl₂N₃O₄S•0.33H₂O: | | | |
|---|---|---|---|
| | C, 53.93; | H, 5.97; | N, 7.86. |
| Found | C, 53.93; | H, 5.77; | N, 7.92. |

### EXAMPLE 425

### 3,4-Dihydro-1'-[2-(2,3-dihydro-1-methanesulfonamido-1H-indol-5-yl)ethyl]-6-methanesulfonamido-spiro[(2H)-benzopyran-2,4'-piperidine]-4-one hydrochloride

Methanesulfonyl chloride (55 mg, 0.48 mmol) was added to a suspension of 3,4-dihydro-1'-[2-(2,3-dihydro-1H-indol-5-yl)ethyl]-6-methanesulfonamidospiro[(2H)-benzopyran-2,4'-piperidine]-4-one (crude, 84% pure, 217 mg, 0.4 mmol) in pyridine (5 ml). The mixture was stirred for 22 hours, then the solvent was evaporated under reduced pressure. Aqueous sodium hydrogen carbonate (saturated, 20 ml) and water (5 ml) were added and the mixture was extracted with dichloromethane (3 x 25 ml). The combined organic fractions were dried (Na₂SO₄) and evaporated under reduced pressure. The residue was purified by flash column chromatography on silica gel, eluting with CH₂Cl₂ /MeOH/NH₃(Aq.) (96: 4: 0.4) to give a yellow glass which was suspended in ethanol (5 ml) and treated with ethanolic HCl (6N, 0.5 ml). The mixture was stirred for 1 hr, cooled and the precipitate was collected and dried in vacuo to give the hydrochloride as a white solid (91 mg, 40%), m.p. > 285°C.

| Anal. Calc'd. for C₂₅H₃₂ClN₃O₆S₂: | | | |
|---|---|---|---|
| | C, 52.67; | H, 5.66; | N, 7.37. |
| Found | C, 53.00; | H, 5.71; | N, 7.30. |

### EXAMPLE 426

### 3,4-Dihydro-1'-[2-(1H-indol-5-yl)ethyl]-6-methane-sulfonamido-spiro[(2H)-benzopyran-2,4'-piperidine]-4-one hydrochloride

DMSO (0.32 g, 0.36 g, 4.6 mmol) in dichloromethane (5 ml) was added dropwise to a cooled (-60°C) solution of oxalyl chloride (0.20 ml, 0.29 g, 2.3 mmol) in dichloromethane (5 ml). The mixture was stirred at -60°C for 3 minutes, then 3,4-dihydro-1'-[2-(2,3-dihydro-1H-indol-5-yl)ethyl]-6-methanesulfonamido-spiro[(2H)-benzopyran-2,4'-piperidine]-4-one (crude, 84% pure, 1.03 g, 1.9 mmol) in DMSO (5 ml) was added. The mixture was stirred at -60°C for 10 minutes, then triethylamine (5 ml) was added and the mixture was allowed to warm to room temperature. The mixture was poured into aqueous sodium hydrogen carbonate (saturated, 100 ml) and the mixture was extracted with dichloromethane (3 x 100 ml). The combined organic fractions were dried (Na₂SO₄) and evaporated under reduced pressure. The residue was purified by repeated flash column chromatography on silica gel, eluting with CH₂Cl₂/MeOH/NH₃(Aq.) (96: 4: 0.4) to give a blue foam which was dissolved in ethanol (5 ml) and treated with ethanolic HCl (6N, 0.5 ml). The mixture was stirred for 1 hour, cooled and the precipitate was collected, washed with hot ethanol and dried in vacuo, to give the hydrochloride as a white solid (232 mg, 25%), m.p. 260-262°C (dec.).

| Anal. Calc'd. for C₂₄H₂₈ClN₃O₄S•0.75H₂O: | | | |
|---|---|---|---|
| | C, 57.25; | H, 5.90; | N, 8.34. |
| Found | C, 57.34; | H, 5.68; | N, 8.30. |

### EXAMPLE 427

### 3,4-Dihydro-1'-[2-(benzofurazan-5-yl)ethyl]-6-methanesulfonamido-4-(methoxyimino)-spiro[(2H)-1-benzopyran-2,4'-piperidine] hydrochloride

3,4-Dihydro-1'-[2-(benzofurazan-5-yl)ethyl]-6-methanesulfonamido-spiro[(2H)-1-benzopyran-2,4'piperidine]-4-one [prepared as described in Example 94, Step B] 0.137 g, 0.3 mmol) was dissolved in DMF (2 ml) and pyridine (1 ml) and methoxylamine hydrochloride (28 mg, 0.33 mmol) were added. The mixture was stirred for 17 hours, then further methoxylamine hydrochloride (112 mg) was added and the mixture was stirred at 50°C for 3 hours. Further methoxylamine hydrochloride (100 mg) was added and the mixture was stirred at 50°C for 3 hours. The mixture was cooled, the solvent was evaporated under reduced pressure, aqueous sodium hydrogen carbonate (Saturated, 20 ml) was added and the mixture was extracted with dichloromethane (3 x 20 ml). The combined organic fractions were dried (Na₂SO₄) and evaporated under reduced pressure. The residue was purified by flash column chromatography on silica gel, eluting with CH₂Cl₂/MeOH/NH₃(Aq.) (98:2:0.2 then 94:6:0.6) to give the isomeric oximes as colorless gums; higher running fraction: 100 mg, 68%; lower running fraction: 46 mg, 32%. The higher running fraction was dissolved in ethyl acetate and ethanolic HCl was added. The precipitate was collected and dried in vacuo to give the hydrochloride as a white solid (83 mg), m.p. 248-250°C.

| Anal. Calc'd. for C₂₃H₂₈ClN₅O₅S•0.4 H₂O: | | | |
|---|---|---|---|
| | C, 52.20; | H, 5.49; | N, 13.23. |
| Found | C, 52.13; | H, 5.42; | N, 12.99. |

### EXAMPLE 428

### 3,4-Dihydro-6-methanesulfonamido-4-(hydroxyimino)-1'-(2-phenylethyl)-spiro[(2H)-1-benzopyran-2,4'-piperidine]

In the manner of Example 427, 3,4-dihydro-6-methane-sulfonamido-1'-(2-phenylethyl)-spiro [(2H)-benzopyran-2,4'-piperidine]-4-one was treated with hydroxylamine hydrochloride to give, after chromatography, the piperidine as a solid, m.p. 95-99°C.

| Anal. Calc'd for C₂₂H₂₇N₃O₄S•0.6CHCl₃: | | | |
|---|---|---|---|
| | C, 54.16; | H, 5.55; | N, 8.39. |
| Found | C, 54.37; | H, 5.49; | N, 8.48. |

### EXAMPLE 429

### (+-)-3,4-Dihydro-1'-[2-(benzofurazan-5-yl)ethyl]-6-methanesulfonamido-spiro[(2H)-1-benzopyran-2,4'-piperidine]-4-ol hydrochloride

Sodium borohydride (7.5 mg, 0.2 mmol) was added to a stirred suspension of 3,4-dihydro-1'-[2-(benzofurazan-5-yl)ethyl]-6-methanesulfonamidospiro[(2H)-1-benzopyran-2,4'-piperidine]-4-one [prepared as described in Example 94, Step B] (45.6 mg, 0.1 mmol) in ethanol (2 ml). The mixture was stirred for 16 hours, then poured into aqueous sodium hydrogen carbonate (saturated, 10 ml) and extracted with dichloromethane (3 x 10 ml). The combined organic fractions were dried (Na₂SO₄) and evaporated under reduced pressure to give a pale yellow oil. The residue was dissolved in ethyl acetate (2 ml) and ethanolic HCl (6N, 0.5 ml) was added. The mixture was cooled and the precipitate collected and dried in vacuo to give the hydrochloride as a white solid (32 mg, 65%), m.p. = 242-244°C.

| Anal. Calc'd. for C₂₂H₂₇ClN₄O₅S: | | | |
|---|---|---|---|
| | C, 53.38; | H, 5.50; | N, 11.32. |
| Found | C, 53.05; | H, 5.51; | N, 11.09. |

### EXAMPLE 431

### 6-Amino-3,4-dihydro-1'-[2-(benzofurazan-5-yl)ethyl]-spiro((2H)-1-benzopyran-2,4'-piperidine]-4-one

1-[2-(Benzofurazan-5-yl)ethyl]-4-piperidinone (0.98 g, 4 mmol), N-(3-acetyl-4-hydroxyphenyl)-acetamide (0.77 g, 4 mmol) and pyrrolidine (0.33 ml, 0.28 g, 4 mmol) in methanol (20 ml) were heated under reflux for 3 hours, cooled and the solvent was evaporated under reduced pressure. Water (50 ml) and aqueous sodium hydroxide (20%, 10 ml) were added and the mixture was extracted with ethyl acetate (3 x 50 ml). The combined organic fractions were dried (Na₂SO₄) and evaporated under reduced pressure to give a yellow solid. Ethanol (40 ml) and aqueous HCl (6N, 40 ml) were added and the mixture was heated under reflux for 2 hours. The mixture was cooled and the ethanol was evaporated under reduced pressure. Water (40 ml) was added and the mixture was washed with ethyl acetate (50 ml). Aqueous sodium hydroxide (20%, 60 ml) was added slowly, with stirring and cooling, and the mixture was extracted with dichloromethane (3 x 100 ml). The combined organic fractions were dried (Na₂SO₄) and evaporated under reduced pressure. The residue was triturated with ethyl acetate-hexane (1: 1, 20 ml) and the solid was collected and dried in vacuo to give a yellow solid (1.17 g, 77%). A sample was recrystallized from chloroform-hexane to give the pyranone as a yellow solid, m.p. 191-193°C.

| Anal. Calc'd. for C₂₁H₂₂N₄O₃•0.75H₂O: | | | |
|---|---|---|---|
| | C, 64.35; | H, 6.04; | N, 14.29. |
| Found | C, 64.35; | H, 5.75; | N, 14.29. |

### EXAMPLE 432

### Ethyl [(3,4-dihydro-1'-[2-(benzofurazan-5-yl)-ethyl]-4-oxo-spiro[(2H)-1-benzopyran-2,4'-piperidine]-6-yl)amino]sulfonyl acetate hydrochloride

Ethyl chlorosulfonylacetate (140 mg, 0.75 mmol) in DMF (1 ml) was added dropwise to a stirred solution of 6-amino-3,4-dihydro-1'-[2-(benzofurazan-5-yl)-ethyl]-spiro[(2H)-1-benzopyran-2,4'-piperidine]-4-one (189 mg, 0.5 mmol) and pyridine (40 mg, 3 mmol) in DMF (5 ml). The mixture was stirred for 4 hours, then additional ethyl chlorosulfonylacetate (46 mg, 0.25 mmol) was added. The mixture was stirred for 1 hour, poured into aqueous sodium hydrogen carbonate (saturated, 25 ml) and extracted with dichloromethane (3 x 25 ml). The combined organic fractions were dried (Na₂SO₄) and evaporated under reduced pressure. The residue was purified by flash column chromatography on silica gel, eluting with CH₂Cl₂/MeOH/NH₃(Aq.) (97:3:0.3) to give a yellow foam which was dissolved in ethyl acetate (2 ml) and treated with ethanolic HCl (6N, 0.5 ml). The mixture was stirred for 1 hour, cooled and the solid was collected and dried in vacuo to give the hydrochloride as a white solid (171 mg, 61%), m.p. 250-251°C.

| Anal. Calc'd. for C₂₅H₂₉ClN₄O₇S: | | | |
|---|---|---|---|
| | C, 53.14; | H, 5.17; | N, 9.92. |
| Found | C, 53.00; | H, 5.39; | N, 9.86. |

### EXAMPLE 433

### [{3,4-Dihydro-1'-[2-(benzofurazan-5-yl)ethyl]-4-oxo-spiro[(2H)-1-benzopyran-2,4'-piperidine]-6-yl}amino] sulfonyl acetic acid

Aqueous lithium hydroxide (1M, 0.55 ml) was added to a stirred suspension of ethyl [{3,4-dihydro-1'-[2-(benzofurazan-5-yl)ethyl]-4-oxo-spiro [(2H)-1-benzopyran-2,4'-piperidine]-6-yl}amino] sulfonyl acetate (264 mg, 0.5 mmol) in methanol-water (1:1, 20 ml) and the mixture was stirred for 2 hours. Further aqueous lithium hydroxide (1M, 1.45 ml) was added and the mixture was stirred for 24 hours. The solvent was evaporated under reduced pressure, water (10 ml) was added and the pH was adjusted to 1.5 with aqueous HCl (3M). The mixture was cooled and the solid was collected and dried in vacuo to give the acid as a white solid (198 mg, 79%), m.p. = 187-189°C.

| Anal. Calc'd. for C₂₃H₂₄N₄O₇S•0.6 H₂O: | | | |
|---|---|---|---|
| | C, 54.03; | H, 4.97; | N, 10.96. |
| Found | C, 53.97; | H, 4.92; | N, 10.83. |

### EXAMPLE 434

### 1'-Benzoyl-3,4-dihydro-6-methoxy-spiro[(2H)-1- benzopyran-2,4'-piperidine]-4-one

Pyrrolidine (8.35 ml, 7.11 g, 0.1 mol) was added to a suspension of 2'-hydroxy-5'-methoxyacetophenone (16.62 g, 0.1 mol) in methanol (100 ml). The mixture was stirred for 10 minutes, then 1-benzoyl-4-piperidone (20.32 g, 0.1 mol) was added and the mixture was heated under reflux for 7 hr. The mixture was cooled and the solvent was evaporated under reduced pressure. Water (400 ml) was added and the mixture was extracted with ethyl acetate (3 x 400 ml). The combined organic fractions were evaporated under reduced pressure and the residue was recrystallized from methanol to give the ketone as a pale yellow solid (29.54 g, 84%).
¹H NMR (CDCl₃): δ 7.41 (5H, s), 7.30 (1H, d, J=3.1 Hz), 7.12 (1H, dd, J=9.0, 3.1 Hz), 6.94 (1H, d, J=9.0 Hz), 4.5 (1H, br s), 3.8 (3H, s), 3.7-3.2 (3H, m), 2.73 (2H, s), 2.3-1.5 (4H, m).

### EXAMPLE 435

### 1'-Benzoyl-3,4-dihydro-6-methanesulfonamido-spiro [(2H)-1-benzopyran-2,4'-piperidine]

Sodium borohydride (1.13 g, 30 mmol) was added in portions over 5 minutes to a stirred, ice cooled suspension of 1'-benzoyl-3,4-dihydro-6-methanesulfonamidospiro[(2H)-1-benzopyran-2,4'-piperidine]-4-one (prepared as described in Example 70, Method 2, Step C) (6.21 g, 15 mmol) in ethanol (150 ml). The mixture was stirred at room temperature for 22 hours, poured into water (150 ml) and the ethanol was evaporated under reduced pressure. The mixture was extracted with dichloromethane (4 x 150 ml) and the combined organic fractions were dried (Na₂SO₄) and evaporated under reduced pressure to give a white foam (6.61 g). A sample (6.41 g) and p-toluenesulfonic acid (Monohydrate, 100 mg) in toluene (150 ml) were heated under reflux for 1 hr, cooled and diluted with methylene chloride (600 ml). The mixture was washed with aqueous sodium hydrogen carbonate (saturated, 150 ml), dried (Na₂SO₄) and evaporated under reduced pressure. The residue was suspended in ethanol-methanol (1: 1, 200 ml), palladium on carbon (5%, 1 g) was added and the mixture was stirred under hydrogen (1 Atm.) for 24 hours. The mixture was filtered through celite, washing with methanol, and the filtrate was evaporated under reduced pressure. The residue was redissolved in dichloromethane, filtered through celite and evaporated under reduced pressure to give the piperidine as a foam (5.44 g, 93%).
¹H NMR (CDCl₃): δ 7.4 (5H, s), 6.98 (2H, m), 6.83 (1H, d, J 8.4 Hz), 4.5 (1H, br m), 3.6-3.2 (3H, m), 2.96 (3H, s), 2.8 (2H, m), and 2.0-1.5 (7H, m).

### EXAMPLE 436

### 1'-Benzoyl-3,4-dihydro-6-methoxy-spiro[(2H)-1-benzopyran-2,4'-piperidine]

Following the procedures as described in Example 435, 1'-benzoyl-3,4-dihydro-6-methoxyspiro[(2H)-1-benzopyran-2,4'-piperidine]-4-one was reduced with sodium borohydride, dehydrated with p-toluenesulfonic acid and hydrogenated with palladium on carbon (5%) under hydrogen (50 psi) to give the piperidine as a foam (54%).
¹H NMR (CDCl₃): δ 7.41 (5H, s), 6.77 (1H, d), 6.69 (1H, dd), 6.61 (1H, d), 4.4 (1H, br s), 3.75 (3H, s), 3.6 (1H, br s), 3.40 (2H, m), 2.78 (2H, m), 1.82 (4H, m), 1.60 (2H, m).

### EXAMPLE 437

### 3,4-Dihydro-6-methanesulfonamido-spiro[(2H)-1-benzopyran-2,4'-piperidine] hydrochloride

1'-Benzoyl-3,4-dihydro-6-methanesulfonamidospiro[(2H)-1-benzopyran-2,4'-piperidine] (5.44 g, 13.6 mmol) was dissolved in methanol (70 ml) and aqueous HCl (6N, 105 ml) was added. The mixture was heated under reflux for 24 hr, then the volume was reduced to 50 ml by distillation. Ethanol (200 ml) was added and the volume was reduced to 50 ml. Further ethanol (200 ml) was added, the volume was reduced to 100 ml and the mixture was cooled. The precipitate was collected and dried in vacuo to give the hydrochloride as a white solid (3.77 g, 83%). A sample was recrystallized from ethanol to give the hydrochloride as a white solid, m.p. = 282-285°C (dec.).
¹H NMR (DMSO): δ 9.35 (1H, s), 9.1 (2H, br s), 6.95 (2H, m), 6.79 (1H, d, J 9.5 Hz), 3.17-2.90 (4H, m), 2.88 (3H, s), 2.72 (2H, m), and 1.84 (6H, m).

| Anal. Calc'd. for C₁₄H₂₁ClN₂O₃S: | | | |
|---|---|---|---|
| | C, 50.52; | H, 6.36; | N, 8.42. |
| Found: | C, 50.69; | H, 6.24; | N, 8.40. |

### EXAMPLE 438

### 3,4-Dihydro-6-methoxy-spiro[(2H)-1-benzopyran-2,4'- piperidine] hydrochloride

1'-Benzoyl-3,4-dihydro-6-methoxy-spiro [(2H)-1 -benzopyran-2,4'-piperidine] (5.23 g, 15.5 mmol) was dissolved in methanol (80 ml) and aqueous HCl (6N, 120 ml) was added. The mixture was heated under reflux for 24 hours, then the volume was reduced to 50 ml by distillation. Ethanol (200 ml) was added and the volume was reduced to 50 ml. Further ethanol (200 ml) was added, the volume was reduced to 100 ml and the mixture was cooled. The mixture was filtered and evaporated under reduced pressure and the residue was crystallized from ethanol-ether to give, in two crops, the hydrochloride as a white solid (1.29 g, 31%), m.p. = 240-242°C.

| Anal. Calc'd. for C₁₄H₂₀ClNO₂•0.3H₂O: | | | |
|---|---|---|---|
| | C, 61.10; | H, 7.55; | N, 5.09. |
| Found | C 61.04; | H 7.22; | N 4.91. |

The compounds of Table XXIX were prepared from 3,4-dihydro-6-methanesulfonamido-spiro[(2H)-1-benzopyran-2,4'-piperidine] hydrochloride by the method described in Example 92, Step B, using the appropriate mesylate or bromide as indicated.

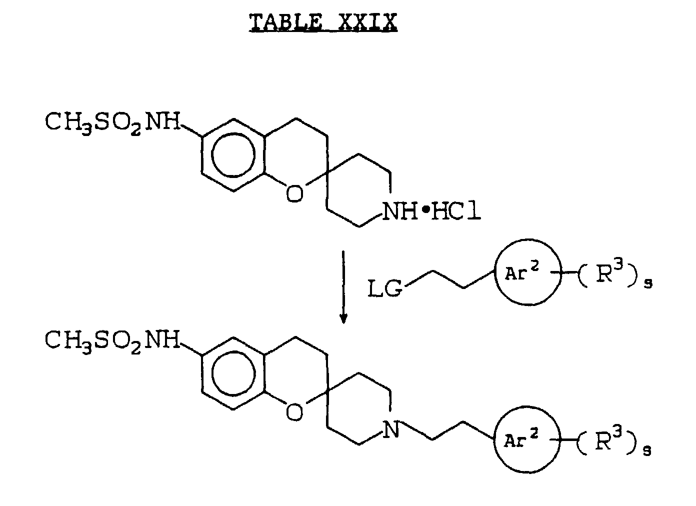

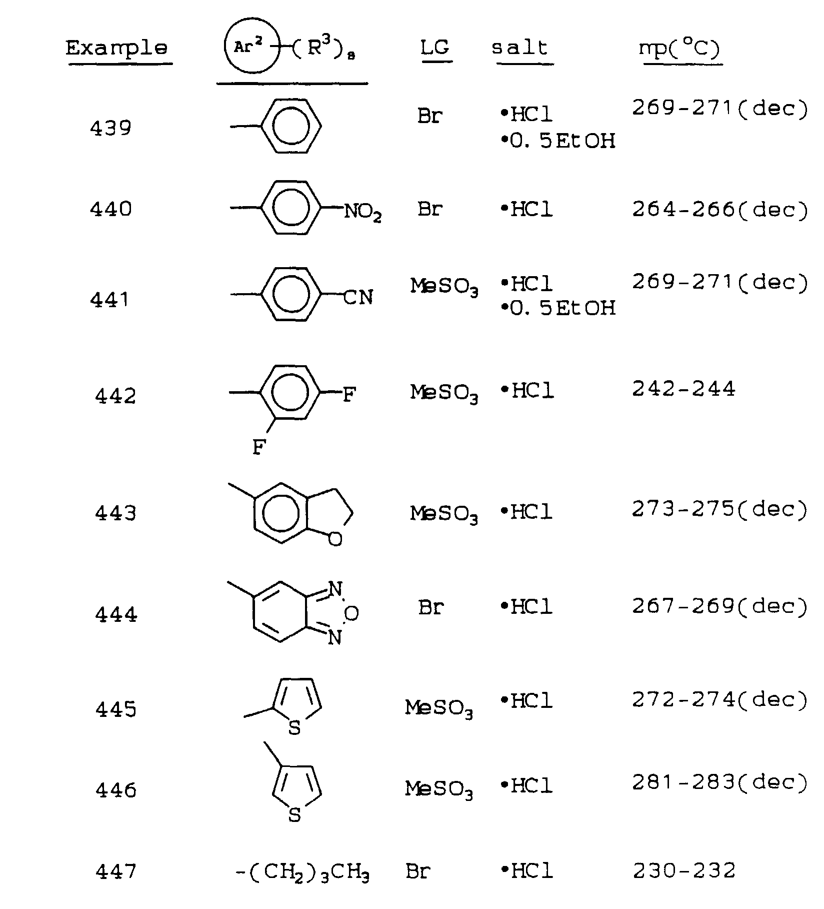

The compounds of Table XXX were prepared from 3,4-dihydro-6-methoxy-spiro[(2H)-1-benzopyran-2,4'-piperidine] hydrochloride by the method described in Example 92, Step B, using the appropriate mesylate or bromide as indicated.

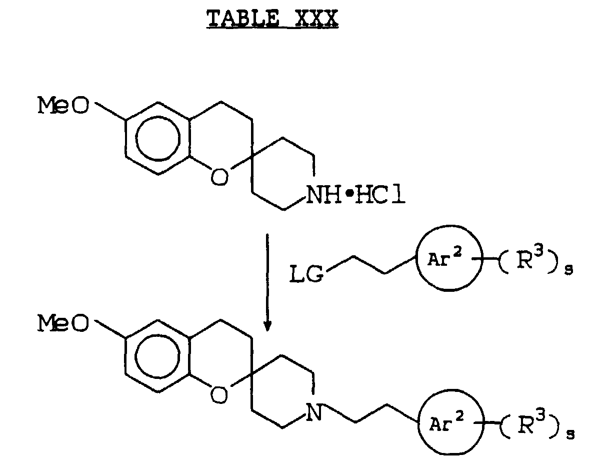

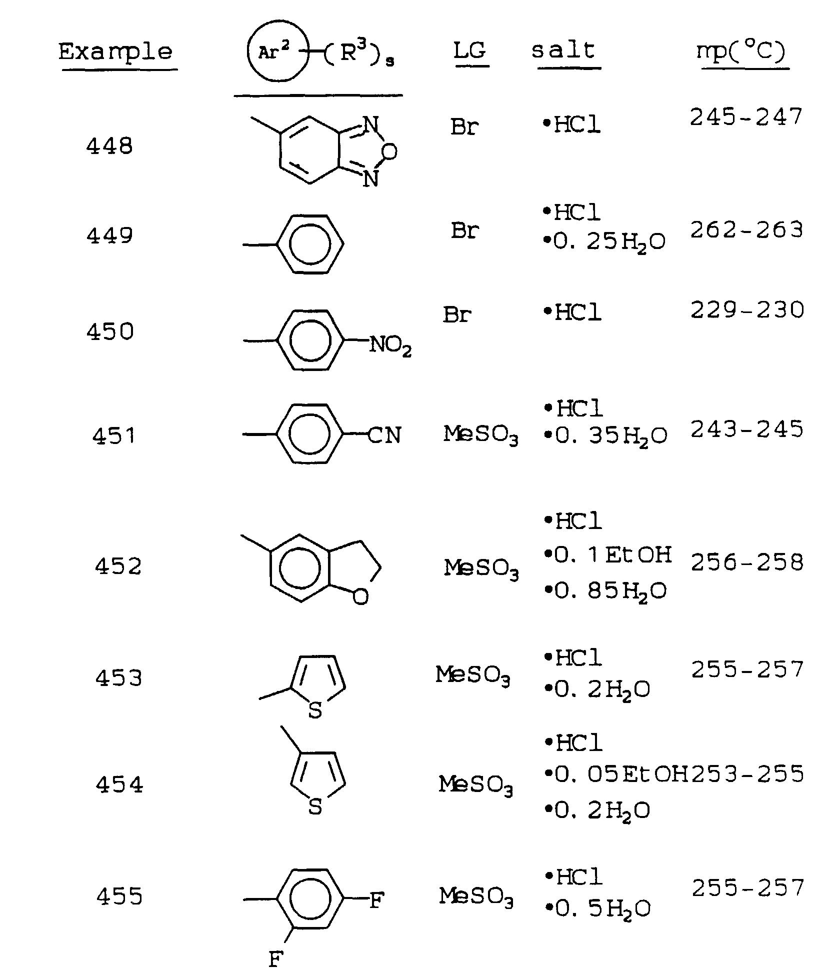

### EXAMPLE 456 (INTERMEDIATE COMPOUNDS)

### N-(4-Acetyl-5-hydroxy-2-nitrophenyl)acetamide and N-(4-Acetyl-3-hydroxy-2-nitrophenyl)acetamide

A mixture of N-(4-acetyl-3-hydroxyphenyl)-acetamide (9.66 g, 50 mmol) and acetic anhydride (14.2 ml, 15.3 g, 150 mmol) in dichloromethane (150 ml) was cooled to 5°C and nitric acid (90%, d 1.49, 4.70 ml, 7.00 g, 100 mmol) was added dropwise. The mixture was stirred at room temperature for 1 hour, poured into water (150 ml) and extracted with dichloromethane (3 x 150 ml). The combined organic fractions were dried (Na₂SO₄) and evaporated under reduced pressure and the residue was purified by repeated flash column chromatography on silica gel, eluting with hexane/30% ethyl acetate then hexane/50% ethyl acetate. The first isomer to elute was N-(4-acetyl-5-hydroxy-2-nitrophenyl)acetamide as a yellow solid (3.87 g, 33%).
¹H NMR (CDCl₃): δ 12.83 (1H, s), 10.78 (1H, br s), 8.78 (1H, s), 8.43 (1H, s), 2.68 (3H, s), 2.32 (3H, s).

The second isomer to elute was N-(4-acetyl-3-hydroxy-2-nitrophenyl)acetamide as a yellow solid (0.41 g, 3%).
¹H NMR (CDCl₃): δ 13.74 (1H, s), 8.94 (1H, br s), 8.09 (1H, d, J 9.1 Hz), 7.88 (1H, d, J 9.1 Hz), 2.66 (3H, s), 2.26 (3H, s).

### EXAMPLE 457

### 7-Amino-3,4-dihydro-6-nitro-1'-[2-(4-cyanophenyl)-ethyl]spiro[(2H)-1-benzopyran-2,4'-piperidine]-4-one hydrochloride

Pyrrolidine (108 µl, 92 mg, 1.3 mmol) was added to N-(4-acetyl-5-hydroxy-2-nitrophenyl)-acetamide (309 mg, 1.3 mmol) in methanol (2 ml). The mixture was stirred for 10 minutes, then 1-[2-(4-cyanophenyl)ethyl]-4-piperidinone (296 mg, 1.3 mmol) in methanol (2 ml) was added and the mixture was heated under reflux for 8 hours, cooled and poured into aqueous sodium hydrogen carbonate (saturated, 25 ml). The mixture was extracted with dichloromethane (3 x 25 ml) and the combined organic fractions were dried (Na₂SO₄) and evaporated under reduced pressure. The residue was purified by flash column chromatography on silica gel, eluting with CH₂Cl₂/MeOH /NH₃(Aq.) (97:3:0.3) to give the pyranone as a yellow solid (300 mg, 57%). A sample (30 mg) was suspended in ethanol (1 ml) and treated with ethanolic HCl (6N, 0.2 ml). Ether (5 ml) was added dropwise and the precipitate was collected and dried in vacuo to give the hydrochloride as a yellow solid (17 mg), m.p. 174-177°C (dec.).

| Anal. Calc'd. for C₂₃H₂₃ClN₄O₄•0.33H₂O: | | | |
|---|---|---|---|
| | C, 58.86; | H, 5.31; | N, 12.48. |
| Found | C, 58.87; | H, 5.24; | N, 12.34. |

### EXAMPLE 458

### 7-Amino-3,4-dihydro-8-nitro-1'-[2-(4-cyanophenyl)-ethyl]spiro[(2H)-1-benzopyran-2,4'-piperidine]-4-one

In the manner of Example 457, N-(4-acetyl-3-hydroxy-2-nitrophenyl)acetamide (405 mg, 1.7 mmol), pyrrolidine (142 ml, 121 mg, 1.7 mmol) and 1-[2-(4-cyanophenyl)ethyl]-4-piperidinone (388 mg, 1.7 mmol) in methanol (2 ml) gave, after chromatography, the title compound as an orange foam, (302 mg, 44%) ¹H NMR (CDCl₃): δ 7.80 (1H, d, J=8.8 Hz), 7.58 (2H, d, J=8.2 Hz), 7.31 (2H, d, J=8.2 Hz), 6.38 (1H, d, J=8.8 Hz), 5.82 (2H, br s), 2.90-2.60 (10H, m), 2.13 (2H, m), 1.76 (2H, m).

### EXAMPLE 459

### (±)-7-Amino-3,4-dihydro-6-nitro-1'-[(5,6,7,8-tetrahydro-2-napthalenecarbonitrile)-6-yl]-spiro[(2H)-1-benzopyran-2,4'-piperidine]-4-one

In the manner of Example 457, N-(4-acetyl-5-hydroxy-2-nitrophenyl)acetamide (643 mg, 2.7 mmol) and (±)-1-[(5,6,7,8-tetrahydro-2-napthalenecarbonitrile)-6-yl]-4-piperidinone (687 mg, 2.7 mmol) in methanol (16 ml) gave, after chromatography, the title compound as an orange foam, (730 mg, 62%) ¹H NMR (CDCl₃): δ 8.80 (1H, s), 7.38 (2H, m), 7.18 (1H, d, J 9 Hz), 6.42 (2H, br s), 6.30 (1H, s), 3.42 (1H, m), 3.02-2.78 (6H, m), 2.72 (2H, m), 2.70 (2H, s), 2.08 (2H, m), and 1.85-1.60 (4H, m).

### EXAMPLE 460

### 7-Amino-3,4-dihydro-1'-hexyl-6-nitrospiro[(2H)-1-benzopyran-2,4'-piperidin]-4-one

In the manner of Example 457, N-(4-acetyl-5-hydroxy-2-nitrophenyl)acetamide (1.07 g, 4.5 mmol), pyrrolidine (380 µl, 320 mg, 4.5 mmol) and 1-hexyl-4-piperidone (820 mg, 4.5 mmol) in methanol (15 ml) gave, after chromatography, the title compound as an orange gum (1.07 g, 66%).
¹H NMR (CDCl₃): δ 8.78 (1H, s), 6.4 (2H, br s), 6.28 (1H, s), 2.68 (2H, s), 2.63 (2H, m), 2.35 (4H, m), 2.00 (2H, m), 1.77 (2H, m), 1.50 (2H, m), 1.29 (6H, m), 0.88 (3H, t).

### EXAMPLE 461

### 7-Amino-3,4-dihydro-1'-heptyl-6-nitrospiro[(2H)-1-benzopyran-2,4'-piperidin]-4-one

In the manner of Example 457, N-(4-acetyl-5-hydroxy-2-nitrophenyl)acetamide (1.07 g, 4.5 mmol), pyrrolidine (380 µl, 320 mg, 4.5 mmol) and 1-heptyl-4-piperidone (890 mg, 4.5 mmol) in methanol (15 ml) gave, after chromatography, the title compound as an orange gum (980 mg, 58%).
¹H NMR (CDCl₃): δ 8.79 (1H, s), 6.4 (2H, br s), 6.28 (1H, s), 2.68 (2H, s), 2.65 (2H, s), 2.35 (4H, m), 2.01 (2H, m), 1.77 (2H, m), 1.50 (2H, m), 1.28 (8H, m), 0.88 (3H, t).

### EXAMPLE 462

### 6,7-Diamino-3,4-dihydro-1'-[2-(4-cyanophenyl)ethyl] spiro[(2H)-1-benzopyran-2,4'-piperidine]-4-one dihydrochloride

Titanium trichloride (15 wt% solution in 20-30% aqueous HCl, 5 ml) was added dropwise to a solution of 7-amino-3,4-dihydro-6-nitro-1'-[2-(4-cyanophenyl)ethyl]spiro[(2H)-1-benzopyran-2,4'-piperidine]-4-one (238 mg, 0.59 mmol) in acetic acid (20 ml). The mixture was stirred for 2 hours, then further titanium chloride (15 wt%, 2.5 ml) was added. The mixture was stirred for 2 hours, poured into aqueous sodium hydroxide (20%, 80 ml) and aqueous sodium hydrogen carbonate (saturated, 200 ml) was added. The mixture was extracted with ethyl acetate (3 x 100 ml) and the combined organic fractions were dried (Na₂SO₄) and evaporated under reduced pressure. The residue was purified by flash column chromatography on silica gel, eluting with CH₂Cl₂/MeOH /NH₃(Aq.) (96:4:0.4) to give the pyranone as an orange foam (152 mg, 69%). A sample (30 mg) was suspended in ethanol (1 ml) and treated with ethanolic HCl (6M, 0.2 ml). The mixture was stirred for I hour, cooled and the precipitate was collected and dried in vacuo to give the dihydrochloride as a pale yellow solid (29 mg), m.p. >250°C.

| Anal. Calc'd. for C₂₂H₂₆Cl₂N₄O₂: | | | |
|---|---|---|---|
| | C, 58.80; | H, 5.83; | N, 12.47. |
| Found | C, 58.63; | H, 5.84; | N, 12.31. |

### EXAMPLE 463

### 7,8-Diamino-3,4-dihydro-1'-[2-(4-cyanophenyl)ethyl]-spiro[(2H)-1-benzopyran-2,4'-piperidine]-4-one

In the manner of Example 462, 7-amino-3,4-dihydro-8-nitro-1'-[2-(4-cyanophenyl)ethyl]-spiro[(2H)-1-benzopyran-2,4'-piperidine]-4-one (291 mg, 0.72 mmol) was reduced to give, after chromatography, the title compound as a pale yellow foam (239 mg, 89%).
¹H NMR (CDCl₃): δ 7.58 (2H, d, J=8.1 Hz), 7.36 (1H, d, J=8.5 Hz), 7.31 (2H, d, J=8.1 Hz), 6.37 (1H, d, J=8.5 Hz), 4.0 (2H, br s), 3.31 (2H, br s), 2.89-2.42 (10H, m), 2.10 (2H, m), 1.76 (2H, m).

### EXAMPLE 464

### (±)-6,7-Diamino-3,4-dihydro-1'-[(5,6,7,8-tetrahydro-2-napthalenecarbonitrile)-6-yl]-spiro[(2H)-1-benzopyran-2,4'-piperidine]-4-one

In the manner of Example 462, (±)-7-amino-3,4-dihydro-6-nitro-1'-[(5,6,7,8-tetrahydro-2-napthalenecarbonitrile)-6-yl]-spiro[(2H)-1-benzopyran-2,4'-piperidine]-4-one (690 mg, 1.6 mmol) was reduced to give, after chromatography, the pyranone as an orange solid (314 mg, 49%), m.p. = 184-185°C.
¹H NMR (CDCl₃): δ 7.37 (2H, m), 7.22 (1H, s), 7.16 (1H, d, J 9 Hz), 6.23 (1H, s), 4.18 (2H, s), 3.10-2.75 (7H, m), 2.71 (4H, m), 2.61 (2H, s), 2.10 (2H, m), 1.23 (4H, m).

### EXAMPLE 465

### 6,7-Diamino-3,4-dihydro-1'-hexylspiro[(2H)-1-benzopyran-2,4'-piperidin]-4-one

In the manner of Example 462, 7-amino-3,4-dihydro-1'-hexyl-6-nitrospiro[(2H)-1-benzopyran-2,4'-piperidin]-4-one (1.05 g, 2.9 mmol) was reduced to give, after chromatography, the title compound as an orange foam (640 mg, 67%).
¹H NMR (CDCl₃): δ 7.21 (1H, s), 6.21 (1H, s), 4.15 (2H, br s), 3.04 (2H, br s), 2.62 (2H, m), 2.59 (2H, s), 2.37 (4H, m), 2.03 (2H, m), 1.70 (2H, m), 1.49 (2H, m), 1.29 (6H, m), 0.88 (3H, t).

### EXAMPLE 466

### 6,7-Diamino-3,4-dihydro-1'-heptylspiro[(2H)-1-benzopyran-2,4'-piperidin]-4-one

In the manner of Example 462, 7-amino-3,4-dihydro-1'-heptyl-6-nitrospiro[(2H)-1-benzopyran-2,4'-piperidin]-4-one (975 mg, 2.6 mmol) was reduced to give, after chromatography, the title compound as an orange foam (560 mg, 62%).
¹H NMR (CDCl₃): δ 7.21 (1H, s), 6.21 (1H, s), 4.15 (2H, br s), 3.05 (2H, br s), 2.62 (2H, m), 2.59 (2H, s), 2.35 (4H, m), 2.03 (2H, m), 1.71 (2H, m), 1.49 (2H, m), 1.28 (6H, m), 0.88 (3H, t).

### EXAMPLE 467

### 1-[2-(4-Cyanophenyl)ethyl]-7',8'-dihydro-8'-oxo-spiro-[piperidine-4,6'(1'H)-pyrano[2,3-f]benzimidazole] dihydrochloride

Triethyl orthoformate (75 ml, 67 mg, 0.45 mmol) was added to a stirred suspension of 6,7-diamino-3,4-dihydro-1'-[2-(4-cyanophenyl)ethyl]-spiro[(2H)-1-benzopyran-2,4'-piperidine]-4-one (56 mg, 0.15 mmol) and p-toluenesulfonic acid (monohydrate, 10 mg) in ethyl acetate (5 ml). The mixture was heated under reflux for 1 hour, further triethyl orthoformate (75 ml) was added and the mixture was heated under reflux for 1.5 hour. The mixture was cooled and aqueous HCl (1N, 5 ml) was added. The mixture was stirred for 15 minutes, poured into aqueous sodium hydrogen carbonate (saturated, 25 ml) and extracted with dichloromethane (3 x 25 ml). The combined organic fractions were dried (Na₂SO₄) and evaporated under reduced pressure. The residue was purified by column chromatography on silica gel, eluting with CH₂Cl₂/MeOH/NH₃(AQ.) (95:5:0.5) to give a yellow solid which was suspended in ethanol (1 ml) and treated with ethanolic HCl (6M, 0.2 ml). The mixture was stirred for 1 hour, cooled and the precipitate was collected and dried in vacuo to give the title compound as a tan solid (40 mg, 55%), m.p. >250°C.

| Anal. Calc'd. for C₂₃H₂₄Cl₂N₄O₂•0.5 EtOH: | | | |
|---|---|---|---|
| | C, 59.76; | H, 5.64; | N, 11.61. |
| Found | C, 59.62; | H, 5.63; | N, 11.88. |

### EXAMPLE 468

### 1-[2-(4-Cyanophenyl)ethyl]-6',7'-dihydro-6'-oxo-spiro-[piperidine-4,8'(1'H)-pyrano[2,3-e]benzimidazole]dihydrochloride

In the manner of Example 467, 7,8-diamino-3,4-dihydro-1'-[2-(4-cyanophenyl)ethyl]spiro[(2H)-1-benzopyran-2,4'-piperidine]-4-one (113 mg, 0.3 mmol) gave, after chromatography and salt formation, the title compound as a white solid (63 mg, 46%), m.p. = 226-229°C.

| Anal. Calc'd. for C₂₃H₂₄Cl₂N₄O₂ | | | |
|---|---|---|---|
| | C, 60.14; | H, 5.27; | N, 12.20. |
| Found | C, 60.24; | H, 5.46; | N, 12.20. |

### EXAMPLE 469

### (±)-1-[(5,6,7,8-Tetrahydro-2-napthalenecarbonitrile)-6-yl]-7',8'-dihydro-8'-oxo-spiro[piperidine-4,6'(1'H)-pyrano[2,3-f]benzimidazole] dihydrochloride

In the manner of Example 467, (±)-6,7-diamino-3,4-dihydro-1'-[(5,6,7,8-tetrahydro-2-napthalenecarbonitrile)-6-yl]-spiro[(2H)-1-benzopyran-2,4'-piperidine]-4-one (161, mg, 0.4 mmol) gave, after chromatography and salt formation, the title compound as a light tan solid (92 mg, 47%), m.p. 228-230°C.

| Anal. Calc'd. for C₂₅H₂₆Cl₂N₄O₂•0.5 EtOH: | | | |
|---|---|---|---|
| | C, 61.41; | H, 5.75; | N, 11.02. |
| Found | C, 61.32; | H, 5.99; | N, 10.94. |

### EXAMPLE 470

### 7',8'-Dihydro-1-hexyl-8'-oxospiro[piperidine-4,6'-(1H)-pyrano[2,3-f]benzimidazole]dihydrochloride

In the manner of Example 467, 6,7-diamino-3,4-dihydro-1'-hexylspiro[(2H)-1-benzopyran-2,4'-piperidin]-4-one (298 mg, 0.90 mmol) gave, after chromatography and salt formation, the title compound as an off-white solid (172 mg, 46%) mp 234-237°.

| Anal. Calc'd for C₂₀H₂₇N₃O₂•2HCl: | | | |
|---|---|---|---|
| | C, 57.97; | H, 7.05; | N, 10.14. |
| Found | C, 58.31; | H, 7.21; | N, 10.19. |

### EXAMPLE 471

### 7',8'-Dihydro-1-heptyl-8'-oxospiro[piperidine-4,6'-(1H)-pyrano[2,3-f]benzimidazole]dihydrochloride

In the manner of Example 467, 6,7-diamino-3,4-dihydro-1'-heptylspiro[(2H)-1-benzopyran-2,4'-piperidin]-4-one (275 mg, 0.80 mmol) gave, after chromatography and salt formation, the title compound as an off-white solid (151 mg, 44%) mp 248-252°.

| Anal. Calc'd for C₂₁H₂₉N₃O₂•2HCl•0.75H₂O: | | | |
|---|---|---|---|
| | C, 57.07; | H, 7.41; | N, 9.51. |
| Found | C, 56.96; | H, 7.14; | N, 9.43. |

### EXAMPLE 472

### 1-[2-(4-Cyanophenyl)ethyl]-2',3',7',8'-tetrahydro-2',8'-dioxo-spiro[piperidine-4,6'(1'H)-pyrano[2,3-f]-benzimidazole] hydrochloride

Carbonyl diimidazole (122 mg, 0.75 mmol) was added to a solution of 6,7-diamino-3,4-dihydro-1'-[2-(4-cyanophenyl)ethyl]-spiro[(2H)-1-benzopyran-2,4'-piperidine]-4-one (188 mg, 0.5 mmol) in THF (5 ml) and the mixture was stirred for 3 hours. The mixture was cooled and the solid was collected and triturated with hot ethanol. The solid was suspended in ethanol (2 ml) and treated with ethanolic HCl (6N, 0.5 ml). The mixture was stirred for 1 hour, cooled and the precipitate was collected and dried in vacuo to give the title compound as a white solid (119 mg, 52%), m.p. >285°C.

| Anal. Calc'd. for C₂₃H₂₃ClN₄O₃•H₂O | | | |
|---|---|---|---|
| | C, 60.46; | H, 5.51; | N, 12.26. |
| Found | C, 60.43; | H, 5.12; | N, 12.08. |

### EXAMPLE 473

### 1-[2-(4-Cyanophenyl)ethyl]-2',3',6',7'-tetrahydro-2',6'-dioxo-spiro[piperidine-4,8'(1'H)-pyrano[2,3-e]-benzimidazole] hydrochloride

In the manner of Example 472, 7,8-diamino-3,4-dihydro-1'-[2-(4-cyanophenyl)ethyl]spiro[(2H)-1-benzopyran-2,4'-piperidine]-4-one (113 mg, 0.3 mmol) gave, after column chromatography on silica gel, eluting with CH₂CL₂/MeOH/NH₃ (Aq.,) (93:7:0.7 then 90:10:1), and salt formation, the title compound as a white solid (78 mg, 58%), m.p. = 262-265°C (dec.).

| Anal. Calc'd. for C₂₃H₂₃ClN₄O₃•0.75H₂O: | | | |
|---|---|---|---|
| | C, 61.06; | H, 5.46; | N, 12.38. |
| Found | C, 60.99; | H, 5.60; | N, 12.07. |

### EXAMPLE 474A

### (±)-1-[(5,6,7,8-Tetrahydro-2-napthalenecarbonitrile)-6-yl]-2',3',7',8'-tetrahydro-2',8'-dioxo-spiro[piperidine-4,6'(1'H)-pyrano[2,3-f]benzimidazole] hydrochloride

In the manner of Example 472, (±)-6,7-diamino-3,4-dihydro-1'-[(5,6,7,8-tetrahydro-2-naptha-lenecarbonitrile)-6-yl]-spiro[(2H)-1-benzopyran-2,4'-piperidine]-4-one (141 mg, 0.35 mmol) gave, after flash column chromatography on silica gel, eluting with CH₂Cl₂/MeOH; 95:5, and salt formation, the title compound as an off-white solid (98 mg, 60%), m.p. = 248-250°C.

| Anal. Calc'd. for C₂₅H₂₅ClN₄O₃•0.25EtOH•0.75H₂O: | | | |
|---|---|---|---|
| | C, 62.50; | H, 5.76; | N, 11.43. |
| Found | C, 62.47; | H, 5.67; | N, 11.43. |

### EXAMPLE 474B

### 1-Hexyl-2',3',7',8'-tetrahydro-2',8'-dioxospiro-[piperidine-4,6'(1H)-pyrano[2,3-f]benzimidazole]-hydrochloride

In the manner of Example 457, 6,7-diamino-3,4-dihydro-1'-hexylspiro[(2H)-1-benzopyran-2,4'-piperidin]-4-one (298 mg, 0.90 mmol) gave, after chromatography and salt formation, the title compound as an pale yellow solid (156 mg, 45%) mp 293-297°.

| Anal. Calc'd for C₂₀H₂₇N₃O₃•HCl•0.3 H₂O: | | | |
|---|---|---|---|
| | C, 60.15; | H, 7.22; | N, 10.52. |
| Found | C, 60.17; | H, 6.98; | N, 10.50. |

### EXAMPLE 474C

### 1-Heptyl-2',3',7',8'-tetrahydro-2',8'-dioxospiro-[piperidine-4,6'(1H)-pyrano[2,3-f]benzimidazole]-hydrochloride

In the manner of Example 457, 6,7-diamino-3,4-dihydro-1'-heptylspiro[(2H)-1-benzopyran-2,4'-piperidin]-4-one (275 mg, 0.80 mmol) gave, after chromatography and salt formation, the title compound as a white solid (137 mg, 42%) mp 299-301°.

| Anal. Calc'd for C₂₁H₂₉N₃O₃•HCl•0.3 H₂O: | | | |
|---|---|---|---|
| | C, 61.02; | H, 7.46; | N, 10.17. |
| Found | C, 61.04; | H, 7.59; | N, 10.16. |

### EXAMPLE 475

### 1'-(2-Phenylethyl)-7,8-dihydro-8-oxospiro[6H-1,3-dioxolo[4,5-g][1]benzopyran-6,4'-piperidine]hydrochloride

Pyrrolidine (83 ml, 71 mg, 1 mmol) was added to a mixture of 1-(6-hydroxy-1,3-benzodioxol-5-yl)-ethanone [prepared as described by K. Fukui and M. Nakayama, Bull. Chem. Soc. Jap., 1963, 37, 300.] (180 mg, 1 mmol) in methanol (1 ml). The mixture was stirred for 10 minutes, then 1-(2-phenylethyl)-4-piperidinone (203 mg, 1 mmol) in methanol (1 ml) was added and the mixture was heated under reflux for 1.5 hours. The mixture was cooled, the solvent was evaporated under reduced pressure and aqueous sodium hydrogen carbonate (saturated, 20 ml) was added. The mixture was extracted with dichloromethane (3 x 20 ml) and the combined organic fractions were dried (Na₂SO₄) and evaporated under reduced pressure. The residue was purified by column chromatography on silica gel, eluting with CH₂Cl₂/MeOH/NH₃(Aq.) (98:2:0.2) to give a yellow glass which was suspended in ethanol (5 ml) and treated with ethanolic HCl (6N, 0.5 ml). The mixture was stirred for 1 hour, cooled and the precipitate was collected and dried in vacuo to give the title compound as a white solid (153 mg, 38%), m.p. >285°C.

| Anal. Calc'd. for C₂₂H₂₄ClNO₄: | | | |
|---|---|---|---|
| | C, 65.75; | H, 6.02; | N, 3.48. |
| Found | C, 65.61; | H, 6.22; | N, 3.42. |

### EXAMPLE 476

### 1'-[2-(4-Cyanophenyl)ethyl]-7,8-dihydro-8-oxospiro[6H-1,3-dioxolo[4,5-g][1]benzopyran-6,4'-piperidine] hydrochloride

As described in Example 475, 1-(6-hydroxy-1,3-benzo-dioxol-5-yl)ethanone (180 mg, 1 mmol) was condensed with 1-[2-(4-cyanophenyl)ethyl]-4-piperidinone (228 mg, 1 mmol) to give, after chromatography and salt formation, the title compound as a white solid (173 mg, 41%), m.p. 268-270°C (dec.).

| Anal. Calc'd. for C₂₃H₂₃ClN₂O₄: | | | |
|---|---|---|---|
| | C, 64.71; | H, 5.43; | N, 6.56. |
| Found | C, 64.61; | H, 5.61; | N, 6.46. |

### EXAMPLE 479

### (±)- and (+)-3,4-Dihydro-1'-[(6-cyano-1,2,3,4-tetrahydronapthalene)-2-yl]-6-methanesulfonamidospiro[(2H)-1-benzopyran-2,4'-piperidine]-4-ol hydrochloride

In the manner of Example 429, (±)-3,4-dihydro-1'-[6-cyano-1,2,3,4-tetrahydronapthalene)-2-yl]-6-methanesulfonamido-spiro[(2H)-1-benzopyran-2,4'-piperidine]-4-one (651 mg, 1.4 mmol) was reduced to give, after chromatography, the alcohol as a foam (618 mg, 95%). A sample (200 mg) was treated with ethanolic HCl to give the (±)-hydrochloride as a white solid (164 mg), m.p. 216-218°C.

| Anal. Calcd. for C₂₅H₃₀ClN₃O₄S•0.33H₂O: | | | |
|---|---|---|---|
| | C 58.87; | H 6.06; | N 8.23. |
| Found | C 58.96; | H 5.95; | N 8.18. |

In the same way, (+)-3,4-dihydro-1'-[(6-cyano-1,2,3,4-tetrahydronapthalene)-2-yl]-6-methanesulfonamido-spiro[(2H)-1-benzopyran-2,4'-piperidine]-4-one gave, after chromatography and salt formation, the (+)-hydrochloride, m.p. 182-184°C, [α]_{d}+43.0° (c 0.128, MeOH). (saturated, 40 ml) and water (10 ml) were added. The mixture was extracted with dichloromethane (3 X 40 ml) and the combined organic fractions were dried (Na₂SO₄) and evaporated under reduced pressure to give a tan foam which was purified by flash column chromatography on silica gel, eluting with CH₂Cl₂/ MeOH/NH₃(Aq.) (97:3:0.3) to give a foam (289 mg, 86%). A sample (115 mg) was treated with ethanolic HCl to give the (±)-hydrochloride as a white solid (104 mg), m.p. 272-274°C. In the same way, (+)-3,4-dihydro-1'-[6-cyano-1,2,3,4-tetrahydronapthalene)-2-yl]-6-methanesulfonamido-spiro[(2H)-1-benzopyran-2,4'-piperidine]-4-ol gave, after chromatography and salt formation, the (+)-hydrochloride, m.p. 276-278°C, [α]_{d}+48.2°(c 0.112,MeOH).

### EXAMPLE 481

### (+)-3,4-Dihydro-1'-[(6-cyano-1,2,3,4-tetrahydronapthalene)-2-yl]-6-methanesulfonamido-spiro[(2H)-1-benzopyran-2,4'-piperidine] hydrochloride

(+)-1'-[(6-Cyano-1,2,3,4-tetrahydronapthalene)-2-yl]-6-methanesulfonamido-spiro[(2H)-1-benzopyran-2,4'-piperidine] (359 mg, 0.8 mmol) was suspended in methanol (17 ml). 5% Palladium on carbon (25 mg) was added and the mixture was stirred under hydrogen (1 Atm.). Further 5% palladium on carbon (20 mg portions) was added after 7, 24 and 31 h. After 48 h, the mixture was filtered through celite, washing with methanol, and evaporated under reduced pressure to give a foam which was purified by flash column chromatography on silica gel, eluting with CH₂Cl₂/MeOH/NH₃(Aq.) (96:4:0.4) to give a foam (312 mg, 86%). A sample (300 mg) was treated with ethanolic HCl to give the (+)-hydrochloride as a white solid (261 mg), m.p. 284-286°C, [α]_{d}+55.6° (c 0.126, MeOH).

### EXAMPLE 482

### 7-Amino-3,4-dihydro-6-nitro-1'-(2-phenylethyl)-spiro[(2H)-1-benzopyran-2,4'-piperidine]-4-one

In the manner of Example 457, N-(4-acetyl-5-hydroxy-2-nitrophenyl)acetamide (1.07 g, 4.5 mmol) and 1-(2-phenylethyl)-4-piperidinone (910 mg, 4.5 mmol) in methanol (15 ml) gave, after chromatography, the pyranone as an orange foam, (1.23 g, 72%).
¹HNMR (CDCl₃) δ: 8.80 (1H, s), 7.28 (2H, m), 7.21 (3H, m), 6.4 (2H, br s), 6.28 (1H, s), 2.75-2.60 (6H, m), 2.70 (2H, s), 2.49 (2H, m), 2.02 (2H, m), and 1.79 (2H, m).

### EXAMPLE 483

### 7-Amino-3,4-dihydro-6-nitro-1'-[(2,3-dihydro-1H-indene)-2-yl]-spiro[(2H)-1-benzopyran-2,4'-piperidine] -4-one dihydrochloride

In the manner of Example 457, N-(4-acetyl-5-hydroxy-2-nitrophenyl)acetamide (1.07 g, 4.5 mmol) and 1-[(2,3-dihydro-1H-indene)-2-yl]-4-piperidinone (970 mg, 4.5 mmol) in methanol (15 ml) gave, after hydrolysis in refluxing ethanol (3 ml) and HCl-H₂O (6M, 1 ml), the dihydrochloride as a yellow solid, (0.87 g, 41%).
¹HNMR (DMSO) δ: 11.5-11.1 (1H, s), 8.47 (1H, s), 8.00 (2H, br s), 7.24 (4H, m), 6.53 (1H, s), 4.15 (1H, m), 3.47 (2H, m), 3.32 (6H, m), 2.84 (2H, s), and 2.28-2.04 (4H, m).

### EXAMPLE 484

### (±)-7-Amino-3,4-dihydro-6-nitro-1'-[(1,2,3,4- tetrahydronapthalene)-2-yl]-spiro[(2H)-1-benzopyran-2,4'-piperidine]-4-one

In the manner of Example 457, N-(4-acetyl-5-hydroxy-2-nitrophenyl)acetamide (1.07 g, 4.5 mmol) and (±)-1-[(1,2,3,4-tetrahydronapthalene)-2-yl]-4-piperidinone (1.03 g, 4.5 mmol) in methanol (9 ml) gave, after acid hydrolysis in refluxing ethanol (20 ml) and HCl-H₂O (6M, 10 ml) and chromatography, the pyranone as an orange foam, (418 mg, 23%).
¹HNMR (CDCl₃) δ: 8.80 (1H, s), 7.11 (4H, m), 6.4 (2H, br s), 6.30 (1H, s), 2.91-2.71 (11H, m), and 2.52-1.60 (6H, m).

### EXAMPLE 485

### 7-Amino-3,4-dihydro-6-nitro-1'-[(6,7,8,9-tetrahydro-5H-benzocycloheptene)-7-yl]-spiro[(2H)-1-benzopyran-2,4'-piperidine]-4-one

In the manner of Example 457, N-(4-acetyl-5-hydroxy-2-nitrophenyl)acetamide (785 mg, 3.3 mmol) and 1-[(6,7,8,9-tetrahydro-5H-benzocycloheptene)-7-yl]-4-piperidinone (802 mg, 3.3 mmol) in methanol (7 ml) gave, after acid hydrolysis in refluxing ethanol (20 ml) and HCl-H₂O (6M, 10 ml) and chromatography, the pyranone as an orange solid, (217 mg, 16%).
¹HNMR (CDCl₃) δ: 8.78 (1H, s), 7.12 (4H, s), 6.4 (2H, br s), 6.26 (1H, s), 2.9-2.5 (11H, m), and 2.2-1.35 (8H, m).

### EXAMPLE 486

### 6,7-Diamino-3,4-dihydro-1'-(2-phenylethyl)-spiro[(2H)-1-benzopyran-2,4'-piperidine]-4-one

In the manner of Example 462, 7-amino-3,4-dihydro-6-nitro-1'-(2-phenylethyl)-spiro[(2H)-1-benzopyran-2,4'-piperidine]-4-one (1.22 g, 3.2 mmol) was reduced to give, after chromatography, the pyranone as a yellow foam (790 mg, 70%).
¹HNMR (CDCl₃) δ: 7.32-7.10 (6H, m), 6.22 (1H, s), 4.16 (2H, br s), 3.05 (2H, br s), 2.80 (2H, m), 2.75-2.53 (4H, m), 2.61(2H, s), 2.48 (2H, m), 2.07 (2H, m), and 1.73 (2H, m).

### EXAMPLE 487

### 6,7-Diamino-3,4-dihydro-1'-[(2,3-dihydro-1H-indene)-2-yl]-spiro[(2H)-1-benzopyran-2,4'-piperidine]-4-one

In the manner of Example 462, 7-amino-3,4-dihydro-6-nitro-1'-[(2,3-dihydro-1H-indene)-2-yl]-spiro[(2H)-1-benzopyran-2,4'-piperidine]-4-one dihydrochloride (863 mg, 1.85 mmol) was reduced to give the pyranone as an orange solid (301 mg, 45%).
¹HNMR (CDCl₃) δ: 7.16 (5H, m), 6.23 (1H, s), 4.16 (2H, br s), 3.22 (1H, m), 3.09 (4H, m), 2.90 (2H, m), 2.72 (2H, m), 2.61 (2H, s), 2.47 (2H, m), 2.08 (2H, m) and 1.75 (2H, m).

### EXAMPLE 488

### (±)-6,7-Diamino-3,4-dihydro-1'-[(1,2,3,4-tetrahydro-napthalene)-2-yl]-spiro[(2H)-1-benzopyran-2,4'-piperidine]-4-one

In the manner of Example 462, (±)-7-amino-3,4-dihydro-6-nitro-1'-[(1,2,3,4-tetrahydronapthalene)-2- yl]-spiro[(2H)-1-benzopyran-2,4'-piperidine]-4-one (407 mg, 1.00 mmol) was reduced to give, after chromatography, the pyranone as an orange foam (331 mg, 88%).
¹HNMR (CDCl₃) δ: 7.26 (1H, s), 7.10 (4H, m), 6.25 (1H, s), 4.17 (2H, br s), 3.05 (2H, br s), 3.00-2.78 (7H, m), 2.72 (4H, m), 2.62 (2H, s), 2.10 (2H, m), and 1.72 (2H, m).

### EXAMPLE 489

### 6,7-Diamino-3,4-dihydro-1'-[(6,7,8,9-tetrahydro-5H-benzocycloheptene)-7-yl]-spiro[(2H)-1-benzopyran-2,4'-piperidine]-4-one

In the manner of Example 462, 7-amino-3,4-dihydro-6-nitro-1'-[(6,7,8,9-tetrahydro-5H-benzocyclo-heptene)-7-yl]-spiro[(2H)-1-benzopyran-2,4'-piperidine]-4-one (215 mg, 0.51 mmol) was reduced to give, after chromatography, the pyranone as an orange foam (146 mg, 73%).
¹HNMR (CDCl₃) δ: 7.20 (1H, s), 7.12 (4H, s), 6.20 (1H, s), 4.14 (2H, br s), 3.03 (2H, br s), 2.82 (2H, m), 2.71 (5H, m), 2.58 (2H, s), 2.53 (2H, m), 2.12 (2H, m), 2.02 (2H, m), and 1.42 (2H, m).

### EXAMPLE 490

### 1-(2-Phenylethyl)-7',8'-dihydro-8'-oxo-spiro[piperidine-4,6'(1'H)-pyrano[2,3-f]benzimidazole] Dihydrochloride

In the manner of Example 467, 6,7-diamino-3,4-dihydro-1'-(2-phenylethyl)-spiro[(2H)-1-benzopyran-2,4'-piperidine]-4-one (316, mg, 0.90 mmol) gave, after chromatography and salt formation, the dihydrochloride as a light tan solid (107 mg, 27%), m.p. 247-249°C.

| Anal Calcd. for C₂₂H₂₅Cl₂N₃O₂•0.05EtOH•0.30H₂O: | | | |
|---|---|---|---|
| | C 60.04; | H 5.91; | N 9.51. |
| Found | C 60.08; | H 5.70; | N 9.45. |

### EXAMPLE 491

### 1-[(2,3-Dihydro-1H-indene)-2-yl]-7',8'-dihydro-8'-oxo- spiro[piperidine-4,6'(1'H)-pyrano[2,3-f]benzimid azole] Dihydrochloride

In the manner of Example 467, 6,7-diamino-3,4-dihydro-1'-[(2,3-dihydro-1H-indene)-2-yl]-spiro [(2H)-1-benzopyran-2,4'-piperidine]-4-one (73, mg, 49 mmol) gave, after chromatography and salt formation, the dihydrochloride as a light tan solid (73 mg, 49%), m.p. 233-236°C.

| Anal. Calcd. for C₂₃H₂₅Cl₂N₃O₂•0.30H₂O: | | | |
|---|---|---|---|
| | C 61.14; | H 5.71; | N 9.30. |
| Found | C 61.14; | H 5.58; | N 9.22. |

### EXAMPLE 492

### (±)-1-[(1,2,3,4-Tetrahydronapthalene)-2-yl]-7',8'-dihydro-8'-oxo-spiro[piperidine-4,6'(1'H)-pyrano[2,3-f]benzimidazole] dihydrochloride

In the manner of Example 467, (±)-6,7-diamino-3,4-dihydro-1'-[(1,2,3,4-tetrahydronapthalene)-2-yl]-spiro[(2H)-1-benzopyran-2,4'-piperidine]-4-one (132 mg, 0.35 mmol) gave, after chromatography and salt formation, the dihydrochloride as a tan solid (90 mg, 55%), m.p. 233-236°C.

| Anal. Calc'd. for C₂₄H₂₇Cl₂N₃O₂•0.55EtOH: | | | |
|---|---|---|---|
| | C 62.06; | H 6.29; | N 8.65. |
| Found | C 62.08; | H 6.66; | N 8.75. |

### EXAMPLE 493

### 1-[(6,7,8,9-Tetrahydro-5H-benzocycloheptene)-7-yl]-7',8'-dihydro-8'-oxospiro[piperidine-4,6'(1'H)-pyrano[2,3-f]benzimidazole] dihydrochloride

In the manner of Example 467, 6,7-diamino-3,4-dihydro-1'-[(6,7,8,9-tetrahydro-5H-benzocyclo-heptene)-7-yl]-spiro[(2H)-1-benzopyran-2,4'-piperidine]-4-one (29, mg, 0.075 mmol) gave, after chromatography and salt formation, the dihydrochloride as a tan solid (17 mg, 47%), m.p. 232-235°C.

| Anal. Calc'd. for C₂₅H₂₉Cl₂N₃O₂: | | | |
|---|---|---|---|
| | C 63.29; | H 6.16; | N 8.86. |
| Found | C 62.91; | H 6.44; | N 8.55. |

### EXAMPLE 494

### 1-(2-Phenylethyl)-2',3',7',8'-tetrahydro-2',8'-dioxospiro[piperidine-4,6'(1'H)-pyrano[2,3-f]benzimidazole] hydrochloride

In the manner of Example 472, (6,7-diamino-3,4-dihydro-1'-(2-phenylethyl)-spiro[(2H)-1-benzopyran-2,4'-piperidine]-4-one (316 mg, 0.90 mmol) gave, after chromatography and salt formation, the hydrochloride as a pale yellow solid (137 mg, 36%), m.p. 298-300°C.

| Anal. Calc'd. for C₂₂H₂₄ClN₃O₃•0.50H₂O | | | |
|---|---|---|---|
| | C 62.48; | H 5.96; | N 9.84. |
| Found | C 62.49; | H 5.69; | N 9.89. |

### EXAMPLE 495

### 1-[(2,3-Dihydro-1H-indene)-2-yl]-2',3',7',8'-tetrahydro-2',8'-dioxo-spiro[piperidine-4,6'(1'H)-pyrano-[2,3-f]benzimidazole] Hydrochloride

In the manner of Example 472, 6,7-diamino-3,4-dihydro-1'-[(2,3-dihydro-1H-indene)-2-yl]-spiro [(2H)-1-benzopyran-2,4'-piperidine]-4-one (180 mg, 0.5 mmol) gave, after chromatography and salt formation, the hydrochloride as a pale orange solid (126 mg, 58%), m.p. 257-260°C.

| Anal. Calc'd. for C₂₃H₂₄ClN₃O₃•0.65EtOH•0.75H₂O: | | | |
|---|---|---|---|
| | C, 62.18; | H, 6.31; | N, 8.95. |
| Found | C, 62.18; | H, 6.10; | N, 8.81. |

### EXAMPLE 496

### (±)-1-[(1,2,3,4-Tetrahydronapthalene)-2-yl]-2',3',7', 8'-tetrahydro-2',8'-dioxo-spiro[piperidine-4,6'(1'H)-pyrano[2,3-f]benzimidazole] Hydrochloride

In the manner of Example 472, (±)-6,7-diamino-3,4-dihydro-1'-[(1,2,3,4-tetrahydronapthalene)-2-yl]spiro[(2H)-1-benzopyran-2,4'-piperidine]-4-one (170 mg, 0.45 mmol) gave, after chromatography and salt formation, the hydrochloride as a yellow solid (89 mg, 45%), m.p. 254-257°C.

| Calcd. for C₂₄H₂₆ClN₃O₃•0.05EtOH•1.20H₂O: | | | |
|---|---|---|---|
| | C 62.40; | H 6.24; | N 9.06. |
| Found | C 62.42; | H 5.97; | N 8.94. |

### EXAMPLE 497

### 1-[(6,7,8,9-Tetrahydro-5H-benzocycloheptene)-7-yl]-2',3',7',8'-tetrahydro-2',8'-dioxo-spiro[piperidine-4,6'(1'H)-pyrano[2,3-f]benzimidazole] Hydrochloride

In the manner of Example 472, 6,7-diamino-3,4-dihydro-1'-[(6,7,8,9-tetrahydro-5H-benzocycloheptene-7-yl]-spiro[(2H)-1-benzopyran-2,4'-piperidine]-4-one (98 mg, 0.25 mmol) gave, after chromatography and salt formation, the hydrochloride as a yellow solid (47 mg, 41%), m.p. 260-263°C.

| Calc'd. for C₂₅H₂₈ClN₃O₃•1.50H₂O: | | | |
|---|---|---|---|
| | C 62.42; | H 6.50; | N 8.74. |
| Found | C 62.32; | H 6.35; | N 8.59. |

In the manner of Example 339, step 8, 1-(3-hydroxypyridinyl)ethanone can be condensed with suitably 4-substituted piperidinones using pyrrolidine in methanol to give the following 1'-substituted 2,4-dihydro(2H-pyrano[3,2-b]pyridine-2,4'-piperidine)-4-ones of Table XXXI.

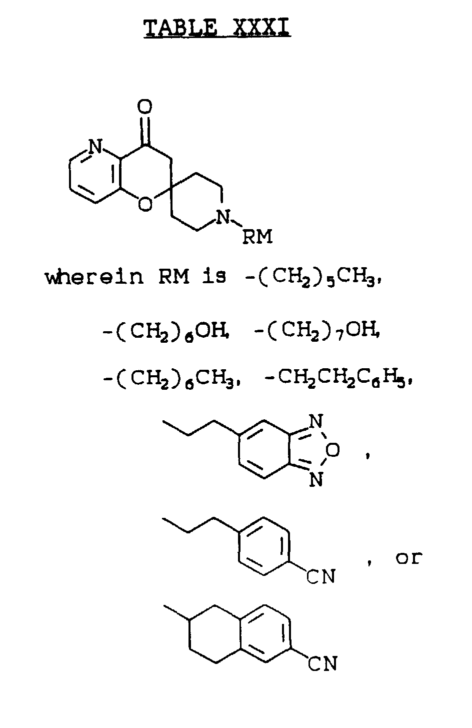

In the manner of Example 429, the appropriate pyran-4-ones can be reduced with sodium borohydride in ethanol to give the following 1'-substituted 3,4-dihydro-6-methanesulfonyl-spiro[(2H)-benzopyran-2,4'-piperidine]-4-ols, 1-substituted 7',8'-dihydro-8'-hydroxy-spiro[piperidine-4,6'(1'H)-pyranol[2,3-f] benzimidazole]'s and 1-substituted 2',3',7',8'-tetrahydro-8'-hydroxy-2'-oxo-spiro[piperidine-4,6'(1'H)-pyrano[2,3-f]benzimidazole]'s of Table XXXII.

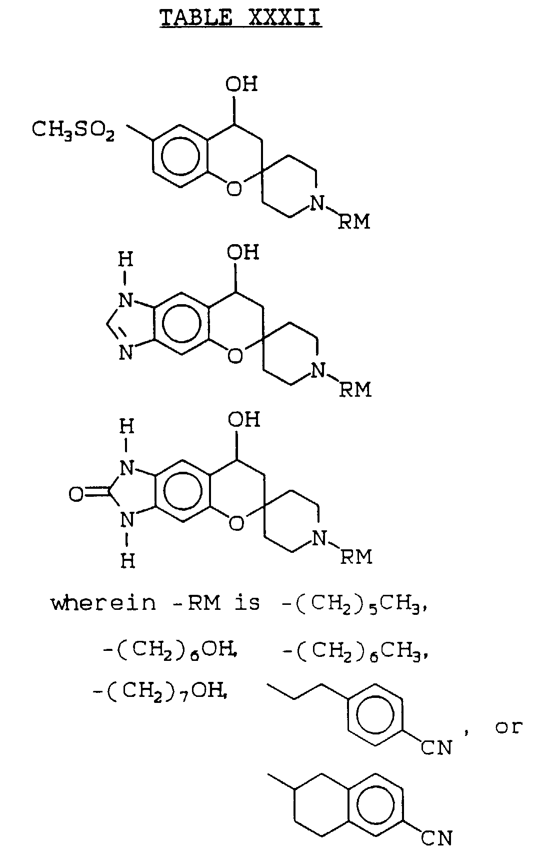

In the manner of Example 480, the appropriate pyran-4-ols can be dehydrated with p-toluenesulfonic acid in toluene to give the following 1'-substituted 6-methanesulfonyl-spiro[(2H)-benzopyran-2,4'-piperidines]'s, 1-substituted spiro[piperidine-4,6'(1'H)-pyrano[2,3-f]-benzimidazole]'s and 1-substituted 2',3'-dihydro-2'-oxo-spiro-[piperidine-4,6'(1'H)-pyrano[2,3-f]benzimidazole]'s of Table XXXIII.

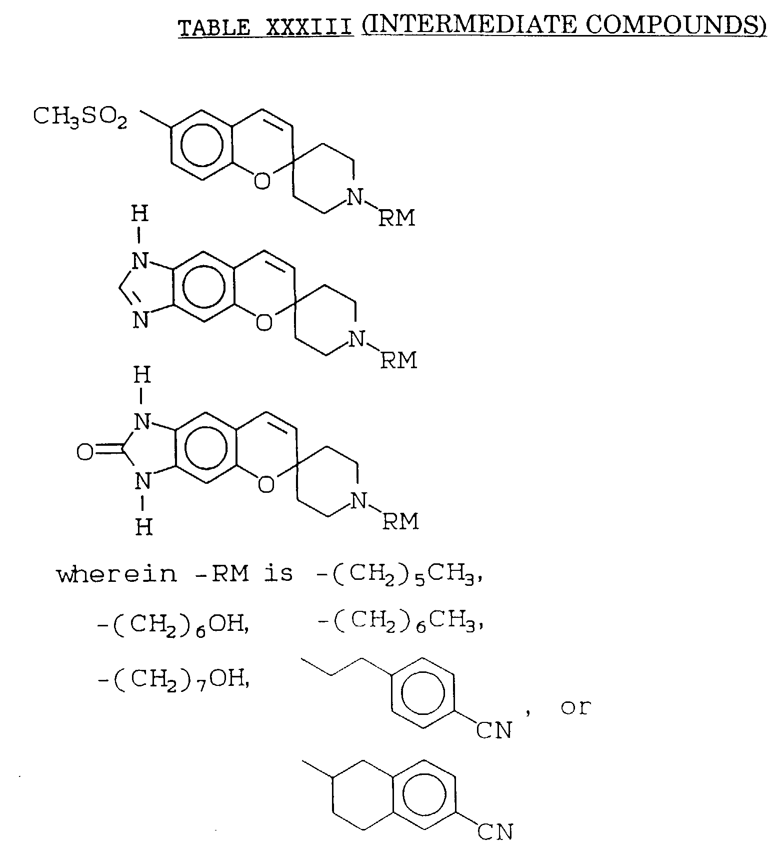

In the manner of Example 481, the appropriate pyrans can be hydrogenated over 5% palladium on carbon to give the following 1'-substituted 3,4-dihydro-6-methanesulfonyl-spiro[(2H)-benzopyran-2,4'-piperidine]'s, 1-substituted 7',8'-dihydro-spiro[piperidine-4,6'(1'H)-pyrano[2,3-f]benzimidazole]'s and 1-substituted 2',3',7',8'-tetrahydro-2'-oxo-spiro [piperidine-4,6'(1'H)-pyrano[2,3-f]benzimidazole]'s of Table XXXIV.

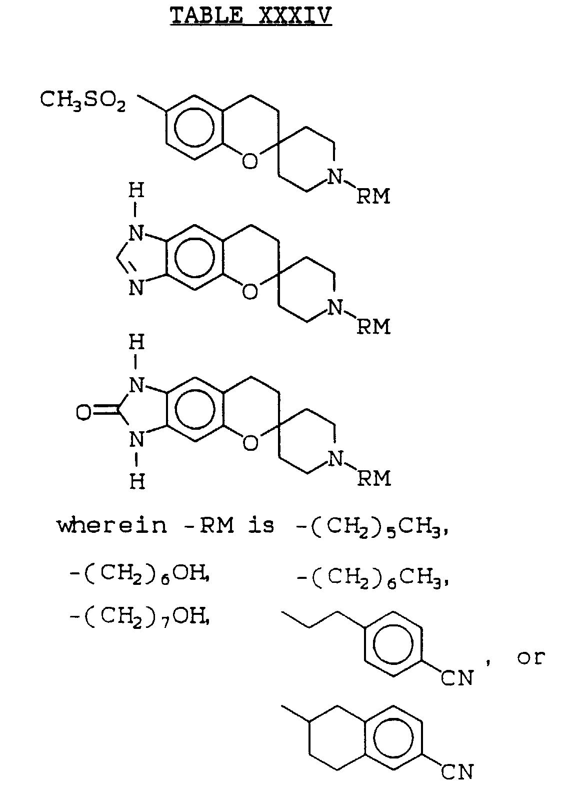

In a manner similar to that of Example 339, step 8, 5-methylsulfonyl-2-hydroxypropiophenone can be condensed with suitably 4-substituted piperidinones using pyrrolidine in methanol to give the following 3,4-dihydro-3-methyl-6-methanesulfonyl-1'-subsituted-spiro[(2H)-1-benzopyran-2,4'-piperidine]-4-ones of Table XXXV.

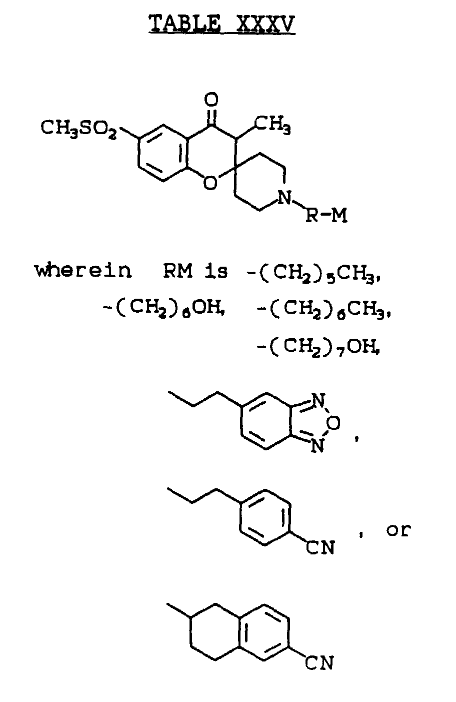

Employing the procedures of Example 416 and substituting the appropriate alcohol and the appropriate nitrile, there are produced the compounds of Table XXXVIII.

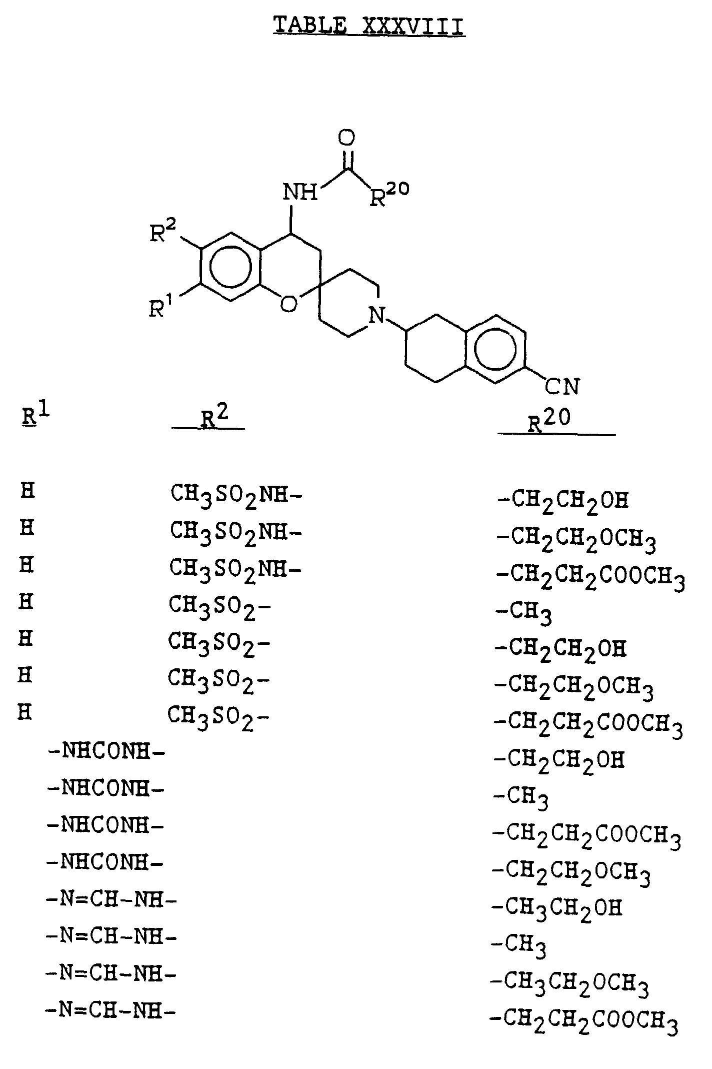

The following compounds are prepared by methods previously described: 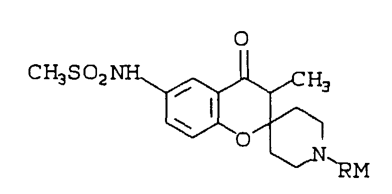 wherein RM is

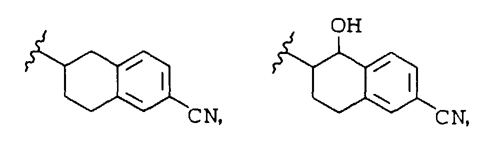

-(CH₂)₆-OH,

-(CH₂)₇OH,

-(CH₂)₆CH₃,

or

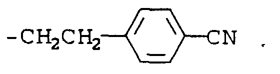

The following compounds are prepared by methods previously described: 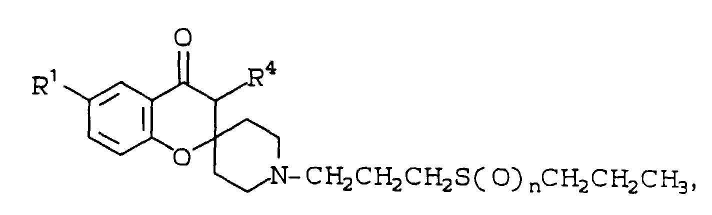 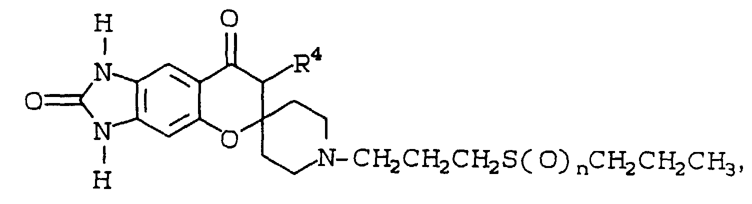 or 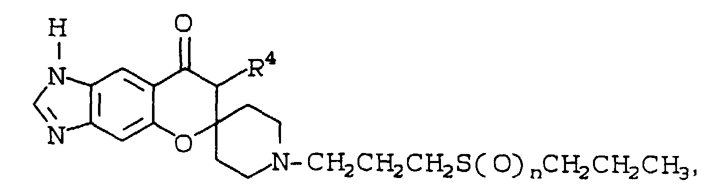 wherein R¹ is CH₃SO₂NH or CH₃SO₂,
n is 0, 1 or 2 and R⁴ is H or C₁₋₆ alkyl.

The following compounds are prepared by methods previously described: 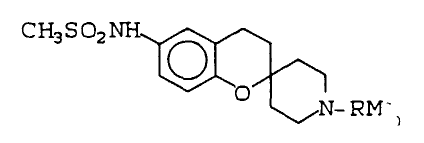 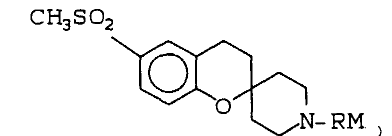 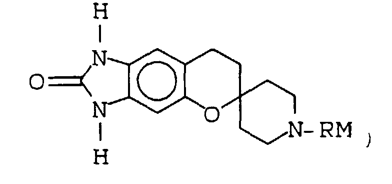 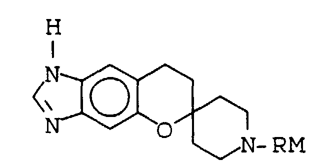 wherein -RM is

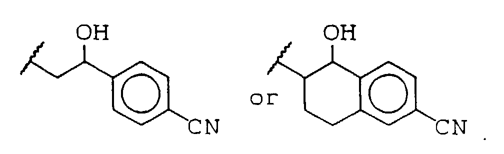

In a manner similar to that of Example 92, Step B, 3,4-dihydro-6-methanesulfonamidospiro-[(2H)-1-benzopyran-2,4'-piperidine] may be reacted with an appropriate bromide to give the compounds of Table XXXIX.

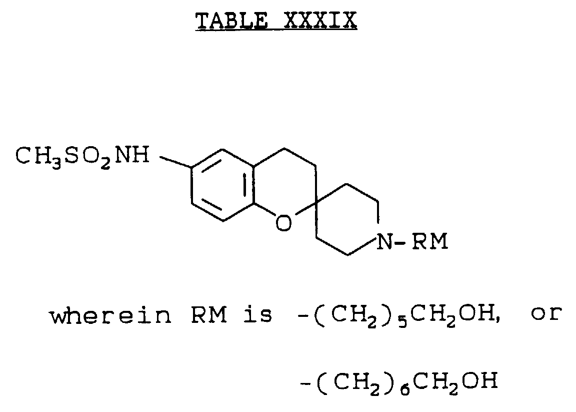

Employing the procedures previously described, there are produced the compounds of Table XXXX.

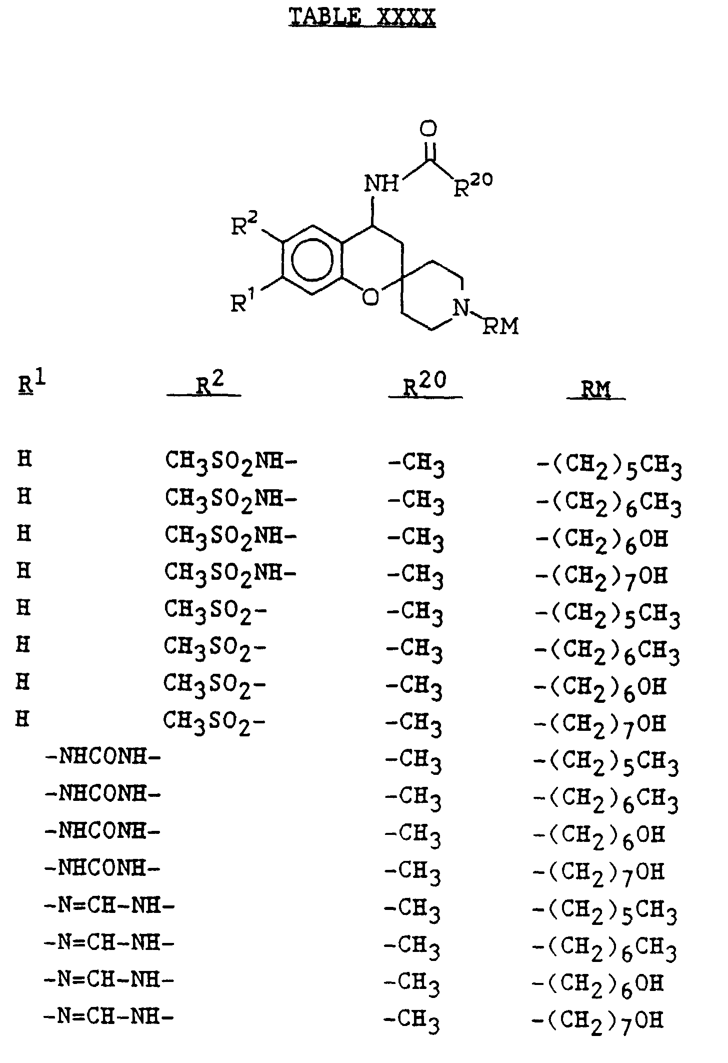

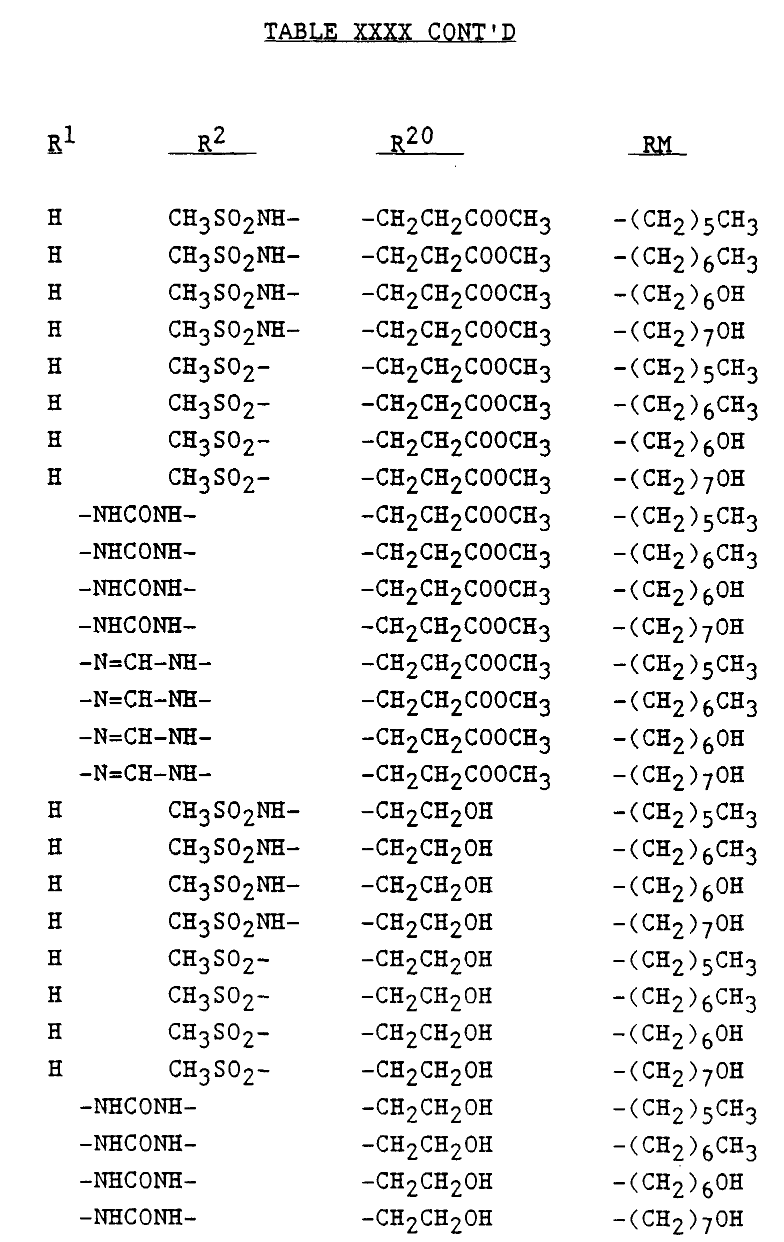

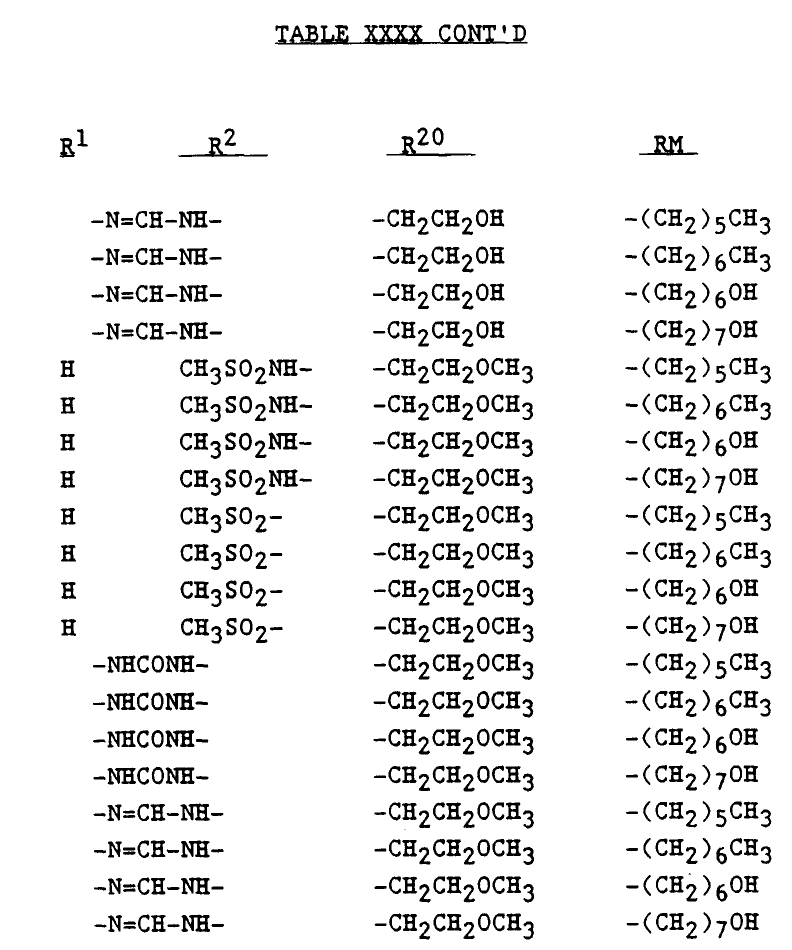

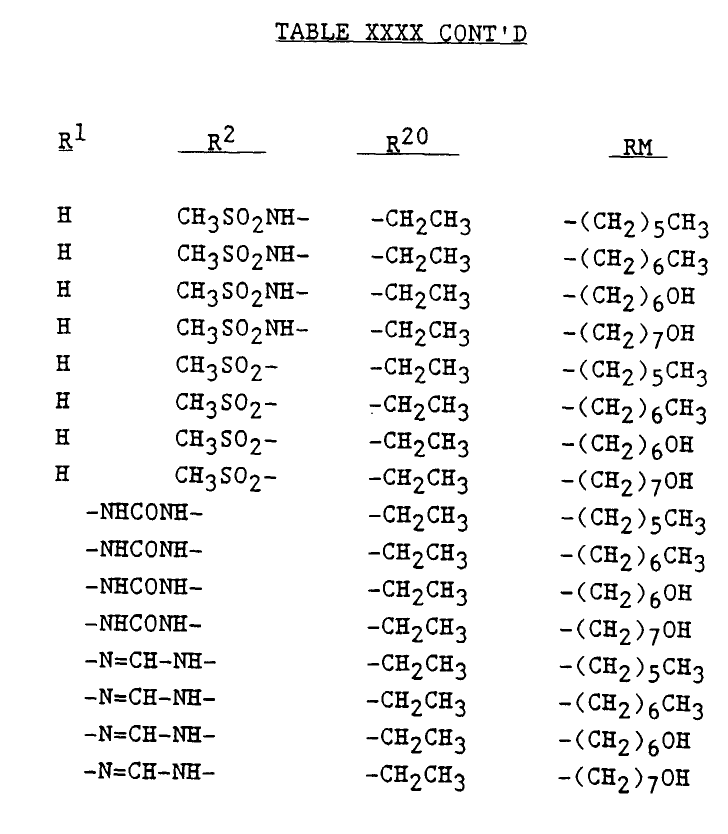

### EXAMPLE 503

### In Vitro Test for Class III Antiarrhythmic Activity

### Purpose:

This in vitro assay is designed to assess possible potassium channel blocking activity of a compound based on its ability to prolong effective refractory period (ERP) in isolated papillary muscle.

### Tissue Preparation:

Ferrets (700 to 1200 grams) are anesthetized with 0.7 ml of a mixture of xylazine and ketamine HCL in 1: 7 ratio. Papillary muscles from the right ventricle are quickly excised from the isolated heart and mounted in 50 ml organ baths containing Krebs-Henseleit solution (pH=7.2-7.4) at 37°C. The composition of the solution in millimoles per liter are as follows: NaCl, 118; KCl, 4.7; NaHCO₃,23; CaCl₂ 2H₂O 2.0; MgSO₄7H₂O, 1.2; KH₂PO₄, 1.2; Dextrose, 11.1. Timolol (10-⁷M) is added to the solution to block the effects of catecholamines released during stimulation of the muscles. This solution is aerated with 95% O₂ and 5% CO₂. The tissue is stimulated at 1 Hz at one msec pulse duration by a square wave stimulator at a voltage 30% above threshold through platinum electrodes that touch the tissue just above the bottom attachment point. The tendenous end of the tissue is connected by thread to an isometric force transducer leading to a polygraph.

### Effective Refractory Period (ERP) Measurement:

The ERP is determined by a standard 2 pulse protocol. Both pulses are stimulated at 1.3 x voltage threshold. While pacing the tissue at a basal frequency of 1 Hz, a single extrastimulus is delivered after a variable time delay. The shortest delay resulting in a propagated response is defined as the ERP.

### Protocol:

1. Tissues are mounted with a resting tension of 0.5 gms, stimulated at 1 Hz, and allowed to equilibrate for 2 hours with washings at 15-20 minute intervals.
2. Voltage is adjusted to 30% above threshold and resting tension is adjusted for maximum developed tension, and the tissue is allowed 5 min. reequilibration time.
3. Effective refractory period is measured at 1 Hz. Changes in resting tension and developed force are noted.
4. After equilibration, ERP's and developed force are measured at 30 minutes following the addition of increasing cumulative concentrations for test agent to the organ bath. Four to five concentrations of test agents were used to generate a concentration-response curve.
5. Four tissues per compound are tested.

### Results

Employing the above protocol, it has been found that the effective concentration of the compounds of this invention required to increase the refractory period by an increment of 25% above base-line is less than or equal to 10 micromolar, i.e. EC₂₅ ≤ 10µM, whereas sotalol in the same protocol has an EC₂₅ ∼20 micromolar.

### EXAMPLE 504

### Preparation of intravenous solutions

A solution containing 0.5 mg of active ingredient per ml of injectable solution is prepared in the following manner.

A mixture of 0.5 mg of active ingredient is dissolved in 1 ml of acetate buffer. The pH is adjusted using hydrochloric acid or aqueous sodium hydroxide to about pH 5.5.

If it is desired that the intravenous solution be used for multi-dose purposes, 1.0 mg of methyl-p-hydroxy benzoate (methyl paraben) and 0.10 mg of n-propyl-p-hydroxy benzoate (propyl paraben) are mixed with the other solids before adding water to dissolve the solids. The solution is prepared and stored in such a manner that it is suitably protected from the deleterious effects of the atmosphere. One method by which this can be accomplished is by preparation and storage of the solution in an atmosphere of nitrogen. The resulting solution is sterilized by autoclaving. injectable solutions comprising 0.001, 0.01, and 0.1 mg, respectively, of active ingredient per ml of solution are similarly prepared substituting the indicated amount for the above-illustrated 10 mg quantity. Bulk injectable solutions of convenient volume for subsequent delivery in unit dosage form are readily prepared following the above procedure.

### EXAMPLE 505

### Tablet Preparation

Tablets containing 1.0, 2.0, 25.0, 26.0, 50.0 and 100.0 mg, respectively, of active ingredient are prepared as illustrated below.

| TABLE FOR DOSES CONTAINING FROM 1-25 MG OF THE ACTIVE COMPOUND | | | |
|---|---|---|---|
| | Amount - mg | | |
| Active ingredient | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100 MG OF THE ACTIVE COMPOUND | | | |
|---|---|---|---|
| | Amount - mg | | |
| Active ingredient | 26.0 | 50.0 | 100.0 |
| Microcrystalline cellulose | 25.0 | 100.0 | 200.0 |
| Modified food corn starch | 2.21 | 4.25 | 8.5 |
| Magnesium stearate | .39 | 0.75 | 1.5 |

All of the active compound, cellulose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.0 mg, 50.0 mg, and 100.0 mg of active ingredient per tablet.

## Claims

1. The use of a compound of structural formula: 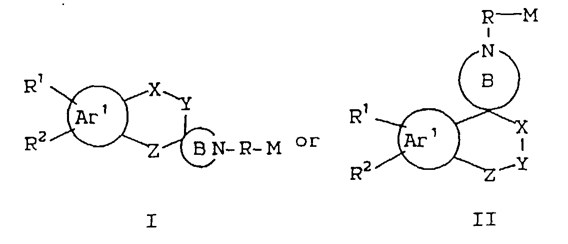 or a pharmaceutically acceptable salt thereof, wherein Ar¹ is an aromatic ring selected from
1) benzo, and
2) thieno,
the ring system comprising X, Y and Z is selected from: 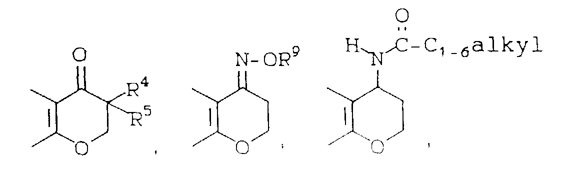 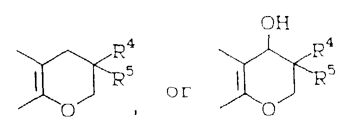 wherein:
R⁴ and R⁵ are independently
a) hydrogen, or
b) C₁₋₆alkyl ;
R⁹ is
a) hydrogen, or
b) C₁₋₆alkyl, unsubstituted or substituted with -COOR⁵;
R-M is n-hexyl, unsubstituted or substituted with hydroxy or cyano; or
R is a bond or C₁₋₈alkyl and
M is Ar²R³, wherein Ar²R³ is:
phenyl,
4-fluorophenyl, 4-cyanophenyl, benzofurazanyl, 2- or 4-pyridyl, 4-methoxyphenyl, 4-chlorophenyl, 3,4-methylenedioxyphenyl, 4-sulfamoylphenyl 4-methanesulfonamidophenyl, cyanotetralin or 2-methyl-6-pyridyl; R¹ and R² are independently selected from:
a) -NO₂,
b) -NHSO₂(C₁₋₆alkyl),
c) -SO₂(C₁₋₆alkyl),
d) -NHSO₂-C₆H₄-R4,
e) -SO₂-C₆H₄-R4,
f) hydrogen, or
R¹ and R² taken together with the Ar¹ group to which they are attached represent: 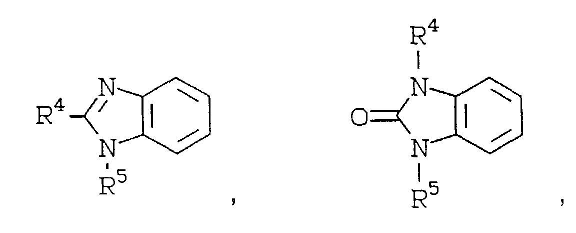 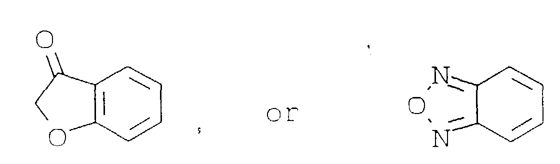
B is a piperidine ring;
with the proviso that if Ar¹ is benzo, and R¹ and R² are hydrogen, hydroxyalkyl or alkoxy, then M is other than unsubstituted phenyl or hydrogen; in the manufacture of a medicament for the treatment of arrythmia and impaired cardiac function.

2. The use as claimed in Claim 1 wherein the cyclic moiety comprising X, Y and Z is:

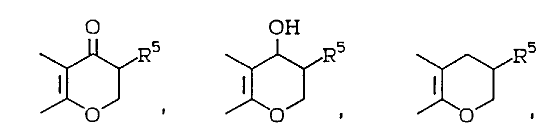

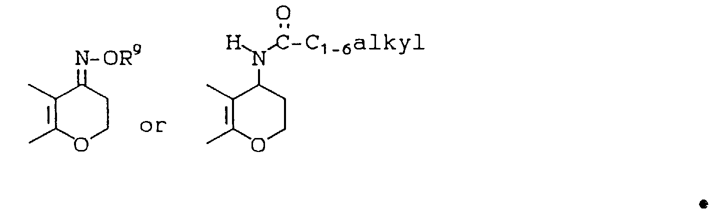

3. The use as claimed in Claim 1 or Claim 2 wherein Ar¹ is benzo.

4. The use as claimed in any one of Claims 1-3 wherein the compound of formula (I) is selected from:
3,4-dihydro-1'-[(6-cyano-1,2,3,4-tetrahydronaphthalene)-2-yl]-6-methanesulfonyl-spiro[(2H)-1-benzopyran-2,4'-piperidine]-4-one; and
3,4-dihydro-1'-[(6-cyano-1,2,3,4-tetrahydronaphthalene)-2-yl]-6-methanesulfonamido-spiro[(2H)-1-benzopyran-2,4'-piperidine]-4-ol;
or a pharmaceutically acceptable salt thereof.

5. The use as claimed in any one of Claims 1 to 3 wherein the compound of formula (I) is selected from:
3,4-dihydro-1'-[(6-cyano-1,2,3,4-tetrahydronaphthalene)-2-yl]-6-methanesulfonamido-spiro[(2H)-1-benzopyran-2,4'-piperidine];
1-[(5,6,7,8-tetrahydro-2-naphthalenecarbonitrile)-6-yl]-7',8'-dihydro-8'-oxo-spiro[piperidine-4,6' (1'H)-pyrano[2,3-f]benzimidazole];
1-[(5,6,7,8-tetrahydro-2-naphthalenecarbonitrile)-6-yl]-2',3',7',8'-tetrahydro-2',8'-dioxo-spiro[piperidine-4,6'(1'H)-pyrano[2,3-f]benzimidazole] ;
3,4-dihydro-1'-[2-(benzofurazan-5-yl)ethyl]-6-methanesulfonamido-spiro[(2H)-1-benzopyran-2,4'-piperidine]-4-ol;
3,4-dihydro-3-methyl-6-methanesulfonamido-1'-[2-(benzofurazan-5-yl)ethyl]spiro[(2H)-1-benzopyran-2,4'-piperidine]-4-ol;
3,4-dihydro-1'-[2-(4-cyanophenyl)ethyl]--6-methanesulfonamido-3-methyl-spiro[(2H)-1-benzopyran-2,4'-piperidine]-4-one;
6-methanesulfonamido-1'-hexyl-4-hydroxy-3,4-dihydrospiro[(2H)-1-benzopyran-2,4'-piperidine] ;
3,4-dihydro-3-methyl-6-methanesulfonamido-1'-[2-(benzofurazan-5-yl)ethyl]spiro[(2H)-1-benzopyran-2,4-piperidine]-4-one;
methanesulfonamide, N-[1'-(6-cyano-1,2,3,4-tetrahydro-2-naphthalenyl)-3,4-dihydro-4-oxospiro[2H-1-benzopyran-2,4'-piperidin]-6-yl]-;
cis or trans-3,4-dihydro-1'-[(6-cyano-1,2,3,4-tetrahydro-1-hydroxy-naphthalene)-2-yl]-6-methanesulfonamido-spiro[(2H)-1-benzopyran-2,4'-piperidine]-4-one;
4-acetamido-3,4-dihydro-1'-[(6-cyano-1,2,3,4-tetrahydronaphthalene)-2-yl]-6-methanesulfonamido-spiro-[(2H)-1-benzopyran-2,4'-piperidine];
3,4-dihydro-1'-[(6-cyano-1,2,3,4-tetrahydronaphthalene)-2-yl]-6-methanesulfonyl-spiro[(2H)-1-benzopyran-2,4'-piperidine]-4-ol;
3,4-dihydro-1'-[(6-cyano-1,2,3,4-tetrahydronaphthalene)-2-yl]-6-methanesulfonyl-spiro[(2H)-1-benzopyran-2,4'-piperidine];
1-[(5,6,7,8-tetrahydro-2-naphthalenecarbonitrile)-6-yl]-7',8'-dihydro-8'-hydroxy-spiro[piperidine-4,6'(1'H)-pyrano[2,3-f]benzimidazole];
1-[(5,6,7,8-tetrahydro-2-naphthalenecarbonitrile)-6-yl]-2',3',7',8'-tetrahydro-8'-hydroxy-2'-oxo-spiro-[piperidine-4,6'(1'H)-pyrano[2,3-f]benzimidazole];
1-[(5,6,7,8-tetrahydro-2-naphthalenecarbonitrile)-6-yl]-7',8'-dihydro-spiro[piperidine-4,6'(1'H)-pyrano[2,3-f]benzimidazole];
1-[(5,6,7,8-tetrahydro-2-naphthalenecarbonitrile)-6-yl]-2',3',7',8'-tetrahydro-2'-oxo-spiro[piperidine-4,6'(1'H)-pyrano[2,3-f]benzimidazole];
4-acetamido-3,4-dihydro-1'-[(6-cyano-1,2,3,4-tetrahydronaphthalene)-2-yl]-6-methanesulfonyl-spiro-[(2H)-1-benzopyran-2,4'-piperidine];
cis or trans-3,4-dihydro-1'-[(6-cyano-1,2,3,4-tetrahydro-l-hydroxy-naphthalene)-2-yl]-6-methanesulfonylspiro[(2H)-1-benzopyran-2,4'-piperidine];
3,4-dihydro-1'-[(6-cyano-1,2,3,4-tetrahydronaphthalene)-2-yl]-6-methanesulfonamido-3-methyl-spiro[(2H)-1-benzopyran-2,4'-piperidine]-4-one;
3,4-dihydro-1'-[(6-cyano-1,2,3,4-tetrahydronaphthalene)-2-yl]-6-methanesulfonyl-3-methyl-spiro[(2H)-1-benzopyran-2,4'-piperidine]-4-one;
cis or trans-3,4-dihydro-1'-[(6-cyano-1,2,3,4-tetrahydro-1-hydroxy-naphthalene)-2-yl]-6-methanesulfonamido-spiro[(2H)-1-benzopyran-2,4'-piperidine];
1-[2-(4-cyanophenyl)ethyl]-7',8'-dihydro-8'-oxospiro[piperidine-4,6'(1'H)-pyrano[2,3-f]benzimidazole];
1-[2-(4-cyanophenyl)ethyl]-2',3',7',8'-tetrahydro-2',8'-dioxo-spiro[piperidine-4,6'(1'H)-pyrano[2,3-f]-benzimidazole;
3,4-dihydro-1'-[2-(4-cyanophenyl)ethyl]-6-methanesulfonyl-spiro[(2H)-1-benzopyran-2,4'-piperidine]-4-one;
3,4-dihydro-1'-hexyl-6-methanesulfonamido-spiro[(2H)-1-benzopyran-2,4'-piperidine] ;
3,4-dihydro-1'-hexyl-6-methanesulfonamido-spiro[(2H)-1-benzopyran-2,4'-piperidine]-4-one;
1-hexyl-2',3',7',8'-tetrahydro-2',8'-dioxospiro-[piperidine-4,6'(1H)-pyrano[2,3-f]benzimidazole];
3,4-dihydro-1'-[2-(benzofurazan-5-yl)ethyl]-6-methanesulfonamido-spiro[2H)-1-benzopyran-2,4'-piperidine]-4-one;
3,4-dihydro-1'-[2-(benzofurazan-5-yl)ethyl]-6-methanesulfonamido-spiro[(2H)-1-benzopyran-2,4'-piperidine];
7',8'-dihydro-1-hexyl-8'-oxospiro[piperidine-4,6'-(1H)-pyrano[2,3-f]benzimidazole];
3,4-dihydro-1'-(7-hydroxyheptyl)-6-methanesulfonamidospiro[(2H)-1-benzopyran-2,4'-piperidine]-4-one;
3,4-dihydro-1'-(6-hydroxyhexyl)-6-methanesulfonamidospiro[(2H)-1-benzopyran-2,4'-piperidine]-4-one;
3,4-dihydro-1-heptyl-6-methanesulfonamido-spiro[(2H)-1-benzopyran-2,4'-piperidine]-4-one;
3,4-dihdro-1'-[2-(4-acetylphenyl)ethyl]-6-methanesulfonamido-spiro[(2H)-1-benzopyran-2,4'-piperidine]-4-one;
3,4-dihydro-1'-hexyl-6-methanesulfonyl-spiro[(2H)-1-benzopyran-2,4'-piperidine]-4-one;
or a pharmaceutically acceptable salt thereof.

6. A compound of the structure (I) as defined in claim 1 wherein Ar¹ is benzo.

7. A compound as claimed in claim 6 wherein the cyclic moiety comprising X, Y and Z is as defined in claim 2.

8. A compound as set forth in claim 4.

9. A compound as set forth in claim 5 .

10. A pharmaceutical composition which comprises a compound as claimed in any of claims 6 to 9 and a pharmaceutically acceptable carrier therefor.

11. A process for the preparation of a compound as claimed in any one of claims 6 to 9 which comprises:
A) where R is -CH₂CH₂- and M is pyridyl, addition of the spiropiperidine of formula (1) 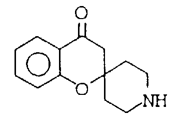 across the vinyl group of the compound of formula (2) 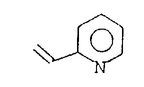 by heating a mixture of said compounds in aqueous lower alkanol, in the presence of sodium or potassium acetate, to obtain the compound of formula (3) 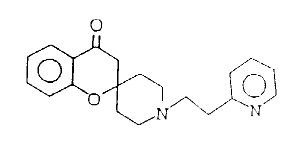
B) where R is -CH₂CH₂- and M is p-nitrophenyl alkylation of the spiropiperidine of formula (1), above, with the phenylalkyl alkylating agent of formula (4) 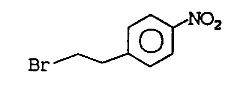 in the presence of an acid scavenger, an organic amine or an appropriate ion exchange resin, to obtain the compound of formula (5) 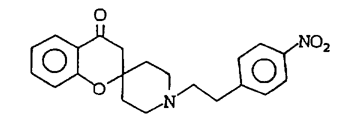
C) where R¹ is CH₃SO₂NH-, sulfonylating the compound of formula (6) 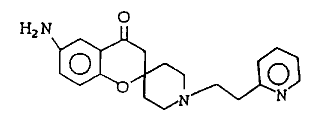 with an alkanesulfonyl chloride by standard procedures, to obtain the compound of formula (7) 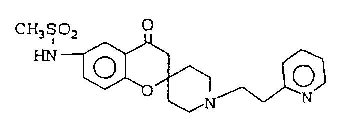
D) where the cyclic moiety comprising X, Y and Z is 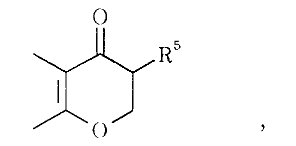
(a) condensation of a compound of formula (8) 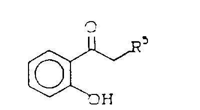 (R⁵ = H or C₁₋₅alkyl) with 1-acetyl-4-piperidinone, in the presence of pyrrolidine in methanol, to obtain a compound of formula (9) 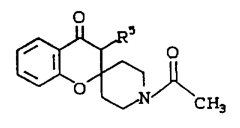 and optionally,
(b) nitrating a compound of formula (9) with nitric acid, reducing the nitro group by standard procedures, and sulfonylating the resultant compound according to the method of process (C), to obtain a compound of formula (10) 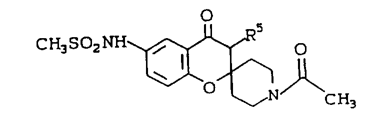 and optionally,
(c) cleaving an acetamide of formula (10) with hydrochloric acid in methanol, to obtain a compound of formula (11) 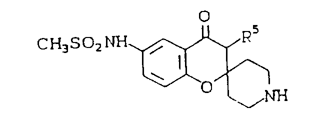 and optionally,
(d) further reacting a compound of formula (11) according to the methods of processes (A) or (B);
E) where the cyclic moiety comprising X, Y and Z is 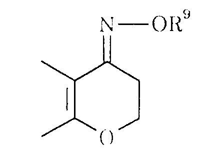 where R⁹ is R⁵, i.e. hydrogen or C₁₋₆alkyl, reacting the compound of formula (12) 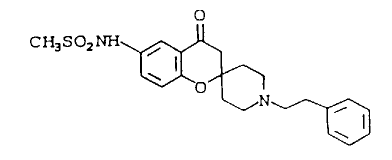 with a compound of the formula H₂N-OR⁵.HCl in the presence of pyridine, to obtain a compound of formula (13) 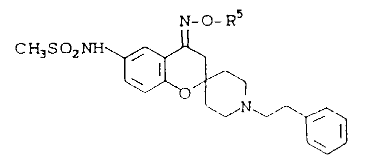 each process being followed, where necessary, by the removal of any protecting group where present; and/or, if desired, converting the resulting compound of formulae (I) or (II) or a salt thereof, into a pharmaceutically acceptable salt thereof.

## Patentansprüche

1. Die Verwendung einer Verbindung der Strukturformel: 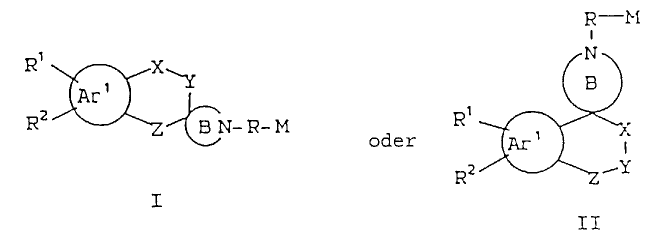 oder eines pharmazeutisch annehmbaren Salzes davon, worin Ar¹ ein aromatischer Ring ist, ausgewählt aus
1) Benzo und
2) Thieno,
das Ringsystem, welches X, Y und Z enthält, ausgewählt ist aus: 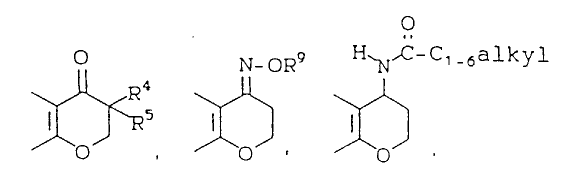 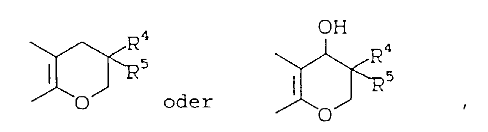 worin:
R⁴ und R⁵ unabhängig voneinander
a) Wasserstoff oder
b) C₁₋₆-Alkyl sind,
R⁹
a) Wasserstoff oder
b) C₁₋₆-Alkyl ist, unsubstituiert oder mit -COOR⁵ substituiert,
R-M n-Hexyl ist, unsubstituiert oder mit Hydroxy oder Cyano substituiert, oder
R eine Bindung oder C₁₋₈-Alkyl ist und
M Ar²R³ ist, wobei Ar²R³ ist:
Phenyl, 4-Fluorphenyl, 4-Cyanophenyl, Benzofurazanyl, 2- oder 4-Pyridyl, 4-Methoxyphenyl, 4-Chlorphenyl, 3,4-Methylendioxyphenyl, 4-Sulfamoylphenyl, 4-Methansulfonamidophenyl, Cyanotetralin oder 2-Methyl-6-pyridyl,
R¹ und R² unabhängig voneinander ausgewählt sind aus:
a) -NO₂,
b) -NHSO₂(C₁₋₆-Alkyl),
c) -SO₂(C₁₋₆-Alkyl),
d) -NHSO₂-C₆H₄-R⁴,
e) -SO₂-C₆H₄-R⁴,
f) Wasserstoff, oder
R¹ und R² zusammen mit der Ar¹-Gruppe, an die sie gebunden sind, 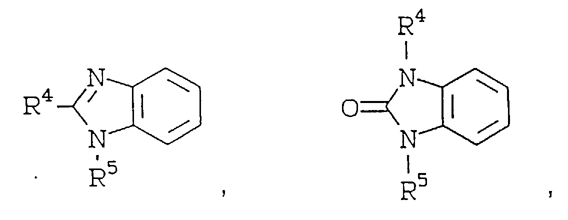 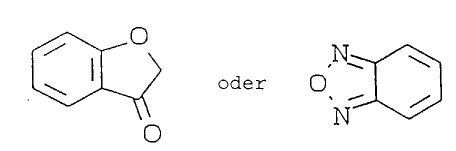 bedeuten,
B ein Piperidinring ist,
mit der Maßgabe, daß, wenn Ar¹ Benzo ist und R¹ und R² Wasserstoff, Hydroxyalkyl oder Alkoxy sind, M dann kein unsubstituiertes Phenyl oder Wasserstoff ist,
zur Herstellung eines Medikaments zur Behandlung von Arrhythmie und gestörter Herzfunktion.

2. Die wie in Anspruch 1 beanspruchte verwendung, worin der cyclische Rest, welcher X, Y und Z enthält, 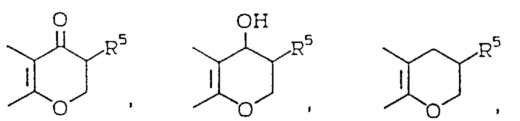 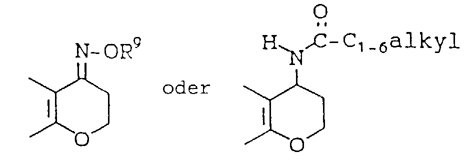 ist.

3. Die wie in Anspruch 1 oder Anspruch 2 beanspruchte Verwendung, worin Ar¹ Benzo ist.

4. Die wie in irgendeinem der Ansprüche 1-3 beanspruchte Verwendung, worin die Verbindung der Formel (I) ausgewählt ist aus:
3,4-Dihydro-1'-[(6-cyano-1,2,3,4-tetrahydronaphthalin)-2-yl]-6-methansulfonylspiro[(2H)-1-benzopyran-2,4'-piperidin]-4-on und
3,4-Dihydro-1'-[(6-cyano-1,2,3,4-tetrahydronaphthalin)-2-yl]-6-methansulfonamidospiro[(2H)-1-benzopyran-2,4'-piperidin]-4-ol oder einem pharmazeutisch annehmbaren Salz davon.

5. Die wie in irgendeinem der Ansprüche 1 bis 3 beanspruchte Verwendung, worin die Verbindung der Formel (I) ausgewählt ist aus:
3,4-Dihydro-1'-[(6-cyano-1,2,3,4-tetrahydronaphthalin)-2-yl]-6-methansulfonamidospiro[(2H)-1-benzopyran-2,4'-piperidin],
1-[(5,6,7,8-Tetrahydro-2-naphthalincarbonitril)-6-yl]-7',8'-dihydro-8'-oxospiro[piperidin-4,6' (1'H)-pyrano[2,3-f]benzimidazol],
1-[(5,6,7,8-Tetrahydro-2-naphthalincarbonitril)-6-yl]-2',3',7',8'-tetrahydro-2',8'-dioxospiro[piperidin-4,6' (1'H)-pyrano[2,3-f]benzimidazol],
3,4-Dihydro-1'-[2-(benzofurazan-5-yl)ethyl]-6-methansulfonamidospiro[(2H)-1-benzopyran-2,4'-piperidin]-4-ol,
3,4-Dihydro-3-methyl-6-methansulfonamido-1'-[2-(benzofurazan-5-yl)ethyl]-spiro[(2H)-1-benzopyran-2,4'-piperidin]-4-ol,
3,4-Dihydro-1'-[2-(4-cyanophenyl)ethyl]-6-methansulfonamido-3-methylspiro[(2H)-1-benzopyran-2,4'-piperidin]-4-on,
6-Methansulfonamido-1'-hexyl-4-hydroxy-3,4-dihydrospiro[(2H)-1-benzopyran-2,4'-piperidin],
3,4-Dihydro-3-methyl-6-methansulfonamido-1'-[2-(benzofurazan-5-yl)ethyl]-spiro[(2H)-1-benzopyran-2,4'-piperidin]-4-on,
N-[1'-(6-Cyano-1,2,3,4-tetrahydro-2-naphthalinyl)-3,4-dihydro-4-oxospiro[(2H)-1-benzopyran-2,4'-piperidin]-6-yl]methansulfonamid,
cis- oder trans-3,4-Dihydro-1'-[(6-cyano-1,2,3,4-tetrahydro-1-hydroxynaphthalin)-2-yl]-6-methansulfonamidospiro[(2H)-1-benzopyran-2,4'-piperidin]-4-on,
4-Acetamido-3,4-dihydro-1'-[(6-cyano-1,2,3,4-tetrahydronaphthalin)-2-yl]-6-methansulfonamidospiro[(2H)-1-benzopyran-2,4'-piperidin],
3,4-Dihydro-1'-[(6-cyano-1,2,3,4-tetrahydronaphthalin)-2-yl]-6-methansulfonylspiro[(2H)-1-benzopyran-2,4'-piperidin]-4-ol,
3,4-Dihydro-1'-[(6-cyano-1,2,3,4-tetrahydronaphthalin)-2-yl]-6-methansulfonylspiro[(2H)-1-benzopyran-2,4'-piperidin],
1-[(5,6,7,8-Tetrahydro-2-naphthalincarbonitril)-6-yl]-7',8'-dihydro-8'-hydroxyspiro[piperidin-4,6' (1'H)-pyrano[2,3-f]benzimidazol],
1-[(5,6,7,8-Tetrahydro-2-naphthalincarbonitril)-6-yl]-2',3',7',8'-tetrahydro-8'-hydroxy-2'-oxospiro[piperidin-4,6' (1'H)-pyrano[2,3-f]benzimidazol],
1-[(5,6,7,8-Tetrahydro-2-naphthalincarbonitril)-6-yl]-7',8'-dihydrospiro-[piperidin-4,6'(1'H)-pyrano[2,3-f]benzimidazol],
1-[(5,6,7,8-Tetrahydro-2-naphthalincarbonitril)-6-yl]-2',3',7',8'-tetrahydro-2'-oxospiro[piperidin-4,6' (1'H)-pyrano[2,3-f]benzimidazol],
4-Acetamido-3,4-dihydro-1'-[(6-cyano-1,2,3,4-tetrahydronaphthalin)-2-yl]-6-methansulfonylspiro[(2H)-1-benzopyran-2,4'-piperidin],
cis- oder trans-3,4-Dihydro-1'-[(6-cyano-1,2,3,4-tetrahydro-1-hydroxynaphthalin)-2-yl]-6-methansulfonylspiro[(2H)-1-benzopyran-2,4'-piperidin],
3,4-Dihydro-1'-[(6-cyano-1,2,3,4-tetrahydronaphthalin)-2-yl]-6-methansulfonamido-3-methylspiro[(2H)-1-benzopyran-2,4'-piperidin]-4-on,
3,4-Dihydro-1'-[(6-cyano-1,2,3,4-tetrahydronaphthalin)-2-yl]-6-methansulfonyl-3-methylspiro[(2H)-1-benzopyran-2,4'-piperidin]-4-on,
cis- oder trans-3,4-Dihydro-1'-[(6-cyano-1,2,3,4-tetrahydro-1-hydroxynaphthalin)-2-yl]-6-methansulfonamidospiro[(2H)-1-benzopyran-2,4'-piperidin],
1-[2-(4-Cyanophenyl)ethyl]-7',8'-dihydro-8'-oxospiro[piperidin-4,6'(1'H)-pyrano[2,3-f]benzimidazol],
1-[2-(4-Cyanophenyl)ethyl]-2',3',7',8'-tetrahydro-2',8'-dioxospiro-[piperidin-4,6'(1'H)-pyrano[2,3-f]benzimidazol],
3,4-Dihydro-1'-[2-(4-cyanophenyl)ethyl]-6-methansulfonylspiro[(2H)-1-benzopyran-2,4'-piperidin]-4-on,
3,4-Dihydro-1'-hexyl-6-methansulfonamidospiro[(2H)-1-benzopyran-2,4'-piperidin],
3,4-Dihydro-1'-hexyl-6-methansulfonamidospiro[(2H)-1-benzopyran-2,4'-piperidin]-4-on,
1-Hexyl-2',3',7',8'-tetrahydro-2',8'-dioxospiro[piperidin-4,6' (1H)-pyrano[2,3-f]benzimidazol],
3,4-Dihydro-1'-[2-(benzofurazan-5-yl)ethyl]-6-methansulfonamidospiro-[(2H)-1-benzopyran-2,4'-piperidin]-4-on,
3,4-Dihydro-1'-[2-(benzofurazan-5-yl)ethyl]-6-methansulfonamidospiro-[(2H)-1-benzopyran-2,4'-piperidin],
7',8'-Dihydro-1-hexyl-8'-oxospiro[piperidin-4,6'-(1H)-pyrano[2,3-f]benzimidazol],
3,4-Dihydro-1'-(7-hydroxyheptyl)-6-methansulfonamidospiro[(2H)-1-benzopyran-2,4'-piperidin]-4-on,
3,4-Dihydro-1'-(6-hydroxyhexyl)-6-methansulfonamidospiro[(2H)-1-benzopyran-2,4'-piperidin]-4-on,
3,4-Dihydro-1'-heptyl-6-methansulfonamidospiro[(2H)-1-benzopyran-2,4'-piperidin]-4-on,
3,4-Dihydro-1'-[2-(4-acetylphenyl)ethyl]-6-methansulfonamidospiro[(2H)-1-benzopyran-2,4'-piperidin]-4-on,
3,4-Dihydro-1'-hexyl-6-methansulfonylspiro[(2H)-1-benzopyran-2,4'-piperidin]-4-on
oder einem pharmazeutisch annehmbaren Salz davon.

6. Eine Verbindung der Struktur (I), wie sie in Anspruch 1 definiert ist, worin Ar¹ Benzo ist.

7. Eine wie in Anspruch 6 beanspruchte Verbindung, worin der cyclische Rest, welcher X, Y und Z enthält, wie in Anspruch 2 definiert ist.

8. Eine wie in Anspruch 4 beschriebene Verbindung.

9. Eine wie in Anspruch 5 beschriebene Verbindung.

10. Eine pharmazeutische Zusammensetzung, die eine wie in irgendeinem der Ansprüche 6 bis 9 beanspruchte Verbindung und einen pharmazeutisch annehmbaren Träger dafür enthält.

11. Ein Verfahren zur Herstellung einer wie in irgendeinem der Ansprüche 6 bis 9 beanspruchten Verbindung, umfassend:
A), wenn R -CH₂CH₂- ist und M Pyridyl ist, die Addition des Spiropiperidins der Formel (1) 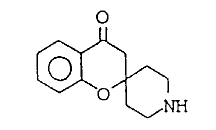 an die Vinylgruppe der Verbindung der Formel (2) 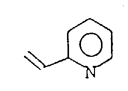 durch Erhitzen einer Mischung aus den Verbindungen in wäßrigem Niedrigalkanol in Gegenwart von Natrium- oder Kaliumacetat, um die Verbindung der Formel (3) zu erhalten 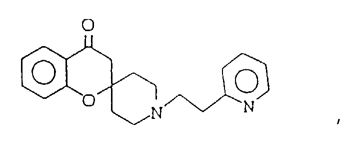
B), wenn R -CH₂CH₂- ist und M p-Nitrophenyl ist, die Alkylierung des Spiropiperidins der obigen Formel (1) mit dem Phenylalkyl-Alkylierungsmittel der Formel (4) 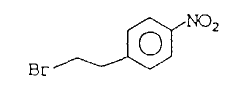 in Gegenwart eines Säurefängers, eines organischen Amins oder eines geeigneten Ionenaustauschharzes, um die Verbindung der Formel (5) zu erhalten 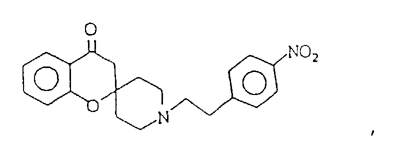
C), wenn R¹ CH₃SO₂NH- ist, die Sulfonylierung der Verbindung der Formel (6) 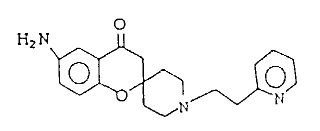 mit einem Alkansulfonylchlorid durch Standardverfahren, um die Verbindung der Formel (7) zu erhalten 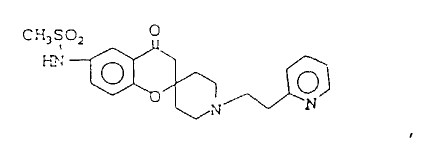
D), wenn der cyclische Rest, welcher X, Y und Z enthält, 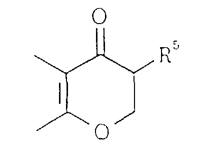 ist,
a) die Kondensation einer Verbindung der Formel (8) 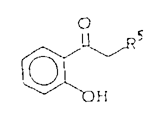 (R⁵ = H oder C₁₋₅-Alkyl) mit 1-Acetyl-4-piperidinon in Gegenwart von Pyrrolidin in Methanol, um eine Verbindung der Formel (9) zu erhalten 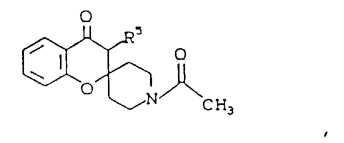 und gegebenenfalls
b), die Nitrierung einer Verbindung der Formel (9) mit Salpetersäure, die Reduzierung der Nitrogruppe durch Standardverfahren und die Sulfonylierung der resultierenden Verbindung gemäß der Methode von Verfahren (C), um eine Verbindung der Formel (10) zu erhalten 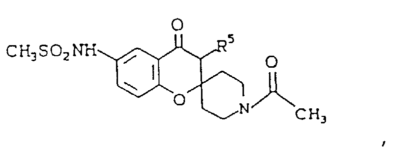 und gegebenenfalls
c) die Spaltung eines Acetamids der Formel (10) mit Salzsäure in Methanol, um eine Verbindung der Formel (11) zu erhalten 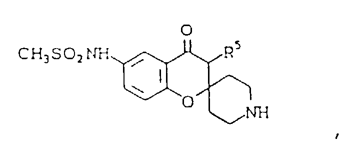 und gegebenenfalls
(d) die weitere Umsetzung einer Verbindung der Formel (11) gemäß den Methoden der Verfahren (A) oder (B),
E), wenn der cyclische Rest, welcher X, Y und Z enthält, 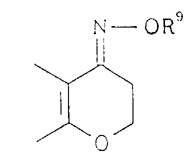 ist,
worin R⁹ R⁵ ist, d.h. Wasserstoff oder C₁₋₆-Alkyl, die Umsetzung der Verbindung der Formel (12) 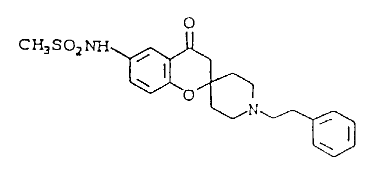 mit einer Verbindung der Formel H₂N-OR⁵.HCl in Gegenwart von Pyridin, um eine Verbindung der Formel (13) zu erhalten 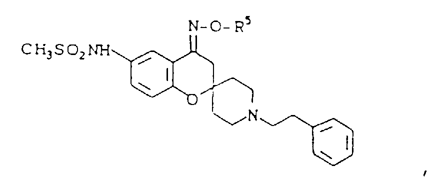 wobei jedem Verfahren, falls notwendig, die Entfernung einer etwaigen Schutzgruppe, falls vorhanden, folgt und/oder falls erwünscht, die Umwandlung der resultierenden Verbindung der Formeln (I) oder (II) oder eines Salzes davon in ein pharmazeutisch annehmbares Salz davon.

## Revendications

1. Utilisation d'un composé répondant à la formule développée suivante : 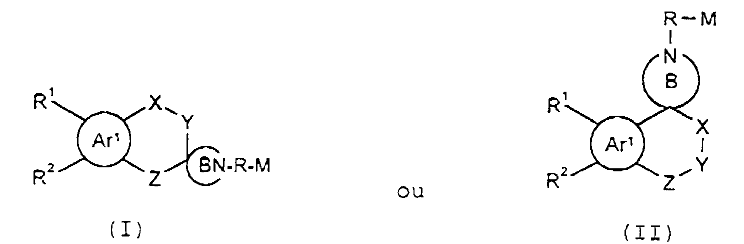 ou d'un de ses sels acceptables en pharmacie,
où Ar¹ est un cycle aromatique choisi parmi
1) benzo, et
2) thiéno,
le système cyclique comprenant X, Y et Z est choisi parmi : 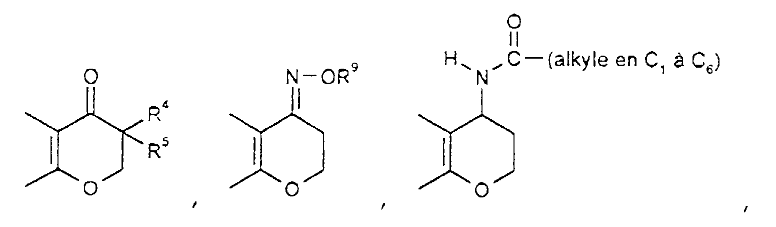 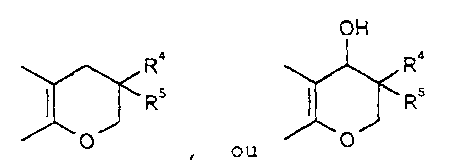 où :
R⁴ et R⁵ sont indépendamment
a) l'hydrogène, ou
b) un groupe alkyle en C₁ à C₆ ;
R⁹ est
a) l'hydrogène, ou
b) un groupe alkyle en C₁ à C₆ éventuellement substitué par -COOR⁵ ;
R-M est un groupe n-hexyle éventuellement substitué par un groupe hydroxy ou cyano ; ou bien
R est une liaison ou un groupe alkyle en C₁ à C₆ et M est un groupe Ar²R³ où Ar²R³ est un groupe phényle, 4-fluorophényle, 4-cyanophényle, benzofurazannyle, 2- ou 4-pyridyle, 4-méthoxyphényle, 4-chlorophényle, 3,4-méthylènedioxyphényle, 4-sulfamoylphényle, 4-méthanesulfonamidophényle, cyanotétraline ou 2-méthyl-6-pyridyle ;
R¹ et R² sont indépendamment choisis parmi :
a) -NO₂,
b) -NHSO₂(alkyle en C₁ à C₆),
c) -SO₂(alkyle en C₁ à C₆),
d) -NHSO₂-C₆H₄-R⁴,
e) -SO₂-C₆H₄-R⁴,
f) l'hydrogène, ou bien
R¹ et R², pris ensemble avec le groupe Ar¹ auquel ils sont attachés, représentent : 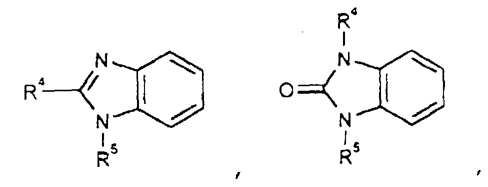 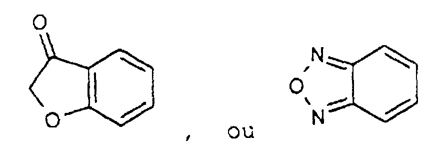
B est un cycle pipéridine ;
avec pour condition que si Ar¹ est un radical benzo et que R¹ et R² sont l'hydrogène ou un radical hydroxyalkyle ou alcoxy, alors M est différent de l'hydrogène et d'un groupe phényle non substitué ;
dans la fabrication d'un médicament pour le traitement de l'arythmie et de l'insuffisance cardiaque.

2. Utilisation selon la revendication 1, dans laquelle le fragment cyclique comprenant X, Y et Z est :

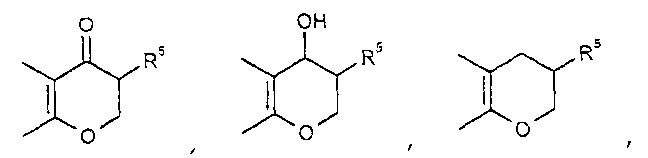

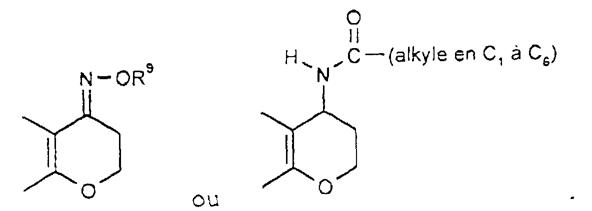

3. Utilisation selon la revendication 1 ou 2, dans laquelle Ar¹ est un radical benzo.

4. Utilisation selon l'une quelconque des revendications 1 à 3, dans laquelle le composé de formule (I) est choisi parmi :
la 3,4-dihydro-1'-[(6-cyano-1,2,3,4-tétrahydronaphtalène)-2-yl]-6-méthanesulfonylspiro[(2H)-1-benzopyranne-2,4'-pipéridine]-4-one ; et
le 3,4-dihydro-1'-[(6-cyano-1,2,3,4-tétrahydronaphtaléne)-2-yl]-6-méthanesulfonylspiro[(2H)-1-benzopyranne-2,4'-pipéridine]-4-ol;
ou un de leurs sels acceptables en pharmacie.

5. Utilisation selon l'une quelconque des revendications 1 à 3, dans laquelle le composé de formule (I) est choisi parmi :
la 3,4-dihydro-1'-[(6-cyano-1,2,3,4-tétrahydronaphtalène)-2-yl]-6-méthanesulfonyl-spiro[(2H)-1-benzopyranne-2,4'-pipéridine] ;
le 1-[(5,6,7,8-tétrahydro-2-naphtalènecarbonitrile)-6-yl]-7',8'-dihydro-8'-oxospiro[pipéridine-4,6'(1'H)-pyranno[2,3-f]benzimidezole] ;
le 1-[(5,6,7,8-tétrahydro-2-naphtalènecarbonitrile)-6-yl]-2',3',7',8'-tétrahydro-2',8'-dioxospiro[pipéridine-4,6'(1'H)-pyranno[2,3-f]benzimidazole] ;
le 3,4-dihydro-1'-[2-(benzofurazanne-5-yl)éthyl]-6-méthanesulfonamido-spiro[(2H)-1-benzopyranne-2,4'-pipéridine]-4-ol ;
le 3,4-dihydro-3-méthyl-6-méthanesulfonamido-1'-[2-(benzofurazanne-5-yl)éthyl]spiro[(2H)-1-benzopyranne-2,4'-pipéridine]-4-ol ;
la 3,4-dihydro-1'-[2-(4-cyanophényl)éthyl]-6-méthanesulfonamido-3-méthyl-spiro[(2H)-1-benzopyranne-2,4'-pipéridine]-4-one ;
la 6-méthanesulfonamido-1'-hexyl-4-hydroxy-3,4-dihydro-spiro[(2H)-1-benzopyranne-2,4'-pipéridine] ;
la 3,4-dihydro-3-méthyl-6-méthanesulfonamido-1'-[2-benzofurazanne-5-yl)éthyl]spiro[(2H)-1-benzopyranne-2,4'-pipéridine]-4-one ;
le N-[1'-(6-cyano-1,2,3,4-tétrahydro-2-naphtalényl)-3,4-dihydro-4-oxospiro[2H-1-benzopyranne-2,4'-pipéridine]-6-yl]méthanesulfonamide ;
l'isomèra cis ou trans de la 3,4-dihydro-1'-[(6-cyano-1,2,3,4-tétrahydro-1-hydroxy-naphtalène)-2-yl]-6-méthanesulfonamido-spiro[(2H)-1-benzopyranne-2,4'-pipéridine]-4-one ;
la 4-acétamido-3,4-dihydro-1'-[(6-cyano-1,2,3,4-tétrahydronaphtalène)-2-yl]-6-méthanesulfonamidospiro[(2H)-1-benzopyranne-2,4'-pipéridine] ;
le 3,4-dihydro-1'-[(6-cyano-1,2,3,4-tétrahydronephtalène)-2-yl]-6-méthanesulfonyl-spiro[(2H)-1-benzopyranne-2,4'-pipéridine]-4-ol ;
la 3,4-dihydro-1'-[(6-cyano-1,2,3,4-tétrahydronaphtalène)-2-yl]-6-méthanesulfonyl-spiro[(2H)-1-benzopyranne-2,4'-pipéridine] ;
le 1-[(5,6,7,8-tétrahydro-2-naphtalène-carbonitrile)-6-yl]-7',8'-dihydro-8'-hydroxyspiro[pipéridine-4, 6' (1'H)pyranno[2,3-f]benzimidazole] ;
le 1-[(5,6,7,8-tétrahydro-2-naphtalènecarbonitrile)-6-yl]-2',3',7',8'-tétrahydro-8'-hydroxy-2'-oxo-spiro[pipéridine-4,6'(1'H)pyranno[2,3-f]benzimidazole] ;
le 1-[(5,6,7,8-tétrahydro-2-naphtalènecarbonitrile)-6-yl]-7,8'-dihydro-spiro[pipéridine-4,6'(1'H)pyranno[2,3-f]benzimidazole];
le 1-[(5,6,7,8-tétrahydro-2-naphtalènecarbonitrile)-6-yl]-2',3',7',8'-tétrahydro-2'-oxospiro[pipéridine-4,6'(1'H)pyranno[2,3-f]benzimidazole] ;
la 4-acétamido-3,4-dihydro-1'-[(6-cyano-1,2,3,4-tétrahydronaphtalène)-2-yl]-6-méthanesulfonylspiro[(2H)-1-benzopyranne-2,4'-pipéridine] ;
l'isomère cis ou trans de la 3,4-dihydro-1'-[(6-cyano-1,2,3,4-tétrahydro-1-hydroxy-naphtelène)-2-yl]-6-méthanesulfonyl-spiro[(2H)-1-benzopyranne-2,4'-pipéridine] ;
la 3,4-dihydro-1'-[(6-cyano-1,2,3,4-tétrahydronaphtalène)-2-yl]-6-méthanesulfonamido-3-méthylspiro[(2H)-1-benzopyranne-2,4'-pipéridine]-4-one ;
la 3,4-dihydro-1'-[(6-cyano-1,2,3,4-tétrahydronaphtalène)-2-yl]-6-méthanesulfonyl-3-méthylspiro[(2H)-1-benzopyranne-2,4'-pipéridine]-4-one ;
l'isomère cis ou trans de la 3,4-dihydro-1'-[(6-cyano-1,2,3,4-tétrahydro-1-hydroxy-naphtalène)-2-yl]-6-méthanesulfonamido-spiro[(2H)-1-benzopyranne-2,4'-pipéridine] ;
le 1-[2-(4-cyanophényl)éthyl]-7',8'-dihydro-8'-oxo-spiro[pipéridine-4',6'(1H)-pyranno[2,3-f]benzimidazole] ;
le 1-[2-(4-cyanophényl)éthyl]-2',3',7',8'-tétrahydro-2',8'-dioxo-spiro[pipéridine-4',6'(1'H)-pyranno[2,3-f]benzimidazole] ;
la 3,4-dihydro-1'-[2-(4-cyanophényl)éthyl]-6-méthanesulfonyl-spiro[(2H)-1-benzopyranne-2,4'-pipéridine]-4-one ;
la 3,4-dihydro-1'-hexyl-6-méthanesulfonamidospiro(2H)-1-benzopyranne-2,4'-pipéridine] ;
la 3,4-dihydro-1'-hexyl-6-méthanesulfonamidospiro(2H)-1-benzopyranne-2,4'-pipéridine]-4-one ;
le 1-hexyl-2',3',7',8'-tétrahydro-2',8'-dioxospiro[piperidine-4,6' (1H)-pyranno[2,3-f]benzimidazole] ;
la 3,4-dihydro-1'-[2-(benzofurazanne-5-yl)éthyl]-6-méthanesulfonamido-spiro(2H)-1-benzopyranne-2,4'-pipéridine]-4-one ;
la 3,4-dihydro-1'-[2-(benzofurazanne-5-yl)éthyl]-6-méthanesulfonamido-spiro(2H)-1-benzopyranne-2,4'-pipéridine] ;
le 7',8'-dihydro-1-hexyl-8'-oxo-spiro[pipéridine-4,6(1H)-pyranno[2,3-f]benzimidazole;
la 3,4-dihydro-1'-(7-hydroxyheptyl)-6-méthenesulfonamido-spiro[(2H)-1-benzopyranne-2,4'-pipéridine]-4-one ;
la 3,4-dihydro-1'-(6-hydroxyheptyl)-6-méthanesulfonamido-spiro[(2H)-1-benzopyranne-2,4'-pipéridine]-4-one ;
la 3,4-dihydro-1'-heptyl-6-méthanesulfonamidospiro[(2H)-1-benzopyranne-2,4'-pipéridine]-4-one ;
la 3,4-dihydro-1'-[2-(4-acétylphényl)éthyl]-6-methanesulfonamido-spiro[(2H)-1-benzopyranne-2,4'-pipéridine]-4-one ;
la 3,4-dihydro-1'-hexyl-6-méthanesulfonyl-spiro-[(2H)-1-benzopyranne-2,4'-pipéridine]-4-one ;
ou un, de leurs sels acceptables en pharmacie.

6. Composé ayant la structure (I) telle que définie dans la revendication 1 dans laquelle Ar¹ est un radical benzo.

7. Composé selon la revendication 6, dans lequel le fragment cyclique comprenant X, Y et Z est tel que défini dans la revendication 2.

8. Composé tel qu'indiqué dans la revendication 4.

9. Composé tel qu'indiqué dans la revendication 5.

10. Composition pharmaceutique qui comprend un composé selon l'une quelconque des revendications 6 à 9 et un véhicule pour celui-ci, acceptable en pharmacie.

11. Procédé pour la préparation d'un composé selon l'une quelconque des revendications 6 à 9, qui comprend :
A) quand R est -CH₂CH₂- et M est un radical pyridyle, l'addition de la spiropipéridine de formule (1) : 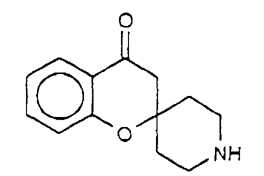 de part et d'autre du groupe vinyle du composé de formule (2) : 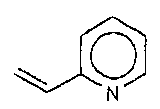 par chauffage d'un mélange desdits composés dans une solution aqueuse d'un alcanol inférieur, en présence d'acétate de sodium ou de potassium, de façon à donner le composé de formule (3) : 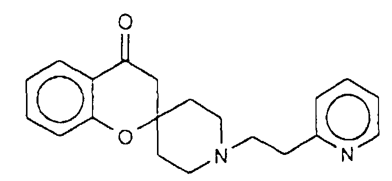
B) quand R est -CH₂CH₂- et M est un radical p-nitrophényle, l'alkylation de la spiropipéridine de formule (i) ci-dessus, avec l'agent d'alkylation de type phénylalkyle de formule (4) : 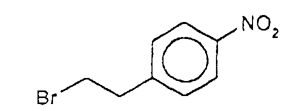 en présence d'un fixateur d'acide, d'une amine organique ou d'une résine échangeuse d'ions appropriée, pour donner le composé de formule (5) : 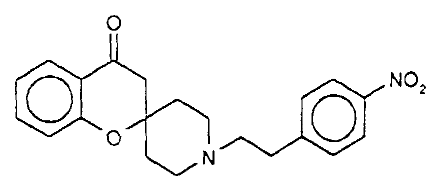
C) quand R¹ est CH₃SO₂NH-, ia sulfonylation du composé de formule (6) : 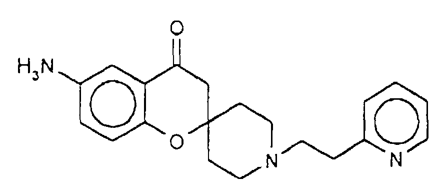 avec un chlorure d'alcanesulfonyle par des modes opératoires classiques, pour donner le composé de formule (7) : 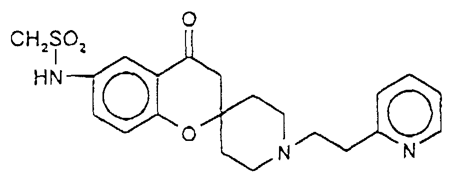
D) quand le fragment cyclique comprenant X, Y et Z est 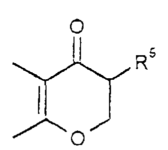
a) la condensation d'un composé de formule (8) : 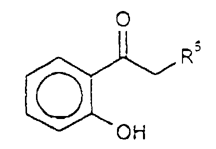 (R⁵ = H ou alkyle en C₁ à C₆)
avec de la 1-acétyl-4-pipéridinone, en présence de pyrrolidine dans du méthanol, pour donner le composé de formule (9) : 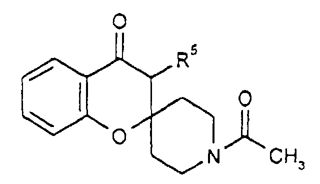 et, éventuellement,
b) la nitration d'un composé de formuie (9) avec de l'acide nitrique, la réduction du groupe micro par des modes opératoires classiques, et la sulfonylation du composé résultant conformément au mode opératoire du procédé (C), pour donner un composé de formule (10) : 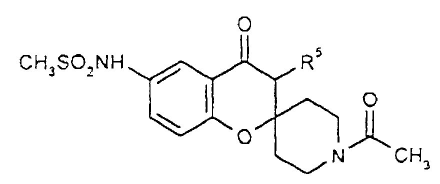 et, éventuellement,
c) la coupure d'un acétamide de formule (10) par de l'acide chlorhydrique dans du méthanol, pour donner un composé de formule (11) : 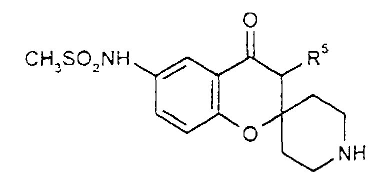 et, éventuellement,
d) la réaction supplémentaire d'un composé de formule (11) selon les modes opératoires du procédé (A) ou (B) ;
E) quand le fragment cyclique comprenant X, Y et Z est 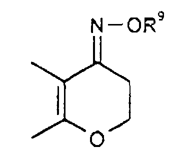 où R⁹ est R⁵, c'est-à-dire l'hydrogène ou un groupe alkyle en C₁ à C₆, la réaction du composé de formule (12) : 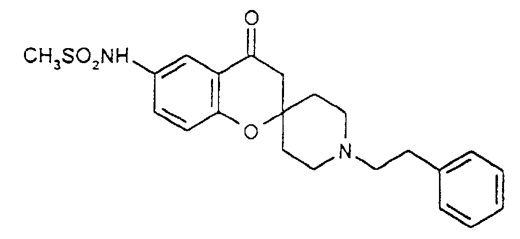 avec un composé de formule H₂N-OR⁵.HCl en présence de pyridine, pour donner le composé de formule (13) : 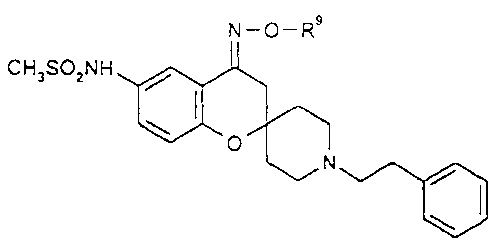 chaque procédé étant suivi, lorsque c'est nécessaire, par l'élimination de tout groupe protecteur éventuellement présent ; et/ou, si on le souhaite, la conversion du composé résultant de formule (I) ou (II) ou d'un de leurs sels en un sel acceptable en pharmacie d'un tel composé. 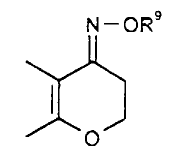 où R⁹ est R⁵, c'est-à-dire l'hydrogène ou un groupe alkyle en C₁ à C₆, la réaction du composé de formule (12) : 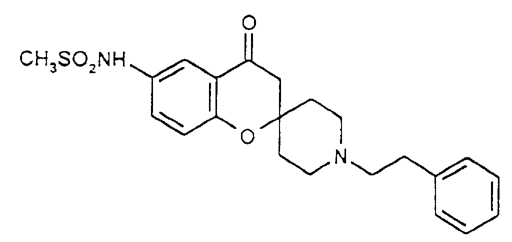 avec un composé de formule H₂N-OR⁵.HCl en présence de pyridine, pour donner le composé de formule (13) : 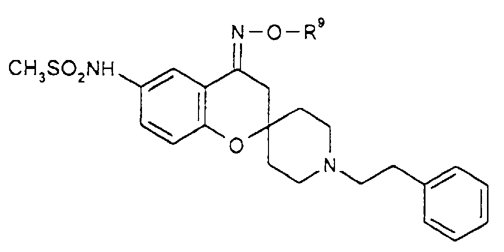 chaque procédé étant suivi, lorsque c'est nécessaire, par l'élimination de tout groupe protecteur éventuellement présent ; et/ou, si on le souhaite, la conversion du composé résultant de formule (I) ou (II) ou d'un de leurs sels en un sel acceptable en pharmacie d'un tel composé.
